# EUROPEAN PATENT SPECIFICATION

(11) **EP 3 319 993 B1**
(45) Date of publication and mention of the grant of the patent: **15.01.2020**
(21) Application number: 16739079.8
(22) Date of filing: 08.07.2016
(51) Int. Cl.: C07K 16/28, A61P 35/00

(54) **AXL-SPECIFIC ANTIBODY-DRUG CONJUGATES FOR CANCER TREATMENT**
AXL-SPEZIFISCHE ANTIKÖRPER-WIRKSTOFF-KONJUGATE ZUR KREBSBEHANDLUNG
CONJUGUÉS ANTICORPS-MÉDICAMENT SPÉCIFIQUES D'AXL POUR LE TRAITEMENT DU CANCER

(30) Priority: 10.07.2015 WO PCT/EP2015/065900; 13.01.2016 US 201662278283 P
(43) Date of publication of application: 16.05.2018
(73) Proprietor: Genmab A/S, 1560 Copenhagen V (DK)
(72) Inventor: BOSHUIZEN, Julia, 1058-NC Amsterdam (NL); JACOBSEN, Kirstine, 5000 Odense (DK); BREIJ, Esther, 3584 CT Utrecht (NL); KOOPMAN, Louise, 3584 CT Utrecht (NL); SATIJN, David, 3584 CT Utrecht (NL); VAN DEN BRINK, Edward, 3584 CT Utrecht (NL); VERZIJL, Dennis, 3584 CT Utrecht (NL); DE JONG, Rob, 3584 CT Utrecht (NL); VAN DIJKHUIZEN RADERSMA, Riemke, 3584 CT Utrecht (NL); PEEPER, Daniel, 1183-DN Amstelveen (NL); DITZEL, Henrik Jørn, 8660 Skanderborg (DK); PARREN, Paul, 3984 PR Odijk (NL)
(74) Representative: Genmab A/S
(86) International application number: PCT/EP2016/066353
(87) International publication number: WO 2017/009258

(56) References cited:
- WO-A1-2014/068139
- WO-A1-2014/174111
- WO-A1-2016/005593
- ESTHER BREIJ: "Novel antibody-drug conjugates targeting Axl show anti-tumor activity in solid cancer xenograft models", AMERICAN ASSOCIATION OF CANCER RESEARCH, 19 April 2015 (2015-04-19), pages 1-2, XP055296816,
- E BREIJ: "Preclinical efficacy studies using HuMax-Axl-ADC, a novel antibody-drug conjugate targeting Axl-expressing solid cancers.", JOURNAL OF CLINICAL ONCOLOGY, vol. 33, 29 May 2015 (2015-05-29), pages 1-2, XP055297040,
- CHEN JIAO ET AL: "PKD2 mediates multi-drug resistance in breast cancer cells through modulation of P-glycoprotein expression", CANCER LETTERS, vol. 300, no. 1, 10 December 2010 (2010-12-10), pages 48-56, XP029881986, ISSN: 0304-3835, DOI: 10.1016/J.CANLET.2010.09.005
- Fryer: "Mechanisms underlying gemcitabine resistance in pancreatic cancer and sensitisation by the iMiD(TM) lenalidomide", ANTICANCER RESEARCH - International Journal of Cancer Research and Treatment, vol. 31, no. 11, 1 November 2011 (2011-11-01), pages 3747-3756, XP055517139, GR ISSN: 0250-7005
- YINXIANG LV ET AL: "TopBP1 contributes to the chemoresistance in non-small cell lung cancer through upregulation of p53", DRUG DESIGN, DEVELOPMENT AND THERAPY, vol. Volume 10, 1 September 2016 (2016-09-01), pages 3053-3064, XP055517145, DOI: 10.2147/DDDT.S90705

## Description

### FIELD OF THE INVENTION

The present invention relates to antibody-drug conjugates (ADCs) binding to human AXL for therapeutic use, particularly for treatment of resistant or refractory cancers.

### BACKGROUND OF THE INVENTION

AXL is a 104-140 kDa transmembrane protein which belongs to the TAM subfamily of mammalian Receptor Tyrosine Kinases (RTKs) and which has transforming abilities (Paccez *et al.,* 2014). The AXL extracellular domain is composed of a combination of two membrane-distal N-terminal immunoglobulin (Ig)-like domains (Ig1 and Ig2 domains) and two membrane-proximal fibronectin type III (FNIII) repeats (the FN1- and FN2-domains) (Paccez *et al.,* 2014). Enhanced or *de novo* expression of AXL has been reported in a variety of cancers, including gastric, prostate, ovarian, and lung cancer (Paccez *et al.,* 2014). Of note, several types of cancer with resistance to tyrosine kinase inhibitors, serine/threonine kinase inhibitors and/or chemotherapy have been found to show enhanced or *de novo* AXL protein. In particular, tumor cells with resistance to Epidermal Growth Factor Receptor (EGFR) targeted therapy (Wilson *et al.,* 2014; Brand *et al.,* 2015; Zhang *et al.,* 2012; Blakely *et al.,* 2012) or inhibitors of the B-raf (BRAF) pathway (Müller *et al.,* 2014) showed enhanced or *de novo* AXL expression. In addition, enhanced or *de novo* expression of AXL was reported in head and neck cancer (SCCHN) cells resistant to the PI3K inhibitor BYL719 (Elkabets *et al.,* 2015), in breast cancer resistant to the HER2-targeting agent lapatinib (Liu *et al.,* 2009), in gastro-intestinal stromal tumors (GIST) resistant to imatinib (Mahadevan *et al.,* 2015), in renal cancer resistant to sunitinib (Zhou *et al.,* 2015), in neuroblastoma cells and non-small cell lung cancer (NSCLC) resistant to the ALK inhibitor crizotinib (Debruyne *et al.,* 2015; Kim *et al.,* 2013), in esophageal cancer resistant to cisplatin (Hong *et al.,* 2013), in rhabdomyosarcoma resistant to the IGF-IR antibody MAB391 (Huang *et al.,* 2010), in acute myeloid leukemia (AML) resistant to the FLT3 inhibitors midostaurin (PKC412) or quizartinib (AC220) (Park *et al.,* 2015), in drug-resistant AML (Hong *et al.,* 2008), and in chronic myeloid leukemia resistant to imatinib (Dufies *et al.,* 2011). AXL expression was also induced in prostate cancer cells with acquired resistance to metformin (Bansal *et al.,* 2015).

AXL can be activated upon binding of its ligand, the vitamin K-dependent growth arrest-specific factor 6 (Gas6). Gas6-binding to AXL leads to AXL dimerization, autophosphorylation and subsequent activation of intracellular signaling pathways, such as the PI3K/AKT, mitogen-activated protein kinase (MAPK), STAT and NF-_{K}B cascades (Leconet *et al.,* 2013). In cancer cells, AXL expression has been associated with tumor cell motility, invasion, migration, and is involved in epithelial-to-mesenchymal transition (EMT) (Linger *et al.,* 2010).

Targeted inhibition of AXL and/or its ligand Gas6 may be effective as anti-tumor therapy using, *e.g.,* small molecules or anti-AXL antibodies (Linger *et al.,* 2010). Anti-AXL antibodies have been described that attenuate NSCLC and breast cancer xenograft growth *in vivo* by downregulation of receptor expression, reducing tumor cell proliferation and inducing apoptosis (Li *et al.,* 2009; Ye *et al.,* 2010; WO 2011/159980, Genentech). Various other anti-AXL antibodies have also been reported (Leconet *et al.,* 2013; lida *et al.,* 2014; WO 2012/175691, INSERM; WO 2012/175692, INSERM; WO 2013/064685, Pierre Fabré Medicaments; WO 2013/090776, INSERM; WO 2009/063965, Chugai Pharmaceuticals and WO 2010/131733), including an ADC based on an anti-AXL antibody and a pyrrolobenzo-diazepine (PBD) dimer (WO 2014/174111, Pierre Fabré Medicament and Spirogen Sari).

Breij et al., "Novel antibody-drug conjgates targeting Axl show anti-tumor activity in solid cancer xenograft models", American Association of Cancer Research, 19 April 2015, pp 1-2 (XP055296816), describes the use of anti-AXL antibody drug conjugates (ADC) to treat lung cancer and epidermoid carcinomas. The anti-AXL antibodies were conjugated to MMAE.

Breij, et al., Journal of Clinical Oncology, Vol. 33, 29 May, 2015, pp 1-2 (XP055297040) describes the use of an AXL antibody conjugated to MMAE to kill lung cancer cells *in vivo.*

WO 2014/068139 describes anti-AXL antibodies, 1003A2 and 1024G11 and conjugates thereof to saporin for killing various tumor cell lines.

WO 2016/005593 describes inhibitory anti-AXL antibodies inhibiting tumor growth and not competing with the ligand GAS6 for binding to AXL, and their use as ADC with MMAE to kill various cancer types *in vivo,* such as breast cancer, lung cancer, cervical carcinoma, esophageal cancer, epidermoid carcinoma and pancreas cancer.

However, there remains a need for improved methods of treating cancers which are, or which have a high tendency to become, resistant to tyrosine kinase inhibitors, serine/threonine kinase inhibitors and/or chemotherapy, particularly using AXL-ADCs.

### SUMMARY OF THE INVENTION

It has been found by the present inventor(s) that ADCs based on anti-AXL antibodies can be used to efficiently treat cancers which are resistant, or which have a high tendency to become resistant, to certain therapeutic agents.

So, in one aspect, the invention relates to an ADC comprising an antibody binding to human AXL for use in treating cancer resistant to at least one therapeutic agent selected from the group consisting of a tyrosine kinase inhibitor, a serine/threonine kinase inhibitor and a chemotherapeutic agent wherein the ADC comprises least one binding region that comprises a VH region comprising the CDR1, CDR2, and CDR3 sequences of SEQ ID Nos.: 36, 37, and 38, respectively; and a VL region comprising the CDR1, CDR2, and CDR3 sequences of SEQ ID Nos.: 39, GAS, and 40, respectively.

This aspect and embodiments and disclosures, including the use of AXL-ADCs based on anti-AXL antibodies characterized by their antigen-binding properties or -sequences, therapeutic moieties suitable for such ADCs, combinations of such ADCs with certain therapeutic agents, and methods of treating resistant neoplasms, are described in further detail below.

### LEGENDS TO THE FIGURES

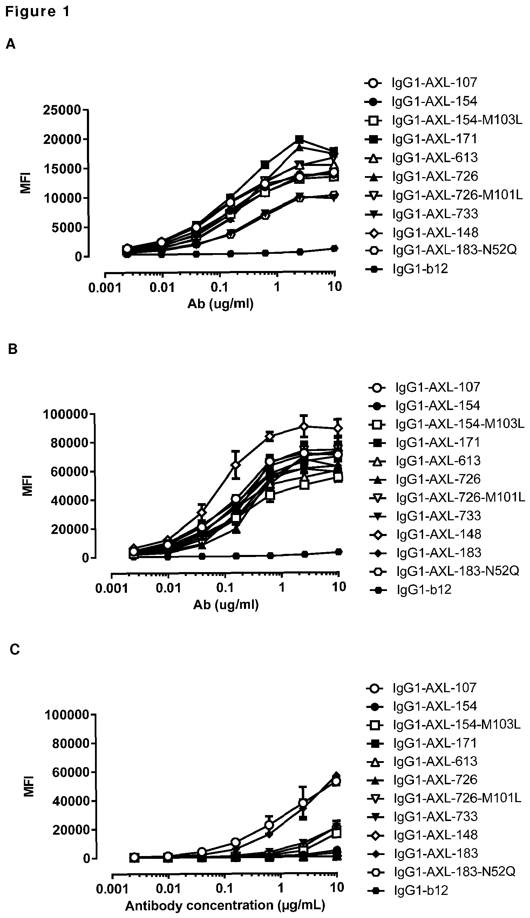

**Figure 1****:** Binding curves of anti-AXL antibodies to HEK293 cells transfected with (A) human AXL-ECD, (B) cynomolgus AXL-ECD, or (C) mouse AXL-ECD. Data shown are mean fluorescence intensities (MFI) of one representative experiment, as described in Example 2.

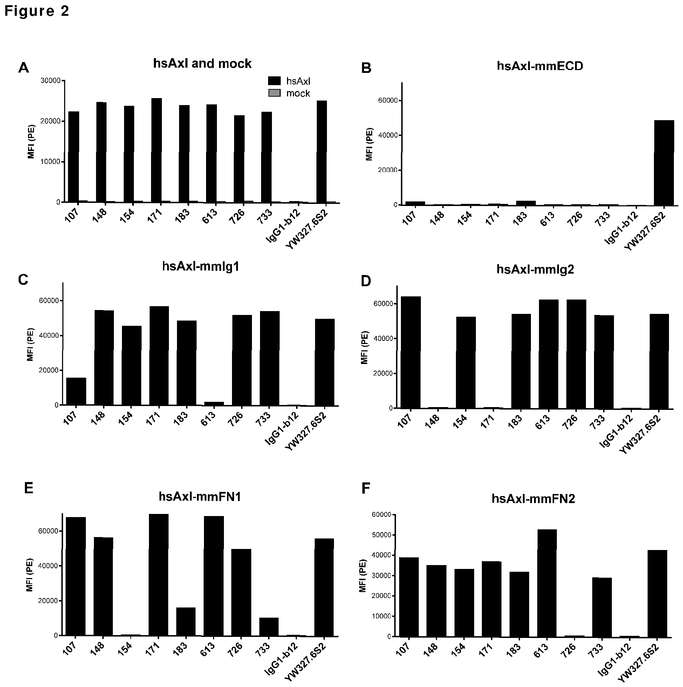

**Figure 2****:** Binding of anti-AXL antibodies to mouse-human AXL chimeras was performed as described in Example 3. The following *Homo sapiens* AXL (hsAXL) and *Mus musculus* AXL (mmAXL) chimeric proteins were tested: (A) hsAXL and mock, (B) hsAXL-mmECD, (C) hsAXL-mmlg1, (D) hsAXL-mmlg2, (E) hsAXL-mmFN1, (F) hsAXL-mmFN2.

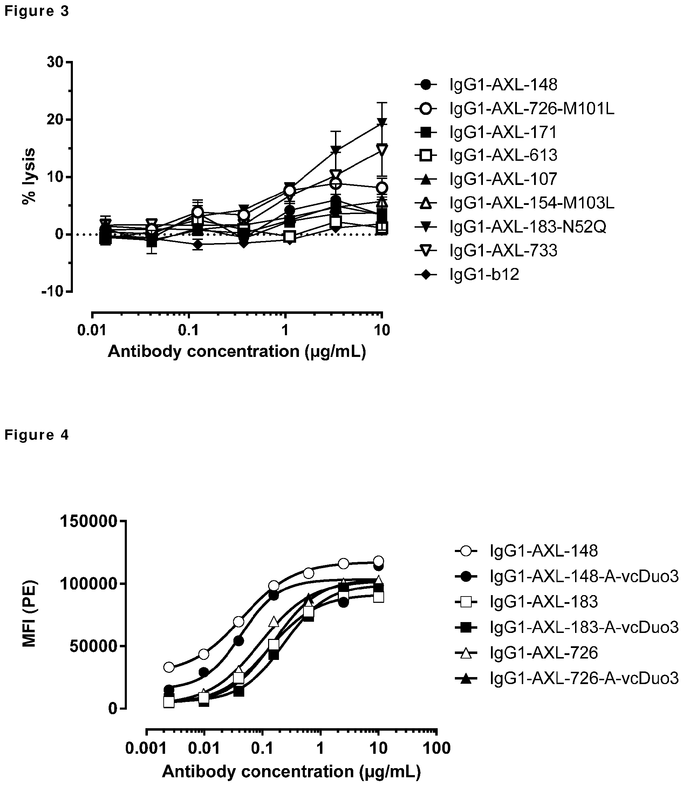

**Figure 3****:** Anti-AXL antibody-dependent cell-mediated cytotoxicity in A431 cells. Antibody-dependent cell-mediated cytotoxicity by anti-AXL antibodies in A431 cells was determined as described in Example 4.
**Figure 4****:** Binding characteristics of AXL antibody-drug conjugates (AXL-ADCs). Binding of AXL-ADCs on HEK293T cells transiently transfected with human AXL was determined as described in Example 5. Data shown are mean fluorescence intensities (MFI) of one representative experiment.

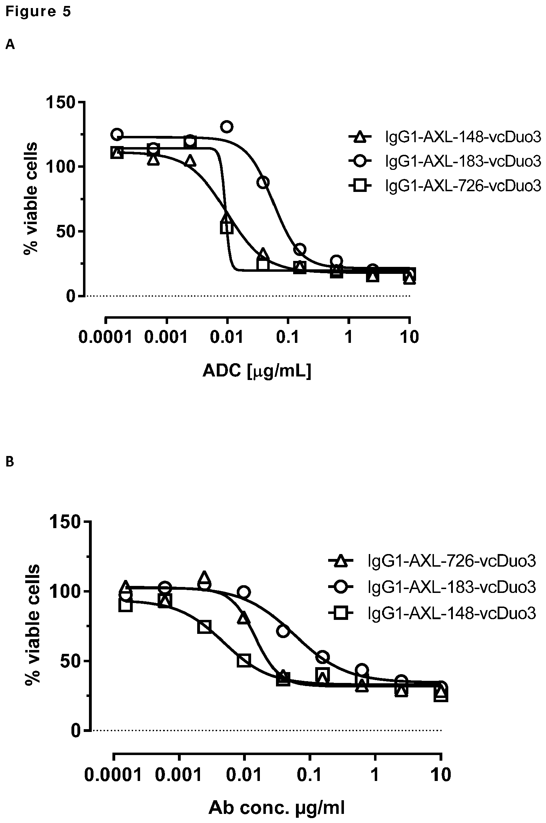

**Figure 5****:** *In vitro* cytotoxicity induced by AXL antibody-drug conjugates. Induction of cytotoxicity by AXL antibody-drug conjugates was determined as explained in Example 6.

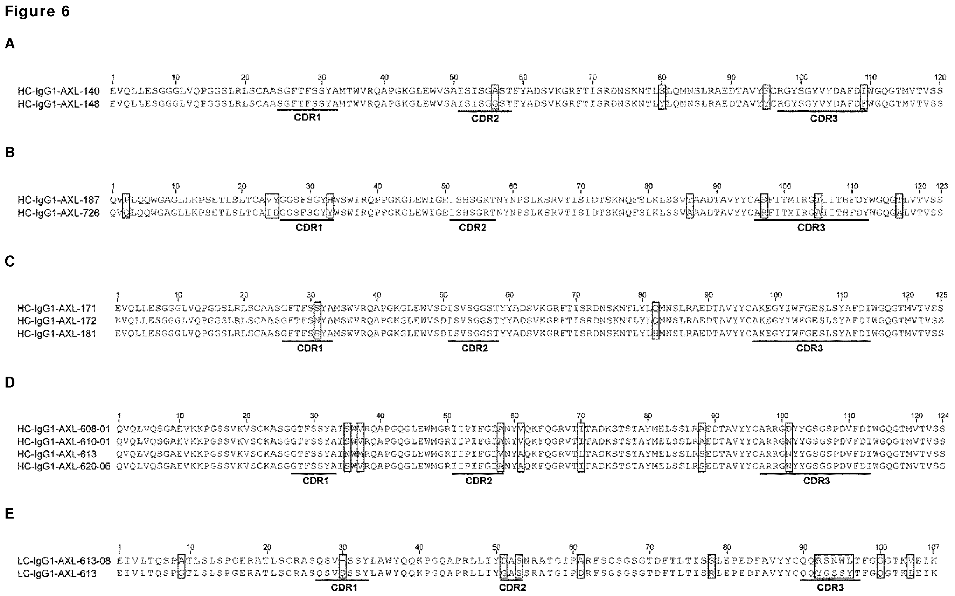

**Figure 6****:** Antibody VH and VL variants that allow binding to AXL. Antibodies with identical VL or VH regions were aligned and differences in VH (Figures A-D) or VL (Figure E) sequences, respectively, were identified and indicated by boxes in the figures. CDR regions are underlined.

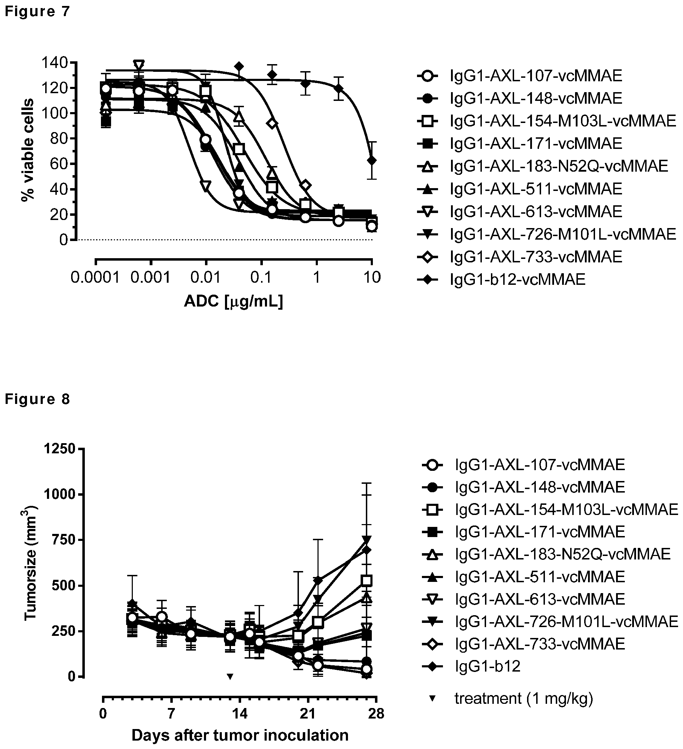

**Figure 7****:** Induction of cytotoxicity by ADCs in LCLC-103H cells was determined as described in Example 8.
**Figure 8****:** Anti-tumor activity by MMAE-conjugated AXL antibodies in a therapeutic LCLC-103H xenograft model as described in Example 9.

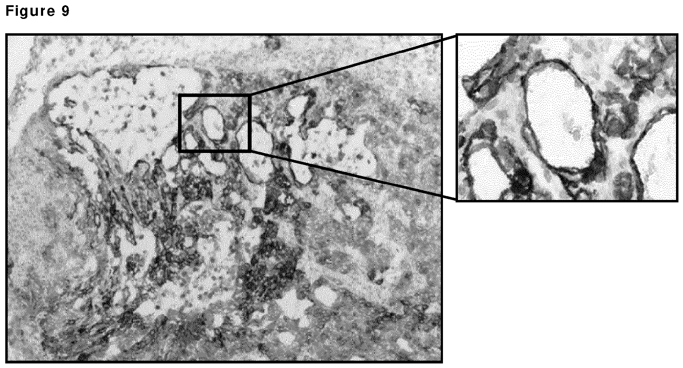

**Figure 9****:** Immunohistochemical staining of frozen PAXF1657 tumor sections (pancreas cancer PDX model) using a pool of AXL monoclonal antibodies as described in Example 10.

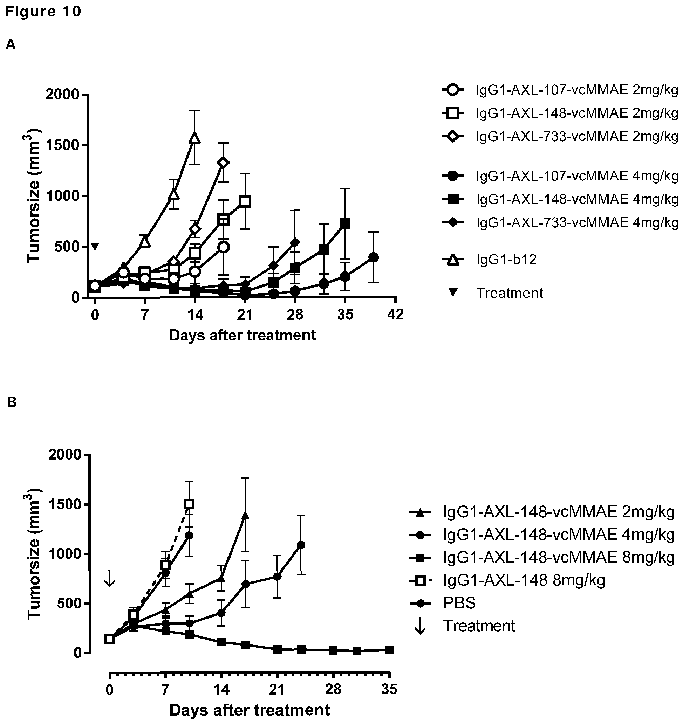

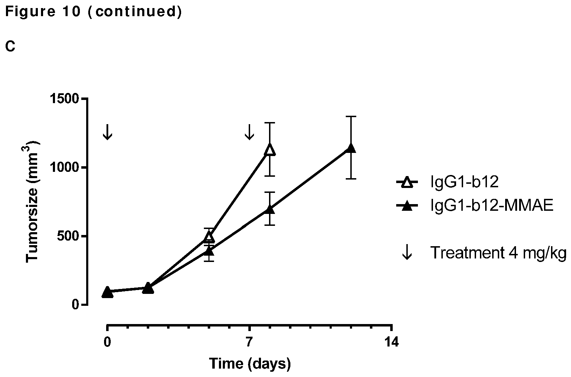

**Figure 10****:** (A) Average tumor size after therapeutic treatment with AXL-ADCs the PAXF1657 model. An unconjugated AXL Humab (C) and an untargeted ADC (D) do not show anti-tumor activity, indicating that the therapeutic capacity of AXL-ADCs was dependent on the cytotoxic activity of MMAE and on target binding, error bars represent S.E.M.

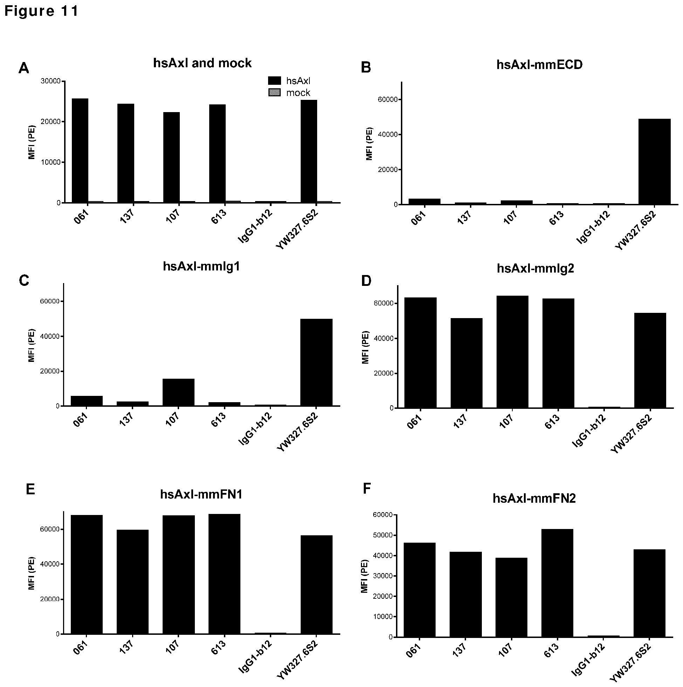

**Figure 11****:** Binding of anti-AXL antibodies to mouse-human AXL chimeras was performed as described in Example 11. The following Homo sapiens AXL (hsAXL) and Mus musculus AXL (mmAXL) chimeric proteins were tested: (A) hsAXL and mock, (B) hsAXL-mmECD, (C) hsAXL-mmIg1, (D) hsAXL-mmIg2, (E) hsAXL-mmFN1, (F) hsAXL-mmFN2.

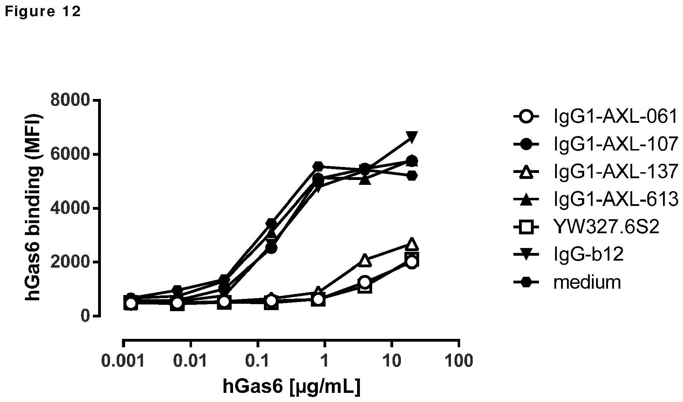

**Figure 12****:** Binding of human Gas6 (hGas6) on A431 cells that had been pre-incubated with antibodies binding to the Ig1 domain of AXL. Data shown are mean fluorescence intensities (MFI) of one representative experiment.

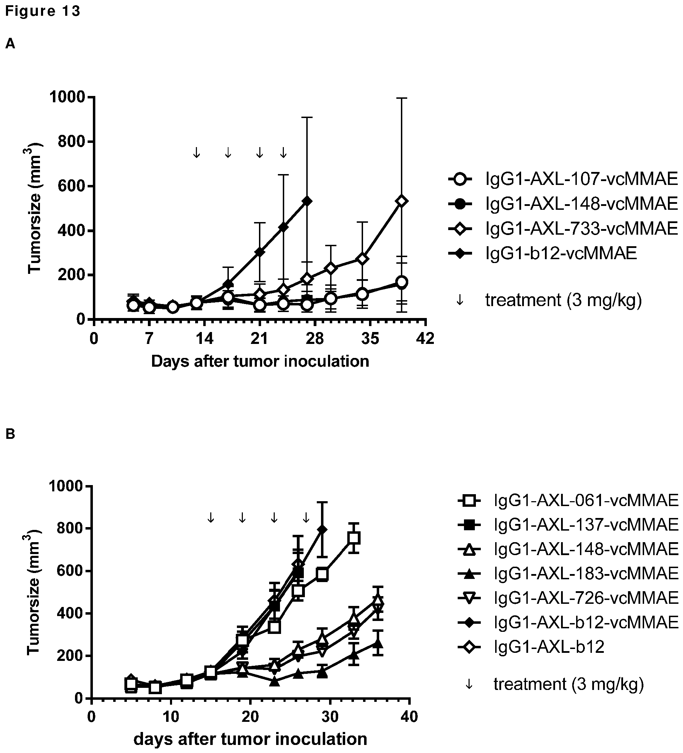

**Figure 13****:** Anti-tumor activity of MMAE-conjugated AXL antibodies in a therapeutic A431 xenograft model, that produces high levels of endogeneous Gas6, as described in Example 13. Panels A and B show results from 2 independent experiments.

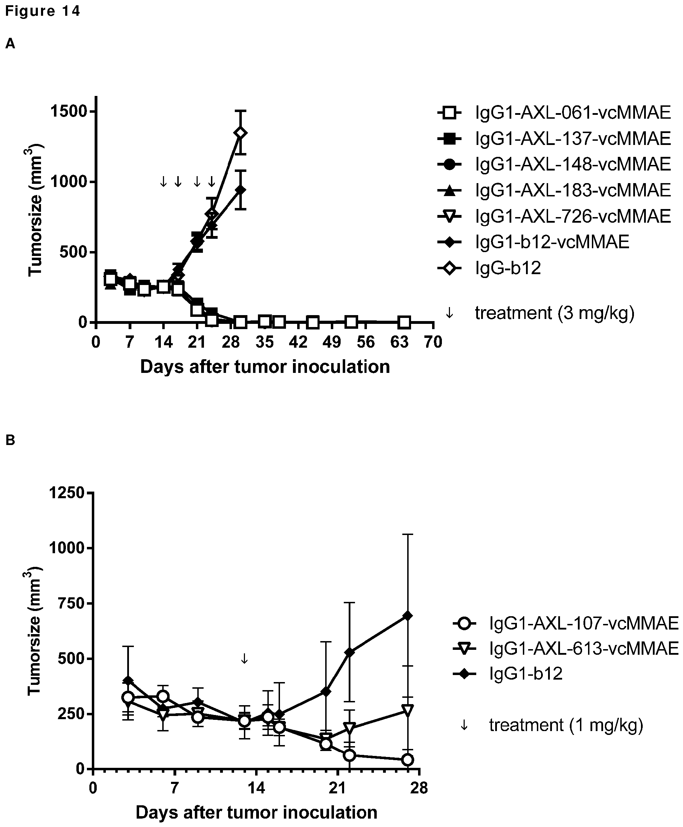

**Figure 14****:** Anti-tumor activity of MMAE-conjugated AXL antibodies in a therapeutic LCLC-103H xenograft model, that expresses low levels of endogenous Gas6, as described in Example 13. Panels A and B show results from 2 independent experiments.

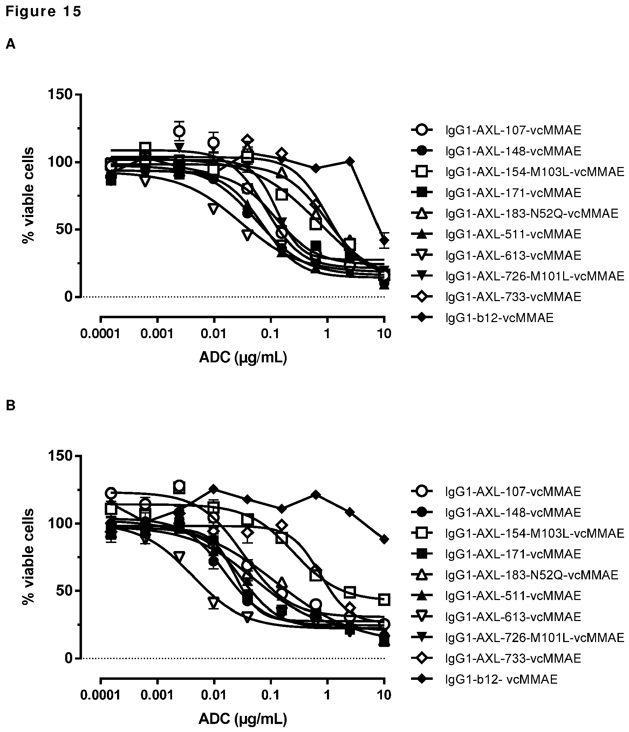

**Figure 15****:** Induction of cytotoxicity by AXL-ADCs in A431 cells (A) and MDA-MB231 cells (B) was determined as described in Example 8.

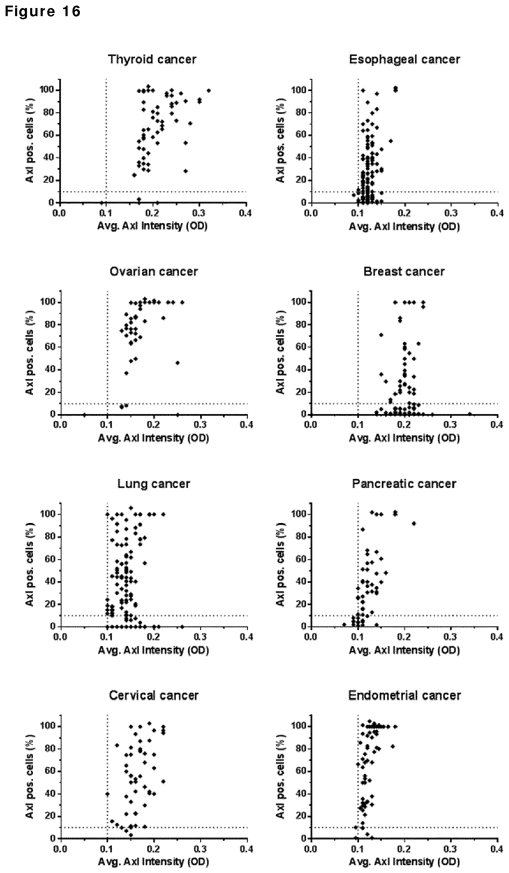

**Figure 16****.** AXL staining in thyroid, esophageal, ovarian, breast, lung, pancreatic, cervical and endometrial cancer. The average AXL staining intensity (OD) of AXL-positive cells is plotted on the X-axis, and the percentage of AXL-positive tumor cells is plotted on the Y-axis. Each dot represents a tumor core, derived from an individual patent.

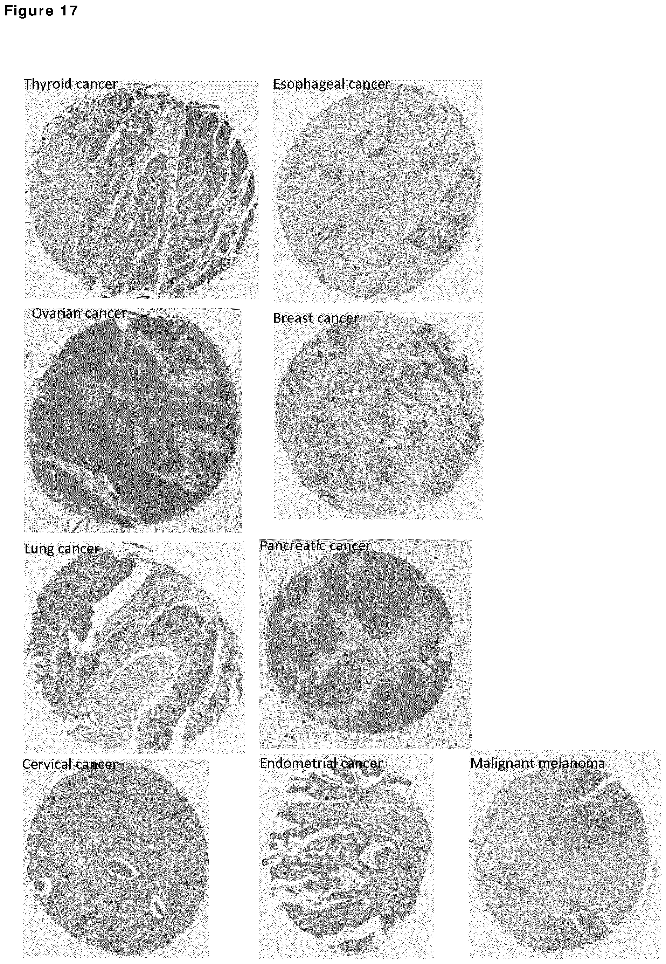

**Figure 17****.** Representative examples of AXL-immunostained tumor cores for different tumor indication.

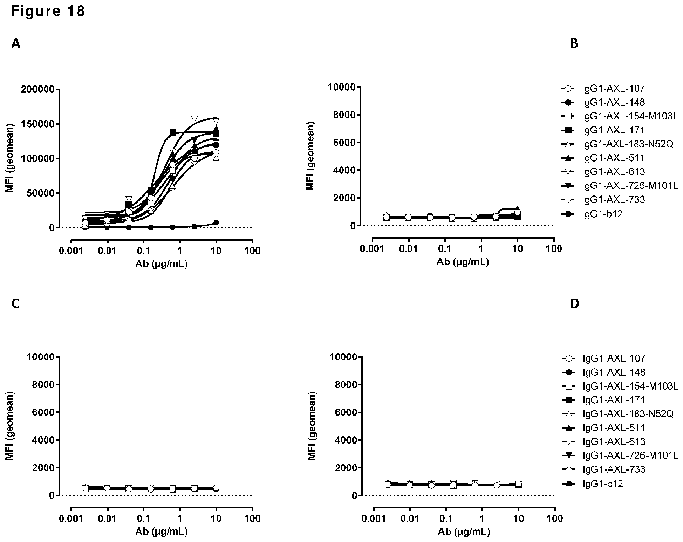

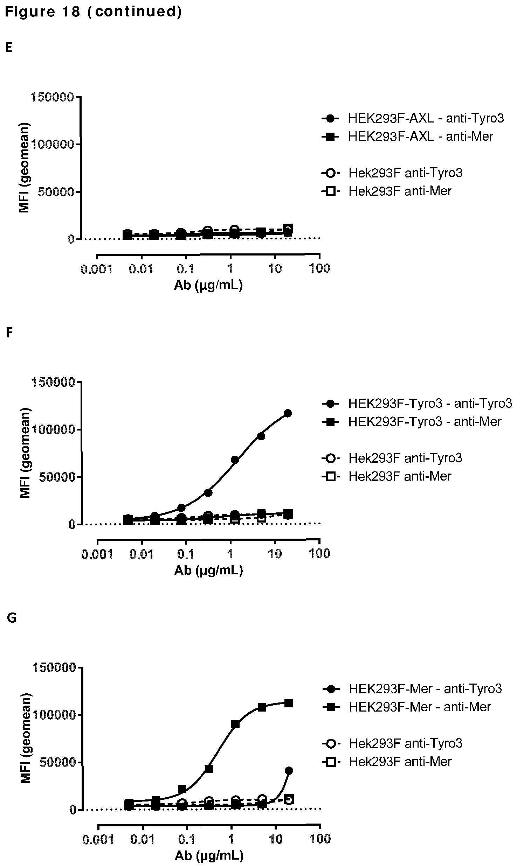

**Figure 18****.** AXL antibodies specifically bind AXL but not to other TAM receptor family members. Binding of HuMab-AXL antibodies to HEK293 cells transfected with human AXL (A), human MER (B), human TYRO3 (C), or untransfected HEK293 cells (D). To confirm proper expression of transfected cells, untransfected HEK293F cells and cells transfected with AXL (E), MER (F), or TYRO3 (G) were stained with MER- and TYRO3-specific antibodies. Data shown are mean fluorescence intensities (MFI) of one representative experiment, as described in Example 15.

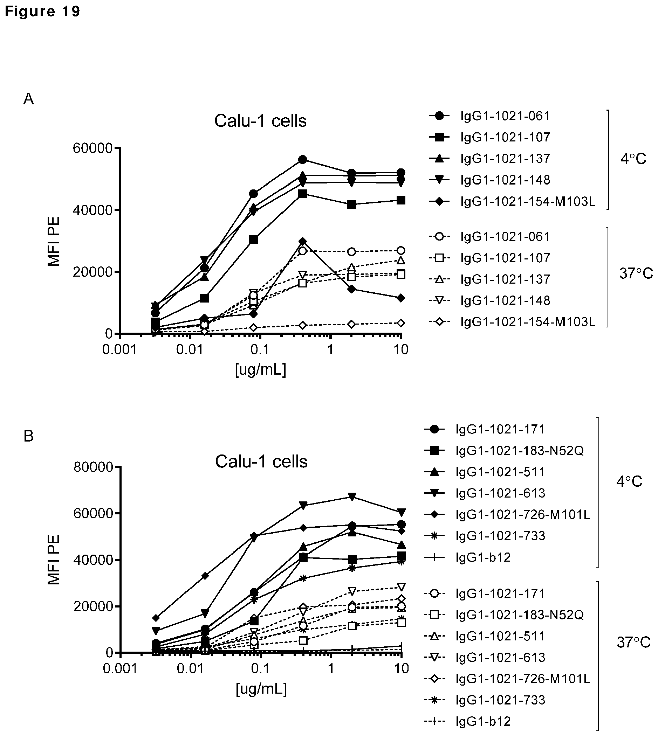

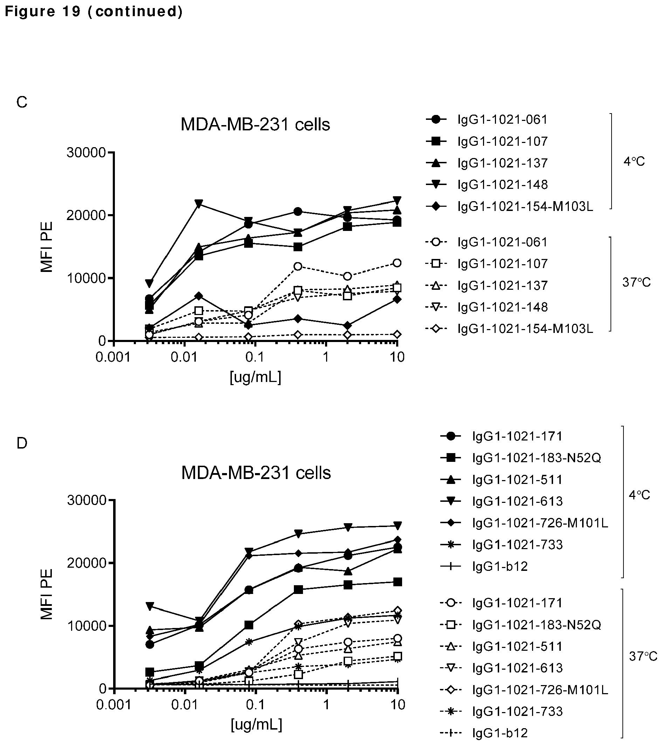

**Figure 19****.** Detection of AXL antibodies on the plasma membrane of tumor cell lines that had been incubated with AXL-antibodies for 1 hour at 4°C, followed by an overnight incubation 4°C or 37°C. In both MDA-MB-231 (A and B) and Calu-1 cells (C and D), more antibody was detected on the plasma membrane of cells that had been incubated at 4°C than on cells that had been incubated at 37°C, illustrating internalization of membrane-bound antibody at 37°C.

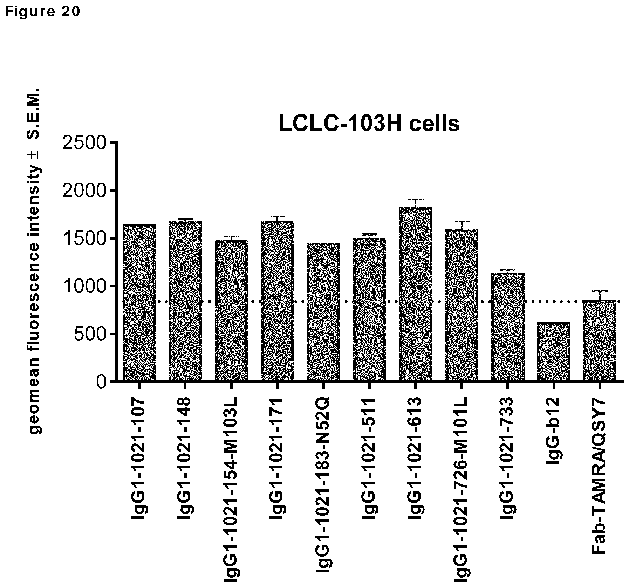

**Figure 20****.** Geomean fluorescence intensity of LCLC-103H cells after incubation with AXL antibodies that had been complexed to Fab-TAMRA/QSY7. IgG1-b12 and Fab-TAMRA/QSY7 alone were included as negative controls.

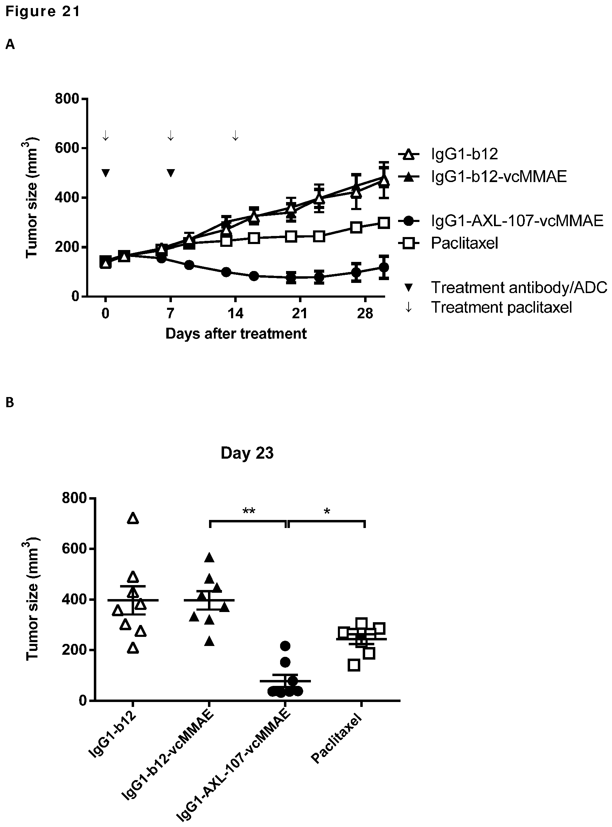

**Figure 21****.** (A) Average tumor size after therapeutic treatment with IgG1-AXL-107-vcMMAE in the esophageal cancer PDX model ES0195. IgG1-b12 and IgG1-b12-MMAE were included as isotype control antibody and isotype control ADC, respectively. (B) Tumor size in individual mice on day 32 after injection of MDA-MB-231-luc D3H2LN tumor cells in the mammary fat pads of female SCID mice. * p<0.05; ** p<0.0001

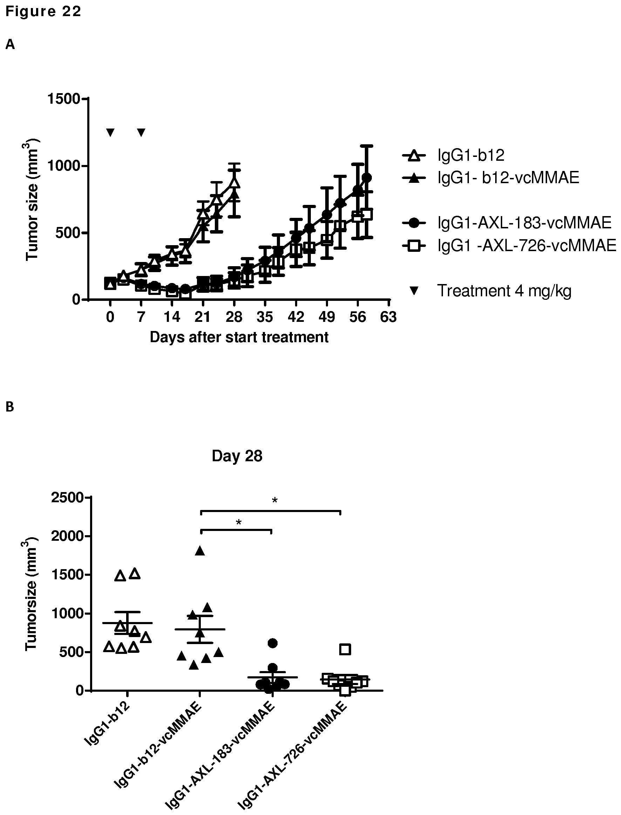

**Figure 22****.** Therapeutic effect of AXL-ADCs in a patient-derived cervical cancer xenograft model. (A) Average tumor size after therapeutic treatment with IgG1-AXL-183-vcMMAE or IgG1-AXL-726-vcMMAE in the cervical cancer PDX model CEXF 773. IgG1-b12 and IgG1-b12-MMAE were included as isotype control antibody and isotype control ADC, respectively. (B) Tumor size in individual mice on day 28 after initiation of treatment in the cervical cancer PDX model CEXF 773. * p<0.001.

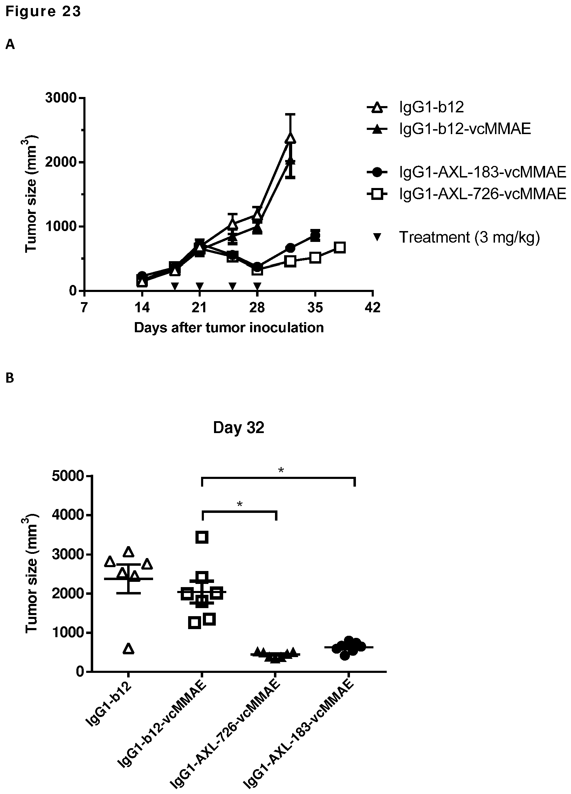

**Figure 23****.** Therapeutic activity of AXL-ADCs in an orthotopic breast cancer xenograft model. (A) Average tumor size after therapeutic treatment with IgG1-AXL-183-vcMMAE or IgG1-AXL-726-vcMMAE in an orthotopic MDA-MB-231-luc D3H2LN xenograft model. IgG1-b12 and IgG1-b12-MMAE were included as isotype control antibody and isotype control ADC, respectively. (B) Tumor size in individual mice on day 32 after injection of MDA-MB-231-luc D3H2LN tumor cells in the mammary fat pads of female SCID mice. * p<0.001.

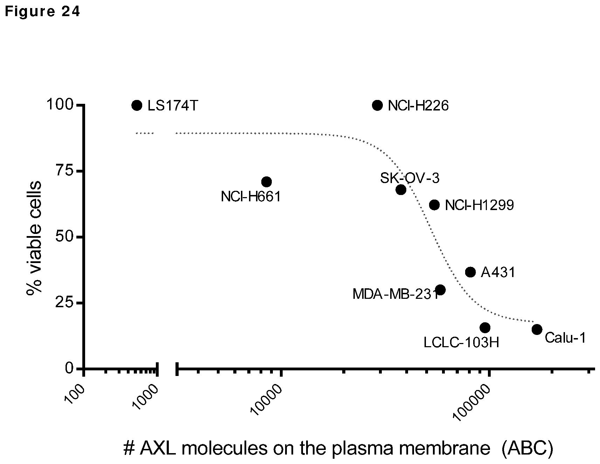

**Figure 24****.** Cytotoxicity of IgG1-AXL-107-vcMMAE in human tumor cell lines with different levels of AXL expression on the plasma membrane. AXL expression in the plasma membrane of human tumor cell lines was assessed using Qifikit analysis, and the cytotoxicity of IgG1-AXL-107-vcMMAE was expressed as the percentage of viable tumor cells that remained in the cell cultures after exposure to 1 µg/mL IgG1-AXL-107-vcMMAE.

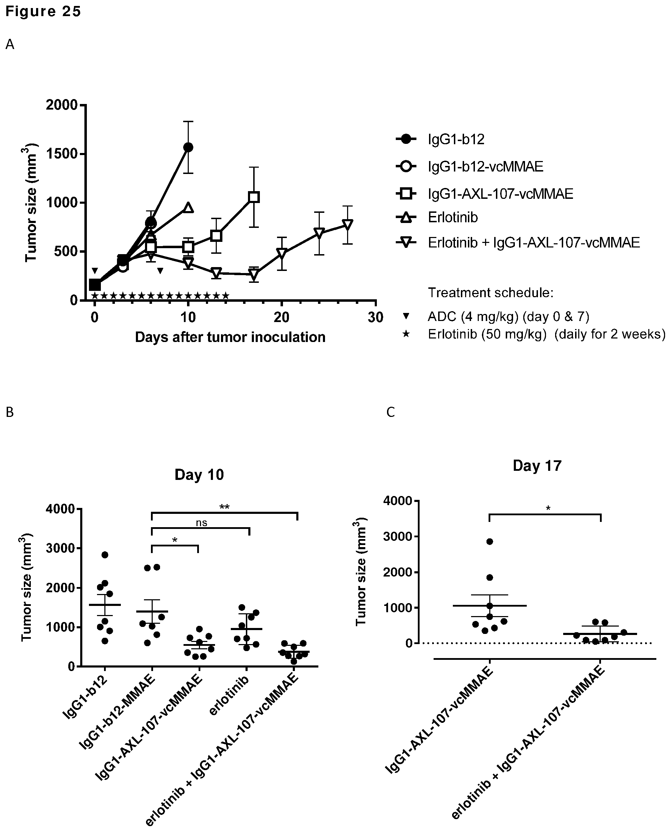

**Figure 25****.** Improved anti-tumor efficacy of IgG1-AXL-107-vcMMAE in an erlotinib-resistant NSCLC patient-derived xenograft (PDX) model in combination with erlotinib. Average tumor size after therapeutic treatment with IgG1-AXL-107-vcMMAE, erlotinib, or erlotinib in combination with IgG1-AXL-107-vcMMAE in the NSCLC PDX model LU2511. IgG1-b12 and IgG1-b12-MMAE were included as isotype control antibody and isotype control ADC, respectively. *, p<0.05; **, p<0.01; ns, not significant (one-way ANOVA test).

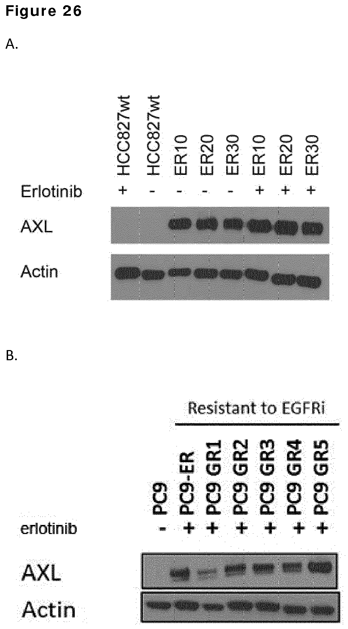

**Figure 26****.** Enhanced Axl protein expression in NSCLC cell lines with acquired resistance to EGFR-TKIs. The expression of Axl protein was determined by Western blotting. Actin staining was used as control for equal protein loading. Expression of Axl was evaluated in cells that had been cultured in the presence (+) or absence (-) of erlotinib.

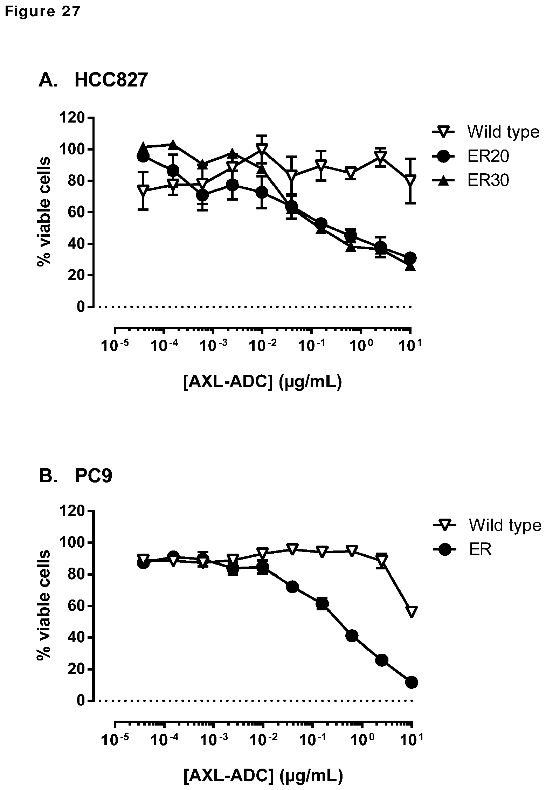

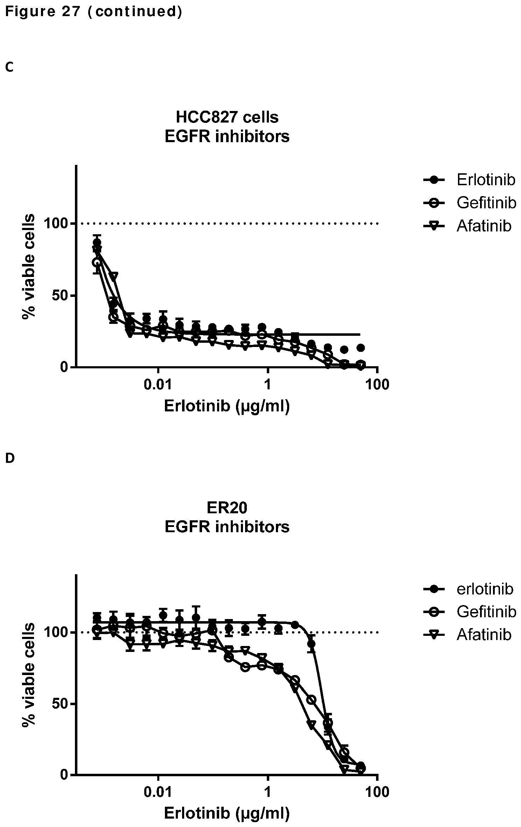

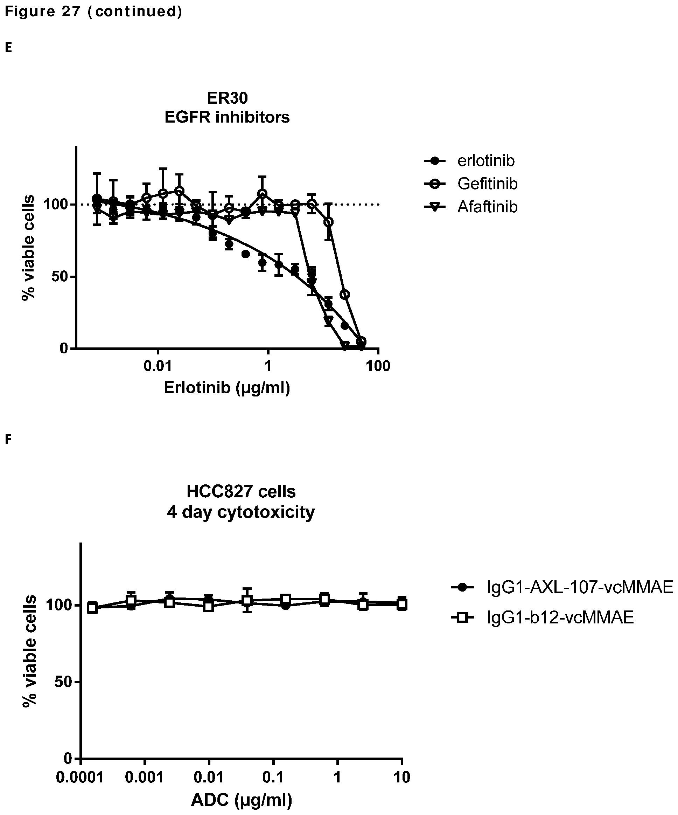

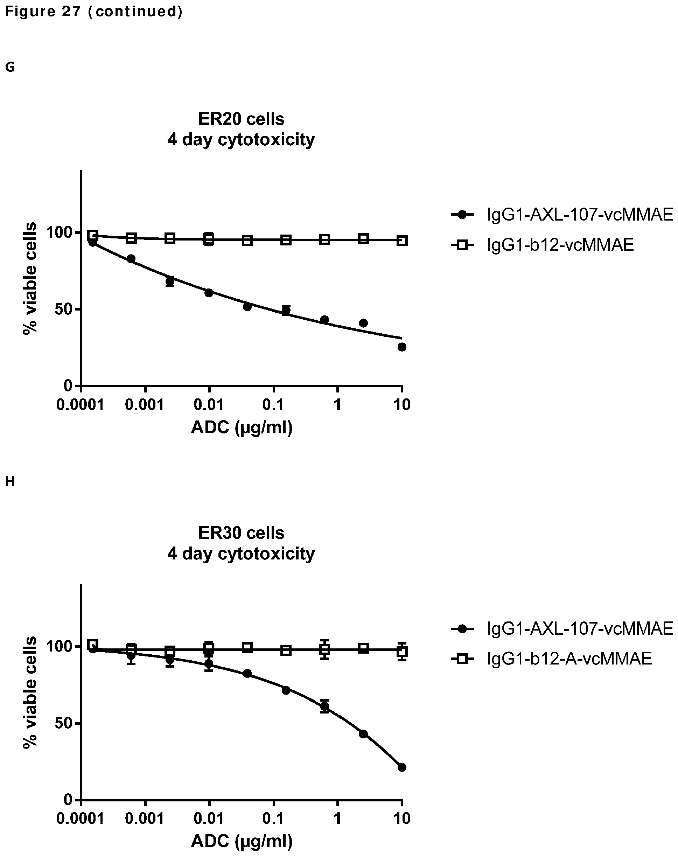

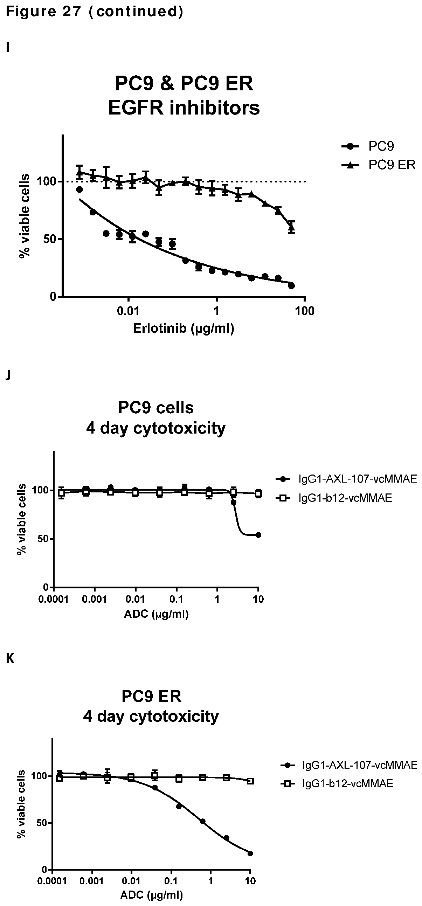

**Figure 27****.** Sensitivity of parental (wild-type) and erlotinib-resistant HCC827 and PC9 cells to IgG1-AXL-107-vcMMAE (A, B, F, G, H, J, K) or EGFR-TKIs (C, D, E, and I) was evaluated in viability assays. Parental (wild-type) and erlotinib-resistant cell lines were exposed to increasing concentrations of IgG1-b12-vcMMAE, IgG1-AXL-107-vcMMAE, erlotinib, gefitinib, or afatinib for 4 or 5 days after which the cell viability was determined as described in Example 22.

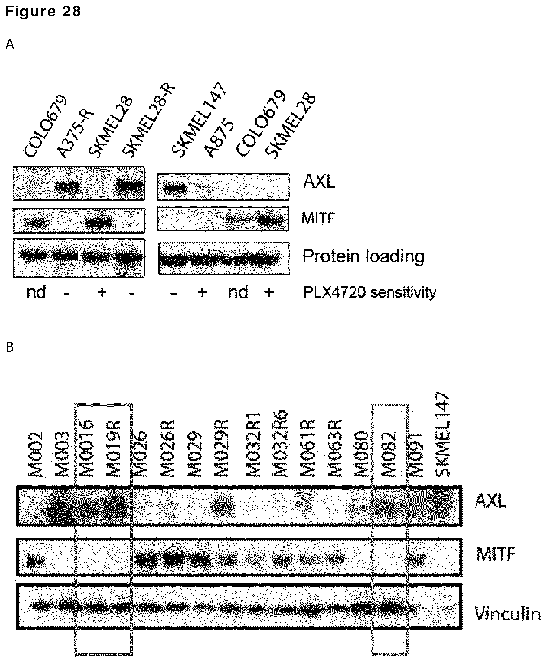

**Figure 28****.** AXL expression in established melanoma cell lines and patient-derived low passage primary melanoma lines (PDX). (A) Variable levels of AXL expression were observed in established melanoma cell lines. Enhanced or de novo AXL expression was observed in PLX4720 resistant cell lines (A375-R, SKMEL28R, SKMEL147). (B) AXL expression was observed in 8/15 patient derived primary melanoma lines. In both established melanoma cell lines and low passage PDX cultures, AXL expression was inversely correlated with MITF expression.

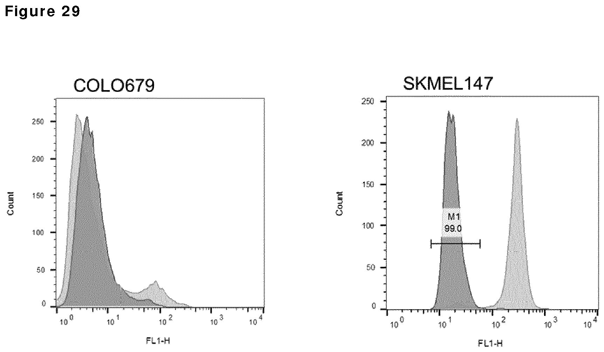

**Figure 29****.** AXL protein expression on the cell surface. Examples of AXL expression as determined by quantitative flow cytometry in an Axl-negative and an Axl-positive melanoma cell line. The light gray plots represent staining with AXL-specific antibodies, while the dark grey plots represent staining with isotype control antibody.

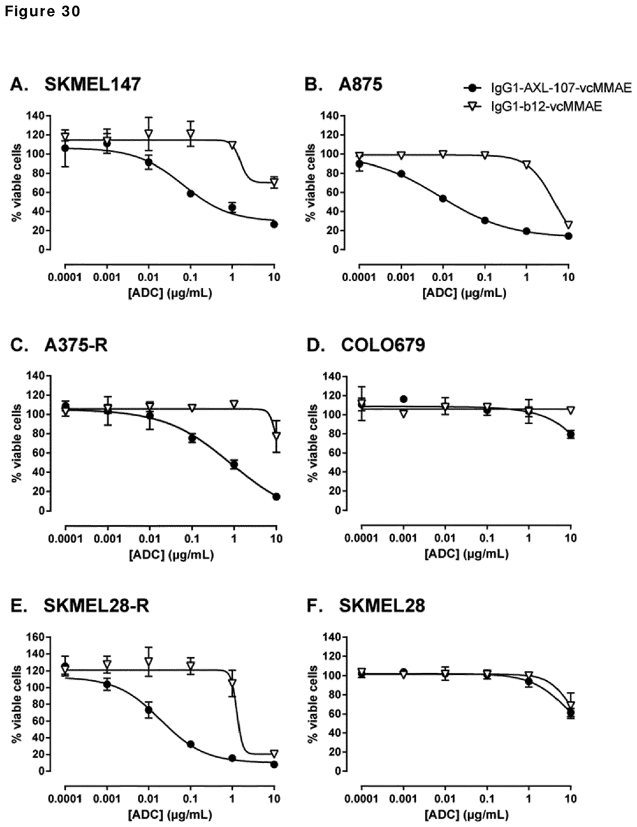

**Figure 30****.** Sensitivity of established melanoma cell lines to IgG1-AXL-107-vcMMAE. Melanoma cell lines (A-F; CDX) were treated with IgG1-AXL-107-vcMMAE or the isotype control ADC IgG1-b12-vcMMAE for 5 days in triplicate. Cell viability was assessed with a CellTiter-Glo assay and plotted against the ADC concentration.

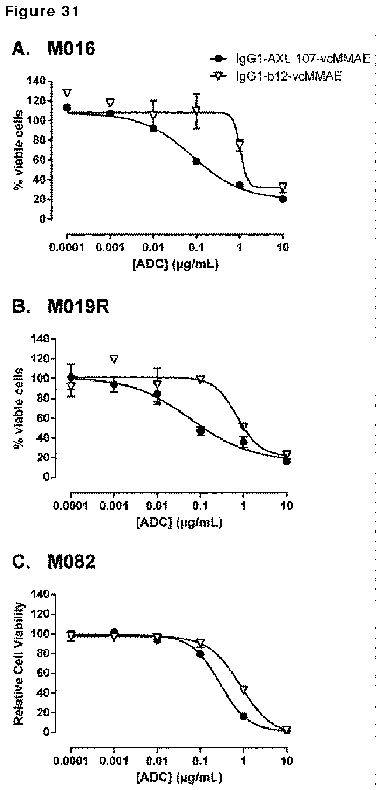

**Figure 31****.** Sensitivity of primary melanoma cell cultures to IgG1-AXL-107-vcMMAE. Low passage primary melanoma cell lines (A-C; PDX) were treated with IgG1-AXL-107-vcMMAE or the isotype control ADC IgG1-b12-vcMMAE for 8 days in triplicate. Cell viability was assessed with a CellTiter-Glo assay and plotted against the ADC concentration.

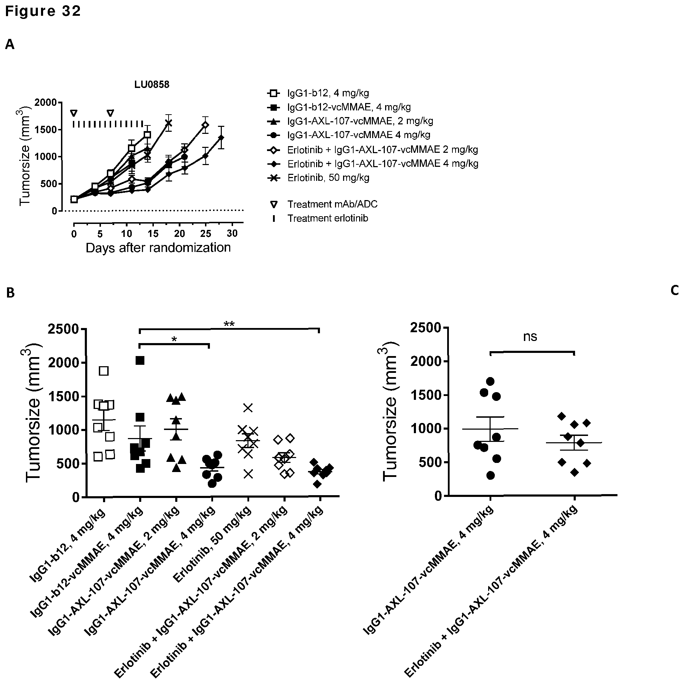

**Figure 32****.** Anti-tumor efficacy of IgG1-AXL-107-vcMMAE in the erlotinib-resistant LU0858 NSCLC patient-derived xenograft (PDX) model. Average tumor size after therapeutic treatment with IgG1-AXL-107-vcMMAE, erlotinib, or erlotinib in combination with IgG1-AXL-107-vcMMAE is shown (A). IgG1-b12 and IgG1-b12-MMAE were included as isotype control antibody and isotype control ADC, respectively. Mean tumor size and SEM of each group per time point and tumor size per individual mouse per group on day 11 (B) and day 21 (C) are shown. *, p<0.05; **, p<0.01; ns, not significant (Mann-Whitney test).

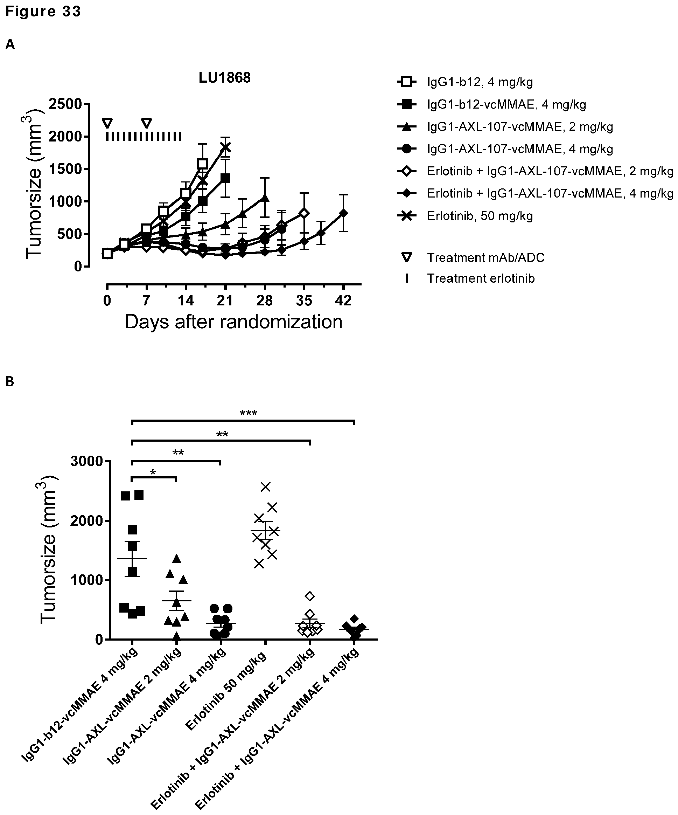

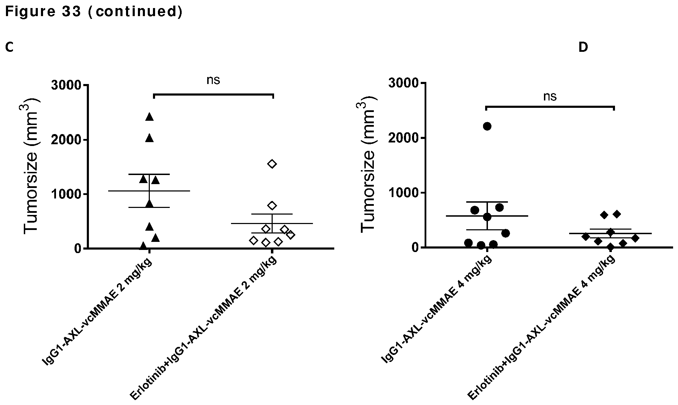

**Figure 33****.** Anti-tumor efficacy of IgG1-AXL-107-vcMMAE in the erlotinib-resistant LU1868 NSCLC patient-derived xenograft (PDX) model. Average tumor size after therapeutic treatment with IgG1-AXL-107-vcMMAE, erlotinib, or erlotinib in combination with IgG1-AXL-107-vcMMAE is shown (A). IgG1-b12 and IgG1-b12-MMAE were included as isotype control antibody and isotype control ADC, respectively. Mean tumor size and SEM of each group per time point and tumor size per individual mouse per group on day 21 (B), day 28 (C) and day 31 (D) are shown. *, p<0.05; **, p<0.01; ns, not significant (Mann-Whitney test).

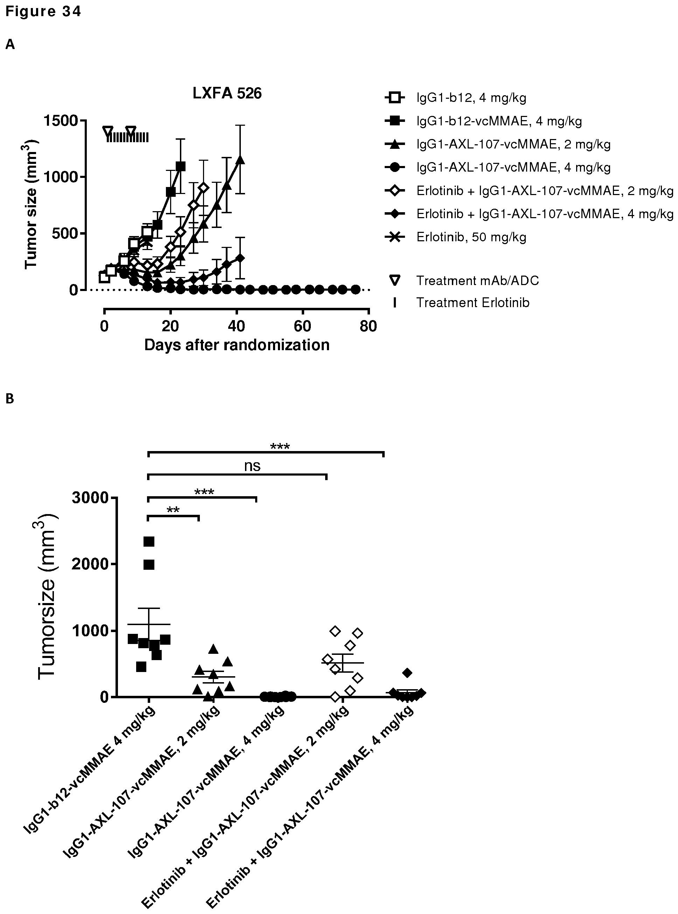

**Figure 34****.** Anti-tumor efficacy of IgG1-AXL-107-vcMMAE in the erlotinib-resistant LXFA 526 NSCLC patient-derived xenograft (PDX) model. (A) Average tumor size after therapeutic treatment with IgG1-AXL-107-vcMMAE, erlotinib, or erlotinib in combination with IgG1-AXL-107-vcMMAE is shown. (B) Mean tumor size and SEM of each group per time point and tumor size per individual mouse per group on day 23. *, p<0.05; **, p<0.01; ns, not significant (Mann-Whitney test).

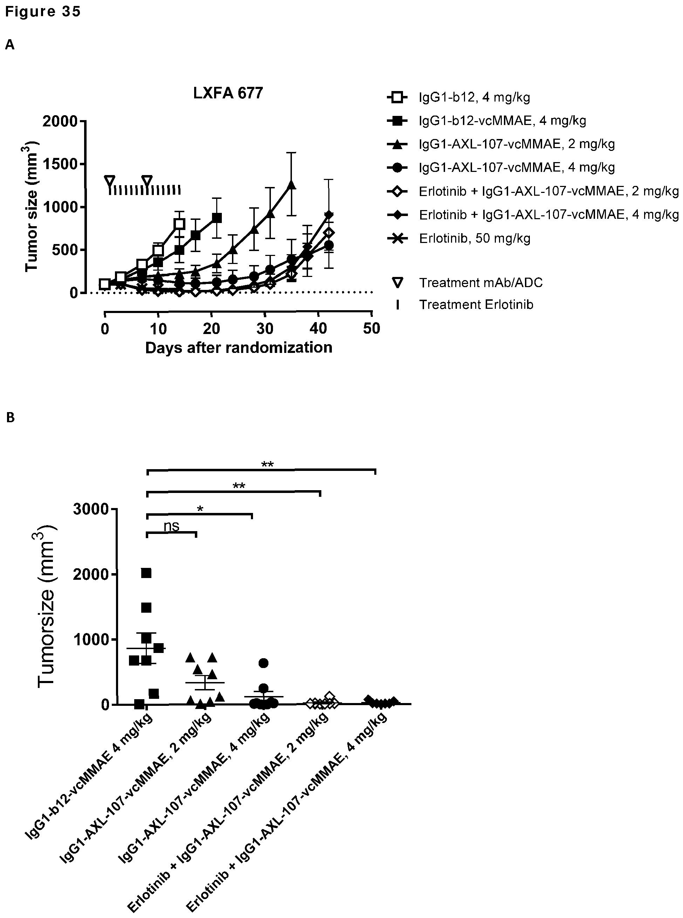

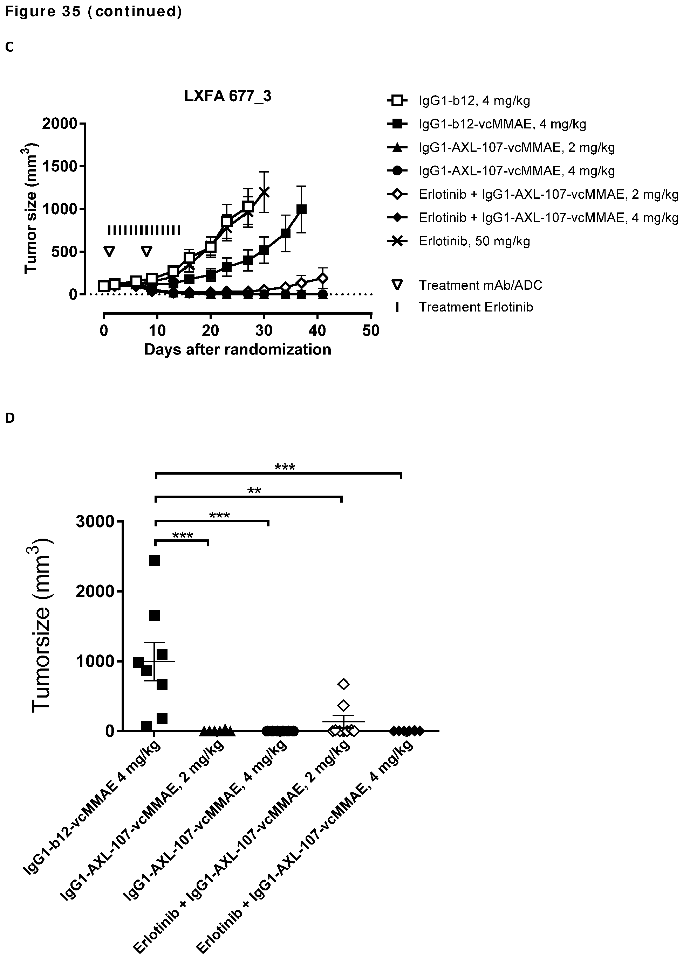

**Figure 35****.** Anti-tumor efficacy of IgG1-AXL-107-vcMMAE in the NSCLC patient-derived xenograft (PDX) model LXFA 677 (A) and LXFA 677_3 (C), which has acquired resistance to erlotinib. Average tumor size after therapeutic treatment with IgG1-AXL-107-vcMMAE, erlotinib, or erlotinib in combination with IgG1-AXL-107-vcMMAE is shown. (B, D) Mean tumor size and SEM of each group per time point and tumor size per individual mouse per group on day 21 of the LXFA 677 model (B) or on day 37 of the LXFA 677_3 model (D). *, p<0.05; **, p<0.01; ns, not significant (Mann-Whitney test).

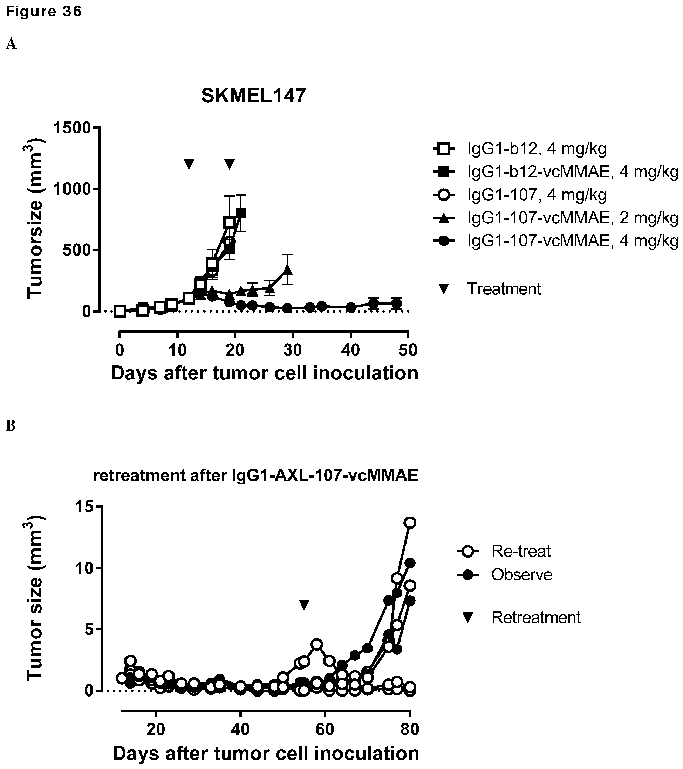

**Figure 36****.** Anti-tumor efficacy of IgG1-AXL-107-vcMMAE in the melanoma model SKMEL147. Average tumor size after therapeutic treatment with IgG1-b12, IgG1-b12-vcMMAE, IgG1-AXL-107, or IgG1-AXL-107-vcMMAE is shown (A). Tumor size in IgG1-AXL-107-vcMMAE mice that were observed (n=2) or retreated with IgG1-AXL-107-vcMMAE (n=4) is shown in (B).

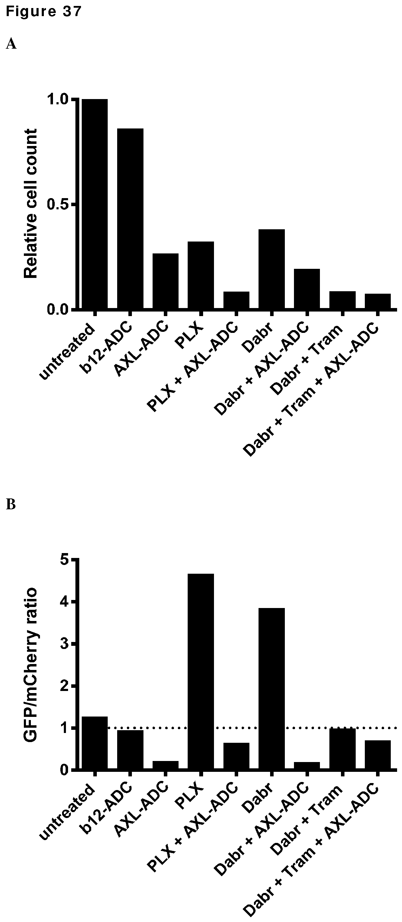

**Figure 37****.** SKMEL28 wild-type cells (red) and PLX4720-resistant SKMEL28-R cells (green) were mixed 1:1 and treated with IgG1-AXL-107-vcMMAE (AXL-ADC), IgG1-b12-MMAE (b12-ADC), PLX4720 (PLX), dabrafenib (dabr), trametinib (tram), or combinations as indicated. (A) Total cell numbers relative to untreated cells. (B) GFP/mCherry ratio corresponding to the ratio SKMEL28-R/SKMEL28 cells.

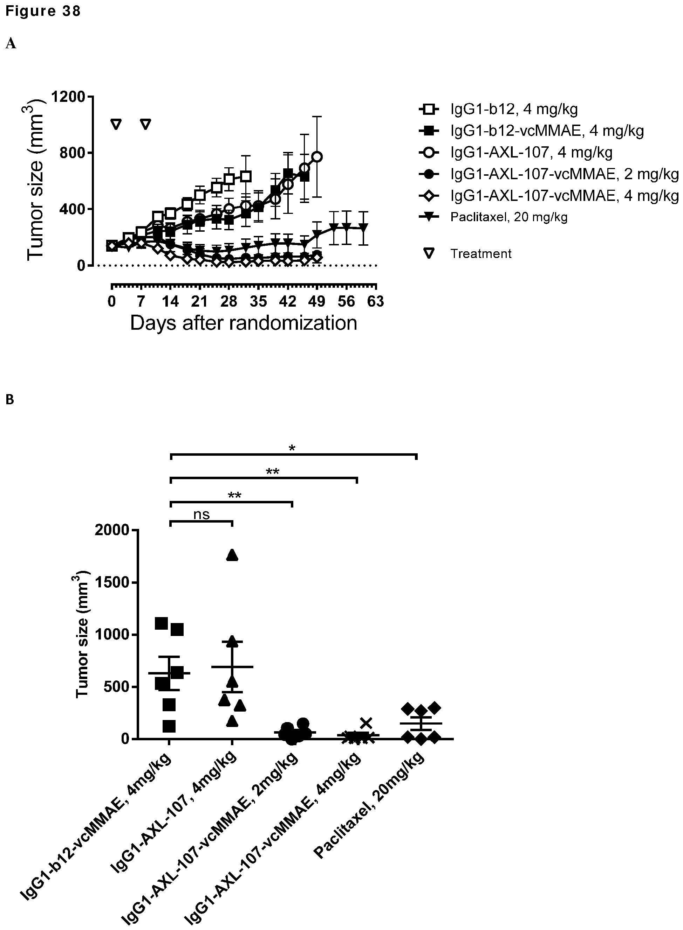

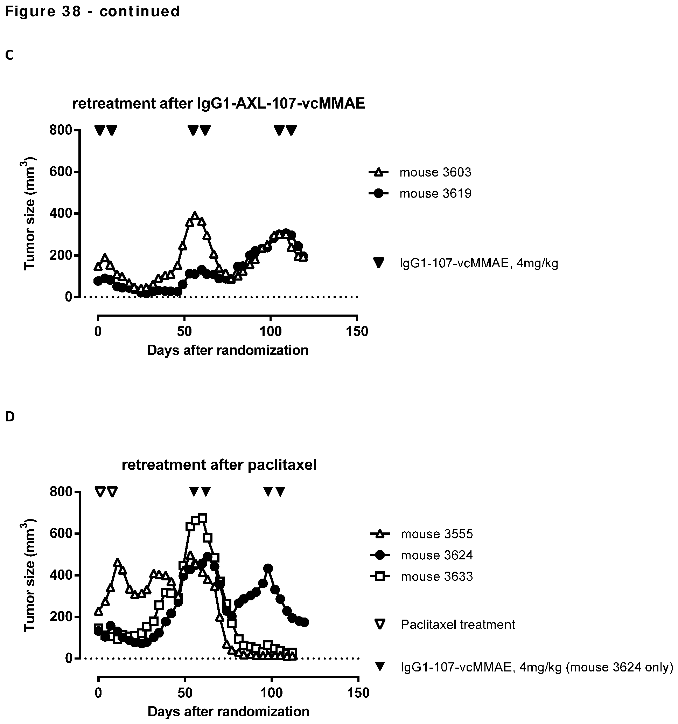

**Figure 38****.** Anti-tumor efficacy of IgG1-AXL-107-vcMMAE in the cervical cancer PDX model CV1664. (A) Average tumor size after therapeutic treatment with IgG1-b12, IgG1-b12-vcMMAE, IgG1-AXL-107, IgG1-AXL-107-vcMMAE, or paclitaxel is shown. (**B**) Mean tumor size and SEM of each group per time point and tumor size per individual mouse per group on day 46 is shown. (**C, D**) Average tumor size in IgG1-AXL-107-vcMMAE - (**C**) or paclitaxel-treated (**D**) mice that were retreated with IgG1-AXL-107-vcMMAE is shown.*, p<0.05; **, p<0.01; ns, not significant (Mann-Whitney test).

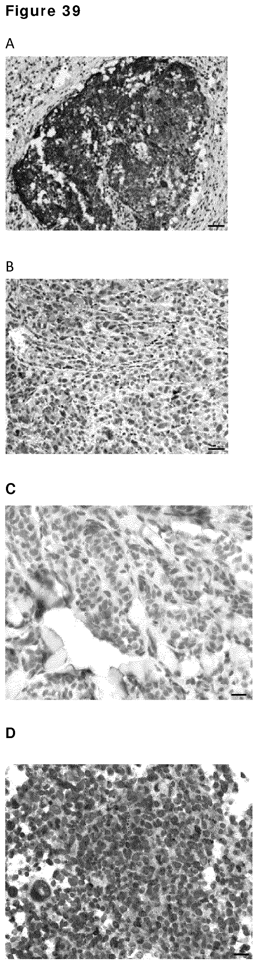

**Figure 39****.** Examples of Axl expression detected by immunohistochemistry in primary melanoma samples. (A) Example of melanoma with positive +++ Axl staining intensity (B) Example of melanoma with positive Axl staining intensity between + and ++ (C) Example of Axl expression in melanoma tissues from the same patient pre- and post- treatment with vemurafenib; left = pre-vemurafenib, Axl staining intensity weakly + ; right = post-vemurafenib, Axl staining intensity weakly + to ++ (D) Example of heterogeneous Axl expression with ++ intensity within primary melanoma tissue.

### DETAILED DISCLOSURE OF THE INVENTION

### Therapeutic applications

The invention relates to AXL-specific ADCs (also referred to as "AXL-ADCs" herein) as defined in any aspect or embodiment herein, for use in treating cancers or tumors which are resistant to certain chemotherapeutics, tyrosine kinase inhibitors (*e.g*., EGFR inhibitors), serine/threonine kinase inhibitors (*e.g*., BRAF inhibitors), as described herein.

The present invention is based, at least in part, on the discovery that AXL-ADCs are effective both *in vitro* and *in vivo* in inducing cytotoxicity in tumor cells resistant to EGFR targeted therapy, BRAF/MEK-targeted therapy or microtubule-targeting agents. For example, NSCLC cells with acquired resistance to the EGFR inhibitors erlotinib, gefitinib and afatinib showed reduced viability upon treatment with AXL-ADC (Example 21), and erlotinib-resistant models with different EGFR gene status showed sensitivity for AXL-ADC (Example 22; Table 17). Notably, in several tumor models where treatment with the EGFR inhibitor erlotinib did not induce anti-tumor activity, treatment with AXL-ADC *or* a combination of AXL-ADC and erlotinib induced potent anti-tumor activity (Example 22). For example, an erlotinib-resistant cell-line derived from an erlotinib-sensitive cell-line was particularly sensitive to AXL-ADC - a stronger anti-tumor activity was obtained at a lower dose (Example 22). In addition, melanoma cell lines resistant to the BRAF-inhibitors PLX4720 (an analog of vemurafenib) or dabrafenib showed enhanced expression of AXL and were sensitive to treatment with AXL-ADC, and AXL-ADC showed strong anti-tumor activity in an *in vivo* melanoma model resistant to PLX4720 (Example 23). Moreover, AXL-ADC induced complete or partial tumor regression in a tumor model of cervical cancer where tumors had progressed after treatment with paclitaxel (Example 24).

So the present disclosure provides an AXL-ADC, e.g., HuMax-AXL-ADC, for use in treating cancer resistant to at least one therapeutic agent selected from the group consisting of a tyrosine kinase inhibitor, a PI3K inhibitor, an antagonistic antibody to a receptor tyrosine kinase, a serine/threonine kinase inhibitor and a chemotherapeutic agent. In one aspect, the therapeutic agent is selected from a tyrosine kinase inhibitor, a serine/threonine kinase inhibitor and a chemotherapeutic agent wherein the ADC comprises least one binding region that comprises a VH region comprising the CDR1, CDR2, and CDR3 sequences of SEQ ID Nos.: 36, 37, and 38, respectively; and a VL region comprising the CDR1, CDR2, and CDR3 sequences of SEQ ID Nos.: 39, GAS, and 40, respectively.In a particular embodiment, the therapeutic agent is selected from a tyrosine kinase inhibitor, a serine/threonine kinase inhibitor and a chemotherapeutic agent.

The present disclosure also provides an AXL-ADC, e.g., HuMax-AXL-ADC, for use in treating a cancer in combination with a therapeutic agent selected from a chemotherapeutic agent, a tyrosine kinase inhibitor, a PI3K inhibitor, an antagonistic antibody to a receptor tyrosine kinase, and a serine/threonine kinase inhibitor, wherein the ADC and therapeutic agent are administered simultaneously, separately or sequentially. The cancer may be resistant to the therapeutic agent and/or may have a high tendency to become resistant to the therapeutic agentThe therapeutic agent may in particular be selected from a tyrosine kinase inhibitor, a serine/threonine kinase inhibitor and a chemotherapeutic agent.

As used herein, a "resistant", "treatment-resistant" or "refractory" cancer, tumor or the like, means a cancer or tumor in a subject, wherein the cancer or tumor did not respond to treatment with a therapeutic agent from the onset of the treatment (herein referred to as "native resistance") or initially responded to treatment with the therapeutic agent but became non-responsive or less responsive to the therapeutic agent after a certain period of treatment (herein referred to as "acquired resistance"), resulting in progressive disease. For solid tumors, also an initial stabilization of disease represents an initial response. Other indicators of resistance include recurrence of a cancer, increase of tumor burden, newly identified metastases or the like, despite treatment with the therapeutic agent. Whether a tumor or cancer is, or has a high tendency of becoming, resistant to a therapeutic agent can be determined by a person of skill in the art. For example, the National Comprehensive Cancer Network (NCCN, www.nccn.org) and European Society for Medical Oncology (ESMO, www.esmo.org/Guidelines) provide guidelines for assessing whether a specific cancer responds to treatment. As described in Table 1 below and elsewhere herein, cancers or tumors developing resistance to certain chemotherapeutics (*e.g*., microtubule-targeting agents ("MTAs") such as taxanes), to tyrosine kinase inhibitors (*e.g*., EGFR inhibitors), to serine/threonine kinase inhibitors (*e.g*., BRAF- or MEK-inhibitors), to PI3K inhibitors and to antagonistic antibodies have been found to express AXL.

As used herein, the term "subject" is typically a human to whom the AXL-ADC is administered, including for instance human patients diagnosed as having a cancer that may be treated by killing of AXL-expressing cells, directly or indirectly.

As used herein, a cancer which "has a high tendency" for resistance to a specific therapeutic agent is a cancer which is known to be associated with a high tendency of being or becoming resistant or refractory to treatment with a certain class of drugs. For example, a cancer patient who is being treated or who has been found to eligible for treatment with a therapeutic agent as described herein for which there is a correlation between resistance and enhanced or *de novo* expression of AXL, suffers from a cancer having a high tendency for resistance. Non-limiting examples of cancers and therapeutic agents known to be associated with enhanced or *de novo* expression of AXL and which are thus may have a high tendency to become resistant to the therapeutic agent, are shown in Table 1 below. Moreover, as shown in Example 24, AXL-ADC induced complete or partial tumor regression in a tumor model of cervical cancer where tumors had progressed after treatment with paclitaxel. Other cancers and tumor types with native or acquired resistance to a therapeutic agent and sensitive to AXL-ADC treatment are also described elsewhere herein.

**Table 1 - Examples of therapeutic agents inducing enhanced or de novo expression of AXL**

| **Tumor type** | **Compound** | **Target/MoA** | **Class** | **Ref** |
|---|---|---|---|---|
| NSCLC | Erlotinib | EGFR | TKI | Zhang (2012), Wilson (2014) |
| NSCLC | Crizotinib | ALK | TKI | Kim (2013) |
| Breast cancer | Lapatinib | HER2, EGFR | TKI | Liu (2009) |
| Breast cancer | Afatinib | EGFR | TKI | Zhang (2012) |
| GIST | Imatinib, sunitinib | ABL/PDGFR/cKIT | TKI | Mahadevan (2015) |
| Renal cancer | Sunitinib | VEGFR/PDGFR/cKIT | TKI | Zhou (2015) |
| Neuroblastoma | Crizotinib | ALK | TKI | Debruyne (2015) |
| AML | midostaurin (PKC412) | FLT3 | TKI | Park (2015) |
| AML | Quizartinib (AC220) | FLT3 | TKI | Park (2015) |
| CML | Imatinib | ABL/PDGFR/cKIT | TKI | Dufies (2011) |
| SCCHN | Alpelisib (BYL719) | PI3K | PI3KI | Elkabets (2015) |
| SCCHN | Cetuximab | EGFR | mAb/rTKI | Brand (2015) |
| Rhabdomyosarcoma | MAB391 | IGF-IR | mAb/rTKI | Huang (2010) |
| Melanoma | Vemurafenib (PLX4032) | BRAF | S/Th KI | Müller (2014) Konieczkowski (2014) |
| | PLX4720;* | BRAF | | |
| | Selumetinib (AZD6244);** | MEK | | |
| | VTX11E*** | ERK2 | | |
| Pancreatic Cancer | Selumetinib (AZD6244) | MEK | S/Th KI | Pettazzoni (2015) |
| Esophageal cancer | Cisplatin | DNA crosslinking | Chemo | Hong (2013) |
| Prostate cancer | Metformin | Diabetic drug, cytostatic | Chemo | Bansal (2015) |
| AML | Doxorubicin, etoposide, cisplatin | Upregulation resistance pumps | Chemo | Hong (2008) |
| SCCHN | Cisplatin/carboplatin | DNA crosslinking | Chemo | Brand (2015) |

| | | | | |
|---|---|---|---|---|
| *N-(3-(5-chloro-1H-pyrrolo[2,3-b]pyridine-3-carbonyl)-2,4-difluorophenyl)propane-1-sulfonamide **6-(4-bromo-2-chloroanilino)-7-fluoro-N-(2-hydroxyethoxy)-3-methylbenzimidazole-5-carboxamide ***4-[2-(2-Chloro-4-fluoroanilino)-5-methylpyrimidin-4-yl]-N-[(1S)-1-(3-chlorophenyl)-2-hydroxyethyl]-1H-pyrrole-2-carboxamide | | | | |

A "tyrosine-kinase inhibitor" or "TKI" as used herein refers to a compound, typically a pharmaceutical, which inhibits tyrosine kinases or down-stream signaling from tyrosine kinases. Tyrosine kinases are enzymes responsible for the addition of a phosphate group to a tyrosine of a protein (phosphorylation), a step that TKIs inhibit, either directly or indirectly. Tyrosine phosphorylation results in the activation of intracellular signal transduction cascades. Many TKIs are useful for cancer therapy. Non-limiting examples of such TKIs and their targets are shown in Table 1 above, and include, *e.g.,* EGFR inhibitors such as erlotinib. In one embodiment, the term tyrosine kinase inhibitor as used herein refer to compounds which specifically inhibit the protein phosphorylation activity of a tyrosine kinase, *e.g.,* the tyrosine kinase activity of the EGFR.

While many TKIs in clinical use are small molecule pharmaceuticals, there are also "receptor tyrosine kinase inhibitors" (rTKIs) such as antagonistic antibodies which bind to the extracellular portion of a receptor tyrosine kinase (herein referred to as "mAb/rTKIs"), thereby inhibiting receptor-mediated signaling. Examples of such antibodies are cetuximab and MAB391.

A "phosphoinositide 3-kinase inhibitor" or "PI3KI" as used herein refers to a compound, typically a pharmaceutical, which inhibits an enzyme in the PI3K/AKT pathway. Examples of PI3KIs include Alpelisib (BYL791).

A "serine/threonine kinase inhibitor" or "S/Th KI", as used herein, refers to a compound, typically a pharmaceutical, which inhibits serine/threonine kinases such as BRAF or MEK or down-stream signaling pathways from such serine/threonine kinases such as via the BRAF/MEK pathways. Serine/threonine kinases are enzymes responsible for the phosphorylation of the hydroxyl-group of a serine or threonine residue, a step that S/Th KIs inhibit, either directly or indirectly. Phosphorylation of serines or threonines results in the activation of intracellular signal transduction cascades. Examples of S/Th KIs useful for cancer therapy, and their targets, are shown in Table 1 above, and include BRAF-inhibitors such as vemurafenib and analogs or derivatives thereof. In one embodiment, the term serine/threonine kinase inhibitor as used herein refer to compounds which specifically inhibit the protein phosphorylation activity of a serine/threonine kinase, e.g., the serine/threonine kinase activity of a mutant BRAF or MEK.

Vemurafenib (PLX4032) is an orally bioavailable, ATP-competitive, small-molecule inhibitor of mutated BRAF kinase, which selectively binds to and inhibits BRAF comprising certain mutations, resulting in an inhibition of an over-activated MAPK signaling pathway downstream in the mutant BRAF kinase-expressing tumor cells. BRAF mutations identified in human cancers are generally located in the glycine-rich P loop of the N lobe and the activation segment and flanking regions within the kinase domain. Vemurafenib binds to and inhibits BRAF kinase having certain of these mutations, such as, but not limited to, an amino acid substitution in residue V600 (*e.g.,* V600E), residue L597 (*e.g.,* L597R; Bahadoran *et al.,* 2013); and residue K601 (Dahlman *et al.,* 2012).

As used herein, a "derivative" of a drug is a compound that is derived or derivable, by a direct chemical reaction, from the drug. As used herein, an "analog" or "structural analog" of a drug is a compound having a similar structure and/or mechanism of action to the drug but differing in at least one structural element. "Therapeutically active" analogs or derivatives of a drug such as, e.g., vemurafenib or erlotinib, have a similar or improved therapeutic efficacy as compared to the drug but may differ in, *e.g.,* one or more of stability, target specificity, solubility, toxicity, and the like.

Tables 2 and 3 below show BRAF and EGFR inhibitors which have a similar mechanism of action (BRAF or EGFR inhibition, respectively) as vemurafenib and erlotinib, respectively.

**Table 2 - BRAF inhibitors**

| **Inhibitor Name** | |
|---|---|
| Vemurafenib (PLX4032) (PLX4720= tool compound) | Bollag (2012) |
| GDC-0879 * | Wong (2009) |
| Dabrafenib (GSK2118436) | Hong (2012) |
| Encorafenib (LGX818) | Li (2016) |
| Sorafenib (BAY 43-9006) | Hilger (2002) |
| RAF265 (CHIR-265) | Mordant (2010) |
| SB590885 ** | King (2006) |
| AZ628 *** | Montagut (2008) |

| | |
|---|---|
| *(E)-5-(1-(2-hydroxyethyl)-3-(pyridin-4-yl)-1H-pyrazol-4-yl)-2,3-dihydroinden-1-one oxime ** (E)-5-(2-(4-(2-(dimethylamino)ethoxy)phenyl)-4-(pyridin-4-yl)-1H-imidazol-5-yl)-2,3-dihydroinden-1-one oxime ***3-(2-cyanopropan-2-yl)-N-(4-methyl-3-(3-methyl-4-oxo-3,4-dihydroquinazolin-6-ylamino)phenyl)benzamide | |

**Table 3 - EGFR inhibitors**

| **Name** | **Class** | |
|---|---|---|
| Erlotinib | TKI | Pollack (1999) |
| Gefitinib | TKI | Sirotnak (2000) |
| Afatinib | TKI | Li (2008) |
| Lapatinib | TKI | Xia (2002) |
| Icotinib | TKI | Tan 2012 |
| Vandetanib | TKI | Herbst (2007) |
| Osimertinib | TKI | Greig (2016) |
| Rociletinib | TKI | Sequist (2015) |
| Cetuximab | mAb/rTKI | Prewett (1996) |
| Panitumumab | mAb/rTKI | Yang (2001) |
| zalutumumab | mAb/rTKI | Bleeker (2004) |
| Nimotuzumab | mAb/rTKI | Talavera (2009) |
| Matuzumab | mAb/rTKI | Kim (2004) |
| necitumumab (IMC-11F8) | mAb/rTKI | Li (2008) |
| sym004 | mAb/rTKI | Pedersen (2010) |
| mab 806 | mAb/rTKI | Mishima (2001) |
| MM-151 | mAb/rTKI | Merrimack |

Accordingly, as shown herein, melanoma resistance to vemurafenib, dabrafenib, trametinib or combinations of any two or more thereof; and NSCLC resistance to erlotinib, gefitinib or afatinib, or combinations of any two or more thereof, may be associated with *de novo* or enhanced expression of AXL by the tumor cells. Thus, such tumors may be eligible for treatment with an AXL-specific ADC.

The present disclosure provides a method of treating a cancer in a subject, wherein the cancer is resistant to at least one therapeutic agent selected from a tyrosine kinase inhibitor, a serine/threonine kinase inhibitor, and a chemotherapeutic agent, the method comprising administering an AXL-ADC. The cancer may for example, have acquired the resistance during a previous or still on-going treatment with the therapeutic agent. Alternatively, the cancer was resistant from the onset of treatment with the therapeutic agent. In one embodiment, the cancer is an AXL-expressing cancerThe therapeutic agent may be a PI3K inhibitor or a mAb/rTKI.

The present disclosure provides a method of treating a cancer in a subject, the method comprising administering an AXL-ADC in combination with at least one therapeutic agent selected from a chemotherapeutic agent, a tyrosine kinase inhibitor or a serine/threonine kinase inhibitor, wherein the ADC and therapeutic agent are administered simultaneously, separately or sequentially. The cancer may have a high tendency for resistance to the therapeutic agent. The cancer may be resistant to the therapeutic agent. The therapeutic agent may be a PI3K inhibitor or a mAb/rTKI.

As shown by the inventors of the present invention and in Table 1 above, in certain types of cancer, the development of resistance has been associated with increased or *de novo* expression of AXL. Such cancers may include, but are not limited to, melanoma, non-small cell lung cancer (NSCLC), cervical cancer, ovarian cancer, squamous cell carcinoma of the head and neck (SCCHN), breast cancer, gastrointestinal stromal tumors (GISTs), renal cancer, neuroblastoma, esophageal cancer, rhabdomyosarcoma, acute myeloid leukaemia (AML), an chronic myeloid leukaemia (CML).

The cancer or tumor may be selected from cervical cancer, melanoma, NSCLC, SCCHN, breast cancer, GIST, renal cancer, neuroblastoma, esophageal cancer and rhabdomyosarcoma. In another embodiment, the cancer is a hematological cancer selected from AML and CML.

The cancer or tumor may be characterized by at least one mutation in the EGFR amino acid sequence selected from L858R and T790M, such as e.g., L858R or T790M/L858R. For example, the cancer or tumor may be an NSCLC.

The at least one therapeutic agent may consist of or may comprise a TKI inhibitor which is an EGFR antagonist, a HER2 antagonist, an ALK-inhibitor, a FLT3 inhibitor, or a combination of two or more thereof. Non-limiting, preferred TKIs include erlotinib, gefitinib, lapatinib, osimertinib, rociletinib, imatinib, sunitinib, afanitib, crizotinib, midostaurin (PKC412) and quizartinib (AC220). In one embodiment, the TKI is an EGFR inhibitor, such as erlotinib or a therapeutically active analog or derivative thereof, *e.g.,* afatinib, lapatinib, osimertinib, rociletinib, or gefitinib.

The tyrosine kinase inhibitor may be erlotinib and the cancer may be an NSCLC, resistant to or having a high tendency for becoming resistant to erlotinib.

The tyrosine kinase inhibitor may be erlotinib and the cancer may be a pancreatic cancer, resistant to or having a high tendency for becoming resistant to erlotinib.

The tyrosine kinase inhibitor may be gefitinib and the cancer may be an NSCLC, resistant to or having a high tendency for becoming resistant to gefitinib.

The tyrosine kinase inhibitor may be crizotinib and the cancer may be a NSCLC, resistant to or having a high tendency for becoming resistant to crizotinib.

The tyrosine kinase inhibitor may be lapatinib and the cancer may be a breast cancer, resistant to or having a high tendency for becoming resistant to lapatinib.

The tyrosine kinase inhibitor may be imatinib and the cancer is a CML, resistant to or having a high tendency for becoming resistant to imatinib.

The tyrosine kinase inhibitor may be imatinib and the cancer may be a GIST, resistant to or having a high tendency for becoming resistant to imatinib.

The tyrosine kinase inhibitor may be sunitinib and the cancer may be a GIST, resistant to or having a high tendency for becoming resistant to sunitinib.

The tyrosine kinase inhibitor may be sunitinib and the cancer may be a renal cancer, resistant to or having a high tendency for becoming resistant to sunitinib.

The tyrosine kinase inhibitor may be crizotinib and the cancer may be a neuroblastoma, resistant to or having a high tendency for becoming resistant to crizotinib.

The tyrosine kinase inhibitor may be midostaurin (PKC412) and the cancer may be AML, resistant to or having a high tendency for becoming resistant to midostaurin.

The tyrosine kinase inhibitor may be quizartinib and the cancer may be an AML resistant to or having a high tendency for becoming resistant to quizartinib.

The tyrosine kinase inhibitor may be afatinib and the cancer may be a breast cancer, resistant to or having a high tendency for becoming resistant to afatinib.

The tyrosine kinase inhibitor may be axitinib and the cancer may be a renal cancer, resistant to or having a high tendency for becoming resistant to axitinib.

The tyrosine kinase inhibitor may be lenvatinib and the cancer is a thyroid cancer, resistant to or having a high tendency for becoming resistant to lenvatinib.

The present disclosure particularly contemplates that the tyrosine kinase inhibitor is an EGFR-inhibiting agent, such as, *e.g.,* erlotinib or a therapeutically active analog or derivative thereof, preferably wherein the cancer is an NSCLC, resistant to or having a high tendency for becoming resistant to the EGFR-inhibiting agent. Specifically, the cancer or tumor (e.g., the NSCLC) may be characterized by at least one mutation in the EGFR selected from L858R and T790M, or a combination thereof.

The at least one therapeutic agent may consist of or comprise a PI3K inhibitor. Non-limiting, preferred PI3K inhibitors include alpelisib and therapeutically active analogs and derivatives thereof.

The PI3Ki may be alpelisib (BYL719) and the cancer may be a SCCHN, resistant to or having a high tendency for becoming resistant to alpelisib.

The at least one therapeutic agent may consist of or comprise an antagonistic antibody which binds to the extracellular portion of a receptor tyrosine kinase. Non-limiting, preferred mAb/rTKIs include cetuximab and anti-IGF-IR MAB391 as well as therapeutically active analogs or derivatives of cetuximab and MAB391.

The mAb/rTKI may be cetuximab and the cancer may be a SCCHN, resistant to or having a high tendency for becoming resistant to cetuximab.

Tthe mAb/rTKI may be anti-IGF-IR antibody MAB391 and the cancer may be an SCCHN, resistant to or having a high tendency for becoming resistant to MAB391.

The at least one therapeutic agent may consists of or comprises a S/Th KI which is a BRAF-inhibitor, MEK-inhibitor or a combination thereof. The S/Th KI may be a BRAF-inhibitor, such as vemurafenib (PLX4032) or a therapeutically effective derivative or analog thereof, e.g., PLX4720 or dabrafenib; or VTXKIIE. The S/Th KI may be a MEK-inhibitor, such as selumetinib (AZD6244) or trametinib.

The S/Th KI may be vemurafenib and the cancer may be a melanoma, resistant to or having a high tendency for becoming resistant to vemurafenib.

The S/Th KI is vemurafenib and the cancer may be a colorectal cancer, resistant to or having a high tendency for becoming resistant to vemurafenib.

The s/Th KI may be dabrafenib and the cancer may be a melanoma, resistant to or having a high tendency for becoming resistant to dabrafenib.

The S/Th KI may be dabrafenib and the cancer may be a colorectal cancer, resistant to or having a high tendency for becoming resistant to dabrafenib.

The S/Th KI may be selumetinib and the cancer may be a pancreatic cancer, resistant to or having a high tendency for becoming resistant to selumetinib.

The S/Th KI may be selumetinib and the cancer may be a melanoma, resistant to or having a high tendency for becoming resistant to selumetinib.

The S/Th KI inhibitor may be trametinib and the tumor may be a melanoma, resistant to or having a high tendency for becoming resistant to trametinib.

The S/Th KI may be VTXKIIE and the cancer may be a melanoma, resistant to or having a high tendency for becoming resistant to VTXKIIE.

The S/Th KI may be PLX4720 and the cancer may be a melanoma, resistant to or having a high tendency for becoming resistant to PLX4720.

The at least one therapeutic agent may consist of or comprise a BRAF inhibitor. In a particular embodiment, the BRAF inhibitor is vemurafenib (PLX4032) or a therapeutically effective analog or derivative thereof, such as dabrafenib or PLX4720. The BRAF inhibitor may be vemurafenib or a therapeutically active derivative or analog thereof, and the tumor may be a melanoma resistant to or having a high tendency for becoming resistant to vemurafenib. Vemurafenib may be an inhibitor of BRAF kinase harboring certain mutations, such as mutations located in the glycine-rich P loop of the N lobe and the activation segment and flanking regions within the kinase domain. The vemurafenib analog may be dabrafenib.

The AXL-ADC provided by the present disclosure may in particular be for use in treating an AXL-expressing melanoma resistant to a therapeutic agent with which the melanoma is being or has been treated, wherein the therapeutic agent is vemurafenib or a therapeutically effective analog or derivative thereof, and wherein the melanoma exhibits a mutation in BRAF. In particular, the melanoma may exhibit a mutation in BRAF which renders the BRAF sensitive for inhibition by vemurafenib or the therapeutically effective analog or derivative. Non-limiting mutations include amino acid substitutions, deletions or insertions; preferably, the mutation is an amino acid substitution. Specific residues for such mutations include, but are not limited to, V600 (*e.g.,* V600E, V600K and V600D), residue L597 (*e.g.,* L597R); and residue K601 (K601E). In one embodiment, the mutation is selected from V600E, V600D, V600K, L597R and K601E.

In one embodiment, the at least one therapeutic agent consists of or comprises a chemotherapeutic agent selected from the group consisting of paclitaxel, docetaxel, cisplatin, doxorubicin, etoposide, carboplatin and metformin or acombination thereof.The therapeutic agent may be a microtubule-targeting agent, such as, e.g., paclitaxel, docetaxel or vincristine, or a therapeutically active analog or derivative of any thereof. The at least one therapeutic agent may be a taxane, such as paclitaxel, docetaxel or a therapeutically active analog or derivative of paclitaxel or docetaxel.

The chemotherapeutic agent may be paclitaxel, and the cancer may be cervical cancer, resistant to or having a high tendency for becoming resistant to paclitaxel.

The chemotherapeutic agent may be paclitaxel, and the cancer may be an NSCLC, resistant to or having a high tendency for becoming resistant to paclitaxel.

The chemotherapeutic agent may be paclitaxel, and the cancer may be an ovarian cancer, resistant to or having a high tendency for becoming resistant to paclitaxel.

The chemotherapeutic may be docetaxel and the cancer may be a head and neck cancer, resistant to or having a high tendency for becoming resistant to docetaxel.

The chemotherapeutic may be docetaxel and the cancer may be a gastric cancer, resistant to or having a high tendency for becoming resistant to docetaxel.

The chemotherapeutic may be docetaxel and the cancer is a breast cancer, resistant to or having a high tendency for becoming resistant to docetaxel.

The chemotherapeutic may be docetaxel and the cancer may be a prostate cancer, resistant to or having a high tendency for becoming resistant to docetaxel.

The chemotherapeutic may be docetaxel and the cancer may be a NSCLC, resistant to or having a high tendency for becoming resistant to docetaxel.

The chemotherapeutic agent may be cisplatin, and the cancer may be an esophageal cancer, resistant to or having a high tendency for becoming resistant to cisplatin.

The chemotherapeutic agent may be cisplatin, and the cancer may be an SCCHN, resistant to or having a high tendency for becoming resistant to cisplatin.

The chemotherapeutic agent may be carboplatin, and the cancer may be an SCCHN, resistant to or having a high tendency for becoming resistant to carboplatin.

The chemotherapeutic agent may be cisplatin, and the cancer may be an AML, resistant to or having a high tendency for becoming resistant to cisplatin.

The chemotherapeutic agent may be doxorubicin, and the cancer may be an AML, resistant to or having a high tendency for becoming resistant to doxorubicin.

The chemotherapeutic agent may be etoposide, and the cancer may be an AML, resistant to or having a high tendency for becoming resistant to etoposide.

The chemotherapeutic agent may be metformin, and the cancer may be a prostate cancer, resistant to or having a high tendency for becoming resistant to metformin.

The chemotherapeutic agent may be cisplatin, and the cancer may be an ovarian cancer, resistant to or having a high tendency for becoming resistant to cisplatin.

The chemotherapeutic agent may be doxorubicin, and the cancer may be a non-small cell lung cancer (NSCLC), resistant to or having a high tendency for becoming resistant to doxorubicin.

The chemotherapeutic agent may be temozolomide, and the tumor may be an astrocytoma, resistant to or having a high tendency for becoming resistant to temozolomide.

The chemotherapeutic agent may be carboplatin, and the tumor may be an astrocytoma, resistant to or having a high tendency for becoming resistant to carboplatin.

The chemotherapeutic agent may be vincristine, and the tumor may be an astrocytoma, resistant to or having a high tendency for becoming resistant to vincristine.

The present disclosure relates to a method of treating a cancer in a subject in need thereof, wherein the cancer is, or has a high tendency for becoming, resistant to a therapeutic agent selected from a chemotherapeutic agent, a tyrosine kinase inhibitor, a PI3K inhibitor, a mAb/rTKI and a serine/threonine kinase inhibitor, comprising administering to the subject a therapeutically effective amount of an ADC comprising an antibody binding to human AXL. In one embodiment, the therapeutic agent is selected from a chemotherapeutic agent, a tyrosine kinase inhibitor and a serine/threonine kinase inhibitor. For example, the chemotherapeutic agent may be a taxane, the tyrosine kinase inhibitor may be an EGFR-inhibitor, and the serine/threonine kinase inhibitor may be a BRAF- or MEK-inhibitor. In one embodiment, the cancer is an AXL-expressing cancer.

The disclosure relates to a method of treating a NSCLC resistant to erlotinib in a subject, the method comprising administering to the subject an ADC comprising an antibody binding to human AXL. In one embodiment, the method further comprises administering erlotinib, or an analog or derivative thereof, to the subject. The cancer may be an AXL-expressing cancer.

The disclosure relates to a method of treating a melanoma resistant to vemurafenib in a subject, wherein the melanoma exhibits a mutation in BRAF and the mutation providing for vemurafenib inhibition of BRAF kinase activity of the mutant BRAF, the method comprising administering to the subject an ADC comprising an antibody binding to human AXL. The mutationmay be an amino acid substitution in residue V600, L597 and/or K601. The mutation may be selected from V600E, V600D, V600K, L597R and K601E. The method may further comprise administering vemurafenib, or an analog or derivative thereof, to the subject. The analog may be dabrafenib. The cancer may be an AXL-expressing cancer.

The present disclosure relates to a method of treating a cervical cancer resistant to paclitaxel in a subject, the method comprising administering to the subject an ADC comprising an antibody binding to human AXL. In one embodiment, the method further comprises administering paclitaxel, or an analog or derivative thereof, to the subject. The cancer may be an AXL-expressing cancer.

As for the AXL-ADC, a physician having ordinary skill in the art may readily determine and prescribe the effective amount of the pharmaceutical composition required. In relation hereto, when referring to a pharmaceutical composition it is to be understood also to comprise a composition as such, or vice versa. For example, the physician could start doses of the AXL-ADC employed in the pharmaceutical composition at levels lower than that required in order to achieve the desired therapeutic effect and gradually increase the dosage until the desired effect is achieved. In general, a suitable dose will be that amount of the compound which is the lowest dose effective to produce a therapeutic effect according to a particular dosage regimen. Such an effective dose will generally depend upon the factors described above.

For example, an "effective amount" for therapeutic use may be measured by its ability to stabilize the progression of disease. The ability of a compound to inhibit cancer may, for example, be evaluated in an animal model system predictive of efficacy in human tumors. Alternatively, this property of a composition may be evaluated by examining the ability of the compound to inhibit cell growth or to induce cytotoxicity by *in vitro* assays known to the skilled practitioner. A therapeutically effective amount of a therapeutic compound may decrease tumor size, or otherwise ameliorate symptoms in a subject. One of ordinary skill in the art would be able to determine such amounts based on such factors as the subject's size, the severity of the subject's symptoms, and the particular composition or route of administration selected. For example, as already indicated, the National Comprehensive Cancer Network (NCCN, www.nccn.org) and European Society for Medical Oncology (ESMO, www.esmo.org/Guidelines) guidelines for assessing cancer treatments can be used.

An exemplary, non-limiting range for a therapeutically effective amount of an AXL-ADC of the invention is 0.02-100 mg/kg, such as about 0.02-30 mg/kg, such as about 0.05-10 mg/kg, 0.1-5 mg/kg or 0.1-3 mg/kg, for example about 0.5-3 mg/kg or 0.5-2 mg/kg.

Administration may e.g. be intravenous, intramuscular, intraperitoneal, or subcutaneous, and for instance administered proximal to the site of the target.

Dosage regimens in the above methods of treatment and uses are adjusted to provide the optimum desired response (*e.g*., a therapeutic response). For example, a single bolus may be administered, several divided doses may be administered over time or the dose may be proportionally reduced or increased as indicated by the exigencies of the therapeutic situation.

In one embodiment, the efficacy-safety window is optimized by lowering specific toxicity such as for example by lowering the drug-antibody ratio (DAR) and/or mixing of AXL-ADC with unlabeled anti-AXL antibody.

The efficacy of the treatment may be monitored during the therapy, e.g. at predefined points in time. Methods for measuring efficacy generally depend on the particular type of cancer and are well known to a person skilled in the art.The efficacy may be monitored, by visualization of the disease area, or by other diagnostic methods described further herein, e.g. by performing one or more PET-CT scans, for example using a labeled anti-AXL antibody, fragment or mini-antibody derived from an AXL-specific antibody.

If desired, an effective daily dose of a an AXL-ADC may be two, three, four, five, six or more sub-doses administered separately at appropriate intervals throughout the day, optionally, in unit dosage forms. The AXL-ADCs may be administered by slow continuous infusion over a long period, such as more than 24 hours, in order to minimize any unwanted side effects.

An effective dose of an AXL-ADC may also be administered using a weekly, biweekly or triweekly dosing period. The dosing period may be restricted to, *e.g.,* 8 weeks, 12 weeks or until clinical progression has been established. In one embodiment, an AXL-ADC is administered either once every 3 weeks (1Q3W) or three administrations over 4 weeks (3Q4W) so that the patient receives sixteen or twelve cycles of AXL-ADC at three week or four-week intervals for, e.g., 48 weeks, extending or repeating the regimen as needed.

For example, in one embodiment, the AXL-ADC may be administered by infusion in a weekly dosage of between 10 and 500 mg/m², such as between 200 and 400 mg/m². Such administration may be repeated, e.g., 1 to 8 times, such as 3 to 5 times. The administration may be performed by continuous infusion over a period of from 1 to 24 hours, such as from 1 to 12 hours.

The AXL-ADC may be administered by infusion every three weeks in a dosage of between 10 and 500 mg/m², such as between 50-200 mg/m². Such administration may be repeated, *e.g.,* 1 to 8 times, such as 3 to 5 times. The administration may be performed by continuous infusion over a period of from 1 to 24 hours, such as from 1 to 12 hours.

An AXL-ADC may be administered as a single dose of about 0.1-10 mg/kg, such as about 1-3 mg/kg, every week or every third week for up to twelve times, up to eight times, or until clinical progression. The administration may be performed by continuous infusion over a period of from 1 to 24 hours, such as from 1 to 12 hours. Such regimens may be repeated one or more times as necessary, for example, after 6 months or 12 months. The dosage may be determined or adjusted by measuring the amount of compound of the present invention in the blood upon administration by for instance taking out a biological sample and using anti-idiotypic antibodies which target the antigen binding region of the anti-AXL antibodies.

The AXL-ADCs may be administered as maintenance therapy, such as, *e.g.,* once a week for a period of six months or more. As used herein, "maintenance therapy" means therapy for the purpose of avoiding or delaying the cancer's progression or return. Typically, if a cancer is in complete remission after the initial treatment, maintenance therapy can be used to avoid to delay return of the cancer. If the cancer is advanced and complete remission has not been achieved after the initial treatment, maintenance therapy can be used to slow the growth of the cancer, e.g., to lengthen the life of the patient.

As non-limiting examples, treatment according to the present disclosure may be provided as a daily dosage of a compound of the present invention in an amount of about 0.1-100 mg/kg, such as about 0.1-50 mg/kg, such as about 0.2, 0.5, 0.9, 1.0, 1.1, 1.5, 2, 3, 4, 5, 6, 7, 8, 9, 10, 11, 12, 13, 14, 15, 16, 17, 18, 19, 20, 21, 22, 23, 24, 25, 26, 27, 28, 29, 30, 40, 45, 50, 60, 70, 80, 90 or 100 mg/kg, per day, on at least one of days 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, or 40, or alternatively, at least one of weeks 1, 2, 3, 4, 5, 6, 7, 8, 9, 10, 11, 12, 13, 14, 15, 16, 17, 18, 19 or 20 after initiation of treatment, or any combination thereof, using single or divided doses every 24, 12, 8, 6, 4, or 2 hours, or any combination thereof.

Parenteral compositions may be formulated in dosage unit form for ease of administration and uniformity of dosage. Dosage unit form as used herein refers to physically discrete units suited as unitary dosages for the subjects to be treated; each unit contains a predetermined quantity of active compound calculated to produce the desired therapeutic effect in association with the required pharmaceutical carrier. The specification for the dosage unit forms of the present invention are dictated by and directly dependent on (a) the unique characteristics of the active compound and the particular therapeutic effect to be achieved, and (b) the limitations inherent in the art of compounding such an active compound for the treatment of sensitivity in individuals.

As described herein, the AXL-ADC can be used in combination with at least one additional therapeutic agent. The at least one additional therapeutic agent may comprise, or consist of, the chemotherapeutic agent, tyrosine kinase inhibitor, PI3K inhibitor, mAb/rTKI and/or serine/threonine kinase inhibitor to which the cancer or tumor is resistant or have a high tendency for developing resistance to, as set forth in the preceding embodiments.

The AXL-ADC and the one or more therapeutic agents can be administered simultaneously, separately or sequentially. For example, in one embodiment, the combination is used for treating a cancer patient which has not received prior treatment with the at least one therapeutic agent. In another embodiment, the combination is used for treating a cancer patient which has failed prior treatment with the at least one therapeutic agent. Efficient dosages and dosage regimens for the AXL-ADC and therapeutic agent(s) depend on the neoplasm, tumor or cancer to be treated and may be determined by the persons skilled in the art.

The dosages and dosage regimens for the one or more therapeutic agents to be used in conjunction with the AXL-ADC may be the same or essentially similar to those normally used in the treatment of such neoplasm, tumor or cancer with the one or more therapeutic agents. The dosages of the therapeutic agent(s) may be lower than those normally used, but the dosage regimen may otherwise be similar. The dosages of the therapeutic agent(s) may be similar to those normally used, but the dosage regimen adjusted to fewer or less frequent administrations.

So, in one aspect, the invention relates to a method of treating a cancer in a subject in need thereof, wherein the cancer is, or has a high tendency for becoming, resistant to a therapeutic agent selected from a chemotherapeutic agent, a tyrosine kinase inhibitor and a serine/threonine kinase inhibitor, comprising administering to the subject (i) an ADC comprising an antibody binding to human AXL and (ii) the therapeutic agent. In one embodiment, the chemotherapeutic agent is a taxane, the tyrosine kinase inhibitor is an EGFR-inhibitor, and the serine/threonine kinase inhibitor a BRAF- or MEK-inhibitor. In one embodiment, the cancer is an AXL-expressing cancer. The AXL-ADC may, e.g., be administered in a therapeutically effective amount according to a dosage regimen described in more detail above. For example, as a non-limiting example, the AXL-ADC may be administered in an amount of about 0.02-100 mg/kg, such as about 0.02-30 mg/kg, such as about 0.05-10 mg/kg either every 1 week (1Q1W), every 2 weeks (1Q2W) or every 3 weeks (1Q3W) or three administrations over 4 weeks (3Q4W) so that the patient receives sixteen or twelve cycles of AXL-ADC at three week or four-week intervals for, e.g., 48 weeks, extending, shortening or repeating the regimen as determined by the physician responsible.

In one embodiment, the invention relates to a method of treating a NSCLC resistant to erlotinib in a subject, the method comprising administering to the subject (i) an ADC comprising an antibody binding to human AXL and (ii) erlotinib, or a therapeutically effective analog or derivative thereof. The erlotinib may, for example, be administered orally at a dose of 50 to 300 mg, such as 100-200 mg, such as about 150 mg, once or twice daily, or every 2 or 3 days. Preferably, the erlotinib is administered once daily at a dose of about 150 mg. In one embodiment, the cancer is an AXL-expressing cancer.

In one embodiment, the invention relates to a method of treating a melanoma resistant to vemurafenib in a subject, wherein the melanoma exhibits a mutation in BRAF and the mutation providing for vemurafenib inhibition of BRAF kinase activity of the mutant BRAF, the method comprising administering to the subject (i) an ADC comprising an antibody binding to human AXL and (ii) vemurafenib, or a therapeutically effective analog or derivative thereof. In one embodiment, the cancer is an AXL-expressing cancer. In one embodiment, the mutation is an amino acid substitution in residue V600, L597 and/or K601. In one embodiment, the mutation is selected from V600E, V600D, V600K, L597R and K601E. The vemurafenib may, for example, be administered orally at a dose of about 200-2000 mg, 500-1500 mg, such as about 1000 mg per day, e.g., 960 mg, administered as 4 x 240 mg tablets q12hr (approximately 12 hr apart).

The present disclosure provides a method of treating a melanoma resistant to dabrafenib in a subject, wherein the melanoma exhibits a mutation in BRAF and the mutation providing for dabrafenib inhibition of BRAF kinase activity of the mutant BRAF, the method comprising administering to the subject (i) an ADC comprising an antibody binding to human AXL and (ii) dabrafenib, or a therapeutically effective analog or derivative thereof. In one embodiment, the cancer is an AXL-expressing cancer. The mutation may be an amino acid substitution in residue V600, L597 and/or K601. The mutation may be selected from V600E, V600D, V600K, L597R and K601E. The dabrafenib may, for example, be administered orally to the subject at a dose of about 50-300 mg, such as about 100-200 mg, such as about 150 mg, once or twice daily or every 2 or 3 days. Preferably, the dabrafenib is administered as 150 mg orally twice daily, e.g., at least 1hr before a meal or at least 2 hrs after a meal.

The present disclosure provides a method of treating a melanoma resistant to dabrafenib, trametinib or both in a subject, wherein the melanoma exhibits a mutation in BRAF and the mutation providing for dabrafenib inhibition of BRAF kinase activity of the mutant BRAF, the method comprising administering to the subject (i) an ADC comprising an antibody binding to human AXL, (ii) dabrafenib, or a therapeutically effective analog or derivative thereof and (iii) trametinib or a therapeutically effective analog or derivative thereof. The cancer may be an AXL-expressing cancer. The mutation may be an amino acid substitution in residue V600, L597 and/or K601. The mutation may be selected from V600E, V600D, V600K, L597R and K601E. The dabrafenib may, for example, be administered orally to the subject at a dose of about 50-300 mg, such as about 100-200 mg, such as about 150 mg, once or twice daily or every 2 or 3 days. Preferably, the dabrafenib is administered as 150 mg orally twice daily, e.g., at least 1hr before a meal or at least 2 hrs after a meal. The tramatenib may, for example, be administered orally at a dose of about 0.5 to 5 mg, such as about 1 to 4 mg, such as about 2-3 mg, such as about 2 mg, once or twice daily or every 2, 3 or 4 days, such as once daily.

The present disclosure provides a method of treating a cervical cancer resistant to a taxane in a subject, the method comprising administering to the subject (i) an ADC comprising an antibody binding to human AXL and (ii) a taxane to the subject. In one embodiment, the cancer is an AXL-expressing cancer. Preferably, the taxane is paclitaxel or a therapeutically effective analog or derivative thereof, such as docetaxel. The paclitaxel may be administered intravenously (iv) to the subject, for example at a dose of about 100-500 mg/m2, such as about 125-400 mg/m2, such as about 135 mg/m2, 175 mg/m2 or 250mg/m2 over a few hours (e.g., 3 hrs), and the treatment repeated every 1, 2, 3, 4, 5 weeks, such as every 3 weeks. Alternatively, the paclitaxel may be administered intravenously as albumin-bound paclitaxel (nab-paclitaxel), e.g., at a dose of about 50-400 mg/m2, such as about 75-300 mg/m2, such as about 100-200 mg/m2, such as about 125 mg/m2 over a period over 30 min to 1 hr or more and the once per week, and repeating the treatment twice per week, or once every 2 or 3 weeks, e.g., once per week. Docetaxel may, in turn, be administered iv at a dose of about 25-500 mg/m2, such as about 50-300 mg/m2, such as about 75-200 mg/m2, such as about 100 mg/m2 over 30 minutes to 2 hrs, such as 1 hr, and the treatment repeated every 1, 2, 3, 4 or 5 weeks, such as every 3 weeks.

In a particular, the AXL-ADC may be used, alone or in combination with the therapeutic agent, to treat recurrent cancer in a subject, where the cancer recurred after an initial treatment with the therapeutic agent. Should the cancer recur yet again after the initial treatment with AXL-ADC, the AXL-ADC can be used again, alone or together with the therapeutic agent, to treat the recurrent cancer.

The present disclosure provides a method of selecting a subject suffering from a cancer for treatment with a combination of an AXL-ADC and a therapeutic agent selected from a chemotherapeutic agent, a TKI, a PI3Ki, a mAb/rTKI and a S/Th KI, comprising determining
(a) whether the subject meets the criteria for treatment with a chemotherapeutic agent, TKI, PI3Ki, mAb/rTKI or S/Th KI;
(b) whether AXL expression in the cancer is associated with resistance to the TKI or S/Th KI; and
(c) selecting a subject meeting the criteria for treatment with the TKI or S/Th KI and suffering from a cancer for which AXL expression is associated with resistance to the TKI or S/Th KI. In one embodiment, the therapeutic agent is a chemotherapeutic agent, a TKI or S/Th KI.

The present disclosure provides a method of treating a subject diagnosed with having a melanoma which is, or has a high tendency for becoming, resistant to vemurafenib or a therapeutically effective analog or derivative thereof, comprising administering a therapeutically effective amount of an ADC comprising an antibody binding to human AXL.

The present disclosure provides a method of determining if a subject suffering from melanoma is suitable for treatment with a combination of (i) vemurafenib or a therapeutically effective analog or derivative thereof and (ii) an ADC comprising an antibody which binds to human AXL, wherein the subject is undergoing or has undergone treatment with vemurafenib (or the analog or derivative), and is determined or suspected to be resistant to the vemurafenib (or the analog or derivative), thus determining that the subject is suitable for the treatment. It may be determined if the melanoma expresses AXL. In one embodiment, the analog is dabrafenib.

The present disclosure provides a method of treating a subject diagnosed with a cervical cancer which is, or has a high tendency for becoming, resistant to paclitaxel or a therapeutically effective analog or derivative thereof, such another taxane (e.g., docetaxel), comprising administering a therapeutically effective amount of an ADC comprising an antibody binding to human AXL.

The present disclosure provides a method of determining if a subject suffering from cervical cancer is suitable for treatment with a combination of (i) paclitaxel or a therapeutically effective analog or derivative thereof, such as another taxane (e.g., docetaxel) and (ii) an ADC comprising an antibody which binds to human AXL, wherein the subject is undergoing or has undergone treatment with paclitaxel and is determined or suspected to be resistant to the paclitaxel, thus determining that the subject is suitable for the treatment. In a further aspect it may be determined if the cervical cancer expresses AXL.

The resistant neoplasm, tumor or cancer to be treated with an anti-AXL-ADC may have been determined to express AXL.

This may be achieved by detecting levels of AXL antigen or levels of cells which express AXL on their cell surface in a sample taken from a patient. The patient may, for example, suffer from a cervical cancer, melanoma or NSCLC. The AXL antigen to be detected can be soluble AXL antigen, cell-associated AXL antigen, or both. The sample to be tested can, for example, be contacted with an anti-AXL antibody under conditions that allow for binding of the antibody to AXL, optionally along with a control sample and/or control antibody binding to an irrelevant antigen. Binding of the antibody to AXL can then be detected (*e.g*., using an ELISA). When using a control sample along with the test sample, the level of anti-AXL antibody or anti-AXL antibody AXL complex is analyzed in both samples and a statistically significant higher level of anti-AXL antibody or anti-AXL antibody-AXL complex in the test sample shows a higher level of AXL in the test sample compared with the control sample, indicating a higher expression of AXL. Examples of conventional immunoassays useful for such purposes include, without limitation, ELISA, RIA, FACS assays, plasmon resonance assays, chromatographic assays, tissue immunohistochemistry, Western blot, and/or immunoprecipitation.

A tissue sample may be taken from a tissue known or suspected of containing AXL antigen and/or cells expressing AXL. For example, in situ detection of AXL expression may be accomplished by removing a histological specimen such as a tumor biopsy or blood sample from a patient, and providing the anti-AXL antibody to such a specimen after suitable preparation of the specimen. The antibody may be provided by applying or by overlaying the antibody to the specimen, which is then detected using suitable means.

In the above assays, the anti-AXL antibody can be labeled with a detectable substance to allow AXL-bound antibody to be detected.

The level of AXL expressed on cells in a sample can also be determined according to the method described in Example 23, where AXL expression on the plasma membrane of human tumor cell lines was quantified by indirect immunofluorescence using QIFIKIT analysis (DAKO, Cat nr K0078), using a monoclonal anti-AXL antibody (here: mouse monoclonal antibody ab89224; Abcam, Cambridge, UK). Briefly, a single-cell suspension is prepared, and optionally washed. The next steps are performed on ice. The cells are seeded, e.g., at 100,000 cells per well or tube, and thereafter pelleted and resuspended in 50 µL antibody sample at a concentration of 10 µg/mL, optionally adding a control antibody to a parallel sample. After an incubation of 30 minutes at 4ºC, cells are pelleted and resuspended in 150 µL FACS buffer, and the amount of AXL determined by FACS analysis using, e.g., a secondary, FITC-labelled antibody binding to the anti-AXL and control antibodies. For each cell line, the antibody binding capacity (ABC), an estimate for the number of AXL molecules expressed on the plasma membrane, was calculated using the mean fluorescence intensity of the AXL antibody-stained cells, based on the equation of a calibration curve as described in Example 23 (interpolation of unknowns from the standard curve). Using the method of Example 23, the level of AXL on AXL-expressing cells may be estimated to at least 5000, such as at least 8000, such as at least 13000.

The presence or level of AXL-expressing cells in a neoplasm, tumor or cancer may be assessed by in vivo imaging of detectably labelled anti-AXL antibodies according to methods known in the art. A significantly higher signal from a site, such as the known or suspected site of a tumor, than background or other control indicates overexpression of AXL in the tumor or cancer.

### AXL-ADCs

ADCs suitable for use in the context of the present invention can be prepared from any anti-AXL antibody. Preferred anti-AXL antibodies are characterized by one or more of the AXL-binding properties, variable or hypervariable sequences, or a combination of binding and sequence properties, set out in the aspects and embodiments below. In particular, the antibody may be one that binds to AXL but does not compete for AXL binding with the ligand Growth Arrest-Specific 6 (Gas6). Most preferred are the specific anti-AXL antibodies whose sequences are described in Table 4, in particular the antibody designated 107 and antibodies sharing one or more AXL-binding properties or CDR, VH and/or VL sequences with antibody 107.

So, in one particular embodiment of any preceding aspect or embodiment, the anti-AXL antibody comprises at least one binding region comprising a VH region and a VL region, wherein the VH region comprises the CDR1, CDR2 and CDR3 sequences of SEQ ID Nos.: 36, 37 and 38, and the VL region comprises the CDR1, CDR2 and CDR3 sequences of SEQ ID Nos.: 39, GAS, and 40.

The ADC may comprise such an anti-AXL antibody linked to a cytotoxic agent which is an auristatin or a functional peptide analog or derivate thereof, such as, e.g., monomethyl auristatin E, preferably via a maleimidocaproyl-valine-citrulline-p-aminobenzyloxy-carbonyl (mc-vc-PAB) linker.

The term "antibody" as used herein is intended to refer to an immunoglobulin molecule, a fragment of an immunoglobulin molecule, or a derivative of either thereof, which has the ability to specifically bind to an antigen under typical physiological and/or tumor-specific conditions with a half-life of significant periods of time, such as at least about 30 minutes, at least about 45 minutes, at least about one hour, at least about two hours, at least about four hours, at least about 8 hours, at least about 12 hours, about 24 hours or more, about 48 hours or more, about 3, 4, 5, 6, 7 or more days, etc., or any other relevant functionally-defined period (such as a time sufficient to induce, promote, enhance, and/or modulate a physiological response associated with antibody binding to the antigen and/or time sufficient for the antibody to be internalized). The binding region (or binding domain which may be used herein, both having the same meaning) which interacts with an antigen, comprises variable regions of both the heavy and light chains of the immunoglobulin molecule. The constant regions of the antibodies (Abs) may mediate the binding of the immunoglobulin to host tissues or factors, including various cells of the immune system (such as effector cells) and components of the complement system such as C1q, the first component in the classical pathway of complement activation. As indicated above, the term antibody as used herein, unless otherwise stated or clearly contradicted by context, includes fragments of an antibody that retain the ability to specifically interact, such as bind, to the antigen. It has been shown that the antigen-binding function of an antibody may be performed by fragments of a full-length antibody. Examples of binding fragments encompassed within the term "antibody" include (i) a Fab' or Fab fragment, a monovalent fragment consisting of the VL, VH, CL and CH1 domains, or a monovalent antibody as described in WO 2007/059782; (ii) F(ab')₂ fragments, bivalent fragments comprising two Fab fragments linked by a disulfide bridge at the hinge region; (iii) an Fd fragment consisting essentially of the VH and CH1 domains; (iv) an Fv fragment consisting essentially of the VL and VH domains of a single arm of an antibody, (v) a dAb fragment (Ward *et al.,* 1989), which consists essentially of a VH domain and is also called domain antibody (Holt *et al.,* 2003); (vi) camelid or nanobodies (Revets *et al.,* 2005) and (vii) an isolated complementarity determining region (CDR). Furthermore, although the two domains of the Fv fragment, VL and VH, are coded for by separate genes, they may be joined, using recombinant methods, by a synthetic linker that enables them to be made as a single protein chain in which the VL and VH regions pair to form monovalent molecules (known as single chain antibodies or single chain Fv (scFv), see for instance Bird *et al.* (1988) and Huston *et al.* (1988). Such single chain antibodies are encompassed within the term antibody unless otherwise noted or clearly indicated by context. Although such fragments are generally included within the meaning of antibody, they collectively and each independently are unique features of the present invention, exhibiting different biological properties and utility. These and other useful antibody fragments in the context of the present invention are discussed further herein. It also should be understood that the term antibody, unless specified otherwise, also includes polyclonal antibodies, monoclonal antibodies (mAbs), antibody-like polypeptides, such as chimeric antibodies and humanized antibodies, as well as 'antibody fragments' or 'fragments thereof' retaining the ability to specifically bind to the antigen (antigen-binding fragments) provided by any known technique, such as enzymatic cleavage, peptide synthesis, and recombinant techniques, and retaining the ability to be conjugated to a toxin. An antibody as generated can possess any isotype.

The term "immunoglobulin heavy chain" or "heavy chain of an immunoglobulin" as used herein is intended to refer to one of the heavy chains of an immunoglobulin. A heavy chain is typically comprised of a heavy chain variable region (abbreviated herein as VH) and a heavy chain constant region (abbreviated herein as CH) which defines the isotype of the immunoglobulin. The heavy chain constant region typically is comprised of three domains, CH1, CH2, and CH3. The term "immunoglobulin" as used herein is intended to refer to a class of structurally related glycoproteins consisting of two pairs of polypeptide chains, one pair of light (L) low molecular weight chains and one pair of heavy (H) chains, all four potentially inter-connected by disulfide bonds. The structure of immunoglobulins has been well characterized (see for instance Fundamental Immunology Ch. 7 (Paul, W., ed., 2nd ed. Raven Press, N.Y. (1989). Within the structure of the immunoglobulin, the two heavy chains are inter-connected via disulfide bonds in the so-called "hinge region". Equally to the heavy chains each light chain is typically comprised of several regions; a light chain variable region (abbreviated herein as VL) and a light chain constant region. The light chain constant region typically is comprised of one domain, CL. Furthermore, the VH and VL regions may be further subdivided into regions of hypervariability (or hypervariable regions which may be hypervariable in sequence and/or form of structurally defined loops), also termed complementarity determining regions (CDRs), interspersed with regions that are more conserved, termed framework regions (FRs). Each VH and VL is typically composed of three CDRs and four FRs, arranged from amino-terminus to carboxy-terminus in the following order: FR1, CDR1, FR2, CDR2, FR3, CDR3, FR4. CDR sequences are defined according to IMGT (see Lefranc *et al.* (1999) and Brochet *et al.* (2008)).

The term "antigen-binding region" or "binding region" as used herein, refers to a region of an antibody which is capable of binding to the antigen. The antigen can be any molecule, such as a polypeptide, *e.g.* present on a cell, bacterium, or virion. The terms "antigen" and "target" may, unless contradicted by the context, be used interchangeably in the context of the present invention.

The term "binding" as used herein refers to the binding of an antibody to a predetermined antigen or target, typically with a binding affinity corresponding to a K_{D} of about 10⁻⁶ M or less, *e.g.* 10⁻⁷ M or less, such as about 10⁻⁸ M or less, such as about 10⁻⁹ M or less, about 10⁻¹⁰ M or less, or about 10⁻¹¹ M or even less when determined by for instance surface plasmon resonance (SPR) technology in a BIAcore 3000 instrument using the antigen as the ligand and the protein as the analyte, and binds to the predetermined antigen with an affinity corresponding to a K_{D} that is at least ten-fold lower, such as at least 100 fold lower, for instance at least 1,000 fold lower, such as at least 10,000 fold lower, for instance at least 100,000 fold lower than its affinity for binding to a non-specific antigen (*e.g.*, BSA, casein) other than the predetermined antigen or a closely-related antigen. The amount with which the affinity is lower is dependent on the K_{D} of the protein, so that when the K_{D} of the protein is very low (that is, the protein is highly specific), then the amount with which the affinity for the antigen is lower than the affinity for a non-specific antigen may be at least 10,000 fold. The term "K_{D}" (M), as used herein, refers to the dissociation equilibrium constant of a particular antibody-antigen interaction, and is obtained by dividing k_{d} by kₐ.

The term "k_{d}" (sec⁻¹), as used herein, refers to the dissociation rate constant of a particular antibody-antigen interaction. Said value is also referred to as the k_{off} value or off-rate.

The term "kₐ" (M⁻¹ x sec⁻¹), as used herein, refers to the association rate constant of a particular antibody-antigen interaction. Said value is also referred to as the kₒₙ value or on-rate.

The term "K_{A}" (M⁻¹), as used herein, refers to the association equilibrium constant of a particular antibody-antigen interaction and is obtained by dividing kₐ by k_{d}.

The term "AXL" as used herein, refers to the protein entitled AXL, which is also referred to as UFO or JTK11, a 894 amino acid protein with a molecular weight of 104-140 kDa that is part of the subfamily of mammalian TAM Receptor Tyrosine Kinases (RTKs). The molecular weight is variable due to potential differences in glycosylation of the protein. The AXL protein consists of two extracellular immunoglobulin-like (Ig-like) domains on the N-terminal end of the protein, two membrane-proximal extracellular fibronectin type III (FNIII) domains, a transmembrane domain and an intracellular kinase domain. AXL is activated upon binding of its ligand Gas6, by ligand-independent homophilic interactions between AXL extracellular domains, by autophosphorylation in presence of reactive oxygen species (Korshunov *et al.,* 2012) or by transactivation through EGFR (Meyer *et al.,* 2013), and is aberrantly expressed in several tumor types. In humans, the AXL protein is encoded by a nucleic acid sequence encoding the amino acid sequence shown in SEQ ID NO:130 (human AXL protein: Swissprot P30530; cynomolgus AXL protein: Genbank accession HB387229.1)).

The term "ligand-independent homophilic interactions" as used herein, refers to association between two AXL molecules (expressed on neighboring cells) that occurs in absence of the ligand.

The term "antibody binding AXL" as used herein, refers to any antibody binding an epitope on the extracellular part of AXL.

The term "epitope" means a protein determinant capable of specific binding to an antibody. Epitopes usually consist of surface groupings of molecules such as amino acids, sugar side chains or a combination thereof and usually have specific three dimensional structural characteristics, as well as specific charge characteristics. Conformational and non-conformational epitopes are distinguished in that the binding to the former but not the latter is lost in the presence of denaturing solvents. The epitope may comprise amino acid residues which are directly involved in the binding, and other amino acid residues, which are not directly involved in the binding, such as amino acid residues which are effectively blocked or covered by the specific antigen binding peptide (in other words, the amino acid residue is within the footprint of the specific antigen binding peptide).

The term "ligand" as used herein, refers to a substance, such as a hormone, peptide, ion, drug or protein, that binds specifically and reversibly to another protein, such as a receptor, to form a larger complex. Ligand binding to a receptor may alter its chemical conformation, and determines its functional state. For instance, a ligand may function as agonist or antagonist.

The term "Growth Arrest-Specific 6" or "Gas6" as used herein, refers to a 721 amino acid protein, with a molecular weight of 75-80 kDa, that functions as a ligand for the TAM family of receptors, including AXL. Gas6 is composed of an N-terminal region containing multiple gamma-carboxyglutamic acid residues (Gla), which are responsible for the specific interaction with the negatively charged phospholipid membrane. Although the Gla domain is not necessary for binding of Gas6 to AXL, it is required for activation of AXL. Gas6 may also be termed as the "ligand to AXL".

The terms "monoclonal antibody", "monoclonal Ab", "monoclonal antibody composition", "mAb", or the like, as used herein refer to a preparation of antibody molecules of single molecular composition. A monoclonal antibody composition displays a single binding specificity and affinity for a particular epitope. Accordingly, the term "human monoclonal antibody" refers to antibodies displaying a single binding specificity which have variable and constant regions derived from human germline immunoglobulin sequences. The human monoclonal antibodies may be produced by a hybridoma which includes a B cell obtained from a transgenic or transchromosomal non-human animal, such as a transgenic mouse, having a genome comprising a human heavy chain transgene and a light chain transgene, fused to an immortalized cell.

In the context of the present invention the term "ADC" refers to an antibody drug conjugate, which in the context of the present invention refers to an anti-AXL antibody which is coupled to a therapeutic moiety, *e.g.,* a cytotoxic moiety as described in the present application. It may *e.g.* be coupled with a linker to *e.g.* cysteine or with other conjugation methods to other amino acids. The moiety may *e.g.* be a drug or a toxin or the like.

As used herein, a "therapeutic moiety" means a compound which exerts a therapeutic or preventive effect when administered to a subject, particularly when delivered as an ADC as described herein. A "cytotoxic" or "cytostatic" moiety is a compound that is detrimental to (*e.g.,* kills) cells. Some cytotoxic or cytostatic moieties for use in ADCs are hydrophobic, meaning that they have no or only a limited solubility in water, e.g., 1 g/L or less (very slightly soluble), such as 0.8 g/L or less, such as 0.6 g/L or less, such as 0.4 g/L or less, such as 0.3 g/L or less, such as 0.2 g/L or less, such as 0.1 g/L or less (practically insoluble). Exemplary hydrophobic cytotoxic or cytostatic moieties include, but are not limited to, certain microtubulin inhibitors such as auristatin and its derivatives, *e.g.,* MMAF and MMAE, as well as maytansine and its derivatives, *e.g.,* DM1.

The antibody may have a binding affinity (K_{D}) in the range of 0.3x10⁻⁹ to 63x10⁻⁹ M to AXL, and wherein said binding affinity is measured using a Bio-layer Interferometry using soluble AXL extracellular domain.

The binding affinity may be determined as described in Example 2. Thus, in one embodiment, the antibody has a binding affinity of 0.3x10⁻⁹ to 63x10⁻⁹ M to the antigen, wherein the binding affinity is determined by a method comprising the steps of;
i) loading anti-human Fc Capture biosensors with anti-AXL antibodies, and
ii) determining association and dissociation of soluble recombinant AXL extracellular domain by Bio-Layer Interferometry at different concentrations.

The term "soluble recombinant AXL extracellular domain" as used herein, refers to an AXL extracellular domain, corresponding to amino acids 1-447 of the full-length protein (SEQ ID NO:130; see Example 1) that has been expressed recombinantly. Due to absence of the transmembrane and intracellular domain, recombinant AXL extracellular domain is not attached to a, *e.g.* cell surface and stays in solution. It is well-known how to express a protein recombinantly, see *e.g.* Sambrook (1989), and thus, it is within the knowledge of the skilled person to provide such recombinant AXL extracellular domain.

The antibody may have a dissociation rate of 6.9x10⁻⁵ s⁻¹ to 9.7x10⁻³ s⁻¹ to AXL, and wherein the dissociation rate is measured by Bio-layer Interferometry using soluble recombinant AXL extracellular domain.

The binding affinity may be determined as described above (and in Example 2). Thus, in one embodiment, the antibody has a dissociation rate of 6.9x10⁻⁵ s⁻¹ to 9.7x10⁻³ s⁻¹ to AXL, and wherein the dissociation rate is measured by a method comprising the steps of
i) loading anti-human Fc Capture biosensors with anti-AXL antibodies, and
ii) determining association and dissociation of recombinant AXL extracellular domain by Bio-Layer Interferometry at different concentrations.

The term "dissociation rate" as used herein, refers to the rate at which an antigen-specific antibody bound to its antigen, dissociates from that antigen, and is expressed as s⁻¹. Thus, in the context of an antibody binding AXL, the term "dissociation rate", refers to the antibody binding AXL dissociates from the recombinant extracellular domain of AXL, and is expressed as s⁻¹.

The ADCs for the use of the present invention may comprise an antibody-portion which binds to an extracellular domain of AXL without competing or interfering with Gas6 binding to AXL. In particular, the antibody may bind to the extracellular domain Ig1domain without competing or interfering with Gas6 binding to AXL. The antibody may bind to the extracellular domain and show no more than a 20% reduction in maximal Gas6 binding to AXL. The antibody may show no more than a 15% reduction in maximal Gas6 binding to AXL. The antibody may show no more than a 10% reduction in maximal Gas6 binding to AXL. The antibody may show no more than a 5% reduction in maximal Gas6 binding to AXL. The antibody may show no more than a 4% reduction in maximal Gas6 binding to AXL. The antibody show no more than a 2% reduction in maximal Gas6 binding to AXL. The antibody show no more than a 1% reduction in maximal Gas6 binding. The antibody may bind to the Ig2 domain in the AXL extracellular domain without competing or interfering with Gas6 binding to AXL. The antibody binds to the Ig2 domain in the AXL extracellular domain and show no more than a 20%, such as no more than 15%, such as no more than 10%, such as no more than 5%, such as no more than 4%, such as no more than 2%, such as no more than 1%, reduction in maximal Gas6 binding to AXL. The ability to compete with or reduce Gas6 binding may be determined as disclosed in Example 2 or Example 12. The antibody may bind to the Ig2 domain in the AXL extracellular domain without competing or interfering with maximal Gas6 binding to AXL.

Maximal antibody binding in the presence of Gas6 may be at least 90%, such as at least 95%, such as at least 97%, such as at least 99%, such as 100%, of binding in absence of Gas6 as determined by a competition assay, wherein competition between said antibody binding to human AXL and said Gas6 is determined on A431 cells preincubated with Gas6 and without Gas6.

Competition between anti-AXL and the ligand Gas6 to AXL may be determined as described in Example 2 under the heading "Interference of anti-AXL binding with Gas6 binding". Thus, the antibody may be one that does not compete for AXL binding with the ligand Gas6, wherein the competing for binding is determined in an assay comprising the steps of
i) incubating AXL-expressing cells with Gas6,
ii) adding anti-AXL antibodies to be tested,
iii) adding a fluorescently labelled secondary reagent detecting anti-AXL antibodies and
iv) analyzing the cells by FACS.

The antibody may be one that does not compete for binding with the ligand Gas6, wherein the competing for binding is determined in an assay comprising the steps of
i) incubating AXL-expressing cells with anti-AXL antibodies,
ii) adding Gas6,
iii) adding a fluorescently labelled secondary reagent detecting Gas6, and
iv) analyzing the cells by FACS.

The antibody may modulate AXL-associated signaling in an AXL-expressing cell of the when the cell is contacted with the antibody.

The antibody may be one that does not modulate AXL-associated signaling in an AXL-expressing cell of the when the cell is contacted with the antibody.

Non-limiting examples of modulation of AXL-associated signalling includes modulation of intracellular signaling pathways such as the PI3K/AKT, mitogen-activated protein kinase (MAPK), STAT or NF-κB cascades.

The anti-AXL antibody or AXL-ADC may compete for binding to AXL with an antibody comprising a variable heavy (VH) region and a variable light (VL) region selected from the group consisting of:
(a) a VH region comprising SEQ ID No:1 and a VL region comprising SEQ ID No:2 [**107**];
(b) a VH region comprising SEQ ID No:5 and a VL region comprising SEQ ID No:6 [**148**];
(c) a VH region comprising SEQ ID No:34 and a VL region comprising SEQ ID No:35 [**733**]
(d) a VH region comprising SEQ ID No:7 and a VL region comprising SEQ ID No:9 [**154**];
(e) a VH region comprising SEQ ID No:10 and a VL region comprising SEQ ID No:11 [**171**];
(f) a VH region comprising SEQ ID No:16 and a VL region comprising SEQ ID No:18 [**183**];
(g) a VH region comprising SEQ ID No:25 and a VL region comprising SEQ ID No:26 [**613**];
(h) a VH region comprising SEQ ID No:31 and a VL region comprising SEQ ID No:33 [**726**];
(i) a VH region comprising SEQ ID No:3 and a VL region comprising SEQ ID No:4 [**140**];
(j) a VH region comprising SEQ ID No:8 and a VL region comprising SEQ ID No:9 [**154-M103L**];
(k) a VH region comprising SEQ ID No:12 and a VL region comprising SEQ ID No:13 [**172**];
(l) a VH region comprising SEQ ID No:14 and a VL region comprising SEQ ID No:15 [**181**];
(m)a VH region comprising SEQ ID No:17 and a VL region comprising SEQ ID No:18 [**183-N52Q**];
(n) a VH region comprising SEQ ID No:19 and a VL region comprising SEQ ID No:20 [**187**];
(o) a VH region comprising SEQ ID No:21 and a VL region comprising SEQ ID No:22 [**608-01**] **;**
(p) a VH region comprising SEQ ID No:23 and a VL region comprising SEQ ID No:24 [**610-01**] **;**
(q) a VH region comprising SEQ ID No:27 and a VL region comprising SEQ ID No:28 [**613-08**];
(r) a VH region comprising SEQ ID No:29 and a VL region comprising SEQ ID No:30 [**620-06**]; and
(s) a VH region comprising SEQ ID No:32 and a VL region comprising SEQ ID No:33 [**726-M101L**].

When used herein in the context of an antibody and a Gas6 ligand or in the context of two or more antibodies, the term "competes with" or "cross-competes with" indicates that the antibody competes with the ligand or another antibody, *e.g.,* a "reference" antibody in binding to an antigen, respectively. Example 2 describes an example of how to test competition of an anti-AXL antibody with the AXL-ligand Gas6. Preferred reference antibodies for cross-competition between two antibodies are those comprising a binding region comprising the VH region and VL region of an antibody herein designated 107, 148, 733, 154, 171, 183, 613, 726, 140, 154-M103L, 172, 181, 183-N52Q, 187, 608-01, 610-01, 613-08, 620-06 or 726-M101L, as set forth in Table 4. A particularly preferred reference antibody is the antibody designated 107.

The anti-AXL antibody may bind to the same epitope on AXL as any one or more of the antibodies according to the aforementioned embodiment, as defined by their VH and VL sequences, *e.g.,* a VH region comprising SEQ ID No:1 and a VL region comprising SEQ ID No:2 **[107].**

Methods of determining an epitope to which an antibody binds are well-known in the art. Thus, the skilled person would know how to determine such an epitope. However, one example of determining whether an antibody binds within any epitope herein described may be by introducing point mutations into the extracellular domain of AXL extracellular domain, *e.g.,* for the purpose of identifying amino acids involved in the antibody-binding to the antigen. It is within the knowledge of the skilled person to introduce point mutation(s) in the AXL extracellular domain and test for antibody binding to point mutated AXL extracellular domains, since the effect of point mutations on the overall 3D structure is expected to be minimal.

An alternative method was used in Example 3, wherein the AXL domain specificity was mapped by preparing a panel of human-mouse chimeric AXL mutants where the human , Ig2, FN1 or FN2 domain had been replaced by its murine analog, and determining which mutant an anti-AXL antibody bound to. This method was based on the principle that these human AXL-specific antibodies recognized human but not mouse AXL. So, in separate and specific embodiments, the antibody binds to the domain of AXL, the Ig2 domain of AXL, the FN1 domain of AXL, or the FN2 domain of AXL.

A more high-resolution epitope-mapping method, identifying AXL extracellular domain amino acids involved in antibody binding, was also used in this Example. Specifically, this method analyzed binding of the anti-AXL antibody to a library of AXL sequence variants generated by recombination of AXL sequences derived from species with variable levels of homology with the human AXL sequence (SEQ ID NO:130) in the extracellular domain. This method was based on the principle that these human AXL-specific antibodies recognize human AXL, but not the AXL from any of the other species used in the example.

The antibody may bind to an epitope within the domain of AXL, and the antibody binding may be dependent on one or more or all of the amino acids corresponding to positions L121 to Q129 or one or more or all of T112 to Q124 of human AXL, wherein the numbering of amino acid residues refers to their respective positions in human AXL (SEQ ID NO:130). The antibody may to an epitope within the domain of AXL, and antibody binding may be dependent on the amino acids corresponding to positions L121 to Q129 or T112 to Q124 of human AXL. Antibody binding may be dependent on one or more or all amino acids in position L121, G122, H123, Q124, T125, F126, V127, S128 and Q129, corresponding to the amino acids involved in the binding of the antibody herein designated 107. Antibody binding may be dependent on one or more or all amino acid in position T112, G113, Q114, Y115, Q116, C117, L118,V119, F120, L121, G122, H123 and Q124.

The antibody may bind to an epitope within the Ig2 domain of AXL, and antibody binding may be dependent on one or more or all of the amino acids corresponding to position D170 or the combination of D179 or one or more or all of the amino acids in positions T182 to R190 of human AXL. Antibody binding may be dependent on the amino acids in position T182, A183, P183, G184, H185, G186, P187, Q189 and R190.

The antibody may bind to an the FN1 domain of human AXL, and antibody binding may be dependent on one or more or all of the amino acids corresponding to positions Q272 to A287 and G297 to P301 of human AXL. Antibody binding may be dependent on the amino acids corresponding to positions Q272 to A287 and G297 to P301 of human AXL.

The antibody may bind to the FN2 domain of human AXL and antibody binding may be dependent on one or more or all of the amino acids corresponding to positions A359, R386, and Q436 to K439 of human AXL.

The antibody may bind to an epitope within the Ig1 domain of AXL, and the epitope may comprise or require one or more or all of the amino acids corresponding to positions L121 to Q129 or one or more or all of T112 to Q124 of human AXL, wherein the numbering of amino acid residues refers to their respective positions in human AXL (SEQ ID NO:130). In one embodiment, the antibody binds to an epitope within the domain of AXL, and the epitope comprises or requires the amino acids corresponding to positions L121 to Q129 or T112 to Q124 of human AXL. Preferably, the epitope comprises one or more or all amino acid in position L121, G122, H123, Q124, T125, F126, V127, S128 and Q129, corresponding to the amino acids involved in the binding of the antibody herein designated 107. The epitope may one or more or all amino acid in position T112, G113, Q114, Y115, Q116, C117, L118,V119, F120, L121, G122, H123 and Q124.

The antibody may bind to an epitope within the Ig2 domain of AXL, and the epitope may comprise or requires one or more or all of the amino acids corresponding to position D170 or the combination of D179 or one or more or all of the amino acids in positions T182 to R190 of human AXL. The epitope may comprise or require the amino acids in position T182, A183, P183, G184, H185, G186, P187, Q189 and R190.

The antibody may bind to an epitope within the FN1 domain of human AXL, which epitope comprises or requires one or more or all of the amino acids corresponding to positions Q272 to A287 and G297 to P301 of human AXL. The epitope may comprise or require the amino acids corresponding to positions Q272 to A287 and G297 to P301 of human AXL.

The antibody may bind to an epitope within the FN2 domain of human AXL, which epitope comprises or requires one or more or all of the amino acids corresponding to positions A359, R386, and Q436 to K439 of human AXL.

The antibody may bind to an epitope within the FN1-like domain of human AXL.

The antibody may bind to an epitope on AXL which epitope is recognized by any one of the antibodies defined by
a) ) a VH region comprising the CDR1, CDR2, and CDR3 sequences of SEQ ID Nos.: 36, 37, and 38, respectively; and a VL region comprising the CDR1, CDR2, and CDR3 sequences of SEQ ID Nos.: 39, GAS, and 40, respectively, [107];
b) a VH region comprising the CDR1, CDR2, and CDR3 sequences of SEQ ID Nos.: 46, 47, and 48, respectively; and a VL region comprising the CDR1, CDR2, and CDR3 sequences of SEQ ID Nos.: 49, AAS, and 50, respectively, [**148**];
c) a VH region comprising the CDR1, CDR2, and CDR3 sequences of SEQ ID Nos.: 114, 115, and 116, respectively, and a VL region comprising the CDR1, CDR2, and CDR3 sequences of SEQ ID Nos.: 117, DAS, and 118, respectively [**733**];
d) a VH region comprising the CDR1, CDR2, and CDR3 sequences of SEQ ID Nos.: 51, 52, and 53, respectively; and a VL region comprising the CDR1, CDR2, and CDR3 sequences of SEQ ID Nos.: 55, GAS, and 56, respectively [**154**];
e) a VH region comprising the CDR1, CDR2, and CDR3 sequences of SEQ ID Nos.: 51, 52, and 54, respectively; and a VL region comprising the CDR1, CDR2, and CDR3 sequences of SEQ ID Nos.: 55, GAS, and 56, respectively [**154-M103L**];
f) a VH region comprising the CDR1, CDR2, and CDR3 sequences of SEQ ID Nos.: 57, 58, and 59, respectively; and a VL region comprising the CDR1, CDR2, and CDR3 sequences of SEQ ID Nos.: 60, GAS, and 61, respectively, [**171**];
g) a VH region comprising the CDR1, CDR2, and CDR3 sequences of SEQ ID Nos.: 62, 63, and 64, respectively; and a VL region comprising the CDR1, CDR2, and CDR3 sequences of SEQ ID Nos.: 65, GAS, and 66, respectively, [**172**];
h) a VH region comprising the CDR1, CDR2, and CDR3 sequences of SEQ ID Nos.: 67, 68, and 69, respectively; and a VL region comprising the CDR1, CDR2, and CDR3 sequences of SEQ ID Nos.: 70, GAS, and 71, respectively, [**181**];
i) a VH region comprising the CDR1, CDR2, and CDR3 sequences of SEQ ID Nos.: 72, 73, and 75, respectively; and a VL region comprising the CDR1, CDR2, and CDR3 sequences of SEQ ID Nos.: 76, ATS, and 77, respectively, [**183**];
j) a VH region comprising the CDR1, CDR2, and CDR3 sequences of SEQ ID Nos.: 72, 74, and 75, respectively; and a VL region comprising the CDR1, CDR2, and CDR3 sequences of SEQ ID Nos.: 76, ATS, and 77, respectively, [**183-N52Q**];
k) a VH region comprising the CDR1, CDR2, and CDR3 sequences of SEQ ID Nos.: 78, 79, and 80, respectively; and a VL region comprising the CDR1, CDR2, and CDR3 sequences of SEQ ID Nos.: 81, AAS, and 82, respectively, [**187**];
l) a VH region comprising the CDR1, CDR2, and CDR3 sequences of SEQ ID Nos.: 83, 84, and 85, respectively; and a VL region comprising the CDR1, CDR2, and CDR3 sequences of SEQ ID Nos.: 86, GAS, and 87, respectively, [**608-01**];
m) a VH region comprising the CDR1, CDR2, and CDR3 sequences of SEQ ID Nos.: 88, 89, and 90, respectively; and a VL region comprising the CDR1, CDR2, and CDR3 sequences of SEQ ID Nos.: 91, GAS, and 92, respectively, [**610-01**];
n) a VH region comprising the CDR1, CDR2, and CDR3 sequences of SEQ ID Nos.: 93, 94, and 95, respectively; and a VL region comprising the CDR1, CDR2, and CDR3 sequences of SEQ ID Nos.: 96, GAS, and 97, respectively, [**613**];
o) a VH region comprising the CDR1, CDR2, and CDR3 sequences of SEQ ID Nos.: 98, 99, and 100, respectively; and a VL region comprising the CDR1, CDR2, and CDR3 sequences of SEQ ID Nos.: 10, DAS, and 102, respectively, [**613-08**];
p) a VH region comprising the CDR1, CDR2, and CDR3 sequences of SEQ ID Nos.: 103, 104, and 105, respectively; and a VL region comprising the CDR1, CDR2, and CDR3 sequences of SEQ ID Nos.: 106, GAS, and 107, respectively, [**620-06**];
q) a VH region comprising the CDR1, CDR2, and CDR3 sequences of SEQ ID Nos.: 108, 109, and 110, respectively; and a VL region comprising the CDR1, CDR2, and CDR3 sequences of SEQ ID Nos.: 112, AAS, and 113, respectively, [**726**];
r) a VH region comprising the CDR1, CDR2, and CDR3 sequences of SEQ ID Nos.: 108, 109, and 111, respectively; and a VL region comprising the CDR1, CDR2, and CDR3 sequences of SEQ ID Nos.: 112, AAS, and 113, respectively, [**726-M101L**];
s) a VH region comprising the CDR1, CDR2, and CDR3 sequences of SEQ ID Nos.: 41, 42, and 43, respectively; and a VL region comprising the CDR1, CDR2, and CDR3 sequences of SEQ ID Nos.: 44, AAS, and 45, respectively, [140];
t) a VH region comprising the CDR1, CDR2, and CDR3 sequences of SEQ ID Nos.: 93, 94, and 95, respectively, and a VL region comprising the CDR1, CDR2, and CDR3 sequences of SEQ ID Nos.: 128, XAS, wherein X is D or G, and 129, respectively, [**613** / **613-08**];
u) a VH region comprising the CDR1, CDR2, and CDR3 sequences of SEQ ID Nos.: 46, 119, and 120, respectively; and a VL region comprising CDR1, CDR2, and CDR3 sequences of SEQ ID Nos.: 49, AAS, and 50, respectively, [**148 / 140**];
v) a VH region comprising the CDR1, CDR2, and CDR3 sequences of SEQ ID Nos.: 123, 124, and 125, respectively; and a VL region comprising CDR1, CDR2, and CDR3 sequences of SEQ ID Nos.: 60, GAS, and 61, respectively [**171** / **172** / **181**]; and
w) a VH region comprising the CDR1, CDR2, and CDR3 sequences of SEQ ID Nos.: 121, 109, and 122, respectively; and a VL region comprising the CDR1, CDR2, and CDR3 sequences of SEQ ID Nos.: 112, AAS, and 113, respectively [**726** / **187**]; and
x) a VH region comprising the CDR1, CDR2, and CDR3 sequences of SEQ ID Nos.:93, 126, and 127, respectively; and a VL region comprising the CDR1, CDR2, and CDR3 sequences of SEQ ID Nos.: 96, GAS, and 97, respectively [**613** / **608-01** / **610-01** / **620-06**].

The antibody may bind to an epitope on AXL which epitope is recognized by any one of the antibodies defined by comprising a binding regon comprising the VH and VL sequences of an antibody selected from those herein designated 107, 061, 137, 148, 154-M103L, 171, 183-N52Q, 511, 613, 726-M102L and 733. As shown in Example 16, these anti-AXL antibodies internalize, and are thus suitable for an ADC approach.

The antibody may comprise at least one binding region comprising a VH region and a VL region selected from the group consisting of:
(a) a VH region comprising the CDR1, CDR2, and CDR3 sequences of SEQ ID Nos.:36, 37, and 38, respectively; and a VL region comprising the CDR1, CDR2, and CDR3 sequences of SEQ ID Nos.:39, GAS, and 40, respectively, [**107**];
(b) a VH region comprising the CDR1, CDR2, and CDR3 sequences of SEQ ID Nos.:46, 47, and 48, respectively; and a VL region comprising the CDR1, CDR2, and CDR3 sequences of SEQ ID Nos.:49, AAS, and 50, respectively, [**148**];
(c) a VH region comprising the CDR1, CDR2, and CDR3 sequences of SEQ ID Nos.:114, 115, and 116, respectively, and a VL region comprising the CDR1, CDR2, and CDR3 sequences of SEQ ID Nos.:117, DAS, and 118, respectively [**733**];
(d) a VH region comprising the CDR1, CDR2, and CDR3 sequences of SEQ ID Nos.:51, 52, and 53, respectively; and a VL region comprising the CDR1, CDR2, and CDR3 sequences of SEQ ID Nos.:55, GAS, and 56, respectively [**154**];
(e) a VH region comprising the CDR1, CDR2, and CDR3 sequences of SEQ ID Nos.:51, 52, and 54, respectively; and a VL region comprising the CDR1, CDR2, and CDR3 sequences of SEQ ID Nos.:55, GAS, and 56, respectively [**154-M103L**];
(f) a VH region comprising the CDR1, CDR2, and CDR3 sequences of SEQ ID Nos.:57, 58, and 59, respectively; and a VL region comprising the CDR1, CDR2, and CDR3 sequences of SEQ ID Nos.:60, GAS, and 61, respectively, [**171**];
(g) a VH region comprising the CDR1, CDR2, and CDR3 sequences of SEQ ID Nos.:62, 63, and 64, respectively; and a VL region comprising the CDR1, CDR2, and CDR3 sequences of SEQ ID Nos.:65, GAS, and 66, respectively, [**172**];
(h) a VH region comprising the CDR1, CDR2, and CDR3 sequences of SEQ ID Nos.:67, 68, and 69, respectively; and a VL region comprising the CDR1, CDR2, and CDR3 sequences of SEQ ID Nos.:70, GAS, and 71, respectively, [**181**];
(i) a VH region comprising the CDR1, CDR2, and CDR3 sequences of SEQ ID Nos.:72, 73, and 75, respectively; and a VL region comprising the CDR1, CDR2, and CDR3 sequences of SEQ ID Nos.:76, ATS, and 77, respectively, [**183**];
(j) a VH region comprising the CDR1, CDR2, and CDR3 sequences of SEQ ID Nos.:72, 74, and 75, respectively; and a VL region comprising the CDR1, CDR2, and CDR3 sequences of SEQ ID Nos.:76, ATS, and 77, respectively, [**183-N52Q**];
(k) a VH region comprising the CDR1, CDR2, and CDR3 sequences of SEQ ID Nos.:78, 79, and 80, respectively; and a VL region comprising the CDR1, CDR2, and CDR3 sequences of SEQ ID Nos.:81, AAS, and 82, respectively, [**187**];
(l) a VH region comprising the CDR1, CDR2, and CDR3 sequences of SEQ ID Nos.:83, 84, and 85, respectively; and a VL region comprising the CDR1, CDR2, and CDR3 sequences of SEQ ID Nos.:86, GAS, and 87, respectively, [**608-01**];
(m) a VH region comprising the CDR1, CDR2, and CDR3 sequences of SEQ ID Nos.:88, 89, and 90, respectively; and a VL region comprising the CDR1, CDR2, and CDR3 sequences of SEQ ID Nos.:91, GAS, and 92, respectively, [**610-01**];
(n) a VH region comprising the CDR1, CDR2, and CDR3 sequences of SEQ ID Nos.:93, 94, and 95, respectively; and a VL region comprising the CDR1, CDR2, and CDR3 sequences of SEQ ID Nos.:96, GAS, and 97, respectively, [**613**];
(o) a VH region comprising the CDR1, CDR2, and CDR3 sequences of SEQ ID Nos.:98, 99, and 100, respectively; and a VL region comprising the CDR1, CDR2, and CDR3 sequences of SEQ ID Nos.:101, DAS, and 102, respectively, [**613-08**];
(p) a VH region comprising the CDR1, CDR2, and CDR3 sequences of SEQ ID Nos.:103, 104, and 105, respectively; and a VL region comprising the CDR1, CDR2, and CDR3 sequences of SEQ ID Nos.:106, GAS, and 107, respectively, [**620-06**];
(q) a VH region comprising the CDR1, CDR2, and CDR3 sequences of SEQ ID Nos.:108, 109, and 110, respectively; and a VL region comprising the CDR1, CDR2, and CDR3 sequences of SEQ ID Nos.:112, AAS, and 113, respectively, [**726**];
(r) a VH region comprising the CDR1, CDR2, and CDR3 sequences of SEQ ID Nos.:108, 109, and 111, respectively; and a VL region comprising the CDR1, CDR2, and CDR3 sequences of SEQ ID Nos.:112, AAS, and 113, respectively, [**726-M101L**];
(s) a VH region comprising the CDR1, CDR2, and CDR3 sequences of SEQ ID Nos.:41, 42, and 43, respectively; and a VL region comprising the CDR1, CDR2, and CDR3 sequences of SEQ ID Nos.:44, AAS, and 45, respectively, [**140**];
(t) a VH region comprising the CDR1, CDR2, and CDR3 sequences of SEQ ID Nos.:93, 94, and 95, respectively, and a VL region comprising the CDR1, CDR2, and CDR3 sequences of SEQ ID Nos.:128, XAS, wherein X is D or G, and 129, respectively, [**613** / **613-08**];
(u) a VH region comprising the CDR1, CDR2, and CDR3 sequences of SEQ ID Nos.:46, 119, and 120, respectively; and a VL region comprising CDR1, CDR2, and CDR3 sequences of SEQ ID Nos.:49, AAS, and 50, respectively, [**148 / 140**];
(v) a VH region comprising the CDR1, CDR2, and CDR3 sequences of SEQ ID Nos.:123, 124, and 125, respectively; and a VL region comprising CDR1, CDR2, and CDR3 sequences of SEQ ID Nos.:60, GAS, and 61, respectively [**171 / 172** / **181**]; and
(w) a VH region comprising the CDR1, CDR2, and CDR3 sequences of SEQ ID Nos.:121, 109, and 122, respectively; and a VL region comprising the CDR1, CDR2, and CDR3 sequences of SEQ ID Nos.:112, AAS, and 113, respectively [**726** / **187**]; and
(x) a VH region comprising the CDR1, CDR2, and CDR3 sequences of SEQ ID Nos.:93, 126, and 127, respectively; and a VL region comprising the CDR1, CDR2, and CDR3 sequences of SEQ ID Nos.:96, GAS, and 97, respectively **[613** / **608-01** / **610-01** / **620-06].**

The antibody may comprises at least one binding region comprising a VH region and a VL region selected from the group consisting of:
(a) a VH region comprising SEQ ID No:1 and a VL region comprising SEQ ID No:2 [**107**];
(b) a VH region comprising SEQ ID No:5 and a VL region comprising SEQ ID No:6 [**148**];
(c) a VH region comprising SEQ ID No:34 and a VL region comprising SEQ ID No:35 [**733**]
(d) a VH region comprising SEQ ID No:7 and a VL region comprising SEQ ID No:9 [**154**];
(e) a VH region comprising SEQ ID No:10 and a VL region comprising SEQ ID No:11 [**171**];
(f) a VH region comprising SEQ ID No:16 and a VL region comprising SEQ ID No:18 [**183**];
(g) a VH region comprising SEQ ID No:25 and a VL region comprising SEQ ID No:26 [**613**];
(h) a VH region comprising SEQ ID No:31 and a VL region comprising SEQ ID No:33 [**726**];
(i) a VH region comprising SEQ ID No:3 and a VL region comprising SEQ ID No:4 [**140**];
(j) a VH region comprising SEQ ID No:8 and a VL region comprising SEQ ID No:9 [**154-M103L**];
(k) a VH region comprising SEQ ID No:12 and a VL region comprising SEQ ID No:13 [**172**];
(l) a VH region comprising SEQ ID No:14 and a VL region comprising SEQ ID No:15 [**181**];
(m) a VH region comprising SEQ ID No:17 and a VL region comprising SEQ ID No:18 [**183-N52Q**];
(n) a VH region comprising SEQ ID No:19 and a VL region comprising SEQ ID No:20 [**187**];
(o) a VH region comprising SEQ ID No:21 and a VL region comprising SEQ ID No:22 [**608-01**];
(p) a VH region comprising SEQ ID No:23 and a VL region comprising SEQ ID No:24 **[610-01];**
(q) a VH region comprising SEQ ID No:27 and a VL region comprising SEQ ID No:28 **[613-08];**
(r) a VH region comprising SEQ ID No:29 and a VL region comprising SEQ ID No:30 **[620-06];** and
(s) a VH region comprising SEQ ID No:32 and a VL region comprising SEQ ID No:33 **[726-M101L].**

The present disclosure also provides antibodies comprising functional variants of the VL region, VH region, or one or more CDRs of the antibodies mentioned above. A functional variant of a VL, VH, or CDR used in the context of an AXL antibody still allows the antibody to retain at least a substantial proportion (at least about 50%, 60%, 70%, 80%, 90%, 95%, 99% or more) of the affinity/avidity and/or the specificity/selectivity of the parent antibody and in some cases such an AXL antibody may be associated with greater affinity, selectivity and/or specificity than the parent antibody.

Such functional variants typically retain significant sequence identity to the parent antibody. The percent identity between two sequences is a function of the number of identical positions shared by the sequences (*i.e.,* % homology = # of identical positions/total # of positions x 100), taking into account the number of gaps, and the length of each gap, which need to be introduced for optimal alignment of the two sequences. The comparison of sequences and determination of percent identity between two sequences may be accomplished using a mathematical algorithm, which is well-known in the art.

The sequence identity between two amino acid sequences may, for example, be determined using the Needleman-Wunsch algorithm (Needleman and Wunsch, 1970, J. Mol. Biol. 48: 443-453) as implemented in the Needle program of the EMBOSS package (EMBOSS: The European Molecular Biology Open Software Suite, Rice et al., 2000, Trends Genet. 16: 276-277), preferably version 5.0.0 or later. The parameters used are gap open penalty of 10, gap extension penalty of 0.5, and the EBLOSUM62 (EMBOSS version of BLOSUM62) substitution matrix. The output of Needle labeled "longest identity" (obtained using the -nobrief option) is used as the percent identity and is calculated as follows: $\left(Identical Residues\times 100\right) / \left(Length of Alignment-Total Number of Gaps in Alignment\right) .$

The VH, VL and/or CDR sequences of variants may differ from those of the parent antibody sequences through mostly conservative substitutions; for instance at least about 35%, about 50% or more, about 60% or more, about 70% or more, about 75% or more, about 80% or more, about 85% or more, about 90% or more, (e.g., about 65-95%, such as about 92%, 93% or 94%) of the substitutions in the variant are conservative amino acid residue replacements.

The VH, VL and/or CDR sequences of variants may differ from those of the parent antibody sequences through mostly conservative substitutions; for instance 10 or less, such as 9 or less, 8 or less, 7 or less, 6 or less, 5 or less, 4 or less, 3 or less, 2 or less or 1 of the substitutions in the variant are conservative amino acid residue replacements.

Embodiments of the present disclosuire are also provided wherein mutations or substitutions of up to five mutations or substitutions are allowed across the three CDR sequences in the variable heavy chain and/or variable light chain of the preceding embodiment. The up to five mutations or substitutions may be distributed across the three CDR sequences of the variable heavy chain and the three CDR sequences of the variable light chain. The up to five mutations or substitutions may be distributed across the six CDR sequences of the binding region. The mutations or substitutions may be of conservative, physical or functional amino acids such that mutations or substitutions do not change the epitope or preferably do not modify binding affinity to the epitope more than 30 %, such as more than 20 % or such as more than 10%. The conservative, physical or functional amino acids are selected from the 20 natural amino acids found i.e, Arg, His, Lys, Asp, Glu, Ser, Thr, Asn, Gin, Cys, Gly, Pro, Ala, Ile, Leu, Met, Phe, Trp, Tyr and Val.

So the antibody may comprise at least one binding region comprising a VH region and a VL region selected from the group consisting of VH and VL sequences at least 90%, such as at least 95%, such as at least 97%, such as at least 99% identical to:
(a) a VH region comprising SEQ ID No:1 and a VL region comprising SEQ ID No:2 [**107**];
(b) a VH region comprising SEQ ID No:5 and a VL region comprising SEQ ID No:6 [**148**];
(c) a VH region comprising SEQ ID No:34 and a VL region comprising SEQ ID No:35 [**733**]
(d) a VH region comprising SEQ ID No:7 and a VL region comprising SEQ ID No:9 [**154**];
(e) a VH region comprising SEQ ID No:10 and a VL region comprising SEQ ID No:11 [**171**];
(f) a VH region comprising SEQ ID No:16 and a VL region comprising SEQ ID No:18 [**183**];
(g) a VH region comprising SEQ ID No:25 and a VL region comprising SEQ ID No:26 [**613**] ;
(h) a VH region comprising SEQ ID No:31 and a VL region comprising SEQ ID No:33 [**726**] ;
(i) a VH region comprising SEQ ID No:3 and a VL region comprising SEQ ID No:4 [**140**];
(j) a VH region comprising SEQ ID No:8 and a VL region comprising SEQ ID No:9 [**154-M103L**];
(k) a VH region comprising SEQ ID No:12 and a VL region comprising SEQ ID No:13 [**172**];
(l) a VH region comprising SEQ ID No:14 and a VL region comprising SEQ ID No:15 [**181**];
(m)a VH region comprising SEQ ID No:17 and a VL region comprising SEQ ID No:18 [**183-N52Q**];
(n) a VH region comprising SEQ ID No:19 and a VL region comprising SEQ ID No:20 [**187**] ;
(o) a VH region comprising SEQ ID No:21 and a VL region comprising SEQ ID No:22 [**608-01**] ;
(p) a VH region comprising SEQ ID No:23 and a VL region comprising SEQ ID No:24 [**610-01**] ;
(q) a VH region comprising SEQ ID No:27 and a VL region comprising SEQ ID No:28 [**613-08**] ;
(r) a VH region comprising SEQ ID No:29 and a VL region comprising SEQ ID No:30 [**620-06**]; and
(s) a VH region comprising SEQ ID No:32 and a VL region comprising SEQ ID No:33 [**726-M101L**].

The present disclosure also provides antibodies comprising functional variants of the VL region, VH region, or one or more CDRs of the antibodies of the examples. A functional variant of a VL, VH, or CDR used in the context of an AXL antibody still allows the antibody to retain at least a substantial proportion (at least about 50%, 60%, 70%, 80%, 90%, 95%, 99% or more) of the affinity/avidity and/or the specificity/selectivity of the parent antibody and in some cases such an AXL antibody may be associated with greater affinity, selectivity and/or specificity than the parent antibody.

Such functional variants typically retain significant sequence identity to the parent antibody. The percent identity between two sequences is a function of the number of identical positions shared by the sequences (*i.e.,* % homology = # of identical positions/total # of positions x 100), taking into account the number of gaps, and the length of each gap, which need to be introduced for optimal alignment of the two sequences. The comparison of sequences and determination of percent identity between two sequences may be accomplished using a mathematical algorithm, which is well-known in the art.

The VH, VL and/or CDR sequences of variants may differ from those of the parent antibody sequences through mostly conservative substitutions; for instance at least about 35%, about 50% or more, about 60% or more, about 70% or more, about 75% or more, about 80% or more, about 85% or more, about 90% or more, (*e.g*., about 65-95%, such as about 92%, 93% or 94%) of the substitutions in the variant are conservative amino acid residue replacements.

The VH, VL and/or CDR sequences of variants may differ from those of the parent antibody sequences through mostly conservative substitutions; for instance 10 or less, such as 9 or less, 8 or less, 7 or less, 6 or less, 5 or less, 4 or less, 3 or less, 2 or less or 1 of the substitutions in the variant are conservative amino acid residue replacements.

Embodiments of the present disclosure are also provided wherein mutations or substitutions of up to five mutations or substitutions are allowed across the three CDR sequences in the variable heavy chain and/or variable light chain of the preceding embodiment. The up to five mutations or substitutions may be distributed across the three CDR sequences of the variable heavy chain and the three CDR sequences of the variable light chain. The up to five mutations or substitutions may be distributed across the six CDR sequences of the binding region. The mutations or substitutions may be of conservative, physical or functional amino acids such that mutations or substitutions do not change the epitope or preferably do not modify binding affinity to the epitope more than 30 %, such as more than 20 % or such as more than 10%. The conservative, physical or functional amino acids are selected from the 20 natural amino acids found i.e, Arg, His, Lys, Asp, Glu, Ser, Thr, Asn, Gin, Cys, Gly, Pro, Ala, Ile, Leu, Met, Phe, Trp, Tyr and Val.

The antibody may comprise at least one binding region comprising a VH region and a VL region selected from the group consisting of VH and VL sequences at least 90%, such as at least 95%, such as at least 97%, such as at least 99% identical to:
(t) a VH region comprising SEQ ID No:1 and a VL region comprising SEQ ID No:2 [**107**];
(u) a VH region comprising SEQ ID No:5 and a VL region comprising SEQ ID No:6 [**148**];
(v) a VH region comprising SEQ ID No:34 and a VL region comprising SEQ ID No:35 [**733**]
(w) a VH region comprising SEQ ID No:7 and a VL region comprising SEQ ID No:9 [**154**];
(x) a VH region comprising SEQ ID No:10 and a VL region comprising SEQ ID No:11[**171**];
(y) a VH region comprising SEQ ID No:16 and a VL region comprising SEQ ID No:18 [**183**];
(z) a VH region comprising SEQ ID No:25 and a VL region comprising SEQ ID No:26 [**613**];
(aa)a VH region comprising SEQ ID No:31 and a VL region comprising SEQ ID No:33 [**726**];
(bb) a VH region comprising SEQ ID No:3 and a VL region comprising SEQ ID No:4 [**140**];
(cc) a VH region comprising SEQ ID No:8 and a VL region comprising SEQ ID No:9 [**154-M103L**];
(dd) a VH region comprising SEQ ID No:12 and a VL region comprising SEQ ID No:13 [**172**];
(ee)a VH region comprising SEQ ID No:14 and a VL region comprising SEQ ID No:15 [**181**];
(ff) a VH region comprising SEQ ID No:17 and a VL region comprising SEQ ID No:18 [**183-N52Q**];
(gg)a VH region comprising SEQ ID No:19 and a VL region comprising SEQ ID No:20 [**187**];
(hh) a VH region comprising SEQ ID No:21 and a VL region comprising SEQ ID No:22 [**608-01**];
(ii) a VH region comprising SEQ ID No:23 and a VL region comprising SEQ ID No:24 [**610-01**];
(jj) a VH region comprising SEQ ID No:27 and a VL region comprising SEQ ID No:28 [**613-08**];
(kk)a VH region comprising SEQ ID No:29 and a VL region comprising SEQ ID No:30 [**620-06**]; and
(II) a VH region comprising SEQ ID No:32 and a VL region comprising SEQ ID No:33 [**726-M101L**].

The antibody may comprise at least one binding region comprising the VH and VL CDR1, CDR2, and CDR3 sequences of an anti-AXL antibody known in the art, *e.g.,* an antibody described in any of Leconet *et al.* (2013), Li *et al.* (2009), Ye *et al.* (2010), lida *et al.* (2014), WO 2012/175691 (INSERM), WO 2012/175692 (INSERM), WO 2013/064685 (Pierre Fabré Medicaments), WO 2013/090776 (INSERM), WO 2009/063965 (Chugai Pharmaceuticals), WO 2010/131733, WO 2011/159980 (Genentech), WO09062690 (U3 Pharma), WO2010130751 (U3 Pharma), WO2014093707 (Stanford University) and EP2228392A1 (Chugai),.The antibody may be murine antibody 1613F12 or a chimeric or a humanized variant thereof as described in WO2014174111 (Pierre Fabré Medicament), wherein the VH and VL sequences of the mouse antibody 1613F12 are presented as SEQ ID:8 and SEQ ID:7, respectively. The VH sequence of the humanized antibody variant of 1613F12 is selected from the sequences disclosed therein as SEQ ID NO:29 to 49 and SEQ ID NO:82, and the VL sequence of the humanized antibody variant of 1613F12 is selected from the sequences disclosed therein as SEQ ID NO:17 to 28 and SEQ ID: 81. One specific antibody comprises the VH and VL sequences disclosed therein as SEQ ID NO:29 and 17, respectively. The VH CDR1, CDR2 and CDR3 sequences of mouse, chimeric and humanized 1613F12 are SEQ ID NO:4, 5 and 6, respectively and the VL CDR1, CDR2 and CDR3 sequences of mouse and humanized 1613F12 are disclosed therein as SEQ ID NO:1, 2, and 3, respectively. The antibody may be an antibody described in WO2011159980 (Hoffman-La Roche), particularly paragraphs [0127] through [0229] (pages 28-52). For example, the antibody may comprise the VH and VL hypervariable regions (HVR), or the VH and VL regions, of antibody YW327.6S2, which are disclosed therein as SEQ ID NOS:7, 8 and 9 (VH HVR1, 2 and 3, respectively), SEQ ID NOS:10, 11 and 12 (VL HVR1, 2 and 3, respectively) and SEQ ID NOS:103 and 104 (VH and VL sequences, respectively).

The antibody may mediate antibody-mediated crosslinking or clustering (*e.g*., due to the Fc-region of AXL-bound antibodies binding to FcR-expressing cells) of AXL molecules on the surface of a cell, which can lead to apoptosis of the cell.

The antibody may induce an Fc-dependent cellular response such as ADCC or ADCP against an AXL-expressing cell after binding of the AXL-specific antibody to the plasma membrane of the AXL-expressing cell in the presence of effector cells. The antibody-portion of the antibody is typically full-length and of an isotype leading to an ADCC or ADCP response, such as, *e.g.,* an IgG1,κ isotype.

The antibody may induce a CDC response against an AXL-expressing cell after binding of the AXL-specific antibody to the plasma membrane of the AXL-expressing cell in the presence of complement proteins, such as complement proteins present in normal human serum, that may be activated.The antibody is typically full-length and of an isotype capable of inducing activation of the complement system, such as, *e.g.,* an IgG1,κ isotype.

The antibody and/or ADC may further be characterized by internalization upon binding to AXL. Accordingly, the antibody and/or ADC may be internalized and trafficked to lysosomes for specific (*i.e.* cleavable linker) or non-specific (non-cleavable linker) proteolytic cleavage of the anti-AXL antibody-linker-drug complex.

The antibody may interfere with AXL-mediated regulation of the innate or adaptive immune response, such as by binding of the antibody to AXL-expressing macrophages, dendritic cells or NK cells.

The therapeutic moiety of the ADC may be linked to the antibody moiety via a linker allowing for release of the drug once the ADC is internalized, *e.g.,* by a change in pH or reducing conditions. Suitable linker technology is known in the art, as described herein.

In one embodiment, the antibody comprises a heavy chain of an isotype selected from the group consisting of IgG1, IgG2, IgG3, and IgG4. In a further embodiment, the antibody comprises a heavy chain of an isotype selected from the group consisting of a human IgG1, IgG2, IgG3, and IgG4.

The term "isotype" as used herein refers to the immunoglobulin class (for instance IgG1, IgG2, IgG3, IgG4, IgD, IgA, IgE, or IgM) or any allotype thereof, such as IgG1m(za) and IgG1m(f)) that is encoded by heavy chain constant region genes. Further, each heavy chain isotype can be combined with either a kappa (κ) or lambda (λ) light chain.

In one embodiment, the isotype is IgG1, such as human IgG1, optionally allotype IgG1m(f).

In one embodiment, the antibody is a full-length monoclonal antibody, optionally a full-length human monoclonal IgG1,κ antibody.

The term "full-length antibody" when used herein, refers to an antibody (*e.g*., a parent or variant antibody) which contains all heavy and light chain constant and variable domains corresponding to those that are normally found in a wild-type antibody of that isotype. A full-length antibody according to the present invention may be produced by a method comprising the steps of (i) cloning the CDR sequences into a suitable vector comprising complete heavy chain sequences and complete light chain sequence, and (ii) expressing the complete heavy and light chain sequences in suitable expression systems. It is within the knowledge of the skilled person to produce a full-length antibody when starting out from either CDR sequences or full variable region sequences. Thus, the skilled person would know how to generate a full-length antibody according to the present invention.

The antibody may be a human antibody.

The term "human antibody", as used herein, is intended to include antibodies having variable and framework regions derived from human germline immunoglobulin sequences and a human immunoglobulin constant domain. The human antibodies of the disclosure may include amino acid residues not encoded by human germline immunoglobulin sequences (*e.g*., mutations, insertions or deletions introduced by random or site-specific mutagenesis *in vitro* or by somatic mutation *in vivo*). However, the term "human antibody", as used herein, is not intended to include antibodies in which CDR sequences derived from the germline of another non-human species, such as a mouse, have been grafted onto human framework sequences.

As used herein, a human antibody is "derived from" a particular germline sequence if the antibody is obtained from a system using human immunoglobulin sequences, for instance by immunizing a transgenic mouse carrying human immunoglobulin genes or by screening a human immunoglobulin gene library, and wherein the selected human antibody is at least 90%, such as at least 95%, for instance at least 96%, such as at least 97%, for instance at least 98%, or such as at least 99% identical in amino acid sequence to the amino acid sequence encoded by the germline immunoglobulin gene. Typically, outside the heavy chain CDR3, a human antibody derived from a particular human germline sequence will display no more than 20 amino acid differences, *e.g.* no more than 10 amino acid differences, such as no more than 9, 8, 7, 6 or 5, for instance no more than 4, 3, 2, or 1 amino acid difference from the amino acid sequence encoded by the germline immunoglobulin gene.

The antibody according to the present disclosure may comprise amino acid modifications in the immunoglobulin heavy and/or light chains. In particular, amino acids in the Fc region of the antibody may be modified.

The term "Fc region" as used herein, refers to a region comprising, in the direction from the N- to C-terminal end of the antibody, at least a hinge region, a CH2 region and a CH3 region. An Fc region of the antibody may mediate the binding of the immunoglobulin to host tissues or factors, including various cells of the immune system (such as effector cells) and components of the complement system.

The term "hinge region" as used herein refers to the hinge region of an immunoglobulin heavy chain. Thus, for example the hinge region of a human IgG1 antibody corresponds to amino acids 216-230 according to the Eu numbering as set forth in Kabat *et al.* (1991). However, the hinge region may also be any of the other subtypes as described herein.

The term "CH1 region" or "CH1 domain" as used herein refers to the CH1 region of an immunoglobulin heavy chain. Thus, for example the CH1 region of a human IgG1 antibody corresponds to amino acids 118-215 according to the Eu numbering as set forth in Kabat *et al.* (1991) . However, the CH1 region may also be any of the other subtypes as described herein.

The term "CH2 region" or "CH2 domain" as used herein refers to the CH2 region of an immunoglobulin heavy chain. Thus, for example the CH2 region of a human IgG1 antibody corresponds to amino acids 231-340 according to the Eu numbering as set forth in Kabat *et al.* (1991) . However, the CH2 region may also be any of the other subtypes as described herein.

The term "CH3 region" or "CH3 domain" as used herein refers to the CH3 region of an immunoglobulin heavy chain. Thus for example the CH3 region of a human IgG1 antibody corresponds to amino acids 341-447 according to the Eu numbering as set forth in Kabat *et al.* (1991). However, the CH3 region may also be any of the other subtypes as described herein.

In another embodiment, the antibody is an effector-function-deficient antibody, a stabilized IgG4 antibody or a monovalent antibody.

In one particular embodiment, the heavy chain has been modified such that the entire hinge region has been deleted.

The sequence of the antibody may have been modified so that it does not comprise any acceptor sites for N-linked glycosylation.

The antibody may be a single-chain antibody.

The present disclosure further relates to a multispecific antibody comprising at least a first binding region of an antibody according to any aspect or embodiment herein described, and a second binding region which binds a different target or epitope than the first binding region. The term "multispecific antibody" as used herein, refers to antibodies wherein the binding regions bind to at least two, such as at least three, different antigens or at least two, such as at least three, different epitopes on the same antigen.

Tthe present disclosure relates to the use of an ADC comprising a bispecific antibody comprising a first binding region of an antibody according to any aspect or embodiments herein described, and a second binding region which binds a different target or epitope than the first binding region.

The term "bispecific" as used herein, refers to binding molecules, such as antibodies wherein the binding regions of the binding molecule bind to two different antigens or two different epitopes on the same antigen.

The term "bispecific antibody" refers to an antibody having specificities for at least two different, typically non-overlapping, epitopes. Such epitopes may be on the same or different targets. If the epitopes are on different targets, such targets may be on the same cell or different cells, cell types or structures, such as extracellular tissue.

The term "different target" as used herein, refers to another protein, molecule or the like than AXL or an AXL fragment.

Examples of bispecific antibody molecules which may be used in the present invention comprise (i) a single antibody that has two arms comprising different antigen-binding regions, (ii) a single chain antibody that has specificity to two different epitopes, *e.g*., via two scFvs linked in tandem by an extra peptide linker; (iii) a dual-variable-domain antibody (DVD-Ig™), where each light chain and heavy chain contains two variable domains in tandem through a short peptide linkage (Wu *et al.,* 2010); (iv) a chemically-linked bispecific (Fab')2 fragment; (v) a Tandab®, which is a fusion of two single chain diabodies resulting in a tetravalent bispecific antibody that has two binding sites for each of the target antigens; (vi) a flexibody, which is a combination of scFvs with a diabody resulting in a multivalent molecule; (vii) a so called "dock and lock" molecule (Dock-and-Lock®), based on the "dimerization and docking domain" in Protein Kinase A, which, when applied to Fabs, can yield a trivalent bispecific binding protein consisting of two identical Fab fragments linked to a different Fab fragment; (viii) a so-called Scorpion molecule, comprising, *e.g*., two scFvs fused to both termini of a human Fab-arm; and (ix) a diabody.

The bispecific antibody of the present disclosure may be a diabody, a cross-body, such as CrossMabs, or a bispecific antibody obtained via a controlled Fab arm exchange (such as described in WO 2011/131746, Genmab A/S).

Examples of different classes of bispecific antibodies include but are not limited to (i) IgG-like molecules with complementary CH3 domains to force heterodimerization; (ii) recombinant IgG-like dual targeting molecules, wherein the two sides of the molecule each contain the Fab fragment or part of the Fab fragment of at least two different antibodies; (iii) IgG fusion molecules, wherein full length IgG antibodies are fused to extra Fab fragment or parts of Fab fragment; (iv) Fc fusion molecules, wherein single chain Fv molecules or stabilized diabodies are fused to heavy-chain constant-domains, Fc-regions or parts thereof; (v) Fab fusion molecules, wherein different Fab-fragments are fused together, fused to heavy-chain constant-domains, Fc-regions or parts thereof; and (vi) ScFv-and diabody-based and heavy chain antibodies (e.g., domain antibodies, Nanobodies®) wherein different single chain Fv molecules or different diabodies or different heavy-chain antibodies (*e.g*. domain antibodies, Nanobodies®) are fused to each other or to another protein or carrier molecule fused to heavy-chain constant-domains, Fc-regions or parts thereof.

Examples of IgG-like molecules with complementary CH3 domains molecules include but are not limited to the Triomab® (Trion Pharma/Fresenius Biotech, WO/2002/020039), Knobs-into-Holes (Genentech, WO9850431), CrossMAbs (Roche, WO 2009/080251, WO 2009/080252, WO 2009/080253), electrostatically-matched Fc-heterodimeric molecules (Amgen, EP1870459 and WO2009089004; Chugai, US201000155133; Oncomed, WO2010129304), LUZ-Y (Genentech), DIG-body, PIG-body and TIG-body (Pharmabcine), Strand Exchange Engineered Domain body (SEEDbody) (EMD Serono, WO2007110205), Bispecific IgG1 and IgG2 (Pfizer/Rinat, WO11143545), Azymetric scaffold (Zymeworks/Merck, WO2012058768), mAb-Fv (Xencor, WO2011028952), XmAb (Xencor), Bivalent bispecific antibodies (Roche, WO2009/080254), Bispecific IgG (Eli Lilly), DuoBody® molecules (Genmab A/S, WO 2011/131746), DuetMab (Medimmune, US2014/0348839), Biclonics (Merus, WO 2013/157953), NovImmune (κλBodies, WO 2012/023053), FcΔAdp (Regeneron, WO 2010/151792), (DT)-Ig (GSK/Domantis), Two-in-one Antibody or Dual Action Fabs (Genentech, Adimab), mAb2 (F-Star, WO2008003116), Zybodies™ (Zyngenia), CovX-body (CovX/Pfizer), FynomAbs (Covagen/Janssen Cilag), DutaMab (Dutalys/Roche), iMab (MedImmune), Dual Variable Domain (DVD)-IgTM (Abbott, US 7,612,18), dual domain double head antibodies (Unilever; Sanofi Aventis, WO20100226923), Ts2Ab (MedImmune/AZ), BsAb (Zymogenetics), HERCULES (Biogen Idec, US007951918), scFv-fusions (Genentech/Roche, Novartis, Immunomedics, Changzhou Adam Biotech Inc, CN 102250246), TvAb (Roche, WO2012025525, WO2012025530), ScFv/Fc Fusions, SCORPION (Emergent BioSolutions/Trubion, Zymogenetics/BMS), Interceptor (Emergent), Dual Affinity Retargeting Technology (Fc-DARTTM) (MacroGenics, WO2008/157379, WO2010/080538), BEAT (Glenmark), Di-Diabody (Imclone/Eli Lilly) and chemically crosslinked mAbs (Karmanos Cancer Center), and covalently fused mAbs (AIMM therapeutics).

Examples of recombinant IgG-like dual targeting molecules include but are not limited to Dual Targeting (DT)-Ig (GSK/Domantis), Two-in-one Antibody (Genentech), Cross-linked Mabs (Karmanos Cancer Center), mAb2 (F-Star, WO2008003116), Zybodies™ (Zyngenia), approaches with common light chain (Crucell/Merus, US 7,262,028), κλBodies (NovImmune) and CovX-body (CovX/Pfizer).

Examples of IgG fusion molecules include but are not limited to Dual Variable Domain (DVD)-Ig™ (Abbott, US 7,612,181), Dual domain double head antibodies (Unilever; Sanofi Aventis, WO20100226923), IgG-like Bispecific (ImClone/Eli Lilly), Ts2Ab (MedImmune/AZ) and BsAb (Zymogenetics), HERCULES (Biogen Idec, US 7,951,918), scFv fusion (Novartis), scFv fusion (Changzhou Adam Biotech Inc, CN 102250246) and TvAb (Roche, WO2012025525,WO2012025530).

Examples of Fc fusion molecules include but are not limited to ScFv/Fc Fusions (Academic Institution), SCORPION (Emergent BioSolutions/Trubion, Zymogenetics/BMS), Dual Affinity Retargeting Technology (Fc-DART™) (MacroGenics, WO2008157379 and WO2010080538) and Dual(ScFv)2-Fab (National Research Center for Antibody Medicine - China).

Examples of Fab fusion bispecific antibodies include but are not limited to F(ab)2 (Medarex/AMGEN), Dual-Action or Bis-Fab (Genentech), Dock-and-Lock® (DNL) (ImmunoMedics), Bivalent Bispecific (Biotecnol) and Fab-Fv (UCB-Celltech).

Examples of ScFv-, diabody-based and domain antibodies include but are not limited to Bispecific T Cell Engager (BiTE®) (Micromet, Tandem Diabody (Tandab™) (Affimed), Dual Affinity Retargeting Technology (DART) (MacroGenics), Single-chain Diabody (Academic), TCR-like Antibodies (AIT, ReceptorLogics), Human Serum Albumin ScFv Fusion (Merrimack) and COMBODY (Epigen Biotech), dual targeting nanobodies® (Ablynx), dual targeting heavy chain only domain antibodies.

A bispecific antibody for use as an ADC according the present disclosure may be generated by introducing modifications in the constant region of the antibody.

The bispecific antibody may comprise a first and a second heavy chain, each of the first and second heavy chain comprises at least a hinge region, a CH2 and CH3 region, wherein in the first heavy chain at least one of the amino acids in the positions corresponding to positions selected from the group consisting of K409, T366, L368, K370, D399, F405, and Y407 in a human IgG1 heavy chain has been substituted, and in the second heavy chain at least one of the amino acids in the positions corresponding to a position selected from the group consisting of F405, T366, L368, K370, D399, Y407, and K409 in a human IgG1 heavy chain has been substituted, and wherein the first and the second heavy chains are not substituted in the same positions.

The antibody may be one, wherein in the first heavy chain the amino acid in the position corresponding to K409 in a human IgG1 heavy chain is not K, L or M and optionally the amino acid in the position corresponding to F405 in a human IgG1 heavy chain is F, and in the second heavy chain the amino acid in the position corresponding to F405 in a human IgG1 heavy chain is not F and the amino acid in the position corresponding to K409 in a human IgG1 heavy chain is K.

The antibody may be one, wherein in the first heavy chain, the amino acid in the position corresponding to F405 in a human IgG1 heavy chain is not F, R, and G, and in the second heavy chain the amino acids in the positions corresponding to a position selected from the group consisting of; T366, L368, K370, D399, Y407, and K409 in a human IgG1 heavy chain has been substituted.

The antibody may be one, wherein the amino acid in position corresponding to K409 in a human IgG1 heavy chain is another than K, L or M in the first heavy chain, and in the second heavy chain the amino acid in position corresponding to F405 in a human IgG1 heavy chain is not F and optionally the amino acid in the position corresponding to K409 in a human IgG1 heavy chain is K.

The antibody may be one, wherein the amino acid in the position corresponding to F405 in a human IgG1 heavy chain is L in said first heavy chain, and the amino acid in the position corresponding to K409 in a human IgG1 heavy chain is R in said second heavy chain, or vice versa.

Thus, the amino acid in the position corresponding to K409 in a human IgG1 heavy chain may be R in the first heavy chain, and the amino acid in the position corresponding to F405 in a human IgG1 heavy chain may be L in the second heavy chain.

Unless otherwise stated or contradicted by context, the amino acids of the constant region sequences are herein numbered according to the Eu-index of numbering (described in Kabat, 1991). The terms "Eu-index of numbering" and "Eu numbering as set forth in Kabat" may be used interchangeably and have the same meaning and purpose. Thus, an amino acid or segment in one sequence that "corresponds to" an amino acid or segment in another sequence is one that aligns with the other amino acid or segment using a standard sequence alignment program such as ALIGN, ClustalW or similar, typically at default settings and has at least 50%, at least 80%, at least 90%, or at least 95% identity to a human IgG1 heavy chain. It is well-known in the art how to align a sequence or segment in a sequence and thereby determine the corresponding position in a sequence to an amino acid position according to the present invention.

The term "amino acid corresponding to position" as used herein refers to an amino acid position number in a human IgG1 heavy chain.

The term "amino acid" and "amino acid residue" may herein be used interchangeably, and are not to be understood limiting.

In the context of the present disclosure, the amino acid may be defined by conservative or non-conservative amino acids, and may therefore be classified accordingly. Amino acid residues may also be divided into classes defined by alternative physical and functional properties. Thus, classes of amino acids may be reflected in one or both of the following lists:

**Amino acid residue of conservative class:**

| | |
|---|---|
| Acidic Residues: | D and E |
| Basic Residues: | K, R, and H |
| Hydrophilic Uncharged Residues: | S, T, N, and Q |
| Aliphatic Uncharged Residues: | G, A, V, L, and I |
| Non-polar Uncharged Residues: | C, M, and P |
| Aromatic Residues: | F, Y, and W |

**Alternative Physical and Functional Classifications of Amino Acid Residues:**

| | |
|---|---|
| Alcohol group-containing residues: | S and T |
| Aliphatic residues: | I, L, V, and M |
| Cycloalkenyl-associated residues: | F, H, W, and Y |
| Hydrophobic residues: | A, C, F, G, H, I, L, M, R, T, V, W, and Y |
| Negatively charged residues: | D and E |
| Polar residues: | C, D, E, H, K, N, Q, R, S, and T |
| Positively charged residues: | H, K, and R |
| Small residues: | A, C, D, G, N, P, S, T, and V |
| Very small residues: | A, G, and S |
| Residues involved in turn formation: | A, C, D, E, G, H, K, N, Q, R, S, P, and T |
| Flexible residues: | Q, T, K, S, G, P, D, E, and R |

In the context of the present invention, a substitution in an antibody is indicated as: Original amino acid - position - substituted amino acid;
Referring to the well-recognized nomenclature for amino acids, the three letter code, or one letter code, is used, including the codes "Xaa" or "X" to indicate any amino acid residue. Thus, Xaa or X may typically represent any of the 20 naturally occurring amino acids. The term "naturally occurring" as used herein refers to any one of the following amino acid residues; glycine, alanine, valine, leucine, isoleucine, serine, threonine, lysine, arginine, histidine, aspartic acid, asparagine, glutamic acid, glutamine, proline, tryptophan, phenylalanine, tyrosine, methionine, and cysteine. Accordingly, the notation "K409R" or "Lys409Arg" means, that the antibody comprises a substitution of Lysine with Arginine in amino acid position 409.

Substitution of an amino acid at a given position to any other amino acid is referred to as: Original amino acid - position; or *e.g*. "K409"
For a modification where the original amino acid(s) and/or substituted amino acid(s) may comprise more than one, but not all amino acid(s), the more than one amino acid may be separated by "," or "/". For example, the substitution of Lysine with Arginine, Alanine, or Phenylalanine in position 409 is:
"Lys409Arg,Ala,Phe" or "Lys409Arg/Ala/Phe" or "K409R,A,F" or "K409R/A/F" or "K409 to R, A, or F".

Such designation may be used interchangeably in the context of the invention but have the same meaning and purpose.

Furthermore, the term "a substitution" embraces a substitution into any one or the other nineteen natural amino acids, or into other amino acids, such as non-natural amino acids. For example, a substitution of amino acid K in position 409 includes each of the following substitutions: 409A, 409C, 409D, 409E, 409F, 409G, 409H, 4091, 409L, 409M, 409N, 409Q, 409R, 409S, 409T, 409V, 409W, 409P, and 409Y. This is, by the way, equivalent to the designation 409X, wherein the X designates any amino acid other than the original amino acid. These substitutions may also be designated K409A, K409C, etc. or K409A,C, etc. or K409A/C/etc. The same applies by analogy to each and every position mentioned herein, to specifically include herein any one of such substitutions.

The antibody according to the disclosure may also comprise a deletion of an amino acid residue. Such deletion may be denoted "del", and includes, e.g., writing as K409del. Thus, in such embodiments, the Lysine in position 409 has been deleted from the amino acid sequence.

Both the first and the second binding region of the bispecific antibody may bind AXL. However, the first binding region comprises a different set of CDR sequences than the second binding region. Thus the bispecific antibody may comprise a first and a second binding region, and a first and a second heavy chain, wherein the first and the second binding regions each comprise a VH and VL region selected from the group consisting of;
a) a first VH region comprising the CDR1, CDR2, and CDR3 sequences of SEQ ID Nos.: 36, 37, and 38, respectively; and a first VL region comprising the CDR1, CDR2, and CDR3 sequences of SEQ ID Nos.: 39, GAS, and 40, respectively, [**107**]; and a second VH region comprising the CDR1, CDR2, and CDR3 sequences of SEQ ID Nos.: 46, 47, and 48, respectively; and a second VL region comprising the CDR1, CDR2, and CDR3 sequences of SEQ ID Nos.: 49, AAS, and 50, respectively, [**148**];
b) a first VH region comprising the CDR1, CDR2, and CDR3 sequences of SEQ ID Nos.: 36, 37, and 38, respectively; and a first VL region comprising the CDR1, CDR2, and CDR3 sequences of SEQ ID Nos.: 39, GAS, and 40, respectively, [**107**]; and a second VH region comprising the CDR1, CDR2, and CDR3 sequences of SEQ ID Nos.: 114, 115, and 116, respectively, and a second VL region comprising the CDR1, CDR2, and CDR3 sequences of SEQ ID Nos.: 117, DAS, and 118, respectively [**733**];
c) a first VH region comprising the CDR1, CDR2, and CDR3 sequences of SEQ ID Nos.: 36, 37, and 38, respectively; and a first VL region comprising the CDR1, CDR2, and CDR3 sequences of SEQ ID Nos.: 39, GAS, and 40, respectively, [**107**]; and a second VH region comprising the CDR1, CDR2, and CDR3 sequences of SEQ ID Nos.: 41, 42, and 43, respectively; and a second VL region comprising the CDR1, CDR2, and CDR3 sequences of SEQ ID Nos.: 44, AAS, and 45, respectively, [**140**];
d) a first VH region comprising the CDR1, CDR2, and CDR3 sequences of SEQ ID Nos.: 36, 37, and 38, respectively; and a first VL region comprising the CDR1, CDR2, and CDR3 sequences of SEQ ID Nos.: 39, GAS, and 40, respectively, [**107**]; and a second VH region comprising the CDR1, CDR2, and CDR3 sequences of SEQ ID Nos.: 51, 52, and 55, respectively; and a second VL region comprising the CDR1, CDR2, and CDR3 sequences of SEQ ID Nos.: 55, GAS, and 56, respectively. [**154**];
e) a first VH region comprising the CDR1, CDR2, and CDR3 sequences of SEQ ID Nos.: 36, 37, and 38, respectively; and a first VL region comprising the CDR1, CDR2, and CDR3 sequences of SEQ ID Nos.: 39, GAS, and 40, respectively, [**107**]; and a second VH region comprising the CDR1, CDR2, and CDR3 sequences of SEQ ID Nos.: 51, 52, and 54, respectively; and a second VL region comprising the CDR1, CDR2, and CDR3 sequences of SEQ ID Nos.: 55, GAS, and 56, respectively. [**154-M103L**];
f) a first VH region comprising the CDR1, CDR2, and CDR3 sequences of SEQ ID Nos.: 36, 37, and 38, respectively; and a first VL region comprising the CDR1, CDR2, and CDR3 sequences of SEQ ID Nos.: 39, GAS, and 40, respectively, [**107**]; and a second VH region comprising the CDR1, CDR2, and CDR3 sequences of SEQ ID Nos.: 57, 58, and 59, respectively; and a second VL region comprising the CDR1, CDR2, and CDR3 sequences of SEQ ID Nos.: 60, GAS, and 61, respectively, [**171**];
g) a first VH region comprising the CDR1, CDR2, and CDR3 sequences of SEQ ID Nos.: 36, 37, and 38, respectively; and a first VL region comprising the CDR1, CDR2, and CDR3 sequences of SEQ ID Nos.: 39, GAS, and 40, respectively, [**107**]; and a second VH region comprising the CDR1, CDR2, and CDR3 sequences of SEQ ID Nos.: 62, 63, and 64, respectively; and a second VL region comprising the CDR1, CDR2, and CDR3 sequences of SEQ ID Nos.: 65, GAS, and 66, respectively, [**172**];
h) a first VH region comprising the CDR1, CDR2, and CDR3 sequences of SEQ ID Nos.: 36, 37, and 38, respectively; and a first VL region comprising the CDR1, CDR2, and CDR3 sequences of SEQ ID Nos.: 39, GAS, and 40, respectively, [**107**]; and a second VH region comprising the CDR1, CDR2, and CDR3 sequences of SEQ ID Nos.: 67, 68, and 69, respectively; and a second VL region comprising the CDR1, CDR2, and CDR3 sequences of SEQ ID Nos.: 70, GAS, and 71, respectively, [**181**];
i) a first VH region comprising the CDR1, CDR2, and CDR3 sequences of SEQ ID Nos.: 36, 37, and 38, respectively; and a first VL region comprising the CDR1, CDR2, and CDR3 sequences of SEQ ID Nos.: 39, GAS, and 40, respectively, [**107**]; and a second VH region comprising the CDR1, CDR2, and CDR3 sequences of SEQ ID Nos.: 72, 73, and 75, respectively; and a second VL region comprising the CDR1, CDR2, and CDR3 sequences of SEQ ID Nos.: 76, ATS, and 77, respectively, [**183**];
j) a first VH region comprising the CDR1, CDR2, and CDR3 sequences of SEQ ID Nos.: 36, 37, and 38, respectively; and a first VL region comprising the CDR1, CDR2, and CDR3 sequences of SEQ ID Nos.: 39, GAS, and 40, respectively, [**107**]; and a second VH region comprising the CDR1, CDR2, and CDR3 sequences of SEQ ID Nos.: 72, 74, and 75, respectively; and a second VL region comprising the CDR1, CDR2, and CDR3 sequences of SEQ ID Nos.: 76, ATS, and 77, respectively, [**183-N52Q**];
k) a first VH region comprising the CDR1, CDR2, and CDR3 sequences of SEQ ID Nos.: 36, 37, and 38, respectively; and a first VL region comprising the CDR1, CDR2, and CDR3 sequences of SEQ ID Nos.: 39, GAS, and 40, respectively, [**107**]; and a second VH region comprising the CDR1, CDR2, and CDR3 sequences of SEQ ID Nos.: 78, 79, and 80, respectively; and a second VL region comprising the CDR1, CDR2, and CDR3 sequences of SEQ ID Nos.: 81, AAS, and 82, respectively, [**187**];
I) a first VH region comprising the CDR1, CDR2, and CDR3 sequences of SEQ ID Nos.: 36, 37, and 38, respectively; and a first VL region comprising the CDR1, CDR2, and CDR3 sequences of SEQ ID Nos.: 39, GAS, and 40, respectively, [**107**]; and a second VH region comprising the CDR1, CDR2, and CDR3 sequences of SEQ ID Nos.: 83, 84, and 85, respectively; and a second VL region comprising the CDR1, CDR2, and CDR3 sequences of SEQ ID Nos.: 86, GAS, and 87, respectively, [**608-01**];
m) a first VH region comprising the CDR1, CDR2, and CDR3 sequences of SEQ ID Nos.: 36, 37, and 38, respectively; and a first VL region comprising the CDR1, CDR2, and CDR3 sequences of SEQ ID Nos.: 39, GAS, and 40, respectively, [**107**]; and a second VH region comprising the CDR1, CDR2, and CDR3 sequences of SEQ ID Nos.: 88, 89, and 90, respectively; and a second VL region comprising the CDR1, CDR2, and CDR3 sequences of SEQ ID Nos.: 91, GAS, and 92, respectively, **[610-01];**
n) a first VH region comprising the CDR1, CDR2, and CDR3 sequences of SEQ ID Nos.: 36, 37, and 38, respectively; and a first VL region comprising the CDR1, CDR2, and CDR3 sequences of SEQ ID Nos.: 39, GAS, and 40, respectively, [**107**]; and a second VH region comprising the CDR1, CDR2, and CDR3 sequences of SEQ ID Nos.: 94, 95, and 95, respectively; and a second VL region comprising the CDR1, CDR2, and CDR3 sequences of SEQ ID Nos.: 96, GAS, and 97, respectively, [**613**];
o) a first VH region comprising the CDR1, CDR2, and CDR3 sequences of SEQ ID Nos.: 36, 37, and 38, respectively; and a first VL region comprising the CDR1, CDR2, and CDR3 sequences of SEQ ID Nos.: 39, GAS, and 40, respectively, [**107**]; and a second VH region comprising the CDR1, CDR2, and CDR3 sequences of SEQ ID Nos.: 98, 99, and 100, respectively; and a second VL region comprising the CDR1, CDR2, and CDR3 sequences of SEQ ID Nos.: 101, DAS, and 102, respectively, **[613-08];**
p) a first VH region comprising the CDR1, CDR2, and CDR3 sequences of SEQ ID Nos.: 36, 37, and 38, respectively; and a first VL region comprising the CDR1, CDR2, and CDR3 sequences of SEQ ID Nos.: 39, GAS, and 40, respectively, [**107**]; and a second VH region comprising the CDR1, CDR2, and CDR3 sequences of SEQ ID Nos.: 103, 104, and 105, respectively; and a second VL region comprising the CDR1, CDR2, and CDR3 sequences of SEQ ID Nos.: 106, GAS, and 107, respectively, **[620-06];**
q) a first VH region comprising the CDR1, CDR2, and CDR3 sequences of SEQ ID Nos.: 36, 37, and 38, respectively; and a first VL region comprising the CDR1, CDR2, and CDR3 sequences of SEQ ID Nos.: 39, GAS, and 40, respectively, [**107**]; and a second VH region comprising the CDR1, CDR2, and CDR3 sequences of SEQ ID Nos.: 108, 109, and 110, respectively; and a second VL region comprising the CDR1, CDR2, and CDR3 sequences of SEQ ID Nos.: 112, AAS, and 113, respectively, [**726**];
r) a first VH region comprising the CDR1, CDR2, and CDR3 sequences of SEQ ID Nos.: 36, 37, and 38, respectively; and a first VL region comprising the CDR1, CDR2, and CDR3 sequences of SEQ ID Nos.: 39, GAS, and 40, respectively, [**107**]; and a second VH region comprising the CDR1, CDR2, and CDR3 sequences of SEQ ID Nos.: 108, 109, and 111, respectively; and a second VL region comprising the CDR1, CDR2, and CDR3 sequences of SEQ ID Nos.: 112, AAS, and 113, respectively, [**726-M101L**];
s) a first VH region comprising the CDR1, CDR2, and CDR3 sequences of SEQ ID Nos.: 46, 47, and 48, respectively; and a first VL region comprising the CDR1, CDR2, and CDR3 sequences of SEQ ID Nos.: 49, AAS, and 50, respectively, [**148**]; and a second VH region comprising the CDR1, CDR2, and CDR3 sequences of SEQ ID Nos.: 114, 115, and 116, respectively, and a second VL region comprising the CDR1, CDR2, and CDR3 sequences of SEQ ID Nos.: 117, DAS, and 118, respectively [**733**];
t) a first VH region comprising the CDR1, CDR2, and CDR3 sequences of SEQ ID Nos.: 46, 47, and 48, respectively; and a first VL region comprising the CDR1, CDR2, and CDR3 sequences of SEQ ID Nos.: 49, AAS, and 50, respectively, [**148**]; and a second VH region comprising the CDR1, CDR2, and CDR3 sequences of SEQ ID Nos.: 41, 42, and 43, respectively; and a second VL region comprising the CDR1, CDR2, and CDR3 sequences of SEQ ID Nos.: 44, AAS, and 45, respectively, [**107**];
u) a first VH region comprising the CDR1, CDR2, and CDR3 sequences of SEQ ID Nos.: 46, 47, and 48, respectively; and a first VL region comprising the CDR1, CDR2, and CDR3 sequences of SEQ ID Nos.: 49, AAS, and 50, respectively, [**148**]; and a second VH region comprising the CDR1, CDR2, and CDR3 sequences of SEQ ID Nos.: 51, 52, and 55, respectively; and a second VL region comprising the CDR1, CDR2, and CDR3 sequences of SEQ ID Nos.: 55, GAS, and 56, respectively. [**154**];
v) a first VH region comprising the CDR1, CDR2, and CDR3 sequences of SEQ ID Nos.: 46, 47, and 48, respectively; and a first VL region comprising the CDR1, CDR2, and CDR3 sequences of SEQ ID Nos.: 49, AAS, and 50, respectively, [**148**]; and a second VH region comprising the CDR1, CDR2, and CDR3 sequences of SEQ ID Nos.: 51, 52, and 54, respectively; and a second VL region comprising the CDR1, CDR2, and CDR3 sequences of SEQ ID Nos.: 55, GAS, and 56, respectively. **[154-M103L];**
w) a first VH region comprising the CDR1, CDR2, and CDR3 sequences of SEQ ID Nos.: 46, 47, and 48, respectively; and a first VL region comprising the CDR1, CDR2, and CDR3 sequences of SEQ ID Nos.: 49, AAS, and 50, respectively, [**148**]; and a second VH region comprising the CDR1, CDR2, and CDR3 sequences of SEQ ID Nos.: 57, 58, and 59, respectively; and a second VL region comprising the CDR1, CDR2, and CDR3 sequences of SEQ ID Nos.: 60, GAS, and 61, respectively, [**171**];
x) a first VH region comprising the CDR1, CDR2, and CDR3 sequences of SEQ ID Nos.: 46, 47, and 48, respectively; and a first VL region comprising the CDR1, CDR2, and CDR3 sequences of SEQ ID Nos.: 49, AAS, and 50, respectively, [**148**]; and a second VH region comprising the CDR1, CDR2, and CDR3 sequences of SEQ ID Nos.: 62, 63, and 64, respectively; and a second VL region comprising the CDR1, CDR2, and CDR3 sequences of SEQ ID Nos.: 65, GAS, and 66, respectively, [**172**];
y) a first VH region comprising the CDR1, CDR2, and CDR3 sequences of SEQ ID Nos.: 46, 47, and 48, respectively; and a first VL region comprising the CDR1, CDR2, and CDR3 sequences of SEQ ID Nos.: 49, AAS, and 50, respectively, [**148**]; and a second VH region comprising the CDR1, CDR2, and CDR3 sequences of SEQ ID Nos.: 67, 68, and 69, respectively; and a second VL region comprising the CDR1, CDR2, and CDR3 sequences of SEQ ID Nos.: 70, GAS, and 71, respectively, [**181**];
z) a first VH region comprising the CDR1, CDR2, and CDR3 sequences of SEQ ID Nos.: 46, 47, and 48, respectively; and a first VL region comprising the CDR1, CDR2, and CDR3 sequences of SEQ ID Nos.: 49, AAS, and 50, respectively, [**148**]; and a second VH region comprising the CDR1, CDR2, and CDR3 sequences of SEQ ID Nos.: 72, 73, and 75, respectively; and a second VL region comprising the CDR1, CDR2, and CDR3 sequences of SEQ ID Nos.: 76, ATS, and 77, respectively, [**183**];
aa) a first VH region comprising the CDR1, CDR2, and CDR3 sequences of SEQ ID Nos.: 46, 47, and 48, respectively; and a first VL region comprising the CDR1, CDR2, and CDR3 sequences of SEQ ID Nos.: 49, AAS, and 50, respectively, [**148**]; and a second VH region comprising the CDR1, CDR2, and CDR3 sequences of SEQ ID Nos.: 72, 74, and 75, respectively; and a second VL region comprising the CDR1, CDR2, and CDR3 sequences of SEQ ID Nos.: 76, ATS, and 77, respectively, [**183-N52Q**];
bb) a first VH region comprising the CDR1, CDR2, and CDR3 sequences of SEQ ID Nos.: 46, 47, and 48, respectively; and a first VL region comprising the CDR1, CDR2, and CDR3 sequences of SEQ ID Nos.: 49, AAS, and 50, respectively, [**148**]; and a second VH region comprising the CDR1, CDR2, and CDR3 sequences of SEQ ID Nos.: 78, 79, and 80, respectively; and a second VL region comprising the CDR1, CDR2, and CDR3 sequences of SEQ ID Nos.: 81, AAS, and 82, respectively, [**187**];
cc) a first VH region comprising the CDR1, CDR2, and CDR3 sequences of SEQ ID Nos.: 46, 47, and 48, respectively; and a first VL region comprising the CDR1, CDR2, and CDR3 sequences of SEQ ID Nos.: 49, AAS, and 50, respectively, [**148**]; and a second VH region comprising the CDR1, CDR2, and CDR3 sequences of SEQ ID Nos.: 83, 84, and 85, respectively; and a second VL region comprising the CDR1, CDR2, and CDR3 sequences of SEQ ID Nos.: 86, GAS, and 87, respectively, [**608-01**];
dd) a first VH region comprising the CDR1, CDR2, and CDR3 sequences of SEQ ID Nos.: 46, 47, and 48, respectively; and a first VL region comprising the CDR1, CDR2, and CDR3 sequences of SEQ ID Nos.: 49, AAS, and 50, respectively, [**148**]; and a second VH region comprising the CDR1, CDR2, and CDR3 sequences of SEQ ID Nos.: 88, 89, and 90, respectively; and a second VL region comprising the CDR1, CDR2, and CDR3 sequences of SEQ ID Nos.: 91, GAS, and 92, respectively, [**610-01**];
ee) a first VH region comprising the CDR1, CDR2, and CDR3 sequences of SEQ ID Nos.: 46, 47, and 48, respectively; and a first VL region comprising the CDR1, CDR2, and CDR3 sequences of SEQ ID Nos.: 49, AAS, and 50, respectively, [**148**]; and a second VH region comprising the CDR1, CDR2, and CDR3 sequences of SEQ ID Nos.: 94, 95, and 95, respectively; and a second VL region comprising the CDR1, CDR2, and CDR3 sequences of SEQ ID Nos.: 96, GAS, and 97, respectively, [**613**];
ff) a first VH region comprising the CDR1, CDR2, and CDR3 sequences of SEQ ID Nos.: 46, 47, and 48, respectively; and a first VL region comprising the CDR1, CDR2, and CDR3 sequences of SEQ ID Nos.: 49, AAS, and 50, respectively, [**148**]; and a second VH region comprising the CDR1, CDR2, and CDR3 sequences of SEQ ID Nos.: 98, 99, and 100, respectively; and a second VL region comprising the CDR1, CDR2, and CDR3 sequences of SEQ ID Nos.: 101, DAS, and 102, respectively, [**613-08**];
gg) a first VH region comprising the CDR1, CDR2, and CDR3 sequences of SEQ ID Nos.: 46, 47, and 48, respectively; and a first VL region comprising the CDR1, CDR2, and CDR3 sequences of SEQ ID Nos.: 49, AAS, and 50, respectively, [**148**]; and a second VH region comprising the CDR1, CDR2, and CDR3 sequences of SEQ ID Nos.: 103, 104, and 105, respectively; and a second VL region comprising the CDR1, CDR2, and CDR3 sequences of SEQ ID Nos.: 106, GAS, and 107, respectively, [**620-06**];
hh) a first VH region comprising the CDR1, CDR2, and CDR3 sequences of SEQ ID Nos.: 46, 47, and 48, respectively; and a first VL region comprising the CDR1, CDR2, and CDR3 sequences of SEQ ID Nos.: 49, AAS, and 50, respectively, [**148**]; and a second VH region comprising the CDR1, CDR2, and CDR3 sequences of SEQ ID Nos.: 108, 109, and 110, respectively; and a second VL region comprising the CDR1, CDR2, and CDR3 sequences of SEQ ID Nos.: 112, AAS, and 113, respectively, [**726**];
ii) a first VH region comprising the CDR1, CDR2, and CDR3 sequences of SEQ ID Nos.: 46, 47, and 48, respectively; and a first VL region comprising the CDR1, CDR2, and CDR3 sequences of SEQ ID Nos.: 49, AAS, and 50, respectively, [**148**]; and a second VH region comprising the CDR1, CDR2, and CDR3 sequences of SEQ ID Nos.: 108, 109, and 111, respectively; and a second VL region comprising the CDR1, CDR2, and CDR3 sequences of SEQ ID Nos.: 112, AAS, and 113, respectively, [**726-M101L**];
jj) a first VH region comprising the CDR1, CDR2, and CDR3 sequences of SEQ ID Nos.: 114, 115, and 116, respectively; and a first VL region comprising the CDR1, CDR2, and CDR3 sequences of SEQ ID Nos.: 117, DAS, and 118, respectively, [**733**]; and a second VH region comprising the CDR1, CDR2, and CDR3 sequences of SEQ ID Nos.: 41, 42, and 43, respectively; and a second VL region comprising the CDR1, CDR2, and CDR3 sequences of SEQ ID Nos.: 44, AAS, and 45, respectively, [**140**];
kk) a first VH region comprising the CDR1, CDR2, and CDR3 sequences of SEQ ID Nos.: 114, 115, and 116, respectively; and a first VL region comprising the CDR1, CDR2, and CDR3 sequences of SEQ ID Nos.: 117, DAS, and 118, respectively, [**733**]; and a second VH region comprising the CDR1, CDR2, and CDR3 sequences of SEQ ID Nos.: 51, 52, and 55, respectively; and a second VL region comprising the CDR1, CDR2, and CDR3 sequences of SEQ ID Nos.: 55, GAS, and 56, respectively. [**154**];
II) a first VH region comprising the CDR1, CDR2, and CDR3 sequences of SEQ ID Nos.: 114, 115, and 116, respectively; and a first VL region comprising the CDR1, CDR2, and CDR3 sequences of SEQ ID Nos.: 117, DAS, and 118, respectively, [**733**]; and a second VH region comprising the CDR1, CDR2, and CDR3 sequences of SEQ ID Nos.: 51, 52, and 54, respectively; and a second VL region comprising the CDR1, CDR2, and CDR3 sequences of SEQ ID Nos.: 55, GAS, and 56, respectively. [**154-M103L**];
mm) a first VH region comprising the CDR1, CDR2, and CDR3 sequences of SEQ ID Nos.: 114, 115, and 116, respectively; and a first VL region comprising the CDR1, CDR2, and CDR3 sequences of SEQ ID Nos.: 117, DAS, and 118, respectively, [**733**]; and a second VH region comprising the CDR1, CDR2, and CDR3 sequences of SEQ ID Nos.: 57, 58, and 59, respectively; and a second VL region comprising the CDR1, CDR2, and CDR3 sequences of SEQ ID Nos.: 60, GAS, and 61, respectively, [**171**];
nn) a first VH region comprising the CDR1, CDR2, and CDR3 sequences of SEQ ID Nos.: 114, 115, and 116, respectively; and a first VL region comprising the CDR1, CDR2, and CDR3 sequences of SEQ ID Nos.: 117, DAS, and 118, respectively, [**733**]; and a second VH region comprising the CDR1, CDR2, and CDR3 sequences of SEQ ID Nos.: 62, 63, and 64, respectively; and a second VL region comprising the CDR1, CDR2, and CDR3 sequences of SEQ ID Nos.: 65, GAS, and 66, respectively, [**172**];
oo) a first VH region comprising the CDR1, CDR2, and CDR3 sequences of SEQ ID Nos.: 114, 115, and 116, respectively; and a first VL region comprising the CDR1, CDR2, and CDR3 sequences of SEQ ID Nos.: 117, DAS, and 118, respectively, [**733**]; and a second VH region comprising the CDR1, CDR2, and CDR3 sequences of SEQ ID Nos.: 67, 68, and 69, respectively; and a second VL region comprising the CDR1, CDR2, and CDR3 sequences of SEQ ID Nos.: 70, GAS, and 71, respectively, [**181**];
pp) a first VH region comprising the CDR1, CDR2, and CDR3 sequences of SEQ ID Nos.: 114, 115, and 116, respectively; and a first VL region comprising the CDR1, CDR2, and CDR3 sequences of SEQ ID Nos.: 117, DAS, and 118, respectively, [**733**]; and a second VH region comprising the CDR1, CDR2, and CDR3 sequences of SEQ ID Nos.: 72, 73, and 75, respectively; and a second VL region comprising the CDR1, CDR2, and CDR3 sequences of SEQ ID Nos.: 76, ATS, and 77, respectively, [**183**];
qq) a first VH region comprising the CDR1, CDR2, and CDR3 sequences of SEQ ID Nos.: 114, 115, and 116, respectively; and a first VL region comprising the CDR1, CDR2, and CDR3 sequences of SEQ ID Nos.: 117, DAS, and 118, respectively, [**733**]; and a second VH region comprising the CDR1, CDR2, and CDR3 sequences of SEQ ID Nos.: 72, 74, and 75, respectively; and a second VL region comprising the CDR1, CDR2, and CDR3 sequences of SEQ ID Nos.: 76, ATS, and 77, respectively, [**183-N52Q**];
rr) a first VH region comprising the CDR1, CDR2, and CDR3 sequences of SEQ ID Nos.: 114, 115, and 116, respectively; and a first VL region comprising the CDR1, CDR2, and CDR3 sequences of SEQ ID Nos.: 117, DAS, and 118, respectively, [**733**]; and a second VH region comprising the CDR1, CDR2, and CDR3 sequences of SEQ ID Nos.: 78, 79, and 80, respectively; and a second VL region comprising the CDR1, CDR2, and CDR3 sequences of SEQ ID Nos.: 81, AAS, and 82, respectively, [**187**];
ss) a first VH region comprising the CDR1, CDR2, and CDR3 sequences of SEQ ID Nos.: 114, 115, and 116, respectively; and a first VL region comprising the CDR1, CDR2, and CDR3 sequences of SEQ ID Nos.: 117, DAS, and 118, respectively, [**733**]; and a second VH region comprising the CDR1, CDR2, and CDR3 sequences of SEQ ID Nos.: 83, 84, and 85, respectively; and a second VL region comprising the CDR1, CDR2, and CDR3 sequences of SEQ ID Nos.: 86, GAS, and 87, respectively, **[608-01];**
tt) a first VH region comprising the CDR1, CDR2, and CDR3 sequences of SEQ ID Nos.: 114, 115, and 116, respectively; and a first VL region comprising the CDR1, CDR2, and CDR3 sequences of SEQ ID Nos.: 117, DAS, and 118, respectively, [733]; and a second VH region comprising the CDR1, CDR2, and CDR3 sequences of SEQ ID Nos.: 88, 89, and 90, respectively; and a second VL region comprising the CDR1, CDR2, and CDR3 sequences of SEQ ID Nos.: 91, GAS, and 92, respectively, [**610-01**];
uu) a first VH region comprising the CDR1, CDR2, and CDR3 sequences of SEQ ID Nos.: 114, 115, and 116, respectively; and a first VL region comprising the CDR1, CDR2, and CDR3 sequences of SEQ ID Nos.: 117, DAS, and 118, respectively, [**733**]; and a second VH region comprising the CDR1, CDR2, and CDR3 sequences of SEQ ID Nos.: 94, 95, and 95, respectively; and a second VL region comprising the CDR1, CDR2, and CDR3 sequences of SEQ ID Nos.: 96, GAS, and 97, respectively, [**613**];
vv) a first VH region comprising the CDR1, CDR2, and CDR3 sequences of SEQ ID Nos.: 114, 115, and 116, respectively; and a first VL region comprising the CDR1, CDR2, and CDR3 sequences of SEQ ID Nos.: 117, DAS, and 118, respectively, [**733**]; and a second VH region comprising the CDR1, CDR2, and CDR3 sequences of SEQ ID Nos.: 98, 99, and 100, respectively; and a second VL region comprising the CDR1, CDR2, and CDR3 sequences of SEQ ID Nos.: 101, DAS, and 102, respectively, [**613-08**];
ww) a first VH region comprising the CDR1, CDR2, and CDR3 sequences of SEQ ID Nos.: 114, 115, and 116, respectively; and a first VL region comprising the CDR1, CDR2, and CDR3 sequences of SEQ ID Nos.: 117, DAS, and 118, respectively, [**733**]; and a second VH region comprising the CDR1, CDR2, and CDR3 sequences of SEQ ID Nos.: 103, 104, and 105, respectively; and a second VL region comprising the CDR1, CDR2, and CDR3 sequences of SEQ ID Nos.: 106, GAS, and 107, respectively, [**620-06**];
xx) a first VH region comprising the CDR1, CDR2, and CDR3 sequences of SEQ ID Nos.: 114, 115, and 116, respectively; and a first VL region comprising the CDR1, CDR2, and CDR3 sequences of SEQ ID Nos.: 117, DAS, and 118, respectively, [**733**]; and a second VH region comprising the CDR1, CDR2, and CDR3 sequences of SEQ ID Nos.: 108, 109, and 110, respectively; and a second VL region comprising the CDR1, CDR2, and CDR3 sequences of SEQ ID Nos.: 112, AAS, and 113, respectively, [**726**]; and
yy) a first VH region comprising the CDR1, CDR2, and CDR3 sequences of SEQ ID Nos.: 114, 115, and 116, respectively; and a first VL region comprising the CDR1, CDR2, and CDR3 sequences of SEQ ID Nos.: 117, DAS, and 118, respectively, [**733**]; and a second VH region comprising the CDR1, CDR2, and CDR3 sequences of SEQ ID Nos.: 108, 109, and 111, respectively; and a second VL region comprising the CDR1, CDR2, and CDR3 sequences of SEQ ID Nos.: 112, AAS, and 113, respectively, [**726-M101L**];

Antibodies conjugated to a cytotoxic agent, drug or the like are also known as antibody-drug conjugates (ADC). An ADC may have a half-life of sufficient periods of time for the antibody-drug conjugate to be internalized, degraded and induce cell killing by the released toxin.

Thus, an ADC can comprise an anti-AXL antibody or bispecific antibody and a therapeutic moiety, such as a cytotoxic agent, a chemotherapeutic drug, or the like. The cytotoxic agent, chemotherapeutic drug or the like may be conjugated to the antibody or the bispecific antibody via a linker.

ADCs are often designed such that the cytotoxic payload is inactive when conjugated to the antibody. The cytotoxic payload may be released intracellularly upon internalization of the ADC after binding to the plasma-membrane of cells, or alternatively in response to proteolytic activity in the tumor microenvironment. The term "internalized" or "internalization" as used herein, refers to a biological process in which molecules such as the AXL-ADC are engulfed by the cell membrane and drawn into the interior of the cell. It may also be referred to as "endocytosis".

Accordingly, in some instances it may be desired to use antibodies which undergo internalization. Such antibodies that have good internalization properties may be suited for conjugation to a cytotoxic agent, drug, or the like, optionally via a linker, which is designed to be cleaved intracellularly.

Once internalized, the ADC may be delivered to lysosomes in most cases, where effective drug release takes advantage of the catabolic environment found with these organelles. It is typically a linker that connects the antibody with a cytotoxic agent. Thus, specialized linkers have been designed to be cleaved only in a specific microenvironment found in or on the target tumor cell or in the tumor microenvironment. Examples include linkers that are cleaved by acidic conditions, reducing conditions, or specific proteases.

Stability of the antibody-linker-drug in circulation is important because this allows antibody-mediated delivery of the drug to specific target cells. In addition, the long circulating half-life of the ADC provides exposure for several days to weeks post injection. Drugs that are conjugated through non-cleavable linkers and protease-cleavable linkers are generally more stable in circulation than disulfide and hydrazone linkers, although the stability of the latter two linkers can be tuned by altering the neighboring chemical structure (Alley *et al.,* 2010).

The therapeutic moiety may be a cytotoxic agent. A cytotoxin or cytotoxic agent includes any agent that is detrimental to (*e.g*., kills) cells. Suitable cytotoxic agents for forming ADCs for use in the present invention include taxol, tubulysins, duostatins, cytochalasin B, gramicidin D, ethidium bromide, emetine, mitomycin, etoposide, tenoposide, vincristine, vinblastine, colchicin, doxorubicin, daunorubicin, dihydroxy anthracin dione, maytansine or an analog or derivative thereof, mitoxantrone, mithramycin, actinomycin D, 1-dehydrotestosterone, glucocorticoids, procaine, tetracaine, lidocaine, propranolol, and puromycin; calicheamicin or analogs or derivatives thereof; antimetabolites (such as methotrexate, 6-mercaptopurine, 6-thioguanine, cytarabine, fludarabin, 5-fluorouracil, decarbazine, hydroxyurea, asparaginase, gemcitabine, cladribine), alkylating agents (such as mechlorethamine, thioepa, chlorambucil, melphalan, carmustine (BSNU), lomustine (CCNU), cyclophosphamide, busulfan, dibromomannitol, streptozotocin, dacarbazine (DTIC), procarbazine, mitomycin C, cisplatin and other platinum derivatives, such as carboplatin; as well as duocarmycin A, duocarmycin SA, CC-1065 (a.k.a. rachelmycin), or analogs or derivatives of CC-1065), dolastatin, auristatin, pyrrolo[2,1-c][1,4] benzodiazepins (PDBs), indolinobenzodiazepine (IGNs) or analogues thereof, antibiotics (such as dactinomycin (formerly actinomycin), bleomycin, daunorubicin (formerly daunomycin), doxorubicin, idarubicin, mithramycin, mitomycin, mitoxantrone, plicamycin, anthramycin (AMC)), anti-mitotic agents (*e.g*., tubulin-targeting agents), such as diphtheria toxin and related molecules (such as diphtheria A chain and active fragments thereof and hybrid molecules); ricin toxin (such as ricin A or a deglycosylated ricin A chain toxin), cholera toxin, a Shiga-like toxin (SLT-I, SLT-II, SLT-IIV), LT toxin, C3 toxin, Shiga toxin, pertussis toxin, tetanus toxin, soybean Bowman-Birk protease inhibitor, Pseudomonas exotoxin, alorin, saporin, modeccin, gelanin, abrin A chain, modeccin A chain, alpha-sarcin, Aleurites fordii proteins, dianthin proteins, Phytolacca americana proteins (PAPI, PAPII, and PAP-S), momordica charantia inhibitor, curcin, crotin, sapaonaria officinalis inhibitor, gelonin, mitogellin, restrictocin, phenomycin, and enomycin toxins. Other suitable conjugated molecules include antimicrobial/lytic peptides such as CLIP, Magainin 2, mellitin, Cecropin, and P18; ribonuclease (RNase), DNase I, Staphylococcal enterotoxin-A, pokeweed antiviral protein, diphtherin toxin, and Pseudomonas endotoxin. See, for example, Pastan et al., Cell 47, 641 (1986) and Goldenberg, Calif. A Cancer Journal for Clinicians 44, 43 (1994). Therapeutic agents that may be administered in combination with anti-AXL antibodies or antibody-drug conjugates for use according to the present invention as described elsewhere herein, such as, e.g., anti-cancer cytokines or chemokines, are also candidates for therapeutic moieties useful for conjugation to an antibody for use according to the present invention.

The term "cytotoxic agent" as used herein, refers to any agent that is detrimental to (*e.g.,* kills) cells. For a description of these classes of drugs which are well known in the art, and their mechanisms of action, see Goodman *et al.* (1990). Additional techniques relevant to the preparation of antibody immunotoxins are provided in for instance Vitetta *et al.* (1993) and US 5,194,594.

In one embodiment, the cytotoxic agent is linked to said antibody, or fragment thereof, with a cleavable linker, such as *N*-succinimydyl 4-(2-pyridyldithio)-pentanoate (SSP), maleimidocaproyl-valine-citrulline-p-aminobenzyloxycarbonyl (mc-vc-PAB) or AV-1 K-lock valine-citrulline.

The term "cleavable linker" as used herein, refers to a subset of linkers that are catalyzed by specific proteases in the targeted cell or in the tumor microenvironment, resulting in release of the cytotoxic agent. Examples of cleavable linkers are linkers based on chemical motifs including disulfides, hydrazones or peptides. Another subset of cleavable linker, adds an extra linker motif between the cytotoxic agent and the primary linker, *i.e.* the site that attaches the linker-drug combination to the antibody. The extra linker motif may be cleavable by a cleavable agent that is present in the intracellular environment (e. g. within a lysosome or endosome or caveola). The linker can be, e. g. a peptidyl linker that is cleaved by an intracellular peptidase or protease enzyme, including but not limited to, a lysosomal or endosomal protease. In some embodiments, the peptidyl linker is at least two amino acids long or at least three amino acids long. Cleaving agents can include cathepsins B and D and plasmin, all of which are known to hydrolyze dipeptide drug derivatives resulting in the release of active drug inside the target cells (see e. g. Dubowchik and Walker, 1999, Pharm. Therapeutics 83:67-123). In a specific embodiment, the peptidyl linker cleavable by an intracellular protease is a Val-Cit (valine-citrulline) linker or a Phe-Lys (phenylalanine-lysine) linker (see *e.g*. US6214345, which describes the synthesis of doxorubicin with the Val-Cit linker). An advantage of using intracellular proteolytic release of the therapeutic agent is that the agent is typically attenuated when conjugated and the serum stabilities of the conjugates are typically high.

In another embodiment, the cytotoxic agent is linked to said antibody, or fragment thereof, with a non-cleavable linker, such as succinimidyl-4(*N*-maleimidomethyl)cyclohexane-1-carboxylate (MCC) or maleimidocaproyl (MC).

The term "noncleavable linker" as used herein, refers to a subset of linkers which, in contrast to cleavable linkers, do not comprise motifs that are specifically and predictably recognized by intracellular or extracellular proteases. Thus, ADCs based on non-cleavable linkers are not released or cleaved form the antibody until the complete antibody-linker-drug complex is degraded in the lysosomal compartment. Examples of a non-cleavable linker are thioethers. The linker unit may not be cleavable and the drug may be released by antibody degradation (see US 2005/0238649). Typically, such a linker is not substantially sensitive to the extracellular environment. As used herein, "not substantially sensitive to the extracellular environment" in the context of a linker means that no more than 20%, typically no more than about 15%, more typically no more than about 10%, and even more typically no more than about 5%, no more than about 3%, or no more than about 1% of the linkers, in a sample of antibody drug conjugate compound, are cleaved when the antibody drug conjugate compound is present in an extracellular environment (*e.g*. plasma). Whether a linker is not substantially sensitive to the extracellular environment can be determined for example by incubating with plasma the antibody drug conjugate compound for a predetermined time period (*e.g*. 2, 4, 8, 16 or 24 hours) and then quantitating the amount of free drug present in the plasma.

In one embodiment, cytotoxic agent is selected from the group: DNA-targeting agents, e.g. DNA alkylators and cross-linkers, such as calicheamicin, duocarmycin, rachelmycin (CC-1065), pyrrolo[2,1-c][1,4] benzodiazepines (PBDs), and indolinobenzodiazepine (IGN); microtubule-targeting agents, such as duostatin, such as duostatin-3, auristatin, such as monomethylauristatin E (MMAE) and monomethylauristatin F (MMAF), dolastatin, maytansine, *N*(2')-deacetyl-*N*(2')-(3-marcapto-1-oxopropyl)-maytansine (DM1), and tubulysin; and nucleoside analogs; or an analogs, derivatives, or prodrugs thereof.

In one embodiment, the AXL-ADC comprises a combination of;
i) a cleavable linker and a cytotoxic agent having bystander kill capacity;
ii) a cleavable linker and a cytotoxic agent not having bystander kill capacity;
iii) a non-cleavable linker and a cytotoxic agent having bystander kill capacity; or
iv) a non-cleavable linker and a cytotoxic agent not having bystander kill capacity.

The term "bystander killing effect", "bystander kill", "bystander kill capacity" or "bystander cytotoxicity" as used herein, refers to the effect where the cytotoxic agent that is conjugated to the antibody by either a cleavable or non-cleavable linker has the capacity to diffuse across cell membranes after the release from the antibody and thereby cause killing of neighboring cells. When the cytotoxic agent is conjugated by a cleavable or non-cleavable linker, it may be either the cytotoxic agent only or the cytotoxic agent with a part of the linker that has the bystander kill capacity. The capacity to diffuse across cell membranes is related to the hydrophobicity of the the cytotoxic agent or the combination of the cytotoxic agent and the linker. Such cytotoxic agents may advantageously be membrane-permeable toxins, such as MMAE that has been released from the antibody by proteases. Especially in tumors with heterogeneous target expression and in solid tumors where antibody penetration may be limited, a bystander killing effect may be desirable.

The term "no bystander kill capacity", "no bystander killing effect", "no-bystander kill" or "no bystander cytotoxicity" as used herein, refers to the effect where the cytotoxic agent that is conjugated to the antibody by either a cleavable or non-cleavable linker does not have the capacity to diffuse across cell membranes after release from the antibody. Thus, such cytotoxic agents or combinations of the cytotoxic agent with the linker, will not be able to kill neighboring cells upon release from the antibody. It is believed without being bound by theory, that such combinations of a cytotoxic agent and either a cleavable or non-cleavable linker will only kill cells expressing the target that the antibody binds.

A stable link between the antibody and cytotoxic agent is an important factor of an ADC. Both cleavable and non-cleavable types of linkers have been proven to be safe in preclinical and clinical trials.

The cytotoxic agent may be chosen from the group of microtubule targeting agents, such as auristatins and maytansinoids.

The term "microtubule-targeting agent" as used herein, refers to an agent or drug which inhibits mitosis (cell division). Microtubules are structures that are essential for proper separation of DNA during cell division, and microtubule function critically depends on 'dynamic instability', *i.e.* the process in which microtubule structures are continuously elongated and shortened. Microtubule-targeting agents disrupt or stabilize microtubules, which prevents formation of the mitotic spindle, resulting in mitotic arrest and apoptosis. The microtubule-targeting agents can be derived from *e.g*. natural substances such as plant alkaloids, and prevent cells from undergoing mitosis by disrupting or stabilizing microtubule polymerization, thus preventing formation of the mitotic spindle and subsequent cell division, resulting in inhibition of cancerous growth. Examples of microtubule-targeting agents are paclitaxel, docetaxel, vinblastine, vincristine, vinorelbine, duostatins, auristatins, maytansanoids, tubulysins, and dolastatin.

The cytotoxic agent is auristatins or auristatin peptide analogs and derivates (US 5,635,483;US 5,780,588). Auristatins have been shown to interfere with microtubule dynamics, GTP hydrolysis and nuclear and cellular division (Woyke *et al.,* 2001) and have anti-cancer (US 5,663,149) and anti-fungal activity (Pettit, 1998). The auristatin drug moiety may be attached to the antibody via a linker, through the N (amino) terminus or the C (terminus) of the peptidic drug moiety.

Exemplary auristatin embodiments include the N-terminus-linked monomethyl auristatin drug moieties D_{E} and D_{F}, disclosed in Senter *et al.* (2004) and described in US 2005/0238649.

The cytotoxic agent may in particular be monomethyl auristatin E (MMAE); 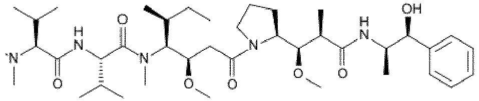 wherein the antibody is linked to MMAE at the nitrogen (N) on the left-hand side of the chemical structure above by the appropriate linker.

The cytotoxic agent monomethyl auristatin E (MMAE) may be linked to the antibody via a valine-citrulline (VC) linker.

The cytotoxic agent monomethyl auristatin E (MMAE) may be linked to the antibody via a valine-citrulline (VC) linker and the maleimidocaproyl (MC)linker, wherein the combination of the cytotoxic agent and the linkers has the chemical structure; 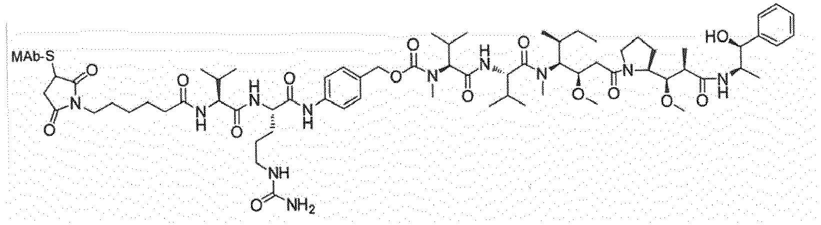 wherein MAb is the antibody.

The cytotoxic agent may be monomethyl auristatin F (MMAF); 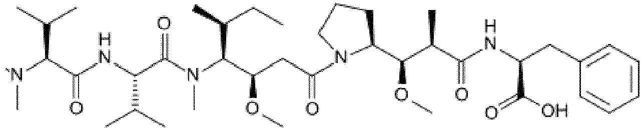 wherein the antibody is linked to MMAF at the nitrogen (N) on the left-hand side of the chemical structure above by the appropriate linker.

The cytotoxic agent monomethyl auristatin F (MMAF) may be linked to the antibody via a maleimidocaproyl (mc)-linker, wherein the combination of the cytotoxic agent and linker has the chemical structure; 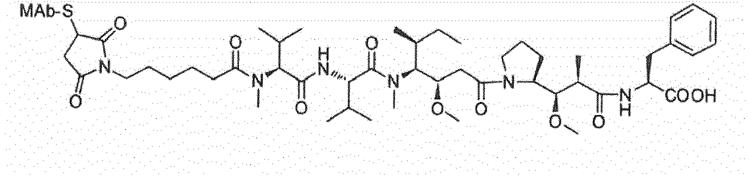 wherein MAb is the antibody.

The cytotoxic agent may be duostatin3.

The cytotoxic agent may be a DNA-targeting agent.

The term "DNA-targeting agent" as used herein, refers to a specific class of cytotoxic agents which are able to alkylate and/or cross-link DNA. An example of such a DNA-acting agent is IGN agents comprising indolino-benzodiazepinedimers and pyrrolo[2,1-c][1,4]benzodiazepines (PBDs) which are highly potent by virtue of their ability to alkylate and cross-link DNA. Another example is IGN agents comprising indolino-benzodiazepinemonomers which are highly potent by virtue of the ability to alkylate only DNA. Duocarmycins are another class of DNA-acting agents. Duocarmycins are small-molecule, synthetic DNA minor groove binding alkylating agents. These compounds are suitable to target solid tumors as well as hematological tumors.

The AXL-ADC comprises two to four cytotoxic molecules per antibody. Depending on the chemical properties of the toxin and the linker-toxin combination, two to four cytotoxic molecules per antibody may be superior to more heavily loaded conjugates that are cleared more rapidly from the circulation than less loaded conjugates. The cytotoxic agent loading is represented by p and is the average number of cytotoxic agent moieties per antibody in a molecule (also designated as the drug to antibody ratio, DAR). The cytotoxic agent loading may range from 1 to 20 drug moieties per antibody and may occur on amino acids with useful functional groups such as, but not limited to, amino or sulfhydryl groups, as in lysine or cysteine.

The number of cytotoxic agents per antibody may be from 1 to 8, such as 2 to 7, such as 2 to 6, such as 2 to 5, such as 2 to 4, and such as 2 to 3.

The AXL-ADC may comprise four to eight cytotoxic molecules per antibody. The AXL-ADC may comprise six to ten cytotoxic molecules per antibody. The AXL-ADC may comprise 10 to 30, such as 15 to 25, such as 20, cytotoxic molecules per antibody.

Depending on the way of conjugation, p may be limited by the number of attachment sites on the antibody, for example where the attachment is a cysteine thiol or a lysine. Generally, antibodies do not contain many free and reactive cysteine thiol groups which may be linked to a drug moiety as most cysteine thiol residues in antibodies exist as disulfide bridges. Therefore, where the cytotoxic agent is conjugated via a cysteine thiol, the antibody may be reduced with reducing agent such as dithiothreitol (DTT) or tricarbonylethylphosphine (TCEP), under partial or fully reducing conditions, to generate reactive cysteine thiol groups. In certain embodiments, the drug loading for an ADC of the invention ranges from 1 to about 8, as a maximum of 8 free cysteine thiol groups becomes available after (partial) reduction of the antibody (there are 8 cysteines involved in inter-chain disulfide bonding).

The drug linker moiety may be vcMMAE. The vcMMAE drug linker moiety and conjugation methods are disclosed in WO 2004/010957; US 7,659,241; US 7,829,531; and US 7,851,437 (Seattle Genetics). vcMMAE is formed by conjugation of the linker mc-vc-PAB and the cytotoxic moiety MMAE, and the vcMMAE drug linker moiety is bound to the anti-AXL antibodies at the cysteine residues using a method similar to those disclosed therein, *e.g*., as described in Example 8.

The drug linker moiety may be mcMMAF. The mcMMAF drug linker moiety and conjugation methods are disclosed in US 7,498,298; US 7,994,135 and WO 2005/081711 (Seattle Genetics), and the mcMMAF drug linker moiety is bound to the anti-AXL antibodies at the cysteine residues using a method similar to those disclosed therein.

The cytotoxic agent may be linked to 1 or 2 lysines within the antibody amino acid sequence by K-Lock™ conjugation as described in WO 2013/173391, WO 2013/173392 and WO 2013/173393 (Concortis Biosystems). Duostatin3 (also known as Duo3) may also be bound to the anti-AXL antibodies at the lysine residues using a method similar to those described therein.

Other linker technologies may be used in the anti-AXL antibody drug conjugates for the use of the invention, such as linkers comprising a hydroxyl group.

The linker may be attached to free cysteine residues of the anti-AXL antibody obtained by (partial) reduction of the anti-AXL antibody.

In a particular embodiment, the linker is mc-vc-PAB and the cytotoxic agent is MMAE. Alternatively, the linker SSP and the cytotoxic agent is DM1.

The linker may be MMC and the cytotoxic agent may be DM1; or the linker may be MC and the cytotoxic agent may be MMAF.

The linker may be the cleavable linker AV1-K lock and the cytotoxic agent may be duostatin3.

The AXL-ADC may comprise the linker mc-vc-PAB, the cytotoxic agent MMAE and an antibody wherein the at least one binding region comprises a VH region and a VL region selected from the group consisting of;

In one embodiment, the antibody comprises at least one binding region comprising a VH region and a VL region selected from the group consisting of:
(y) a VH region comprising the CDR1, CDR2, and CDR3 sequences of SEQ ID Nos.:36, 37, and 38, respectively; and a VL region comprising the CDR1, CDR2, and CDR3 sequences of SEQ ID Nos.:39, GAS, and 40, respectively, [**107**];
(z) a VH region comprising the CDR1, CDR2, and CDR3 sequences of SEQ ID Nos.:46, 47, and 48, respectively; and a VL region comprising the CDR1, CDR2, and CDR3 sequences of SEQ ID Nos.:49, AAS, and 50, respectively, [**148**];
(aa) a VH region comprising the CDR1, CDR2, and CDR3 sequences of SEQ ID Nos.:114, 115, and 116, respectively, and a VL region comprising the CDR1, CDR2, and CDR3 sequences of SEQ ID Nos.:117, DAS, and 118, respectively [**733**];
(bb) a VH region comprising the CDR1, CDR2, and CDR3 sequences of SEQ ID Nos.:51, 52, and 53, respectively; and a VL region comprising the CDR1, CDR2, and CDR3 sequences of SEQ ID Nos.:55, GAS, and 56, respectively [**154**];
(cc) a VH region comprising the CDR1, CDR2, and CDR3 sequences of SEQ ID Nos.:51, 52, and 54, respectively; and a VL region comprising the CDR1, CDR2, and CDR3 sequences of SEQ ID Nos.:55, GAS, and 56, respectively **[154-M103L];**
(dd) a VH region comprising the CDR1, CDR2, and CDR3 sequences of SEQ ID Nos.:57, 58, and 59, respectively; and a VL region comprising the CDR1, CDR2, and CDR3 sequences of SEQ ID Nos.:60, GAS, and 61, respectively, [**171**];
(ee) a VH region comprising the CDR1, CDR2, and CDR3 sequences of SEQ ID Nos.:62, 63, and 64, respectively; and a VL region comprising the CDR1, CDR2, and CDR3 sequences of SEQ ID Nos.:65, GAS, and 66, respectively, [**172**];
(ff) a VH region comprising the CDR1, CDR2, and CDR3 sequences of SEQ ID Nos.:67, 68, and 69, respectively; and a VL region comprising the CDR1, CDR2, and CDR3 sequences of SEQ ID Nos.:70, GAS, and 71, respectively, [**181**];
(gg) a VH region comprising the CDR1, CDR2, and CDR3 sequences of SEQ ID Nos.:72, 73, and 75, respectively; and a VL region comprising the CDR1, CDR2, and CDR3 sequences of SEQ ID Nos.:76, ATS, and 77, respectively, [**183**];
(hh) a VH region comprising the CDR1, CDR2, and CDR3 sequences of SEQ ID Nos.:72, 74, and 75, respectively; and a VL region comprising the CDR1, CDR2, and CDR3 sequences of SEQ ID Nos.:76, ATS, and 77, respectively, [**183-N52Q**];
(ii) a VH region comprising the CDR1, CDR2, and CDR3 sequences of SEQ ID Nos.:78, 79, and 80, respectively; and a VL region comprising the CDR1, CDR2, and CDR3 sequences of SEQ ID Nos.:81, AAS, and 82, respectively, [**187**];
(jj) a VH region comprising the CDR1, CDR2, and CDR3 sequences of SEQ ID Nos.:83, 84, and 85, respectively; and a VL region comprising the CDR1, CDR2, and CDR3 sequences of SEQ ID Nos.:86, GAS, and 87, respectively, [**608-01**];
(kk) a VH region comprising the CDR1, CDR2, and CDR3 sequences of SEQ ID Nos.:88, 89, and 90, respectively; and a VL region comprising the CDR1, CDR2, and CDR3 sequences of SEQ ID Nos.:91, GAS, and 92, respectively, [**610-01**];
(II) a VH region comprising the CDR1, CDR2, and CDR3 sequences of SEQ ID Nos.:93, 94, and 95, respectively; and a VL region comprising the CDR1, CDR2, and CDR3 sequences of SEQ ID Nos.:96, GAS, and 97, respectively, [**613**];
(mm) a VH region comprising the CDR1, CDR2, and CDR3 sequences of SEQ ID Nos.:98, 99, and 100, respectively; and a VL region comprising the CDR1, CDR2, and CDR3 sequences of SEQ ID Nos.:101, DAS, and 102, respectively, [**613-08**];
(nn) a VH region comprising the CDR1, CDR2, and CDR3 sequences of SEQ ID Nos.:103, 104, and 105, respectively; and a VL region comprising the CDR1, CDR2, and CDR3 sequences of SEQ ID Nos.:106, GAS, and 107, respectively, [**620-06**];
(oo) a VH region comprising the CDR1, CDR2, and CDR3 sequences of SEQ ID Nos.:108, 109, and 110, respectively; and a VL region comprising the CDR1, CDR2, and CDR3 sequences of SEQ ID Nos.:112, AAS, and 113, respectively, [**726**];
(pp) a VH region comprising the CDR1, CDR2, and CDR3 sequences of SEQ ID Nos.:108, 109, and 111, respectively; and a VL region comprising the CDR1, CDR2, and CDR3 sequences of SEQ ID Nos.:112, AAS, and 113, respectively, [**726-M101L**];
(qq) a VH region comprising the CDR1, CDR2, and CDR3 sequences of SEQ ID Nos.:41, 42, and 43, respectively; and a VL region comprising the CDR1, CDR2, and CDR3 sequences of SEQ ID Nos.:44, AAS, and 45, respectively, [1**40**];
(rr) a VH region comprising the CDR1, CDR2, and CDR3 sequences of SEQ ID Nos.:93, 94, and 95, respectively, and a VL region comprising the CDR1, CDR2, and CDR3 sequences of SEQ ID Nos.:128, XAS, wherein X is D or G, and 129, respectively, [**613 / 613-08**];
(ss) a VH region comprising the CDR1, CDR2, and CDR3 sequences of SEQ ID Nos.:46, 119, and 120, respectively; and a VL region comprising CDR1, CDR2, and CDR3 sequences of SEQ ID Nos.:49, AAS, and 50, respectively, [**148 / 140**];
(tt) a VH region comprising the CDR1, CDR2, and CDR3 sequences of SEQ ID Nos.:123, 124, and 125, respectively; and a VL region comprising CDR1, CDR2, and CDR3 sequences of SEQ ID Nos.:60, GAS, and 61, respectively [**171** / **172** / **181**]; and
(uu) a VH region comprising the CDR1, CDR2, and CDR3 sequences of SEQ ID Nos.:121, 109, and 122, respectively; and a VL region comprising the CDR1, CDR2, and CDR3 sequences of SEQ ID Nos.:112, AAS, and 113, respectively [**726 / 187**]; and
(vv) a VH region comprising the CDR1, CDR2, and CDR3 sequences of SEQ ID Nos.:93, 126, and 127, respectively; and a VL region comprising the CDR1, CDR2, and CDR3 sequences of SEQ ID Nos.:96, GAS, and 97, respectively [**613** / **608-01** / **610-01** / **620-06**].

An anti-AXL antibody drug conjugate may comprise a conjugated nucleic acid or nucleic acid-associated molecule. The conjugated nucleic acid may be a cytotoxic ribonuclease, an antisense nucleic acid, an inhibitory RNA molecule (*e.g*., a siRNA molecule) or an immunostimulatory nucleic acid (*e.g.,* an immunostimulatory CpG motif-containing DNA molecule).

An anti-AXL antibody may be conjugated to an aptamer or a ribozyme or a functional peptide analog or derivate thereof.

Anti-AXL antibody drug conjugates comprising one or more radiolabeled amino acids are provided. A radiolabeled anti-AXL antibody may be used for both diagnostic and therapeutic purposes (conjugation to radiolabeled molecules is another possible feature). Non-limiting examples of labels for polypeptides include ³H, ¹⁴C, ¹⁵N, ³⁵S, ⁹⁰Y, ⁹⁹Tc, and ¹²⁵I, ¹³¹I, and ¹⁸⁶Re. Methods for preparing radiolabeled amino acids and related peptide derivatives are known in the art (see for instance Junghans *et al.* (1996); US 4,681,581; US 4,735,210; US 5,101,827; US 5,102,990; US 5,648,471; and US 5,697,902. For example, a halogen radioisotope may be conjugated by a chloramine T method.

The antibody may be conjugated to a radioisotope or to a radioisotope-containing chelate. For example, the antibody can be conjugated to a chelator linker, *e.g*. DOTA, DTPA or tiuxetan, which allows for the antibody to be complexed with a radioisotope. The antibody may also or alternatively comprise or be conjugated to one or more radiolabeled amino acids or other radiolabeled molecules. A radiolabeled anti-AXL antibody may be used for both diagnostic and therapeutic purposes. Non-limiting examples of radioisotopes include ³H, ¹⁴C, ¹⁵N, ³⁵S, ⁹⁰Y, ⁹⁹Tc, ¹²⁵I, ¹¹¹In, ¹³¹I, ¹⁸⁶Re, ²¹³Bs, ²²⁵Ac and ²²⁷Th.

Anti-AXL antibodies may also be chemically modified by covalent conjugation to a polymer to for instance increase their circulating half-life. Exemplary polymers, and methods to attach them to peptides, are illustrated in for instance US 4,766,106; US 4,179,337; US 4,495,285 and US 4,609,546. Additional polymers include polyoxyethylated polyols and polyethylene glycol (PEG) *(*e*.g.,* a PEG with a molecular weight of between about 1,000 and about 40,000, such as between about 2,000 and about 20,000). This may for example be used if the anti-AXL antibody is a fragment.

Any method known in the art for conjugating the anti-AXL antibody to the conjugated molecule(s), such as those described above, may be employed, including the methods described by Hunter *et al.* (1974), Pain *et al.* (1981) and Nygren (1982). Such antibodies may be produced by chemically conjugating the other moiety to the N-terminal side or C-terminal side of the anti-AXL antibody (*e.g.,* an anti-AXL antibody H or L chain) (see, *e.g.,* Kanemitsu, 1994). Such conjugated antibody derivatives may also be generated by conjugation at internal residues or sugars, or non-naturally occurring amino acids or additional amino acids that have been introduced into the antibody constant domain, where appropriate.

The agents may be coupled either directly or indirectly to an anti-AXL antibody. One example of indirect coupling of a second agent is coupling via a spacer moiety to cysteine or lysine residues in the antibody. In one embodiment, an anti-AXL antibody is conjugated, via a spacer or linker, to a prodrug molecule that can be activated *in vivo* to a therapeutic drug. After administration, the spacers or linkers are cleaved by tumor cell-associated enzymes or other tumor-specific conditions, by which the active drug is formed. Examples of such pro-drug technologies and linkers are described in WO 2002/083180, WO 2004/043493, WO 2007/018431, WO 2007/089149, WO 2009/017394 and WO 2010/62171 (Syngenta BV). Suitable antibody-pro-drug technology and duocarmycin analogs can also be found in US 6,989,452 (Medarex).

The anti-AXL antibody may be attached to a chelator linker, e.g. tiuxetan, which allows for the antibody to be conjugated to a radioisotope.

### Combinations, compositions and kits

The AXL-ADC for use according to the present disclosure can be administered in the form of a composition.The composition may be a pharmaceutical composition comprising the AXL-ADC and a pharmaceutical carrier.

The AXL-ADC or pharmaceutical composition comprising the AXL-ADC may be for use in treating a neoplasm in combination with the at least one therapeutic agent with which the neoplasm is being or has been treated, *i.e.,* the chemotherapeutic agent, tyrosine kinase inhibitor, PI3K inhibitor, mAb/rTKI and/or serine/threonine kinase inhibitor according to any preceding aspect or embodiment. For example, the therapeutic agent may be a chemotherapeutic agent, a TKI or a S/Th TKI according to any preceding aspect or embodiment. Typically, the AXL-ADC and the therapeutic agent are separately administered.

The pharmaceutical composition comprising the AXL-ADC may further comprise the at least one therapeutic agent with which the neoplasm is being or has been treated, *i.e.,* the chemotherapeutic agent, tyrosine kinase inhibitor, PI3K inhibitor, mAb/rTKI and/or serine/threonine kinase inhibitor according to any preceding aspect or embodiment. For example, the therapeutic agent may be a chemotherapeutic agent, a TKI or a S/Th TKI according to any preceding aspect or embodiment. The AXL-ADCs for use according to the present invention in combination with the at least one therapeutic agent can be also be provided in the form of a kit, for simultaneous, separate or sequential administration, wherein the kit may further comprise instructions for use. The ADC and the at least one therapeutic agent are typically formulated as separate pharmaceutical compositions.

The tyrosine kinase inhibitor in the combination, composition or kit may be an EGFR antagonist.

The tyrosine kinase inhibitor in the combination, composition or kit may be selected from the group consisting of erlotinib, gefitinib, lapatinib, imatinib, sunitinib, crizotinib, midostaurin (PKC412) and quizartinib (AC220), such as , e.g., erlotinib or an analog or derivative thereof such as lapatinib, gefitinib or. Preferably, the tyrosine kinase inhibitor is erlotinib.

The serine/threonine kinase inhibitor in the combination, composition or kit may be selected from vemurafenib, dabrafenib, selumetinib (AZD6244), VTX11E, trametinib and PLX4720.

The BRAF inhibitor in the combination, composition or kit may bes vemurafenib (PLX4032) or a therapeutically effective analog or derivative thereof, such as dabrafenib or PLX4720. The BRAF inhibitor may be vemurafenib. In one embodiment, the BRAF-inhibitor may be dabrafenib.

The serine/threonine kinase inhibitor in the combination, composition or kit may comprise at least one BRAF-inhibitor and at least one MEK-inhibitor, wherein the at least one BRAF-inhibitor is selected from vemurafenib, dabrafenib and a combination thereof, and wherein the MEK-inhibitor is selected from selumetinib (AZD6244) and trametinib, and a combination thereof. For example, the combination, composition or kit may comprise dabrafenib and trametinib; vemurafenib and trametinib; dabrafenib, vemurafenib and trametinib; dabrafenib and selumetinib; or vemurafenib and selumetinib.

The at least one chemotherapeutic agent in the combination, composition or kit may be a taxane, for example selected from paclitaxel and docetaxel.

The at least one chemotherapeutic agent in the combination, composition or kit may be selected from the group consisting of cisplatin, carboplatin, doxorubicin, etoposide and metformin.

The PI3K inhibitor in the combination, composition or kit may be alpelisib (BYL719).

The mAb/rTKiin the combination, composition or kit may be Cetuximab or MAB391.

The kits can further include, if desired, one or more of various conventional pharmaceutical kit components, such as, for example, containers with one or more pharmaceutically acceptable carriers, additional containers, etc., as will be readily apparent to those skilled in the art. Printed instructions, either as inserts or as labels, indicating quantities of the components to be administered, guidelines for administration, and/or guidelines for mixing the components, can also be included in the kit.

The pharmaceutical compositions may be formulated with pharmaceutically acceptable carriers or diluents as well as any other known adjuvants and excipients in accordance with conventional techniques such as those disclosed in Remington: The Science and Practice of Pharmacy (1995).

The pharmaceutically acceptable carriers or diluents as well as any other known adjuvants and excipients should be suitable for the AXL-ADC and the chosen mode of administration. Suitability for carriers and other components of pharmaceutical compositions is determined based on the lack of significant negative impact on the desired biological properties of the chosen compound or pharmaceutical composition (*e.g*., less than a substantial impact (10% or less relative inhibition, 5% or less relative inhibition, etc.) upon antigen binding).

A pharmaceutical composition may also include diluents, fillers, salts, buffers, detergents (e. g., a nonionic detergent, such as Tween-20 or Tween-80), stabilizers (*e.g*., sugars or protein-free amino acids), preservatives, tissue fixatives, solubilizers, and/or other materials suitable for inclusion in a pharmaceutical composition.

The actual dosage levels of the active ingredients in the pharmaceutical compositions may be varied so as to obtain an amount of the active ingredient which is effective to achieve the desired therapeutic response for a particular patient, composition, and mode of administration, without being toxic to the patient. The selected dosage level will depend upon a variety of pharmacokinetic factors including the activity of the particular compositions, the route of administration, the time of administration, the rate of excretion of the particular compound being employed, the duration of the treatment, other drugs, compounds and/or materials used in combination with the particular compositions employed, the age, sex, weight, condition, general health and prior medical history of the patient being treated, and like factors well known in the medical arts.

The pharmaceutical composition may be administered by any suitable route and mode. Suitable routes of administering a compound of the present invention *in vivo* and *in vitro* are well known in the art and may be selected by those of ordinary skill in the art.

The pharmaceutical composition may be administered parenterally.

The terms "parenteral administration" and "administered parenterally" as used herein refers to modes of administration other than enteral and topical administration, usually by injection, and include epidermal, intravenous, intramuscular, intra-arterial, intrathecal, intracapsular, intra-orbital, intracardiac, intradermal, intraperitoneal, intratendinous, transtracheal, subcutaneous, subcuticular, intra-articular, subcapsular, subarachnoid, intraspinal, intracranial, intrathoracic, epidural and intrasternal injection and infusion.

The pharmaceutical composition may be administered by intravenous or subcutaneous injection or infusion.

Pharmaceutically acceptable carriers include any and all suitable solvents, dispersion media, coatings, antibacterial and antifungal agents, isotonicity agents, antioxidants and absorption-delaying agents, and the like that are physiologically compatible with an AXL-ADC or therapeutic agent for the use according to the present invention.

Examples of suitable aqueous and non-aqueous carriers which may be employed in the pharmaceutical compositions include water, saline, phosphate-buffered saline, ethanol, dextrose, polyols (such as glycerol, propylene glycol, polyethylene glycol, and the like), and suitable mixtures thereof, vegetable oils, such as olive oil, corn oil, peanut oil, cottonseed oil, and sesame oil, carboxymethyl cellulose colloidal solutions, tragacanth gum and injectable organic esters, such as ethyl oleate, and/or various buffers. Other carriers are well known in the pharmaceutical arts.

Pharmaceutically acceptable carriers include sterile aqueous solutions or dispersions and sterile powders for the extemporaneous preparation of sterile injectable solutions or dispersion. The use of such media and agents for pharmaceutically active substances is known in the art. Except insofar as any conventional media or agent is incompatible with the active compound, use thereof in the pharmaceutical compositions is contemplated.

Proper fluidity may be maintained, for example, by the use of coating materials, such as lecithin, by the maintenance of the required particle size in the case of dispersions, and by the use of surfactants.

Pharmaceutical compositions may also comprise pharmaceutically acceptable antioxidants for instance (1) water-soluble antioxidants, such as ascorbic acid, cysteine hydrochloride, sodium bisulfate, sodium metabisulfite, sodium sulfite and the like; (2) oil-soluble antioxidants, such as ascorbyl palmitate, butylated hydroxyanisole (BHA), butylated hydroxytoluene (BHT), lecithin, propyl gallate, alpha-tocopherol, and the like; and (3) metal-chelating agents, such as citric acid, ethylenediamine tetraacetic acid (EDTA), sorbitol, tartaric acid, phosphoric acid, and the like.

Pharmaceutical compositions may also comprise isotonicity agents, such as sugars, polyalcohols, such as mannitol, sorbitol, glycerol or sodium chloride in the compositions.

The pharmaceutical compositions may also contain one or more adjuvants appropriate for the chosen route of administration such as preservatives, wetting agents, emulsifying agents, dispersing agents, preservatives or buffers, which may enhance the shelf life or effectiveness of the pharmaceutical composition. The AXL-ADCs or therapeutic agents for the uses of the present invention may be prepared with carriers that will protect the compound against rapid release, such as a controlled release formulation, including implants, transdermal patches, and micro-encapsulated delivery systems. Such carriers may include gelatin, glyceryl monostearate, glyceryl distearate, biodegradable, biocompatible polymers such as ethylene vinyl acetate, polyanhydrides, polyglycolic acid, collagen, poly-ortho-esters, and polylactic acid alone or with a wax, or other materials well known in the art. Methods for the preparation of such formulations are generally known to those skilled in the art. See e.g., Robinbson: Sustained and Controlled Release Drug Delivery Systems (1978).

The compounds may be formulated to ensure proper distribution *in vivo.* Pharmaceutically acceptable carriers for parenteral administration include sterile aqueous solutions or dispersions and sterile powders for the extemporaneous preparation of sterile injectable solutions or dispersion. The use of such media and agents for pharmaceutically active substances is known in the art. Except insofar as any conventional media or agent is incompatible with the active compound, use thereof in the pharmaceutical compositions is contemplated. Other active or therapeutic compounds may also be incorporated into the compositions.

Pharmaceutical compositions for injection must typically be sterile and stable under the conditions of manufacture and storage. The composition may be formulated as a solution, micro-emulsion, liposome, or other ordered structure suitable to high drug concentration. The carrier may be an aqueous or a non-aqueous solvent or dispersion medium containing for instance water, ethanol, polyols (such as glycerol, propylene glycol, polyethylene glycol, and the like), and suitable mixtures thereof, vegetable oils, such as olive oil, and injectable organic esters, such as ethyl oleate. The proper fluidity may be maintained, for example, by the use of a coating such as lecithin, by the maintenance of the required particle size in the case of dispersion and by the use of surfactants. In many cases, it will be preferable to include isotonic agents, for example, sugars, polyalcohols such as glycerol, mannitol, sorbitol, or sodium chloride in the composition. Prolonged absorption of the injectable compositions may be brought about by including in the composition an agent that delays absorption, for example, monostearate salts and gelatin. Sterile injectable solutions may be prepared by incorporating the active compound in the required amount in an appropriate solvent with one or a combination of ingredients *e.g*. as enumerated above, as required, followed by sterilization microfiltration. Generally, dispersions are prepared by incorporating the active compound into a sterile vehicle that contains a basic dispersion medium and the required other ingredients *e.g*. from those enumerated above. In the case of sterile powders for the preparation of sterile injectable solutions, examples of methods of preparation are vacuum-drying and freeze-drying (lyophilization) that yield a powder of the active ingredient plus any additional desired ingredient from a previously sterile-filtered solution thereof.

Sterile injectable solutions may be prepared by incorporating the active compound in the required amount in an appropriate solvent with one or a combination of ingredients enumerated above, as required, followed by sterilization microfiltration. Generally, dispersions are prepared by incorporating the active compound into a sterile vehicle that contains a basic dispersion medium and the required other ingredients from those enumerated above. In the case of sterile powders for the preparation of sterile injectable solutions, examples of methods of preparation are vacuum-drying and freeze-drying (lyophilization) that yield a powder of the active ingredient plus any additional desired ingredient from a previously sterile-filtered solution thereof.

### Production of anti-AXL antibodies

The antibodies for use as ADCs according to the invention can be prepared recombinantly in a host cell, using nucleic acid constructs, typically in the form of one or more expression vectors. In one embodiment, the nucleic acid construct encodes one or more sequences set out in Table 1. In a further embodiment, the expression vector further comprises a nucleic acid sequence encoding the constant region of a light chain, a heavy chain or both light and heavy chains of an antibody, *e.g*. a human IgG1, κ monoclonal antibody.

The expressed anti-AXL antibody may subsequently be conjugated to a moiety as described herein. In another embodiment the anti-AXL antibody may subsequently be used to generate a bispecific antibody as described herein, before conjugation.

The expression vector may be any suitable vector, including chromosomal, non-chromosomal, and synthetic nucleic acid vectors (a nucleic acid sequence comprising a suitable set of expression control elements). Examples of such vectors include derivatives of SV40, bacterial plasmids, phage DNA, baculovirus, yeast plasmids, vectors derived from combinations of plasmids and phage DNA, and viral nucleic acid (RNA or DNA) vectors. An anti-AXL antibody-encoding nucleic acid may be comprised in a naked DNA or RNA vector, including, for example, a linear expression element (as described in for instance Sykes and Johnson (1997), a compacted nucleic acid vector (as described in for instance US 6,077,835 and/or WO 00/70087), a plasmid vector such as pBR322, pUC 19/18, or pUC 118/119, a "midge" minimally-sized nucleic acid vector (as described in for instance Schakowski *et al.* (2001)), or as a precipitated nucleic acid vector construct, such as a calcium phosphate-precipitated construct (as described in for instance WO 00/46147; Benvenisty and Reshef, 1986; Wigler *et al.,* 1978; and Coraro and Pearson, 1981). Such nucleic acid vectors and the usage thereof are well known in the art (see for instance US 5,589,466 and US 5,973,972).

The vector may be one that is suitable for expression of the anti-AXL antibody in a bacterial cell. Examples of such vectors include expression vectors such as BlueScript (Stratagene), pIN vectors (Van Heeke and Schuster, 1989), pET vectors (Novagen, Madison WI) and the like).

An expression vector may also or alternatively be a vector suitable for expression in a yeast system. Any vector suitable for expression in a yeast system may be employed. Suitable vectors include, for example, vectors comprising constitutive or inducible promoters such as alpha factor, alcohol oxidase and PGH (reviewed in Ausubel *et al.,* 1987, and Grant *et al.,* 1987).

A nucleic acid construct and/or vector may also comprise a nucleic acid sequence encoding a secretion/localization sequence, which can target a polypeptide, such as a nascent polypeptide chain, to the periplasmic space or into cell culture media. Such sequences are known in the art, and include secretion leader or signal peptides, organelle targeting sequences (e. g., nuclear localization sequences, ER retention signals, mitochondrial transit sequences, chloroplast transit sequences), membrane localization/anchor sequences (e. g., stop transfer sequences, GPI anchor sequences), and the like.

In an expression vector, the anti-AXL antibody-encoding nucleic acids may comprise or be associated with any suitable promoter, enhancer, and other expression-facilitating elements. Examples of such elements include strong expression promoters (e.g., human CMV IE promoter/enhancer as well as RSV, SV40, SL3-3, MMTV, and HIV LTR promoters), effective poly (A) termination sequences, an origin of replication for plasmid product in E. coli, an antibiotic resistance gene as selectable marker, and/or a convenient cloning site (*e.g.,* a polylinker). Nucleic acids may also comprise an inducible promoter as opposed to a constitutive promoter such as CMV IE (the skilled artisan will recognize that such terms are actually descriptors of a degree of gene expression under certain conditions).

The anti-AXL-antibody-encoding expression vector may be positioned in and/or delivered to the host cell or host animal via a viral vector.

The host cell can be a recombinant eukaryotic or prokaryotic host cell, such as a transfectoma, which produces an anti-AXL antibody as defined herein or a bispecific molecule of the invention as defined herein. Examples of host cells include yeast, bacterial and mammalian cells, such as CHO or HEK cells or derivatives thereof. For example, the cell may comprise a nucleic acid stably integrated into the cellular genome that comprises a sequence coding for expression of the anti-AXL antibody. The cell may comprises a non-integrated nucleic acid, such as a plasmid, cosmid, phagemid, or linear expression element, which comprises a sequence coding for expression of the anti-AXL antibody.

The term "recombinant host cell" (or simply "host cell"), as used herein, is intended to refer to a cell into which an expression vector has been introduced. It should be understood that such terms are intended to refer not only to the particular subject cell, but also to the progeny of such a cell. Because certain modifications may occur in succeeding generations due to either mutation or environmental influences, such progeny may not, in fact, be identical to the parent cell, but are still included within the scope of the term "host cell" as used herein. Recombinant host cells include, for example, transfectomas, such as CHO cells, HEK-293 cells, PER.C6, NSO cells, and lymphocytic cells, and prokaryotic cells such as *E. coli* and other eukaryotic hosts such as plant cells and fungi.

The term "transfectoma", as used herein, includes recombinant eukaryotic host cells expressing the antibody or a target antigen, such as CHO cells, PER.C6, NSO cells, HEK-293 cells, plant cells, or fungi, including yeast cells.

The antibody may alternatively be produced from a hybridoma prepared from murine splenic B cells obtained from mice immunized with an antigen of interest, for instance in form of cells expressing the antigen on the surface, or a nucleic acid encoding an extracellular region of AXL. Monoclonal antibodies may also be obtained from hybridomas derived from antibody-expressing cells of immunized humans or non-human mammals such as rabbits, rats, dogs, primates, etc.

Human antibodies may be generated using transgenic or transchromosomal mice, e.g. HuMAb mice, carrying parts of the human immune system rather than the mouse system. The HuMAb mouse contains a human immunoglobulin gene minilocus that encodes unrearranged human heavy (µ and γ) and κ light chain immunoglobulin sequences, together with targeted mutations that inactivate the endogenous µ and κ chain loci (Lonberg *et al.,* 1994a). Accordingly, the mice mount a human antibody response upon immunization, the introduced human heavy and light chain transgenes, undergo class switching and somatic mutation to generate high affinity human IgG,κ monoclonal antibodies (Lonberg *et al.,* 1994b; Lonberg and Huszar, 1995; Harding and Lonberg, 1995). The preparation of HuMAb mice is described in detail in Taylor *et al.,* 1992; Chen *et al.,* 1993; Tuaillon *et al.,* 1994; and Fishwild *et al.,* 1996. See also US 5,545,806; US 5,569,825; US 5,625,126; US 5,633,425; US 5,789,650; US 5,877,397; US 5,661,016; US 5,814,318; US 5,874,299; US 5,770,429; US 5,545,807; WO 98/024884; WO 94/025585; WO 93/001227; WO 92/022645; WO 92/003918; and WO 01/009187. Splenocytes from these transgenic mice may be used to generate hybridomas that secrete human monoclonal antibodies according to well-known techniques. In addition human antibodies may be generated from transgenic mice or rats to produce human-rat chimeric antibodies that can be used as a source for the recombinant production of fully human monoclonal antibodies.

Further, human antibodies may be identified through display-type technologies, including, without limitation, phage display, retroviral display, ribosomal display, mammalian display, yeast display and other techniques known in the art, and the resulting molecules may be subjected to additional maturation, such as affinity maturation, as such techniques are well known in the art.

**Table 4 - Sequences**

| SEQ ID NO: | Name | Amino acid sequence | Comments |
|---|---|---|---|
| 1 | 107 VH | | HCo12-BalbC Ig1 domain binding Ab |
| 2 | 107 VL | | |
| 3 | 140 VH | | |
| 4 | 140 VL | | |
| 5 | 148 VH | | HCo12-BalbC Ig2 domain binding Ab |
| 6 | 148 VL | | |
| 7 | 154 VH | | HCo12-BalbC FN1 domain binding Ab |
| 8 | 154-M103L VH | | |
| | | | |
| 9 | 154 VL | | |
| 10 | 171 VH | | HCo17-BalbC Ig2 domain binding Ab |
| 11 | 171 VL | | |
| 12 | 172 VH | | |
| 13 | 172 VL | | |
| 14 | 181 VH | | |
| 15 | 181 VH | | |
| 16 | 183 VH | | HCo17-BalbC FN1 domain binding Ab |
| 17 | 183-N52Q VH | | |
| 18 | 183 VL | | |
| 19 | 187 VH | | |
| 20 | 187 VL | | |
| 21 | 608-01 VH | | |
| 22 | 608-01 VL | | |
| 23 | 610-01 VH | | |
| 24 | 610-01 VL | | |
| | | | |
| 25 | 613 VH | | HCo20 Ig1 domain binding Ab |
| 26 | 613 VL | | |
| 27 | 613-08 VH | | |
| 28 | 613-08 VL | | |
| 29 | 620-06 VH | | |
| 30 | 620-06 VL | | |
| 31 | 726 VH | | HCo17-BalbC FN2 domain binding Ab |
| 32 | 726-M101L VH | | |
| 33 | 726 VL | | |
| 34 | 733 VH | | HCo17-BalbC FN1 domain binding Ab |
| 35 | 733 VL | | |
| 36 | 107 VH CDR1 | GFTFSSYA | |
| 37 | 107 VH CDR2 | TSGSGAST | |
| 38 | 107 VH CDR3 | AKIWIAFDI | |
| 39 | 107 VL CDR1 | QSVSSSY | |
| | 107 VL CDR2 | GAS | |
| 40 | 107 VL CDR3 | QQYGSSPYT | |
| 41 | 140 VH CDR1 | GFTFSSYA | |
| 42 | 140 VH CDR2 | ISISGAST | |
| 43 | 140 VH CDR3 | RGYSGYVYDAFDI | |
| 44 | 140 VL CDR1 | QGISNW | |
| | 140 VL CDR2 | AAS | |
| 45 | 140 VL CDR3 | QQYNSYPLT | |
| 46 | 148 VH CDR1 | GFTFSSYA | |
| 47 | 148 VH CDR2 | ISISGGST | |
| 48 | 148 VH CDR3 | RGYSGYVYDAFDF | |
| 49 | 148 VL CDR1 | QGISNW | |
| | 148 VL CDR2 | AAS | |
| 50 | 148 VL CDR3 | QQYNSYPLT | |
| 51 | 154 VH CDR1 | GFTFSSYA | |
| 52 | 154 VH CDR2 | ISIGGGNA | |
| 53 | 154 VH CDR3 | AKPGFIMVRGPLDY | |
| 54 | 154-M103L VH CDR3 | AKPGFILVRGPLDY | |
| 55 | 154 VL CDR1 | QSVSNSY | |
| | 154 VL CDR2 | GAS | |
| 56 | 154 VL CDR3 | QQYGSSPYT | |
| 57 | 171 VH CDR1 | GFTFSSYA | |
| 58 | 171 VH CDR2 | ISVSGGST | |
| 59 | 171 VH CDR3 | AKEGYIWFGESLSYAFDI | |
| 60 | 171 VL CDR1 | QSVSSSY | |
| | 171 VL CDR2 | GAS | |
| 61 | 171 VL CDR3 | QQYGRSFT | |
| 62 | 172 VH CDR1 | GFTFSNYA | |
| 63 | 172 VH CDR2 | ISVSGGST | |
| 64 | 172 VH CDR3 | AKEGYIWFGESLSYAFDI | |
| 65 | 172 VL CDR1 | QSVSSSY | |
| | 172 VL CDR2 | GAS | |
| 66 | 172 VL CDR3 | QQYGRSFT | |
| 67 | 181 VH CDR1 | GFTFSSYA | |
| 68 | 181 VH CDR2 | ISVSGGST | |
| 69 | 181 VH CDR3 | AKEGYIWFGESLSYAFDI | |
| 70 | 181 VLCDR1 | QSVSSSY | |
| | 181 VL CDR2 | GAS | |
| 71 | 181 VL CDR3 | QQYGRSFT | |
| 72 | 183 VH CDR1 | GGSFSGYY | |
| 73 | 183 VH CDR2 | INQSGST | |
| 74 | 183-N52Q VH CDR2 | IQQSGST | |
| 75 | 183 VH CDR3 | ASGNWDHFFDY | |
| 76 | 183 VL CDR1 | QGISSW | |
| | 183 VL CDR2 | ATS | |
| 77 | 183 VL CDR3 | QQAKSFPWT | |
| 78 | 187 VH CDR1 | GGSFSGYH | |
| 79 | 187 VH CDR2 | ISHSGRT | |
| 80 | 187 VH CDR3 | ASFITMIRGTIITHFDY | |
| 81 | 187 VL CDR1 | QGISSW | |
| | 187 VL CDR2 | AAS | |
| 82 | 187 VL CDR3 | QQYHSYPYT | |
| 83 | 608-01 VH CDR1 | GGTFSSY A | |
| 84 | 608-01 VH CDR2 | IIPIFGIA | |
| 85 | 608-01 VH CDR3 | ARRGDYYGSGSPDVFDI | |
| 86 | 608-01 VL CDR1 | QSVSSSY | |
| | 608-01 VL CDR2 | GAS | |
| 87 | 608-01 VL CDR3 | QQYGSSYT | |
| 88 | 610-01 VH CDR1 | GGTFSSYA | |
| 89 | 610-01 VH CDR2 | IIPIFGIA | |
| 90 | 610-01 VH CDR3 | ARRGNYYGSGSPDVFDI | |
| 91 | 610-01 VL CDR1 | QSVSSSY | |
| | 610-01 VL CDR2 | GAS | |
| 92 | 610-01 VL CDR3 | QQYGSSYT | |
| 93 | 613 VH CDR1 | GGTFSSYA | |
| 94 | 613 VH CDR2 | IIPIFGIV | |
| 95 | 613 VH CDR3 | ARRGNYYGSGSPDVFDI | |
| 96 | 613 VL CDR1 | QSVSSSY | |
| | 613 VL CDR2 | GAS | |
| 97 | 613 VL CDR3 | QQYGSSYT | |
| 98 | 613-08 VH CDR1 | GGTFSSYA | |
| 99 | 613-08 VH CDR2 | IIPIFGIV | |
| 100 | 613-08 VH CDR3 | ARRGNYYGSGSPDVFDI | |
| 101 | 613-08 VLCDR1 | QSVSSY | |
| | 613-08 VL CDR2 | DAS | |
| 102 | 613-08 VLCDR3 | QQRSNWLT | |
| 103 | 620-06 VH CDR1 | GGTFSSYA | |
| 104 | 620-06 VH CDR2 | IIPIFGIA | |
| 105 | 620-06 VH CDR3 | ARRGNYYGSGSPDVFDI | |
| 106 | 620-06 VL CDR1 | QSVSSSY | |
| | 620-06 VL CDR2 | GAS | |
| 107 | 620-06 VL CDR3 | QQYGSSYT | |
| 108 | 726 VH CDR1 | GGSFSGYY | |
| 109 | 726 VH CDR2 | ISHSGRT | |
| 110 | 726 VH CDR3 | ARFITMIRGAIITHFDY | |
| 111 | 726-M101L VH CDR3 | ARFITLIRGAIITHFDY | |
| 112 | 726 VL CDR1 | QGISSW | |
| | 726 VL CDR2 | AAS | |
| 113 | 726 VL CDR3 | QQYHSYPYT | |
| 114 | 733 VH CDR1 | GFSFSTYA | |
| 115 | 733 VH CDR2 | ISYDGDNK | |
| 116 | 733 VH CDR3 | ARGRKLGIDAFDI | |
| 117 | 733 VL CDR1 | QGISSA | |
| | 733 VL CDR2 | DAS | |
| 118 | 733 VL CDR3 | QQFNSYPFT | |
| 119 | Ig2 domain VH CDR2 | ISISGXST - wherein X is A or G | |
| 120 | Ig2 domain VH CDR3 | RGYSGYVYDAFDX - wherein X is I or F | |
| 121 | FN2 domain VH CDR1 | GGSFSGYX - wherein X is H or Y | |
| 122 | FN2 domain VH CDR3 | AX1FITMIRGX2IITHFDY - wherein X1 is S or R; and X2 is T or A | |
| 123 | FN1 domain VH CDR1 | GFTFSXYA - wherein X is S or N | |
| 124 | FN1 domain VH CDR2 | ISVSGGST | |
| 125 | FN1 domain VH CDR3 | AKEGYIWFGESLSYAFDI | |
| 126 | Ig1 domain VH CDR2 | IIPIFGIX-wherein X is A or V | |
| 127 | Ig1 domain VH CDR3 | ARRGXYYGSGSPDVFDI -wherein X is D or N | |
| 128 | Ig1 domain VL CDR1 | QSVXSSY - wherein X is S or del | |
| | Ig1 domain VL CDR2 | XAS - wherein X is D or G | |
| 129 | Ig1 domain VL CDR3 | QQX1X2X3X4X5T - wherein X1 is R or Y; X2 is S or G; X3 is N or S; X4 is W or S; and X5 is L or Y | |
| 130 | Human AXL protein (Swissprot P30530) | | |
| 131 | Mus musculus AXL | | |
| | | | |
| 132 | Homo sapiens AXL - Mus musculus Ig1 domain | | |
| 133 | Homo sapiens AXL - Mus musculus Ig2 domain | | |
| | | | |
| 134 | Homo sapiens AXL - Mus musculus FN1 domain | | |
| 135 | Homo sapiens AXL - Mus musculus FN2 domain | | |
| 136 | 511 VH | | Ig2 domain binding Ab |
| 137 | 511 VH CDR1 | GFTFSSYA | |
| 138 | 511 VH CDR2 | ISGSGGHT | |
| 139 | 511 VH CDR3 | AKDRYDILTGYYNLLDY | |
| 140 | 511 VL | | |
| 141 | 511 VL CDR1 | QGISSW | |
| | 511 VL CDR2 | AAS | |
| 142 | 511 VL CDR3 | QQYNSYPLT | |
| 143 | 061 VH | | Ig1 domain binding Ab |
| 144 | 061 VL | | |
| 145 | 137 VH | | |
| 146 | 137 VL | | |
| 147 | Cynomolgus monkey AXL (GenBank number HB387229.1) | | |
| 148-153 | | See Example 3 | |

The present invention is further illustrated by the following examples which should not be construed as further limiting.

### EXAMPLES

### Example 1 - Immunization and generation of AXL antibodies

### Expression constructs for AXL

The following codon-optimized constructs for expression of various full-length AXL variants were generated: human (*Homo sapiens*) AXL (Genbank accession no. NP_068713.2), human-cynomolgus monkey chimeric AXL in which the human extracellular domain (ECD) was replaced with the ECD of cynomolgus monkey (*Macaca fascicularis*) AXL (translation of Genbank accession HB387229.1; aa 1-447), human-mouse chimeric AXL in which the human ECD was replaced with the ECD of mouse (*Mus musculus*) AXL (Genbank accession NP_033491.2; aa 1-441), human-mouse chimeric AXL in which the human Ig-like domain I (aa 1-134, also termed "Ig1 domain" herein) was replaced with the Ig-like domain I of mouse AXL, human-mouse chimeric AXL in which the human Ig-like domain II (aa 148-194, also termed "Ig2 domain" herein) was replaced by the Ig-like domain II of mouse AXL, human-mouse chimeric ALX in which the human FNIII-like domain I (aa 227-329, also termed "FN1 domain" herein) was replaced with the FNIII-like domain I of mouse AXL, human-mouse chimeric AXL in which the human FNIII-like domain II (aa 340-444, also termed "FN2 domain" herein) was replaced by the FNIII-like domain II of mouse AXL. In addition, the following codon-optimized constructs for various AXL ECD variants were generated: the extracellular domain (ECD) of human AXL (aa 1-447) with a C-terminal His tag (AXLECDHis), the FNIII-like domain II of human AXL (aa 327-447) with a N-terminal signal peptide and a C-terminal His tag (AXL-FN2ECDHis), and the Ig1 and Ig2 domains of human AXL (aa 1-227) with a C-terminal His tag (AXL-Ig12ECDHis).

The constructs contained suitable restriction sites for cloning and an optimal Kozak (GCCGCCACC) sequence (Kozak *et al.,* 1999). The constructs were cloned in the mammalian expression vector pcDNA3.3 (Invitrogen).

### AXL expression in EL4 cells

EL4 cells were stable transfected with the pcDNA3.3 vector containing the full human AXL coding sequence and stable clones were selected after selection with the antibiotic agent, G418, (Geneticin).

### Purification of His-tagged AXL

AXLECDHis, AXL-FN2ECDHis, and AXL-Ig12ECDHis were expressed in HEK-293F cells. The His-tag enables purification with immobilized metal affinity chromatography. In this process, a chelator fixed onto the chromatographic resin is charged with Co²⁺ cations. His-tagged protein containing supernatants were incubated with the resin in batch mode (i.e. solution). The His-tagged protein binds strongly to the resin beads, while other proteins present in the culture supernatant do not bind or bind weakly compared to the His-tagged proteins. After incubation the beads are retrieved from the supernatant and packed into a column. The column is washed in order to remove weakly bound proteins. The strongly bound His-tagged proteins are then eluted with a buffer containing imidazole, which competes with the binding of His to Co²⁺. The eluent is removed from the protein by buffer exchange on a desalting column.

### Immunization

Antibodies IgG1-AXL-061, IgG1-AXL-107, IgG1-AXL-183, IgG1-AXL-613, and IgG1-AXL-726 were derived from the following immunizations: HCo12-BalbC (IgG1-AXL-107), HCo17-BalbC (IgG1-AXL-183, IgG1-AXL-726) and HCo20 (IgG1-AXL-061, IgG1-AXL-613) transgenic mice (Medarex, San Jose, CA, USA) which were immunized alternatingly intraperitoneally (IP) with 20 µg of the AXLECDHis protein (IgG1-AXL-511, IgG1-AXL-613, IgG1-AXL-183) *et al.,* 20 µg AXL-FN2ECDHIS plus 20 µg AXL-Ig12ECDHis (IgG1-AXL-726), or 20 µg AXL-Ig12ECDHis (IgG1-AXL-107) and subcutaneously (SC; at the tail base) with the same protein, with an interval of 14 days. In total 8 immunizations were performed: 4 IP and 4 SC immunizations. For most immunizations, the first immunization was performed in complete Freunds' adjuvant (CFA; Difco Laboratories, Detroit, MI, USA) and all subsequent immunizations in incomplete Freunds' adjuvant (IFA; Difco Laboratories, Detroit, MI, USA). Antibody IgG1-AXL-183 was derived from immunizations that were all performed in Sigma adjuvant system (Sigma-Aldrich, St. Louis, MO, USA).

Antibodies IgG1-AXL-137, IgG1-AXL-148, IgG1-AXL-154, IgG1-AXL-171, and IgG1-AXL-733 were derived from the following immunizations: HCo12-BalbC (IgG1-AXL-137, IgG1-AXL-148), HCo17-BalbC (IgG1-AXL-154, IgG1-AXL-733), and HCo20-BalbC (IgG1-AXL-171) transgenic mice (Medarex, San Jose, CA, USA) were immunized with 20 µg of the AXLECDHis protein in CFA. Subsequently, mice were immunized alternating intraperitoneally (IP) with EL4 cells transfected with full length human AXL in PBS and subcutaneously (SC; at the tail base) with the AXLECDHis protein in IFA, with an interval of 14 days.

Mice with at least two sequential AXL specific antibody titers of 200 (serum dilutions of 1/200) or higher, detected in the antigen specific screening FMAT assay as described below, were boosted 3-4 days prior to fusion (10 µg of AXL-derived protein in PBS injected intravenously).

### Homogeneous antigen specific screening assay

The presence of anti-AXL antibodies in sera of immunized mice or HuMab (human monoclonal antibody) hybridoma or transfectoma culture supernatant was determined by homogeneous antigen specific screening assays using Fluorometric Micro volume Assay Technology (FMAT; Applied Biosystems, Foster City, CA, USA). For this, two different test designs with combinations of either 4 or 8 cell based assays were used.

The 4 cell based assay test design was used for the testing of sera from immunized mice and as primary screening test for hybridoma or transfectoma culture supernatant. In the 4 assay test design samples were analyzed for binding of human antibodies to A431 (DSMZ) and MDA-MB-231 cells (both expressing AXL at the cell surface) as well as binding to TH1021-AXL (HEK-293F cells transiently expressing full length human AXL; produced as described above) and HEK293 wild-type cells (negative control which does not express AXL), respectively.

Hybridoma or transfectoma culture supernatant samples were additionally subjected to an 8 cell based assay test design. In the 8 assay test design samples were analyzed for binding of human antibodies to TH1021-hAXL (HEK-293F cells transiently expressing the human AXL), TH1021-cAXL (HEK-293F cells transiently expressing human-cynomolgus AXL chimeras in which the human ECD had been replaced with the ECD of cynomolgus monkey AXL), TH1021-mAXL (HEK-293F cells transiently expressing human-mouse AXL chimeras in which the human ECD had been replaced with the ECD of mouse AXL), TH1021-mIg1 (HEK-293F cells transiently expressing the human AXL with the Ig-like domain I being replaced by the Ig-like domain I of mouse AXL), TH1021-mIg2 (HEK-293F cells transiently expressing human AXL with the Ig-like domain II being replaced by the Ig-like domain II of mouse AXL), TH1021-mFN1 (HEK-293F cells transiently expressing human AXL with the FNIII-like domain I being replaced by the FNIII-like domain I of mouse AXL), TH1021-mFN2 (HEK-293F cells transiently expressing human AXL with the FNIII-like domain II being replaced by the FNIII-like domain II of mouse AXL), and HEK293 wild-type cells (negative control which does not express AXL), respectively.

Samples were added to the cells to allow binding to AXL. Subsequently, binding of HuMab was detected using a fluorescent conjugate (Goat anti-Human IgG Fc gamma-DyLight649; Jackson ImmunoResearch). The AXL specific humanized mouse antibody A0704P (produced in HEK-293F cells) was used as a positive control and HuMab-mouse pooled serum and ChromPure Human IgG, whole molecule (Jackson ImmunoResearch), respectively, were used as negative controls. The samples were scanned using an Applied Biosystems 8200 Cellular Detection System (8200 CDS) and mean fluorescence was used as read-out. Samples were stated positive when counts were higher than 50 and counts x fluorescence was at least three times higher than the negative control.

### HuMab hybridoma generation

The HuMab mouse with sufficient antigen-specific titer development (described above) was sacrificed and the spleen and lymph nodes flanking the abdominal aorta and vena cava were collected. Fusion of splenocytes and lymph node cells to a mouse myeloma cell line (SP2.0 cells) was done by electrofusion using a CytoPulse CEEF 50 Electrofusion System (Cellectis, Paris, France), essentially according to the manufacturer's instructions. Next, the primary wells were subcloned using the ClonePix system (Genetix, Hampshire, UK). To this end, specific primary well hybridomas were seeded in semisolid medium made from 40% CloneMedia (Genetix, Hampshire, UK) and 60% HyQ 2x complete media (Hyclone, Waltham, USA). The sub clones were retested according to the antigen-specific binding assay as described above and scanned using the IsoCyte sytem (Molecular Devices, LLC, Sunnyvale, CA). IgG levels were measured using an Octet (Fortebio, Menlo Park, USA) in order to select the best producing clone per primary well for further expansion. Further expansion and culturing of the resulting HuMab hybridomas were done based upon standard protocols (*e.g*. as described in Coligan J.E., Bierer, B.E., Margulies, D.H., Shevach, E.M. and Strober, W., eds. Current Protocols in Immunology, John Wiley & Sons, Inc. et al., 2006). Clones derived by this process were designated PC1021.

### Mass Spectrometry of purified antibodies

Small 0.8 ml aliquots of antibody containing hybridoma supernatant from 6-well or Hyperflask stage were purified using PhyTip columns containing Protein G resin (PhyNexus Inc., San Jose, USA) on a Sciclone ALH 3000 workstation (Caliper Lifesciences, Hopkinton, USA). The PhyTip columns were used according to manufacturer's instructions, but buffers were replaced by: Binding Buffer PBS (B. Braun, Medical B.V., Oss, Netherlands) and Elution Buffer 0.1M Glycine-HCI pH 2.7 (Fluka Riedel-de Haën, Buchs, Germany). After purification, samples were neutralized with 2M Tris-HCI pH 9.0 (Sigma-Aldrich, Zwijndrecht, Netherlands). Alternatively, in some cases larger volumes of culture supernatant were purified using Protein A affinity column chromatography.

After purification, the samples were placed in a 384-well plate (Waters, 100 µl square well plate, part# 186002631). Samples were deglycosylated overnight at 37°C with N-glycosidase F. DTT (15 mg/ml) was added (1 µl / well) and incubated for 1 h at 37°C. Samples (5 or 6 µl) were desalted on an Acquity UPLC™ (Waters, Milford, USA) with a BEH300 C18, 1.7µm, 2.1x 50 mm column at 60 °C. MQ water and LC-MS grade acetonitrile (Biosolve, cat no 01204101, Valkenswaard, The Netherlands) with both 0.1% formic acid (Fluka, cat no 56302, Buchs, Germany), were used as Eluent A and B, respectively. Time-of-flight electrospray ionization mass spectra were recorded on-line on a micrOTOF™ mass spectrometer (Bruker, Bremen, Germany) operating in the positive ion mode. Prior to analysis, a 900-3000 m/z scale was calibrated with ES tuning mix (Agilent Technologies, Santa Clara, USA). Mass spectra were deconvoluted with DataAnalysis™ software v. 3.4 (Bruker) using the Maximal Entropy algorithm searching for molecular weights between 5 and 80 kDa.

After deconvolution the resulting heavy and light chain masses (under reducing conditions) for all samples were compared in order to find duplicate antibodies. In the comparison of the heavy chains the possible presence of C-terminal lysine variants was taken into account. This resulted in a list of unique antibodies, where unique is defined as a unique combination of heavy and light chains. In case duplicate antibodies were found, the results from other tests were used to decide which antibody was the best material to continue experiments with.

### Sequence analysis of the AXL antibody variable domains and cloning in expression vectors

Total RNA was prepared from 0.2 to 5x10⁶ hybridoma cells and 5'-RACE-Complementary DNA (cDNA) was prepared from 100 ng total RNA, using the SMART RACE cDNA Amplification kit (Clontech), according to the manufacturer's instructions. VH and VL coding regions were amplified by PCR and cloned directly, in frame, in the pGlf and pKappa expression vectors, by ligation independent cloning (Aslanidis, C. and P.J. de Jong, Nucleic Acids Res 1990;18(20): 6069-74). For each antibody, 12 VL clones and 12 VH clones were sequenced. The resulting sequences are shown in Table 4. CDR sequences were defined according to IMGT (Lefranc *et al.,* 1999 and Brochet, 2008). Clones with a correct Open Reading Frame (ORF) were selected for further study and expression. Vectors of all combinations of heavy chains and light chains that were found were transiently co-expressed in FreestyleTM 293-F cells using 293fectin.

For antibodies IgG1-AXL-154, IgG1-AXL-183 and IgG1-AXL-726, the following variants with point mutations in the variable domains were generated: IgG1-AXL-154-M103L, IgG1-AXL-183-N52Q and IgG1-AXL-726-M101L. Mutants were generated by site-directed mutagenesis using the Quickchange II mutagenesis kit (Stratagene).

### AXL control antibodies

In some of the Examples a comparison antibody against AXL was used (IgG1-YW327.6S2) that has been previously described (EP 2 220 131, U3 Pharma; WO 2011/159980, Genentech). The VH and VL sequences for these AXL-specific antibodies were cloned into the pGlf and pKappa expression vectors.

### b12 antibody

In some of the examples the antibody b12, a gp120 specific antibody (Barbas, 1993) was used as a negative control.

### Expression

Antibodies were expressed as IgG1,κ. Plasmid DNA mixtures encoding both heavy and light chains of antibodies were transiently transfected to Freestyle HEK293F cells (Invitrogen, US) using 293fectin (Invitrogen, US) essentially as described by the manufacturer.

### Purification of antibodies

Culture supernatant was filtered over 0.2 µm dead-end filters, loaded on 5 mL MabSelect SuRe columns (GE Health Care) and eluted with 0.1 M sodium citrate-NaOH, pH 3. The eluate was immediately neutralized with 2M Tris-HCI, pH 9 and dialyzed overnight to 12.6 mM NaH2PO4, 140 mM NaCl, pH 7.4 (B.Braun). Alternatively, subsequent to purification, the eluate was loaded on a HiPrep Desalting column and the antibody was exchanged into 12.6 mM NaH2PO4, 140 mM NaCl, pH 7.4 (B.Braun) buffer. After dialysis or exchange of buffer, samples were sterile filtered over 0.2 µm dead-end filters. Purity was determined by SDS-PAGE and IgG concentration was measured using an Octet (Fortebio, Menlo Park, USA). Purified antibodies were stored at 4°C.

The antibody IgG1-AXL-511 was generated by the following method:

### Expression constructs for AXL

The following codon-optimized constructs for expression of various full-length AXL variants were generated: human (*Homo sapiens*) AXL (Genbank accession no. NP_068713.2), human-cynomolgus monkey chimeric AXL in which the human extracellular domain (ECD) was replaced with the ECD of cynomolgus monkey (*Macaca fascicularis*) AXL (translation of Genbank accession HB387229.1; aa 1-447), human-mouse chimeric AXL in which the human ECD was replaced with the ECD of mouse (*Mus musculus*) AXL (Genbank accession NP_033491.2; aa 1-441), human-mouse chimeric AXL in which the human Ig-like domain I (aa 1-147, also termed "Ig1 domain" herein) was replaced with the Ig-like domain I of mouse AXL, human-mouse chimeric AXL in which the human Ig-like domain II (aa 148-227, also termed "Ig2 domain" herein) was replaced by the Ig-like domain II of mouse AXL, human-mouse chimeric ALX in which the human FNIII-like domain I (aa 228-326, also termed "FN1 domain" herein) was replaced with the FNIII-like domain I of mouse AXL, human-mouse chimeric AXL in which the human FNIII-like domain II (aa 327-447, also termed "FN2 domain" herein) was replaced by the FNIII-like domain II of mouse AXL. In addition, the following codon-optimized constructs for various AXL ECD variants were generated: the extracellular domain (ECD) of human AXL (aa 1-447) with a C-terminal His tag (AXLECDHis), the FNIII-like domain II of human AXL (aa 327-447) with a N-terminal signal peptide and a C-terminal His tag (AXL-FN2ECDHis), and the Ig1 and Ig2 domains of human AXL (aa 1-227) with a C-terminal His tag (AXL-Ig12ECDHis).

The constructs contained suitable restriction sites for cloning and an optimal Kozak (GCCGCCACC) sequence (Kozak et al. (1999) Gene 234: 187-208). The constructs were cloned in the mammalian expression vector pcDNA3.3 (Invitrogen).

### AXL expression in EL4 cells

EL4 cells were stable transfected with the pcDNA3.3 vector containing the full length human AXL coding sequence and stable clones were selected after selection with the antibiotic agent, G418, (Geneticin).

### Purification of His-tagged AXL

AXLECDHis, AXL-FN2ECDHis, and AXL-Ig12ECDHis were expressed in HEK293F cells and purified with immobilized metal affinity chromatography.

### Immunization

Material from 4 transgenic mice expressing human antibody gene sequences was used for selecting antibodies. Mice immunized with various immunization protocols and with various antibody responses and yielding various numbers of antibodies from the traditional hybridoma process were chosen. Mouse A (3.5 % hits in the hybridoma process) was an HCo17- BALB/c transgenic mouse (Bristol-Myers Squibb, Redwood City, CA, USA) was immunized alternatingly intraperitoneally (IP) with 20 µg AXL-FN2ECDHIS plus 20 µg AXL-Ig12ECDHis) and subcutaneously (SC) at the tail base) with the same protein, with an interval of 14 days. In total 8 immunizations were performed: 4 IP and 4 SC immunizations. For most immunizations, the first immunization was performed in complete Freunds' adjuvant (CFA; Difco Laboratories, Detroit, MI, USA) and all subsequent immunizations in incomplete Freunds' adjuvant (IFA; Difco Laboratories, Detroit, MI, USA). Mouse B (0 % hits in the hybridoma process) was a HCo12 transgenic mouse (Medarex) immunized with 20 µg of the AXLECDHis protein using a similar immunization protocol as mouse A. Mouse C (38 % hits in the hybridoma process) was a HCo12- BALB/c mouse immunized alternating intraperitoneally (IP) with EL4 cells transfected with full length human AXL in PBS and subcutaneously (SC; at the tail base) with the AXLECDHis protein in IFA, with an interval of 14 days. Mouse D (0 % hits in the hybridoma process) was a HCo12 transgenic mouse (Medarex) immunized with 20 µg of the AXL-Ig12ECDHis protein in using a similar immunization protocol as mouse A.

Mice with at least two sequential AXL specific antibody titers of 200 (serum dilutions of 1/200) or higher, were boosted 3-4 days prior to fusion (10 µg of AXL-derived protein in PBS injected intravenously).

### Isolation of RNA from spleen cells

Total RNA was isolated from spleen cells using the Mini RNA easy kit (Qiagen). First strand cDNA for 5'-RACE was synthesized using 150 ng of RNA using the SMART RACE cDNA Amplification kit (Clontech, Mountain View, CA, USA), PrimeScript Reverse Transcriptase (Clontech) and the SMART IIA oligo and oligodT as primers. VL encoding regions were amplified by PCR using Advantage 2 polymerase (Clontech), the primers RACEkLIC4shortFW2 (320 nM), RACEkLIC4LongFW2 (80 nM) and RACEkLICRV_PmIA3 (400 nM), performing 35 cycles of 30 seconds at 95 °C, and 1 minute at 68 °C. VH encoding regions were amplified by PCR using Pfu Ultra II Fusion HS DNA polymerase (Stratagene), the primers RACEG1LIC3shortFW (320 nM), RACEG1LIC3longFW (80 nM) and RACEG1LIC3RV2 (400 nM), performing 40 cycles of 20 seconds at 95 °C *et al.,* 20 seconds at 66 °C and 30 seconds at 72 °C, ending with a finale extension step of 3 minutes at 72 °C. VH or VL encoding PCR products were separated using agarose gel electrophoresis and DNA products of the expected size were cut from the gel and purified using the Qiagen MiniElute kit. VH and VL coding regions amplified by PCR were cloned, in frame, in the mammalian expression vectors pGlf (containing the human IgG1 constant region encoding DNA sequence) for the VH region and pKappa (containing the kappa light chain constant region encoding DNA sequence) for the VL region, by ligation independent cloning (Aslanidis, C. and P.J. de Jong, Nucleic Acids Res 1990;18(20): 6069-74) in E.coli strain DH5αT1R (Life technologies), yielding single bacterial colonies each containing a single HC or LC expression vector.

### Primer sequences

| **Primer name** | **Primer sequence** |
|---|---|
| SMARTIIA | 5'-AAGCAGTGGTATCAACGCAGAGTACGCGGG (SEQ ID NO:154) |
| RACEkLIC4shortFW2 | 5'-ACGGACGGCAGGACCACT (SEQ ID NO:155) |
| RACEkLIC4LongFW2 | 5'-ACGGACGGCAGGACCACTAAGCAGTGGTATCAACGCAGA (SEQ ID NO: 156) |
| RACEkLICRV_PmIA3 | 5'-CAGCAGGCACACCACTGAGGCAGTTCCAGATTTC (SEQ ID NO:157) |
| RACEG1LIC3shortFW | 5'-ACGGACGGCAGGACCAGT (SEQ ID NO:158) |
| RACEG1LIC3longFW | 5'-ACGGACGGCAGGACCAGTAAGCAGTGGTATCAACGCAGAGT (SEQ ID NO: 159) |
| RACEG1LIC3RV2 | 5'-GGAGGAGGGCGCCAGTGGGAAGACCGA (SEQ ID NO:160) |
| CMV P f (RRA2) | 5'-GCCAGATATACGCGTTGACA (SEQ ID NO:161) |
| TK pA r (RRA2) | 5'-GATCTGCTATGGCAGGGCCT (SEQ ID NO:162) |

### LEE PCR

Linear expression elements (LEE's) were produced by amplifying the fragment containing the CMV promoter, HC or LC encoding regions and the poly A signal containing elements from the expression plasmids. For this the regions were amplified using Accuprime Taq DNA polymerase (Life Technologies) and the primers CMVPf(Bsal)2 and TkpA(Bsal)r, performing 35 cycles of 45 seconds at 94 °C, 30 seconds at 55 °C and 2 (LC) or 3 (HC) minutes at 68 °C, using material of E.coli (strain DH5α) colonies, containing the plasmids, as a DNA template.

### Transient expression in HEK-293 cells

Antibodies were expressed as IgG1,κ. Plasmid DNA mixtures encoding both heavy and light chains of antibodies were transiently transfected in Freestyle 293-F (HEK293F) cells (Life technologies, USA) using 293fectin (Life technologies) essentially as described by Vink, T., et al. (2014) ('A simple, robust and highly efficient transient expression system for producing antibodies', Methods, 65 (1), 5-10).

For LEE expression of Abs 1 µl of the HC LEE PCR reaction mixture, 1 µl of the LC PCR reaction mixture and 1 µl of a 30 ng/ µl enhancing mix containing a mix of 3 expression enhancing plasmids as described in Vink, T., *et al.* (2014), were mixed and transfected in HEK293F cells in a total volume of 100 µl using 293 fectin as transfection reagent, according to the instructions of the manufacturer (Life technologies), using 96 well plates as vessel, essentially as described supra.

### AXLECDHis ELISA

ELISA plates (Greiner, Netherlands) were coated with 100 µl / well of 0.5 µg/ ml AXLECDHis in Phosphate buffered saline (PBS) and incubated for 16 hours at room temperature (RT). The coating solution was removed and the wells were blocked by adding 150 µl PBSTC (PBS containing 0.1 % tween-20 and 2% chicken serum) well and incubating for 1 hour at RT. The plates were washed three times with 300 µl PBST (PBS containing 0.1 % tween-20)/well and 100 µl of test solution was added, followed by an incubation of 1 hour at RT. After washing three times with 300 µl of PBST/well, 100 µl antibody goat anti human IgG coupled with horse radish peroxidase (diluted 1/3000) was added and incubated for 1 hour at RT. After washing three times with 300 µl of PBST/well, 100 µl of ABTS (1mg/ml) solution was added and incubated at RT until sufficient signal was observed and the reaction was stopped by adding 100 µl of 2 % oxalic acid solution. 96 well plates were measured on an ELISA reader at 405 nm.

### Diversity screen

Samples were analyzed for binding of antibodies to TH1021-hAXL (HEK293F cells transiently expressing the human AXL), TH1021-cAXL (HEK293F cells transiently expressing human-cynomolgus AXL chimeras in which the human ECD had been replaced with the ECD of cynomolgus monkey AXL), TH1021-mAXL (HEK293F cells transiently expressing human-mouse AXL chimeras in which the human ECD had been replaced with the ECD of mouse AXL), TH1021-mlg1 (HEK293F cells transiently expressing the human AXL with the Ig-like domain I being replaced by the Ig-like domain I of mouse AXL), TH1021-mlg2 (HEK293F cells transiently expressing human AXL with the Ig-like domain II being replaced by the Ig-like domain II of mouse AXL), TH1021-mFN1 (HEK293F cells transiently expressing human AXL with the FNIII-like domain I being replaced by the FNIII-like domain I of mouse AXL), TH1021-mFN2 (HEK293F cells transiently expressing human AXL with the FNIII-like domain II being replaced by the FNIII-like domain II of mouse AXL), and HEK293F cells (negative control which does not express AXL), respectively.

Samples from the LEE expression were added to the cells to allow binding to the various AXL constructs. Subsequently, binding of antibodies was detected using a fluorescent conjugate (Goat anti-Human IgG Fc gamma-DyLight649; Jackson ImmunoResearch). The samples were scanned using an Applied Biosystems 8200 Cellular Detection System (8200 CDS) and mean fluorescence was used as read-out. Samples were stated positive when counts were higher than 50 and counts x fluorescence was at least three times higher than the negative control.

### Provision of HC and LC pools:

For each mouse, 352 HC expression vector containing bacterial colonies and 384 LC expression vector containing bacterial colonies were picked and amplified by LEE PCR. Part of the LEE reaction was sequenced (AGOWA). The percentage proper VH insert containing constructs differed largely between the 4 mice, mouse A (50 %), mouse B (23 %), mouse C (90 %) and mouse D (14 %) and resembled the variation of hits obtained in the hybridoma process, see supra. The HC diversity in the mice with only a limited amount of proper inserts were dominated by a large group of identical HCs, 65/83 in mouse B and 46/49 in mouse D. For mouse B and D the unique HCs (9 for mouse B, 4 for mouse D) were selected. For mouse A and C no selection was made.

### Co-transfection of HCs with a LC pool

The single HC encoding LEE's were co-transfected with a pool of 96 LC encoding LEE's using the LEE transfection protocol.

### HC selection of AXL binding antibodies

For mouse B and D, supernatants from the LEE co-transfections of the single HC with the pooled LCs were analyzed for AXL binding of the produced antibody mixtures by the AXL ELISA. 7 of the 9 HCs from mouse B resulted in AXL binding and 4 out of 4 of the HC from mouse D resulted in AXL binding.

For mouse A and C supernatants from the LEE co-transfections of the single HC with the pooled LCs were analyzed for AXL binding of the produced antibody mixtures by the diversity screen. This screen enabled both the identification of AXL binding HCs and a rough epitope mapping, by identifying the loss of binding of antibodies to AXL variants. From mouse A approximately 40 % of the HCs bound to human AXL, most of which lost binding either to the Ig1 or FNIII-2 domain, when these domains were replaced by the mouse equivalent. From mouse C approximately 70 % of the HCs bound to human AXL, most of which lost binding either to the Ig1 or Ig2 domain, when these domains were replaced by the mouse equivalent. Based on binding as determined by AXL ELISA or the diversity screen, HC sequence information and loss of binding to specific AXL domains in the diversity screen a total of 12 unique HCs were selected for determination of the best LC.

### Co-transfection of HCs with single LCs

Each single HC LEE of the 12 unique selected HCs was co-transfected with 96 single LC LEEs from the LC pool of the corresponding mice.

### LC selection of AXL binding antibodies

Supernatants of the LEE expression of the single HC / LC combinations were analyzed for AXL binding of the produced antibody by the AXL ELISA. For each HC at least 6 LCs were found and a single LC was selected as best, based on both the ELISA results and the LC sequence information. AXL binding antibodies were identified from all 4 mice, even the mice which were not successful in the hybridoma process.

### Binding affinity of antibody 511

The affinity of one anti-AXL antibody (clone 511) was determined.

Affinity was determined using Bio-Layer Interferometry on a ForteBio OctetRED384. Anti-human Fc Capture (AHC) biosensors (ForteBio, Portsmouth, UK; cat no. 18-5064) were loaded for 150 s with hlgG (1 µg/mL) aiming at a loading response of 1 nm. After a baseline (150 s) the association (1000 s) and dissociation (2000 s) of AXLECDHis (as described in Example 1) was determined, using a concentration range of 10 µg/mL - 0.16 µg/mL (218 nM - 3 nM) with 2-fold dilution steps. For calculations, the theoretical molecular mass of AXLECDHis based on the amino acid sequence was used, *i.e.* 46 kDa. Experiments were carried out on an OctetRED384, while shaking at 1000 rpm and at 30°C. Each antibody was tested in three independent experiments.

Data was analyzed with ForteBio Data Analysis Software v7.0.3.1, using the 1:1 model and a global full fit with 1000 s association time and 1000 s dissociation time unless stated otherwise. A dissociation time of 1000 s (instead of the 2000 s dissociation time that was acquired) was used since this resulted in better fits. Data traces were corrected by subtraction of a reference curve (antibody without AXLECDHis), the Y-axis was aligned to the last 5 s of the baseline, and interstep correction as well as Savitzky-Golay filtering was applied.

The affinity (K_{D}) of clone 511 for AXL was 23^{∗}10⁻⁹M (kₒₙ 1.7^{∗}10⁵ 1/Ms and a k_{dis} of 3.9^{∗}10⁻³ 1/s).

### Duostatin-3 synthesis.

### Preparation of compound 3:

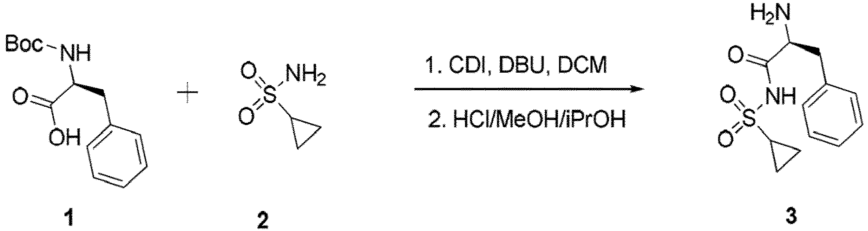

To a solution of Boc-L-phenylalanine 1 (5.36 g *et al.,* 20.2 mmol) in 30 mL of methylene chloride (DCM), carbonyldiimidazole (CDI, 4.26 g, 26.3 mmol) was added and stirred for 1 hour. Then added a solution of 2 (3.67 g, 30.3 mmol) and 2,4-diaminobutyric acid (DBU, 4.5 mL, 30 mmol) in 15 mL of DCM. The mixture was heated at 40°C for 16 hours. The mixture was diluted with 60 mL of DCM and 40 mL of water, then neutralized to pH 7 with conc. HCl.

The DCM extract was collected, washed with 0.2M HCI (60 mL), then with brine (60 mL), dried over Na2SO4, and evaporated to give 7.47 g of Boc protected sulfonamide. This material was suspended in 40 mL of methanol, then 200 mL of 6N HCl/isopropanol was added and the mixture was stirred for 2 hours. The solvent was evaporated under vacuum, 100 mL of ether was then added. The precipitate was collected by filtration and dried to give compound 3 as HCl salt (5.93 g, 96%); MS m/z 269.1 (M+H).

### Preparation of compound 5:

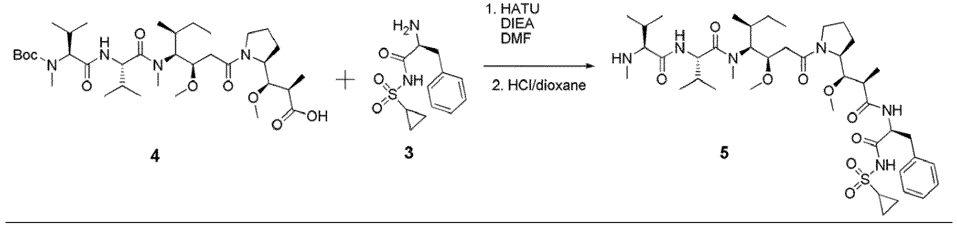

To a solution of compound 4 (1.09 g, 1.6 mmol) in 10 mL of N,N-Dimethylformamide (DMF) was added 2-(IH-7-azabenzotriazol-1-yl)-I,I ,3,3-tetramethyl uranium hexafluorophosphate (HATU, 0.61 g, 1.6 mmol), diisopropylethylamine (DIEA, 0.56 mL), and compound 3 (0.49 g, 1.6 mmol) in that order. The mixture was stirred for 1 hour and diluted with 100 mL of water and 4 mL of acetic acid. The precipitate was collected by filtration, dried under vacuum and added to 10 mL of 4M HCl/dioxane. After 30 min *et al.,* 200 mL of ether was added and insoluble precipitate was collected and purified by HPLC to give compound 5 as tetrahydrofuran salt (TFA, 1.3 g, 88%); MS m/z 835.5 (M+H). Compound 5 is referred to as duostatin-3 throughout the manuscript.

### Preparation of compound 7:

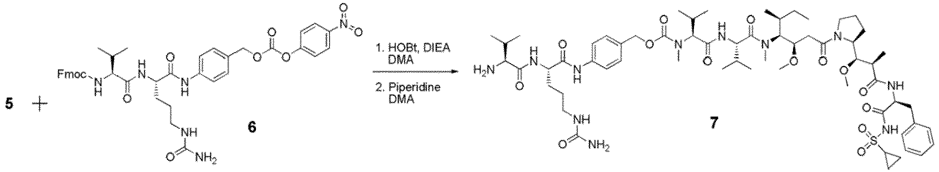

To a solution of compound 5 (500 mg, 0.527 mmol) in 5 mL of DMF was added compound 6 (483 mg, 0.631 mmol), N-Hydroxybenzotriazole (HOBt, 40 mg, 0.296 mmol), and DIEA (0.27 mL). The mixture was stirred for 16 hours after which 0.4 mL of piperidine was added. After 1 hour, the mixture was diluted with 100 mL of ether and the precipitate was collected and dried to give compound 7 as HCl salt (640 mg, 95 %); MS m/z 1240.7 (M+H).

### Preparation of compound 9:

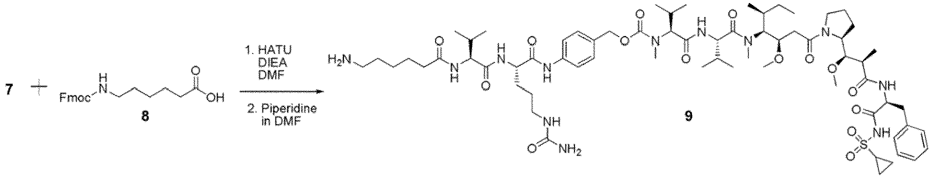

To a solution of compound 8 (219 mg, 0.62 mmol) in 5 mL of DMF was added HATU (236 mg, 0.62 mmol), DIEA (0.15 mL), and compound 7 (316 mg, 1.6 mmol), respectively. After 1 hour, 0.2 mL of piperidine was added and the mixture was stirred for 30 min, then purified by HPLC to give compound 9 as TFA salt (235 mg, 64 %); MS m/z 1353.8 (M+H).

### Preparation of compound 11:

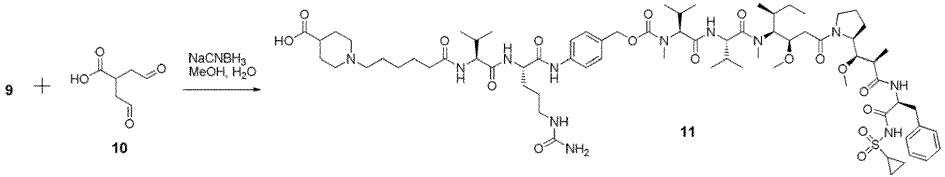

To a solution of compound 9 (235 mg, 0.16 mmol) in 2 mL of methanol and 1 mL of water was added a solution of dialdehyde 10 (1.6 mL of 0.3M in iPrOH) and NaCNBH3 (180 mg, 2.85 mmol). The mixture was stirred for 2 hours at RT, and then purified by HPLC giving rise to compound 11 as TFA salt (126 mg, 50 %); MS m/z 1465.8 (M+H)

### Generation of AXL-specific antibody-drug conjugates (ADC).

Purified AXL antibodies IgG1-AXL-148, IgG1-AXL-183 and IgG1-AXL-726 as well as the negative control antibody IgG1-b12 were conjugated with Duostatin-3 by Concortis Biosystems, Inc. (San Diego, CA) through covalent conjugation using the K-lock AV1-valine-citruline (vc) linker (WO 2013/173391, WO 2013/173392 and WO 2013/173393 by Concortis Biosystems).

The anti-AXL antibody drug conjugates were subsequently analyzed for concentration (by absorbance at 280 nm), the drug to antibody ratio (the 'DAR') by reverse phase chromatography (RP-HPLC) and hydrophobic interaction chromatography (HIC), the amount of unconjugated drug (by reverse phase chromatography), the percentage aggregation (by size-exclusion chromatography, SEC-HPLC) and the endotoxin levels (by LAL). The results were as follows (Table 5):

**Table 5**

| | IgG1-AXL-148-vcDuostatin3 | IgG1-AXL-183-vcDuostatin3 | IgG1-AXL-726-vcDuostatin3 | IgG1-b12-vcDuostatin3 |
|---|---|---|---|---|
| Concentration (mg/mL) | 6.57 | 3.40 | 5.93 | 3.36 |
| DAR by HIC-HPLC | 1.71 | 1.79 | 1.77 | 2.05 |
| % unconjugated drug | 6.67 | 4.16 | 5.38 | 4.19 |
| % aggregate by SEC-HPLC | 3.71% | 3.35 | 3.42 | 1.75 |

### Example 2 - Binding characteristics of AXL antibodies

### Binding affinity of AXL antibodies

The affinities of the panel of 9 anti-AXL antibodies as well as 3 variants of these antibodies with single amino acid mutations in the variable domains (IgG1-AXL-154-M103L, IgG1-AXL-183-N52Q, IgG1-AXL-726-M101L), were determined.

Affinities were determined using Bio-Layer Interferometry on a ForteBio OctetRED384. Anti-human Fc Capture (AHC) biosensors (ForteBio, Portsmouth, UK; cat no. 18-5064) were loaded for 150 s with hlgG (1 µg/mL) aiming at a loading response of 1 nm. After a baseline (150 s) the association (1000 s) and dissociation (2000 s) of AXLECDHis (as described in Example 1) was determined, using a concentration range of 10 µg/mL - 0.16 µg/mL (218 nM - 3 nM) with 2-fold dilution steps. For calculations, the theoretical molecular mass of AXLECDHis based on the amino acid sequence was used, *i.e.* 46 kDa. Experiments were carried out on an OctetRED384, while shaking at 1000 rpm and at 30°C. Each antibody was tested in three independent experiments.

Data was analyzed with ForteBio Data Analysis Software v7.0.3.1, using the 1:1 model and a global full fit with 1000 s association time and 1000 s dissociation time unless stated otherwise. A dissociation time of 1000 s (instead of the 2000 s dissociation time that was acquired) was used since this resulted in better fits. For antibody IgG1-AXL-154 and IgG1-AXL-154-M103L a dissociation time of 500 s was used. For IgG1-AXL-012 and IgG1-AXL-094 dissociation times of 200 s were used. Data traces were corrected by subtraction of a reference curve (antibody without AXLECDHis), the Y-axis was aligned to the last 5 s of the baseline, and interstep correction as well as Savitzky-Golay filtering was applied.

The affinities (K_{D}) of the anti-AXL antibodies ranged from 0.3^{∗}10⁻⁹M to 63^{∗}10⁻⁹M (Table 6). For mutant IgG1-AXL-183-N52Q the K_{D} was lower than for wild-type IgG1-AXL-183, due to an approximately 2.5-fold higher dissociation rate. The observed kinetics of the other two mutants were similar to the kinetics of the wild-type IgGs.

**Table 6**

| Antibody | Binding affinity (OCTET) | | |
|---|---|---|---|
| | KD (M) | Kon (1/Ms) | Kdis (1/s) |
| IgG1-AXL-107 | 16^{∗}10⁻⁹ | 2.8^{∗}10⁵ | 4.1^{∗}10⁻³ |
| IgG1-AXL-148 | 20^{∗}10⁻⁹ | 2.3^{∗}10⁵ | 4.4^{∗}10⁻³ |
| IgG1-AXL-154 | 7.2^{∗}10⁻⁹ | 2.6^{∗}10⁵ | 1.9^{∗}10⁻³ |
| IgG1-AXL-154-M103L | 7.8^{∗}10^{~9} | 2.7^{∗}10⁵ | 2.0^{∗}10⁻³ |
| IgG1-AXL-171 | 17^{∗}10⁻⁹ | 1.1^{∗}10⁵ | 1.8^{∗}10⁻³ |
| IgG1-AXL-183 | 10.2^{∗}10⁻⁹ | 4.1^{∗}10⁴ | 4.2^{∗}10^{~4} |
| IgG1-AXL-183-N52Q | 24^{∗}10⁻⁹ | 4.2^{∗}10⁴ | 1.0^{∗}10⁻³ |
| IgG1-AXL-613 | 1.5^{∗}10⁻⁹ | 5.4^{∗}10⁵ | 8.0^{∗}10⁻⁴ |
| IgG1-AXL-726 | 0.6^{∗}10⁻⁹ | 2.4^{∗}10⁵ | 1.3^{∗}10⁻⁴ |
| IgG1-AXL-726-M101L | 0.3^{∗}10⁻⁹ | 2.1^{∗}10⁵ | 6.9^{∗}10⁻⁵ |
| IgG1-AXL-733 | 63^{∗}10⁻⁹ | 1.6^{∗}10⁵ | 9.7^{∗}10⁻³ |

### Binding of AXL antibodies to human, mouse and cynomolgus AXL

HEK293T cells were transiently transfected with expression constructs for full length human AXL, human AXL with a cynomolgus monkey extracellular domain (ECD) or human AXL with a mouse ECD (see Example 1). Binding of HuMab-AXL antibodies to these cells was evaluated by flow cytometry. Transfected HEK293 cells were incubated with serial dilutions of AXL-antibodies (final concentration range 0.0024-10 µg/mL) for 30 minutes at 4ºC. After washing three times in PBS/0.1% BSA/0.02% azide, cells were incubated with R-Phycoerythrin (PE)-conjugated goat-anti-human IgG F(ab')2 (Jackson ImmunoResearch Laboratories, Inc., West Grove, PA; cat. No. 109-116-098) diluted 1/100 in PBS/0.1% BSA/0.02% azide (final volume 100 µL). Next, cells were washed twice in PBS/0.1% BSA/0.02% azide, resuspended in 120 µL PBS/0.1% BSA/0.02% azide and analyzed on a FACS Cantoll (BD Biosciences).

Binding curves were analyzed using non-linear regression (sigmoidal dose-response with variable slope) using GraphPad Prism V5.04 software (GraphPad Software, San Diego, CA, USA).

**Figure 1A** shows that the HuMab-AXL antibodies showed dose-dependent binding to the HEK293 cells expressing human AXL-ECD. Furthermore, HuMab-AXL antibodies recognized AXL with a cynomolgus monkey ECD, with EC₅₀ values in the same range as for fully human AXL (**Figure 1B**). In contrast, binding of HuMabs to AXL with a mouse ECD was low (IgG1-AXL-107, IgG1-AXL-154, IgG1-AXL-154-M103L, IgG1-AXL-733, IgG1-AXL-183, IgG1-AXL-183-N52Q) or not detectable (IgG1-AXL-171, IgG1-AXL-613, IgG1-AXL-726, IgG1-AXL-726-M101L, IgG1-AXL-148; **Figure 1C**)**.** As expected, the negative control antibody IgG1-b12 showed (**Figure 1**) no binding to cells expressing any of the AXL variants. Table 7 shows the EC50 values and standard deviations for binding of the anti-AXL antibodies to human AXL or human AXL with a cynomolgus AXL ECD (determined in at least 3 experiments). EC50 values for binding to human AXL with a mouse AXL ECD could not be determined to very low or absent binding.

**Table 7**

| Antibody | Binding EC50 (µg/mL) | |
|---|---|---|
| | human AXL Average (s.d.) | cynomolgus AXL Average (s.d.) |
| IgG1-AXL-107 | 0.050 (0.004) | 0.149 (0.021) |
| IgG1-AXL-154 | 0.105 (0.003) | 0.160 (0.027) |
| IgG1-AXL-154-M103L | 0.110 (0.038) | 0.161 (0.042) |
| IgG1-AXL-171 | 0.073 (0.023) | 0.157 (0.057) |
| IgG1-AXL-613 | 0.040 (0.023) | 0.146 (0.023) |
| IgG1-AXL-726 | 0.288 (0.206) | 0.349 (0.160) |
| IgG1-AXL-726-M101L | 0.184 (0.117) | 0.250 (0.066) |
| IgG1-AXL-733 | 0.176 (0.094) | 0.254 (0.114) |
| IgG1-AXL-148 | 0.094 (0.059) | 0.152 (0.080) |
| IgG1-AXL-183 | 0.526 (0.177) | 0.309 (0.086) |
| IgG1-AXL-183-N52Q | 0.350 (0.206) | 0.324 (0.121) |

### Competition between AXL antibodies and Gas6 for AXL binding

It was tested whether the AXL ligand Gas6 interfered with binding of the AXL antibodies to AXL. Therefore, AXL-positive A431 cells were incubated for 15 minutes at 4°C with 10 µg/mL recombinant human Gas6 (R&D Systems, Abingdon, UK; cat. No. 885-GS). Subsequently, serial dilutions of AXL antibodies were prepared (final concentration range 0.014-10 µg/mL), added to the cells and incubated for 30 minutes at 4ºC. After washing three times in PBS/0.1% BSA/0.02% azide, cells were incubated in 100 µL with secondary antibody at 4°C for 30 min in the dark. As a secondary antibody binding the Fc region, R-Phycoerythrin (PE)-conjugated goat-anti-human IgG F(ab')2 (Jackson ImmunoResearch Laboratories, Inc., West Grove, PA; cat. No. 109-116-098) diluted 1/100 in PBS/0.1% BSA/0.02% azide, was used. Next, cells were washed twice in PBS/0.1% BSA/0.02% azide, resuspended in 120 µL PBS/0.1% BSA/0.02% azide and analyzed on a FACS Cantoll (BD Biosciences).

Alternatively, A431 cells were pre-incubated with 10 µg/mL AXL antibodies (15 minutes, 4°C) to assess if the AXL ligand Gas6 could still bind in presence of AXL antibodies. After antibody pre-incubation, serial dilutions of recombinant human Gas6 (R&D Systems, Abingdon, UK; cat. No. 885-GS) were added to the cells at final concentrations of 0.001-20 µg/mL and incubated for 30 minutes at 4ºC. After washing three times in PBS/0.1% BSA/0.02% azide, cells were incubated with mouse anti-Gas6 IgG2a (R&D Systems; cat no. MAB885) at 4°C for 30 min. After washing three times in PBS/0.1% BSA/0.02% azide, cells were incubated with FITC-labelled goat anti-mouse IgG (Dako, Heverlee, Belgium; cat no. F049702) at 4°C for 30 min in the dark. Next, cells were washed twice in PBS/0.1% BSA/0.02% azide, resuspended in 120 µL PBS/0.1% BSA/0.02% azide and analyzed on a FACS Cantoll (BD Biosciences).

Binding curves were analyzed using non-linear regression (sigmoidal dose-response with variable slope) using GraphPad Prism V5.04 software (GraphPad Software, San Diego, CA, USA).

In experiments (n=3) in which A431 cells were pre-incubated with Gas6, the maximal binding values of anti-AXL antibodies was comparable to antibody binding in absence of Gas6 (maximal binding after Gas6 pre-incubation was 90-108% of binding without Gas6 pre-incubation) (Table 7). The EC₅₀ values for AXL antibody binding with or without Gas6 pre-incubation were in the same range, or somewhat enhanced after Gas6 pre-incubation (Table 8).

The binding of control AXL antibody YW327.6S2 to A431 cells was greatly reduced in the presence of Gas6 compared to binding without Gas. Maximal binding of YW327.6S2 in the presence of Gas6 was 19% of binding without Gas6, and the EC50 value for binding to A431 cells was 21-fold higher when cells had been pre-incubated with Gas6.

In experiments in which A431 cells were pre-incubated with anti-AXL antibodies, Gas6 binding was evaluated (n=3). Binding of Gas6 to A431 cells was similar with or without pre-incubation with HuMab-AXL antibodies. Average EC50 concentrations of Gas6 binding when cells were pre-incubated with HuMabs (0.34-0.83 µg/mL) and maximal Gas6 binding were similar to Gas6 binding in the presence of negative control antibody b12 (EC50 concentration: 0.40 µg/mL; 95-115% of Gas6 binding in the presence of the b12 control antibody). The binding of Gas6 to A431 cells was greatly reduced in the presence of control AXL antibody YW327.6S2 compared to pre-incubation with b12 (the EC50 concentration was 14-fold higher). Maximal binding of Gas6 in the presence of control antibody YW327.6S2 was 17% of binding in the presence of negative control antibody b12.

**Table 8**

| Antibody | Antibody binding to A431 cells | | | Gas6 binding to A431 cells | |
|---|---|---|---|---|---|
| | EC50 w/o Gas6 EC50 (µg/mL) mean (s.d.) | EC50 in presence of Gas6 (µg/mL) mean (s.d.) | Maximal binding in presence of Gas6 (% of binding in absence of Gas6) mean (s.d.) | EC50 in presence of AXL antibodies (µg/mL) mean (s.d.) | Maximal binding in presence of AXL antibodies (% of binding in prescence of control antibody) mean (s.d.) |
| IgG1-AXL-107 | 0.16 (0.17) | 0.94 (1.18) | 91 (5) | 0.78 (0.54) | 96 (8) |
| IgG1-AXL-148 | 0.11 (0.13) | 0.20 (0.30) | 93 (5) | 0.73 (0.52) | 106 (7) |
| IgG1-AXL-154 | 0.42 (0.55) | 0.76 (0.78) | 99 (13) | 0.44 (0.28) | 95 (10) |
| IgG1-AXL-171 | 0.18 (0.21) | 0.32 (0.40) | 95 (5) | 0.69 (0.42) | 108 (5) |
| IgG1-AXL-183 | 0.69 (0.72) | 1.19 (1.11) | 90 (19) | 0.34 (0.13) | 115 (8) |
| IgG1-AXL-511 | 0.12 (0.11) | 0.30 (0.31) | 93 (15) | 0.74 (0.44) | 113 (6) |
| IgG1-AXL-613 | 0.09 (0.09) | 0.10 (0.10) | 108 (22) | 0.57 (0.36) | 100 (11) |
| IgG1-AXL-726 | 0.32 (0.35) | 0.55 (0.69) | 97 (10) | 0.77 (0.58) | 98(10) |
| IgG1-AXL-733 | 0.49 (0.51) | 0.62 (0.23) | 93 (5) | 0.83 (0.54) | 96 (5) |
| YW327.6S2 | 0.09 (0.09) | 1.90 (1.04)* | 41 (24) | 5.53 (7.09)* | 17 (10) |
| b12 | n.a.^{a} | n.a. | n.a. | 0.40(0.11) | 100 |

| | | | | | |
|---|---|---|---|---|---|
| ^{a}n.a., not applicable * EC50 values less accurate due to low binding. | | | | | |

### Example 3 - Epitope mapping studies anti-AXL antibody panel

### Determining the AXL domain specificity using human-mouse AXL chimeric molecules

The AXL domain specificity of the AXL antibodies was determined using a panel of human-mouse chimeric AXL mutants. Five different chimeric AXL molecules were generated, in which either the human Ig-like domain I (Ig1), the Ig-like domain II (Ig2), the human FNIII-like domain I (FN1) or the human FNIII-like domain II domain (FN2) were replaced with their murine homologs.

The following codon-optimized constructs for expression of the AXL human-mouse chimeras were generated and expressed in HEK293F cells as described in Example 1: ***Homo sapiens* AXL - *Mus musculus* Ig1 domain (p33-AXL-mIg1):** (SEQ ID NO:150) ***Homo sapiens* AXL - *Mus musculus* Ig2 domain (p33-AXL-mIg2):** (SEQ ID NO:151) ***Homo sapiens* AXL - *Mus musculus* FN1 domain (p33-AXL-mFN1):** (SEQ ID NO:152) ***Homo sapiens* AXL - *Mus musculus* FN2 domain (p33-AXL-mFN2):** (SEQ ID NO:153)

Binding of 1 µg/mL anti-AXL antibody to the human-mouse AXL chimeras was determined by flow cytometry, as described in Example 2. IgG1-b12 was included as an isotype control IgG1.

All anti-AXL antibodies showed binding to human AXL (**Figure 2A**)**,** whereas binding was abrogated or strongly reduced when the human AXL ECD was replaced with its murine homolog **(****Figure 2B****).** The human-mouse cross-reactive monoclonal AXL antibody YW327.6S2 was included to confirm expression of hsAXL-mmECD.

Anti-AXL antibody 107 and 613 showed strongly reduced binding to hsAXL-mmIg1 (**Figure 2C**)**,** indicating recognition of an epitope in the AXL Ig1 domain. IgG1-AXL-148 and IgG1-AXL-171 showed strongly reduced binding to hsAXL-mmIg2 (**Figure 2D**), indicating recognition of an epitope in the AXL Ig2 domain. IgG1-AXL-154, IgG1-AXL-183 and IgG1-AXL-733 showed reduced binding to hsAXL-mmFN1 (**Figure 2E**), indicative of a binding epitope in the AXL FN1 domain. Finally, binding of IgG1-AXL-726 was lost in hsAXL-mmFN2 (**Figure 2F**)**,** indicating recognition of an epitope within the FN2 domain.

AXL domain specificity for all anti-AXL antibodies is summarized in Table 9.

**Table 9**

| Antibody | AXL domain specificity | AXL aa's involved in binding |
|---|---|---|
| IgG1-AXL-107 | Ig1 | L121-Q129 |
| IgG1-AXL-148 | Ig2 | D170-R190 |
| IgG1-AXL-154 | Fn1 | Q272-A287, G297-P301 |
| IgG1-AXL-154-M103L | n.d.^{a} | n.d. |
| IgG1-AXL-171 | Ig2 | P170, T182-R190 |
| IgG1-AXL-183 | Fn1 | Not resolved |
| IgG1-AXL-183-N52Q | n.d. | n.d. |
| IgG1-AXL-613 | Ig1 | T112-Q124 |
| IgG1-AXL-726 | Fn2 | A359, R386, Q436-K439 |
| IgG1-AXL-726-M101L | n.d. | n.d. |
| IgG1-AXL-733 | Fn1 | Not resolved |
| IgG1-AXL-061 | Ig1 | I97-Q124 |
| IgG1-AXL-137 | Ig1 | Q57, E92-T105 |
| YW327.6S2 | Ig1 | G39-D59 |

| | | |
|---|---|---|
| ^{a} n.d., not determined | | |

### High resolution epitope mapping to identify amino acids in the AXL extracellular domain involved in binding of AXL antibodies

To identify amino acids in the AXL extracellular domain involved in binding of anti-AXL antibodies, a library of AXL sequence variants was generated by recombination of AXL sequences derived from species with variable levels of homology with the human AXL sequence in the extracellular domain. Briefly, an expression plasmid encoding human AXL (Hs) was mixed with cloning plasmids encoding *Mus musculus* (Mm), *Monodelphis domestica* (Md; opossum) *Anolis carolinensis* (Ac; lizard) and *Tetraodon nigroviridis* (Tn; pufferfish) AXL homologs or vice versa. A combination of two primers specific to either the cloning or the expression vector was used to perform a PCR amplifying the AXL extracellular domain (ECD) with abbreviated elongation time, forcing melting and reannealing of nascent DNA replication strands during PCR cycling. Full length ECD was amplified using a nested PCR, again specific to recombination products containing termini originating from both vectors.

Resulting AXL ECD PCR products were cloned into an expression vector creating full length AXL, and resulting plasmids were sequenced, ranked by maximal difference to the template vectors and selected to create a minimal ensemble with maximal differentiation power. Plasmids encoding AXL homologs from Hs, Mm, Md, Ac and Tn, four human/mouse chimeric plasmids encoding Hs AXL with murine Ig1, Ig2, Fn1 or Fn2 domains, and the sixteen most differentiating plasmids from the recombination library were transfected into HEK293-F cells according to the specifications supplied by the manufacturer (Life technologies). FACS binding data using 1 µg/mL anti-AXL antibodies were deconvoluted by scoring per amino acid if mutation did (+1) or did not (-1) correlate with loss of binding, after which a baseline correction and normalization to a scale of -5 to +5 was applied, resulting in an impact score per amino acid over the full ECD.

The deconvoluted binding data is summarized in Table 9 as the amino acids involved in binding. Antibodies whose binding sites could not be mapped to high resolution due to a lack of recombination events in the proximity of the binding site, are indicated as not resolved.

### Example 4 - Fc-mediated effector functions

### Antibody-dependent cell-mediated cytotoxicity (ADCC)

The ability of anti-AXL antibodies to induce ADCC of A431 epidermoid carcinoma cells was determined as explained below. As effector cells, peripheral blood mononuclear cells from healthy volunteers (UMC Utrecht, The Netherlands) were used.

### Labeling of target cells

A431 cells were collected (5x10⁶ cells) in culture medium (RPMI 1640 culture medium supplemented with 10% fetal calf serum (FSC)), to which 100 µCi ⁵¹Cr (Chromium-51; Amersham Biosciences Europe GmbH, Roosendaal, The Netherlands) had been added, and the mixture was incubated in a 37°C water bath for 1 hour (hr) while shaking. After washing of the cells (twice in PBS, 1200 rpm, 5 min), the cells were resuspended in RPMI1640/10% FSC and counted by trypan blue exclusion. Cells were diluted to a density of 1x10⁵ cells/mL.

### Preparation of effector cells

Peripheral blood mononuclear cells (healthy volunteers, UMC Utrecht, Utrecht, The Netherlands) were isolated from 45 mL of freshly drawn heparin blood by Ficoll (Bio Whittaker; lymphocyte separation medium, cat 17-829E) according to the manufacturer's instructions. After resuspension of cells in RPMI1640/10% FSC, cells were counted by trypan blue exclusion and diluted to a density of 1x10⁷ cells/mL.

### ADCC set up

50 µl of ⁵¹Cr-labeled targets cells were pipetted into 96-well plates, and 50 µl of antibody were added, diluted in RPMI1640/10% FSC (3-fold dilutions at final concentrations range 0.01-10 µg/mL). Cells were incubated (room temperature (RT), 15 min), and 50 µl effector cells were added, resulting in an effector to target ratio of 100:1 (for determination of maximal lysis, 100 µl 5% Triton-XlOO was added instead of effector cells; for determination of spontaneous lysis, 50 µL target cells and 100 µL RPMI1640/10% FSC were used). Cells were incubated overnight at 37°C and 5% CO₂. After spinning down cells (1200 rpm, 10 min), 70 µL of supernatant was harvested into micronic tubes, and counted in a gamma counter. The percentage specific lysis was calculated as follows: $% specific lysis = \left(cpm sample-cpm target cells only\right) / \left(cpm maximal lysis-cpm target cells only\right) , wherein cpm is counts per minute .$

IgG1-AXL-183-N52Q, and IgG1-AXL-733 induced 15 to 21% ADCC in A431 cells at a concentration of 10 µg/mL (**Figure 3**)**.** IgG1-AXL-148, IgG1-AXL-726-M101L, IgG1-AXL-171, IgG1-AXL-613, IgG1-AXL-107, and IgG1-AXL-154-M103L did not induce significant ADCC in A431 cell at concentrations up to 10 µg/mL (**Figure 3**).

### Example 5 - Binding characteristics of AXL antibody-drug conjugates (AXL-ADCs)

HEK293T cells were transiently transfected with expression constructs for full-length human AXL (see Example 1). Binding of anti-AXL antibodies and AXL-ADCs to these cells was evaluated by flow cytometry. Transiently transfected HEK293 cells were incubated with serial dilutions of anti-AXL antibodies or AXL-ADCs (4-fold dilutions; final concentration range 0.003-10 µg/mL) for 30 minutes at 4ºC. After washing three times in PBS/0.1% BSA/0.02% azide, cells were incubated in 100 µL with secondary antibody at 4°C for 30 min in the dark. As a secondary antibody, R-Phycoerythrin (PE)-conjugated goat-anti-human IgG F(ab')2 (Jackson ImmunoResearch Laboratories, Inc., West Grove, PA; cat. No. 109-116-098) diluted 1/100 in PBS/0.1% BSA/0.02% azide, was used. Next, cells were washed twice in PBS/0.1% BSA/0.02% azide, resuspended in 120 µL PBS/0.1% BSA/0.02% azide and analyzed on a FACS Cantoll (BD Biosciences).

Binding curves were analyzed using non-linear regression (sigmoidal dose-response with variable slope) using GraphPad Prism V5.04 software (GraphPad Software, San Diego, CA, USA).

**Figure 4** shows that binding of the anti-AXL antibodies to the HEK293 cells expressing human AXL-ECD was similar to the binding of the AXL-ADCs.

### Example 6 - In vitro cytotoxicity induced by AXL-specific antibody drug conjugates

LCLC-103H cells (human large cell lung cancer) cells were cultured in RPMI 1640 with L-Glutamine (Cambrex; cat.no. BE12-115F) supplemented with 10% (vol/vol) heat inactivated Cosmic Calf Serum (Perbio; cat.no. SH30087.03), 2 mM L-glutamine (Cambrex; cat.no. US17-905C), 50 IU/mL penicillin, and 50 µg/mL streptomycin (Cambrex; cat.no. DE17-603E). MDA-MB-231 cells (human breast cancer) were cultured in DMEM (Cambrex; cat.no. BE12-709F) supplemented with 10% (vol/vol) heat inactivated Cosmic Calf Serum (Perbio; cat.no. SH30087.03), 1 mM Sodium Pyruvate (Cambrex; cat.no. BE13-115E), 2 mM L-glutamine (Cambrex; cat.no. US17-905C), 100 µM MEM NEAA (Invitrogen; cat.no. 11140), 50 IU/mL penicillin, and 50 µg/mL streptomycin (Cambrex; cat.no. DE17-603E). The cell lines were maintained at 37°C in a 5% (vol/vol) CO2 humidified incubator. LCLC-103H and MDA-MB-231 cells were cultured to near confluency, after which cells were trypsinized, resuspended in culture medium and passed through a cell strainer (BD Falcon, cat.no. 352340) to obtain a single cell suspension. 1x10³ cells were seeded in each well of a 96-well culture plate, and cells were incubated for 30 min at room temperature and subsequently for 5 hrs at 37ºC, 5% CO2 to allow adherence to the plate.

Serial dilutions (4-fold; final concentrations ranging from 0.00015 to 10 µg/mL) of AXL antibody drug conjugates (AXL-ADCs; see Example 1) were prepared in culture medium and added to the plates. Incubation of cells with 1 µM staurosporin (#S6942-200, Sigma) was used as reference for 100% tumor cell kill. Untreated cells were used as reference for 0% tumor cell kill. Plates were incubated for 5 days at 37ºC, 5% CO2. Next, CellTiter-Glo Reagent (Promega; cat.no. G7571) was added to the wells (20 µL per well) and plates were incubated for 1.5 hours at 37ºC, 5% CO2. Subsequently, 180 µL per well was transferred to white 96-well Optiplate™ plates (PerkinElmer, Waltham, MA; cat.no. 6005299), which were incubated for 30 min at room temperature. Finally, luminescence was measured on an EnVision multiplate reader (Envision, Perkin Elmer).

AXL-ADCs IgG1-AXL-148-vcDuo3, IgG1-AXL-183-vcDuo3, and IgG1-AXL-726-vcDuo3 induced cytotoxicity in LCLC-103H cells, with IC50 values between 0.01 and 0.06 µg/mL, as shown in **Figure 5A****.** Similarly, **Figure 5B** shows that these AXL-ADCs induced cytoxicity of MDA-MB-231 cells with IC50 values between 0.005 and 0.015 µg/mL.

### Example 7-Antibody VH and VL variants that allow binding to AXL

Protein sequences of the VH and VL regions of the anti-AXL antibody panel (described in Example 1) were aligned and compared for AXL binding to identify critical or permissive changes of amino acid residues in the VH or VL regions. Therefore, antibodies with identical VH or VL regions were grouped and compared for binding to human AXL and differences in VL or VH sequences, respectively. Binding to human AXL transiently expressed by HEK-293F cells was assessed in the homogeneous antigen specific screening assay as described in Example 1. Numbering of amino acid positions for the alignments done in the present example was done based on the sequences put forth in **Figure 6****,** *i.e.* the first amino acid in the shown sequence was numbered as position '1', the second as position '2', etc.

First, antibodies with identical VL sequences were grouped.

IgG1-AXL-148 and IgG1-AXL-140 were found to have an identical VL sequence, and showed 1 amino acid difference in the HC CDR3 region (F for I at amino acid position 109; **Figure 6A**). Both antibodies bound to human AXL (Table 10), indicating that the amino acid at position 109 is not essential for antibody binding, assuming that a mutation identified in the CDR2 region (G for A at the amino acid position 56) does not compensate for loss of binding (**Figure 6A**).

IgG1-AXL-726 and IgG1-AXL-187 were found to have an identical VL sequence and both antibodies bound to human AXL (Table 10). Two amino acid residue changes in the HC CDR3 region (R for S at position 97 and A for T at position 105; **Figure 6B**) were allowed without losing binding, assuming that mutations identified in the CDR1 (Y for H at position 32) and/or in the framework regions (P3Q, V24I, Y25D, T86A and T117A) do not compensate for loss of binding (**figure 6B****).**

IgG1-AXL-171, IgG1-AXL-172 and IgG1-AXL-181 were found to have an identical VL sequence and all antibodies bound to human AXL (Table 10). The CDR3 regions of these three antibodies were identical, but an amino acid residue change in the HC CDR1 (S for N at position 31) or the framework region (H for Q at position 82) was allowed without losing binding (**Figure 6C**).

IgG1-AXL-613, IgG1-AXL-608-01, IgG1-AXL-610-01 and IgG1-AXL-620-06 were found to have an identical VL sequence, and showed one amino acid difference in the HC CDR3 region (N for D at amino acid position 101; **Figure 6D**). All antibodies bound to human AXL (Table 10), indicating that the amino acid at position 101 is not essential, assuming that mutations identified in the HC CDR2 (V for A at position 58) and/or in the framework regions (N35S, M37V, A61V, L70I, S88A) do not compensate for loss of binding (**Figure 6D**).

Next, antibodies with identical VH sequences were grouped.

IgG1-AXL-613 and IgG1-AXL-613-08 were found to have an identical VH sequence, and showed five amino acid differences in the CDR3 region of the LC (RSNWL for YGSSY at positions 92 to 96; **Figure 6E**). Both antibodies bound to human AXL (Table 10), indicating that the variation of amino acid at positions 92 to 96 are allowed and do not affect antibody binding, assuming that mutations identified in the CDR1 (deletion of the S at position 30), CDR2 (G51D), and/or in the framework regions (G9A, S54N, R78S, Q100G, L104V) do not compensate for loss of binding (**Figure 6E****).**

**Table 10**

| **Antibody** | **EC50 (µg/mL)** | **Maximal binding (Arbitrary units)** |
|---|---|---|
| IgG1-AXL-140 | 0.0026 | 2889 |
| IgG1-AXL-148 | 0.0036 | 3499 |
| IgG1-AXL-171 | 0.003 | 2575 |
| IgG1-AXL-172 | 0.0055 | 5378 |
| IgG1-AXL-181 | 0.008 | 3598 |
| IgG1-AXL-187 | 0.0065 | 2563 |
| IgG1-AXL-608-01 | 0.0035 | 3318 |
| IgG1-AXL-610-01 | 0.0023 | 2947 |
| IgG1-AXL-613 | 0.0072 | 5211 |
| IgG1-AXL-613-08 | 0.0242 | 2209 |
| IgG1-AXL-620-06 | 0.0034 | 4352 |
| IgG1-AXL-726 | 0.0471 | 3154 |

### Example 8 - In vitro cytotoxicity induced by MMAE-conjugated AXL antibodies

### Conjugation of MMAE to anti-AXL antibodies

Anti-AXL antibodies were purified by Protein A chromatography according to standard procedures and conjugated to vcMMAE. The drug-linker vcMMAE was alkylated to the cysteines of the reduced antibodies according to procedures described in the literature (see Sun *et al.,* 2005; McDonagh *et al.,* 2006; and Alley *et al.,* 2008). The reaction was quenched by the addition of an excess of N-acetylcysteine. Any residual unconjugated drug was removed by purification and the final anti-AXL antibody drug conjugates were formulated in PBS. The anti-AXL antibody drug conjugates were subsequently analyzed for concentration (by absorbance at 280 nm), the drug to antibody ratio (DAR) by reverse phase chromatography (RP-HPLC) and hydrophobic interaction chromatography (HIC), the amount of unconjugated drug (by reverse phase chromatography), the percentage aggregation (by size-exclusion chromatography, SEC-HPLC) and the endotoxin levels (by LAL). The results are shown below in **Table 11.**

**Table 11 - Overview of different characteristics of the antibody-drug conjugates.**

| | **ADC** | | | | | | | | | |
|---|---|---|---|---|---|---|---|---|---|---|
| **Assay** | **IgG1 -AXL-107** | **IgG 1-AXL 148** | **IgG1-AXL-154-M103L** | **IgG1-AXL-171** | **IgG1 AXL-183-N52Q** | **IgG1-AXL-511** | **IgG1-AXL-613** | **IgG1-AXL-726-M101L** | **IgG 1-AXL-733** | **IgG1 -b12** |
| Concentration (mg/mL) | 7.18 | 9.63 | 6.57 | 3.69 | 6.71 | 5.77 | 6.17 | 7.37 | 7.71 | 1.58 |
| DAR by HIC | 3.97 | 3.96 | 3.71 | 3.65 | 3.92 | 3.87 | 4.23 | 4.12 | 4.08 | 4.00 |
| % unconjugated antibody | 4.68 | 5.58 | 6.13 | 7.11 | 8.68 | 8.35 | 5.13 | 4.99 | 3.74 | 1.89 |
| % aggregate by SEC-HPLC | 6.3 | 2.28 | 2.9 | 3.3 | 5.2 | 5.1 | 6.4 | 4.0 | 3.5 | 2.5 |
| Endotoxin (EU/mg) | 2.3 | 1.2 | 2.6 | 3.1 | 5.9 | 4.5 | 2.0 | 3.6 | 7.6 | 11.5 |

### Cell culture

LCLC-103H cells (human large cell lung cancer) and A431 cells (DMSZ, Braunschweig, Germany) were cultured in RPMI 1640 with L-Glutamine (Cambrex; cat.no. BE12-115F) supplemented with 10% (vol/vol) heat inactivated Cosmic Calf Serum (Perbio; cat.no. SH30087.03), 2 mM L-glutamine (Cambrex; cat.no. US17-905C), 50 IU/mL penicillin, and 50 µg/mL streptomycin (Cambrex; cat.no. DE17-603E). MDA-MB231 cells were cultured in DMEM with high glucose and HEPES (Lonza #BE12-709F), Donor Bovine Serum with Iron (Life Technologies #10371-029), 2 mM L-glutamine (Lonza #BE17 -605E), 1 mM Sodium Pyruvate (Lonza #BE13-115E), and MEM Non-Essential Amino Acids Solution (Life Technologies #11140). The cell lines were maintained at 37°C in a 5% (vol/vol) CO₂ humidified incubator. LCLC-103H, A431 and MDA-MB231 cells were cultured to near confluency, after which cells were trypsinized, resuspended in culture medium and passed through a cell strainer (BD Falcon, cat.no. 352340) to obtain a single cell suspension. 1x10³ cells were seeded in each well of a 96-well culture plate, and cells were incubated for 30 min at room temperature and subsequently for 5 hrs at 37°C, 5% CO₂ to allow adherence to the plate.

### Cytotoxicity assay

Serial dilutions (final concentrations ranging from 0.00015 to 10 µg/mL) of MMAE-conjugated AXL-antibodies were prepared in culture medium and added to the plates. Incubation of cells with 1 µM staurosporin (#S6942-200, Sigma) was used as reference for 100% tumor cell kill. Untreated cells were used as reference for 100% cell growth. Plates were incubated for 5 days at 37°C, 5% CO₂. Next, CellTiter-Glo Reagent (Promega; cat.no. G7571) was added to the wells (20 µL per well) and plates were incubated for 1.5 hours at 37°C, 5% CO₂. Subsequently, 180 µL per well was transferred to white 96-well Optiplate™ plates (PerkinElmer, Waltham, MA; cat.no. 6005299), which were incubated for 30 min at room temperature. Finally, luminescence was measured on an EnVision multiplate reader (Envision, Perkin Elmer).

MMAE-conjugated AXL-antibodies induced 50% cell kill in LCLC-103H cells at concentrations between 0.004 and 0.219 µg/mL as shown in **Table 12** and **Figure 7****.**

Similarly, AXL-ADCs efficiently induced cytotoxicity in A431 cells (**Table 13**) and **Figure 15A**) and MDA-MB231 cells (**Table 13** and **Figure 15B**)**.**

**Table 12 - Cytotoxicity of MMAE-conjugated -AXL-antibodies in LCLC-103H cells (EC50 values)**

| **ADC** | **EC50 (µg/mL)** |
|---|---|
| IgG1-AXL-613-vcMMAE | 0.004 |
| IgG1-AXL-148-vcMMAE | 0.012 |
| IgG1-AXL-171-vcMMAE | 0.018 |
| IgG1-AXL-726-M101L-vcMMAE | 0.018 |
| IgG1-AXL-107-vcMMAE | 0.022 |
| IgG1-AXL-511-vcMMAE | 0.032 |
| IgG1-AXL-154-M103L-vcMMAE | 0.044 |
| IgG1-AXL-183-N52Q-vcMMAE | 0.113 |
| IgG1-AXL-733-vcMMAE | 0.219 |

**Table 13. Cytotoxicity of MMAE-conjugated AXL antibodies in A431 and MDA-MB-231 cells (EC50 values).**

| **ADC** | **EC50 (µg/mL)** | | | |
|---|---|---|---|---|
| | **A431 (n=3)** | | **MDA-MB231 (n=2)** | |
| | **Mean** | **s.d.** | **Mean** | **s.d.** |
| IgG1-AXL -107-vcMMAE | 0.154 | 0.066 | 0.037 | 0.005 |
| IgG1-AXL-148-vcMMAE | 0.070 | 0.013 | 0.012 | 0.004 |
| IgG1-AXL-154-M103L-vcMMAE | 0.719 | 0.091 | 0.396 | 0.195 |
| IgG1-AXL-171-vcMMAE | 0.206 | 0.074 | 0.035 | 0.006 |
| IgG1-AXL-183-N52Q-vcMMAE | 1.157 | 0.160 | 0.139 | 0.028 |
| IgG1-AXL-511-vcMMAE | 0.093 | 0.020 | 0.052 | 0.003 |
| IgG1-AXL-613-vcMMAE | 0.109 | 0.078 | 0.005 | 0.001 |
| IgG1-AXL-726-M101L-vcMMAE | 0.270 | 0.157 | 0.022 | 0.002 |
| IgG1-AXL-733-vcMMAE | 1.253 | 0.228 | 0.881 | 0.182 |

### Example 9 - Therapeutic treatment of LCLC-103H tumor xenografts in SCID mice with MMAE-conjugated anti-AXL antibodies

The *in vivo* efficacy of MMAE-conjugated anti-AXL antibodies was determined in established subcutaneous (SC) LCLC-103H xenograft tumors in SCID mice. 5 x 10⁶ LCLC-103H (large cell lung carcinoma) tumor cells (obtained from Leibniz-Institut DSMZ-Deutsche Sammlung von Mikroorganismen und Zellkulturen GmbH (DSMZ)) in 200 µL PBS were injected subcutaneously in the right flank of female SCID mice. Starting 14-21 days after tumor cell inoculation, when the average tumor size was >100-200 mm³ and distinct tumor growth was observed, a single injection with 1 mg/kg (20 µg/mouse) IgG1-AXL-vcMMAE antibodies (as described in Supplementary Example 1) or control (unconjugated IgG1-b12) was given intraperitoneally (100 µL/mouse). Tumor volume was determined at least two times per week. Tumor volumes (mm³) were calculated from caliper (PLEXX) measurements as: 0.52 x (length) x (width)².

The panel of anti-AXL-vcMMAE antibodies showed a broad range of anti-tumor activity in established SC LCLC-103H tumors (**Figure 8**)**.** Clones IgG1-AXL-733-vcMMAE, IgG1-AXL-107-vcMMAE and IgG1-AXL-148-vcMMAE induced tumor regression, clones AXL-171-vcMMAE, IgG1-AXL-511-vcMMAE and IgG1-AXL-613-vcMMAE induced tumor growth inhibition, and clones IgG1-AXL-154-M103L-vcMMAE, IgG1-AXL-183-N52Q-vcMMAE, and IgG1-AXL-726-M101L-vcMMAE showed no or only minor tumor growth inhibition.

Statistical analysis on the last day that all groups were intact (day 30) using One Way ANOVA (Dunnett's multiple comparisons test versus control IgG1- b12) indicated a highly significant difference (p<0.0001) in tumor volume between IgG1-b12 versus IgG1-AXL-733-vcMMAE, IgG1-AXL-107-vcMMAE and IgG1-AXL-148-vcMMAE. Treatment with these clones led in some mice within these groups to complete tumor reduction. Treatment with clones IgG1-AXL-171-vcMMAE, IgG1-AXL-511-vcMMAE and IgG1-AXL-613-vcMMAE also showed significant tumor growth inhibition compared to IgG1-b12, but the differences were less pronounced (p<0.05 to p<0.001). The tumor growth of mice treated with clones IgG1-AXL-154-M103L-vcMMAE, IgG1-AXL-183-N52Q-vcMMAE, and IgG1-AXL-726-M101L-vcMMAE was not significant affected compared to the IgG1-b12 control.

Anti-tumor activity of anti-AXL-vcMMAE antibodies was observed in various other *in vivo* tumor models. In two cell line-derived xenograft models (A431; epidermoid adenocarcinoma, and MDA-MB-231; breast cancer) anti-AXL-vcMMAE antibodies induced tumor growth inhibition, and tumor regression was induced by anti-AXL-vcMMAE antibodies in two patient-derived xenograft models from patients with pancreas cancer and cervical cancer.

### Example 10 - Anti-tumor efficacy of AXL-ADCs in a pancreas cancer patient-derived xenograft (PDX) model with heterogeneous target expression

The anti-tumor activity of IgG1-AXL-107-vcMMAE, IgG1-AXL-148-vcMMAE, and IgG1-AXL-733-vcMMAE was determined in the PAXF1657 pancreas cancer PDX model (experiments performed by Oncotest, Freiburg, Germany). Human pancreas tumor tissue was subcutaneously implanted in the left flank of 5-7 weeks old female NMRI nu/nu mice. Randomization of animals was performed as follows: animals bearing a tumor with a volume between 50 - 250 mm³, preferably 80 - 200 mm³, were distributed in 7 experimental groups (8 animals per group), considering a comparable median and mean of group tumor volume. At day of randomization (day 0), the 3 ADCs were dosed intravenously (i.v.) at either 4 mg/kg or 2 mg/kg, and the control group received a single dose of IgG1-b12 (4 mg/kg). Tumor volumes (mm³) were monitored twice weekly and were calculated from caliper (PLEXX) measurements as: 0.52 x (length) x (width)².

Staining of PAXF1657 tumors was performed with standard immunohistochemistry techniques. Briefly, frozen tissues were fixated with acetone for 10 minutes and endogenous peroxidase was exhausted using hydrogen peroxidase. Subsequently, tissue sections were blocked with normal mouse serum and staining was performed by incubation with 5 µg/mL of a pool of 5 IgG1-AXL antibodies (IgG1-AXL-061, IgG1-AXL-137, IgG1-AXL-148, IgG1-AXL-183, IgG1-AXL-726). After incubation with the secondary, horseradish peroxidase (HRP) conjugated antibody, HRP was visualized with amino-ethyl carbazole (AEC; resulting in a red color). Each slide was counterstained with hematoxylin (blue) to identify nuclei and coverslipped in glycergel. Immunostained tissue slices were digitized on manual Zeiss microscope (AxioSkop) at 10x and 40x magnifications.

**Figure 9** shows heterogeneous AXL expression in PAXF1657 tumors. Whereas strong AXL staining is observed in some tumor cells, other cells do not show AXL staining. In black and white photo the AXL staining appears as dark grey. Hematoxylin staining (nuclei) appears as light grey.

**Figure 10A** shows that treatment of mice with 2 mg/kg IgG1-AXL-107-vcMMAE, IgG1-AXL-148-vcMMAE and IgG1-AXL-733-vcMMAE significantly reduced the growth of PAXF1657 tumors compared to the control group. At a dose of 4 mg/kg IgG1-AXL-107-vcMMAE, IgG1-AXL-148-vcMMAE and IgG1-AXL-733-vcMMAE induced tumor regression of PAXF1657 tumors. On day 14 after treatment, the average tumor size in mice that had been treated with 2mg/kg or 4 mg/kg IgG1-AXL-107-MMAE, IgG1-AXL-148-MMAE or IgG1-AXL-733-MMAE was significantly smaller than in mice that had been treated with an isotype control IgG (IgG1-b12) (p<0.001; Tukey's multiple comparison test).

Treatment of mice with unconjugated IgG1-AXL-148 did not result in anti-tumor activity in the PAXF1657 model (**Figure 10B**)**.** Conjugated IgG1-AXL-148-vcMMAE, however, induced dose-dependent antitumor activity in this model (**Figure 10B**)**,** illustrating that the therapeutic capacity of AXL-ADCs is dependent on the cytotoxic activity of MMAE.

Moreover, treatment of mice with the untargeted ADC IgG1-b12-vcMMAE did not show anti-tumor activity in the PAXF1657 model (**Figure 10C**)**,** illustrating that the therapeutic capacity of AXL-ADCs also depends on specific target binding.

### Example 11 - AXL antibodies binding to the Ig1 domain

The AXL domain specificity of AXL antibodies IgG1-AXL-061, IgG1-AXL-107, IgG1-AXL-137, and IgG1-AXL-613 was determined using a panel of human-mouse chimeric AXL mutants. The human-mouse cross-reactive monoclonal AXL antibody YW327.6S2 was included to confirm expression of hsAXL-mmECD. IgG1-b12 was included as isotype control antibody. Five different chimeric AXL molecules were generated and expressed in HEK293F as described in Example 3. In brief, the human Ig-like domain I (Ig1), the Ig-like domain II (Ig2), the human FNIII-like domain I (FN1) or the human FNIII-like domain II domain (FN2) were replaced with their murine homologs. Binding of 1 µg/mL anti-AXL antibody to the human-mouse AXL chimeras was determined by flow cytometry, as described in Example 2.

All anti-AXL antibodies showed binding to human AXL (**Figure 11A**)**,** whereas binding was abrogated when the human AXL ECD was replaced with its murine homolog (**Figure 11B**)**.** As expected, the human-mouse cross-reactive monoclonal AXL antibody YW327.6S2 showed binding to hsAXL-mmECD, confirming proper expression of hsAXL-mmECD.

AXL antibodies IgG1-AXL-061, IgG1-AXL-107, IgG1-AXL-137, and IgG1-AXL-613 showed strongly reduced binding to hsAXL-mmIg1 (**Figure 11C**)**,** illustrating recognition of an epitope in the AXL Ig1 domain. In line with this, binding of IgG1-AXL-061, IgG1-AXL-107, IgG1-AXL-137, and IgG1-AXL-613 to hsAXL-mmIg2 (**Figure 11D**)**,** hsAXL-mmFN1 (**Figure 11E**) or hsAXL-mmFN2 (**Figure 11F**) was not affected. The human-mouse cross-reactive monoclonal AXL antibody YW327.6S2 showed binding to all chimeric AXL variants, confirming proper expression of these proteins.

### Example 12 - AXL antibodies IgG1-AXL-107 and IgG1-AXL-613 bind to the Ig1 domain but do not compete with Gas6 binding

It was tested whether the binding of the AXL antibodies IgG1-AXL-061, IgG1-AXL-107, IgG1-AXL-137, or IgG1-AXL-613 interfered with binding of AXL ligand Gas6 to AXL. Therefore, binding of Gas6 to A431 cells that had been pre-incubated with 10 µg/mL AXL antibodies was tested as described in Example 2. Pre-incubation with AXL antibody YW327.6S2, that was described to compete with Gas6 for AXL binding, IgG1-b12 (isotype control) or medium (negative control) were included as controls.

Binding curves were analyzed using non-linear regression (sigmoidal dose-response with variable slope) using GraphPad Prism V5.04 software (GraphPad Software, San Diego, CA, USA).

**Figure 12** and **Table 14** shows that binding of Gas6 to A431 cells that had been pre-incubated with IgG1-AXL-107 and IgG1-AXL-613 antibodies was similar to the IgG1-b12 and medium controls. This illustrates that binding of IgG1-AXL-107 and IgG1-AXL-613 to AXL does not interfere with Gas6 binding, as shown in Example 2. The binding of Gas6 to A431 cells was largely reduced in the presence of IgG1-AXL-061, IgG1-AXL-137 and control AXL antibody YW327.6S2 compared to the IgG1-b12 and medium controls.

In experiments in which A431 cells were pre-incubated with Gas6, the maximal binding values of IgG1-AXL-107 and IgG1-AXL-613 were comparable to antibody binding in absence of Gas6 (maximal binding after Gas6 pre-incubation was 91-108% of binding without Gas6 pre-incubation) (**Table 14**)**.** The EC₅₀ values for IgG1-AXL-107 and IgG1-AXL-613 binding with or without Gas6 pre-incubation were in the same range, or somewhat higher after Gas6 pre-incubation **(Table 14),** illustrating that IgG1-AXL-107 and IgG1-AXL-613 do not compete with Gas6 binding.

Similar to control antibody YW327.6S2, the binding of IgG1-AXL-061 and IgG1-AXL-137 to A431 cells was greatly reduced in the presence of Gas6 compared to binding without Gas6 (maximal binding after Gas6 pre-incubation was 40-43% of binding without Gas6 pre-incubation; **Table 14).** The EC₅₀ values for IgG1-AXL-061 and IgG1-AXL-137 could not properly be determined after Gas6 pre-incubation **(Table 14).** This shows that IgG1-AXL-061 and IgG1-AXL-137 compete with Gas6 for binding to AXL.

These data demonstrate that antibodies binding to the AXL lg1 domain have differential effect on Gas6 binding.

**Table 14**

| Antibody | Antibody binding to A431 cells | | | Gas6 binding to A431 cells | |
|---|---|---|---|---|---|
| | EC₅₀ w/o Gas6 EC₅₀ (µg/mL) mean (s.d.) | EC50 in presence of Gas6 (µg/mL) mean (s.d.) | Maximal binding in presence of Gas6 (% of binding in absence of Gas6) mean (s.d.) | EC50 in presence of AXL antibodies (µg/mL) mean (s.d.) | Maximal binding in presence of AXL antibodies (% of binding in presence of control antibody) mean (s.d.) |
| IgG1-AXL-061 | 0.15 (n.a.) | *n.a.* | 43 (28) | *n.a.* | 22 (8) |
| IgG1-AXL-107 | 0.16 (0.17) | 0.94 (1.18) | 91 (5) | 0.78 (0.54) | 96 (8) |
| IgG1-AXL-137 | 0.11 (0.10) | *n.a.* | 40 (18) | *n.a* | 36 (4) |
| IgG1-AXL-613 | 0.09 (0.09) | 0.10 (0.10) | 108 (22) | 0.57 (0.36) | 100 (11) |
| YW327.6S2 | 0.09 (0.09) | 1.90 (1.04)* | 41 (24) | 5.53 (7.09)* | 17 (10) |
| b12 | n.a.^{a} | n.a. | n.a. | 0.40 (0.11) | 100 |

| | | | | | |
|---|---|---|---|---|---|
| ^{a}n.a., not applicable * EC₅₀ values less accurate due to low binding. | | | | | |

### Example 13 - In vivo anti-tumor efficacy of AXL-ADCs in xenograft models with and without autocrine (endogenous) Gas6 production

### Gas6 production of A431 and LCLC-103H tumor cells

It was tested whether A431 cells and LCLC-103H cells produce Gas6. Therefore, cells were grown in complete culture medium for 3 days. Gas6 levels in supernatant were determined using the Quantikine Human Gas6 ELISA (R&D Systems, Minneapolis, MN) according to manufacturer's instructions. This assay uses the quantitative sandwich ELISA technique. A monoclonal Ab specific for human Gas6 has been pre-coated onto a microplate. Standards and samples are pipetted into the wells and any human Gas6 present is bound by the immobilized Ab. After washing away any unbound substances, an enzyme-linked polyclonal Ab specific for human Gas6 is added to the wells. Following a wash to remove any unbound Ab-enzyme reagent, a substrate is added to the wells and color develops in proportion to the amount of human Gas6 bound in the initial step. The color development is stopped and the intensity of the color is measured.

Cell culture medium conditioned by A431 cells was found to contain 2576 ng/mL Gas6, while the concentration of Gas6 in medium conditioned by LCLC-103H cells was more than 20-fold less **(Table 15).**

**Table 15 - Gas6 production in tumor cell conditioned medium.**

| Cell line | Gas6 in supernatant (ng/mL) |
|---|---|
| LCLC-103H | 126 |
| A431 | 2576 |

### Anti-tumor activity of AXL-ADCs in vivo

The *in vivo* anti-tumor activity of IgG1-AXL-061-vcMMAE (Ig1 binder), IgG1-AXL-107-vcMMAE (Ig1-binder), IgG1-AXL-137-vcMMAE (Ig1-binder), IgG1-AXL-148-vcMMAE (Ig2-binder), IgG1-AXL-183-vcMMAE (FN1-binder), and IgG1-AXL-726-vcMMAE (FN2-binder) was determined in the A431 (epidermoid carcinoma) tumor model, that produces high levels of Gas6, and the LCLC-103H (large cell lung carcinoma) tumor model, that produces low levels of Gas6.

Tumor induction was performed by subcutaneous injection of 5 x 10⁶ A431 or LCLC-103H tumor cells (both obtained from Leibniz-Institut - Deutsche Sammlung von Mikroorganismen und Zellkulturen GmbH (DSMZ)) in 200 µL PBS in the right flank of female SCID mice. Treatment was started 14-21 days after tumor cell inoculation, when the average tumor size was >100-200 mm³ and distinct tumor growth was observed. Mice received a single injection or a total of 4 biweekly intraperitoneal injections with IgG1-AXL-vcMMAE ADCs or control antibody (unconjugated IgG1-b12), as indicated. Tumor volume was determined at least two times per week. Tumor volumes (mm³) were calculated from caliper (PLEXX) measurements as: 0.52 x (length) x (width)².

**Figure 13A** shows that treatment of mice with 3 mg/kg IgG1-AXL-107-vcMMAE, IgG1-AXL-148-vcMMAE and IgG1-AXL-733-vcMMAE induced growth inhibition of A431 tumors.

**Figure 13B** shows that treatment of mice with 3 mg/kg IgG1-AXL-148-vcMMAE, IgG1-AXL-183-vcMMAE (FN1 binder) and IgG1-AXL-726-vcMMAE (FN2 binder) induced growth inhibition of A431 tumors. In contrast, clones IgG1-AXL-061-vcMMAE and IgG1-AXL-137-vcMMAE did not show anti-tumor activity in the A431 xenograft model.

**Figure 14A** shows that treatment of mice with 3 mg/kg IgG1-AXL-061-vcMMAE, IgG1-AXL-137-vcMMAE, IgG1-AXL-148-vcMMAE, IgG1-AXL-183-vcMMAE and IgG1-AXL-726-vcMMAE induced tumor regression in the LCLC-103H xenograft model. Similarly, treatment of mice with 1 mg/kg IgG1-AXL-107-vcMMAE or 1 mg/kg IgG1-AXL-613-vcMMAE induced regression of LCLC-103H tumors **(****Figure 14B****).**

In summary, all AXL-ADCs showed anti-tumor activity in the LCLC-103H xenograft model that produces low levels of Gas6. In the A431 xenograft model, that produces high levels of Gas6, anti-tumor activity was only observed for those AXL-ADCs that did not compete with the AXL ligand Gas6.

### Example 14 - AXL expression in different tumor indications

Expression of AXL was evaluated in freshly cut paraffin embedded and formalin fixated (FFPE) tumor tissue micro arrays (TMA) comprising tissue cores from patients with thyroid, esophageal, ovarian, pancreatic, lung, breast, cervical or endometrial cancer, or malignant melanoma. TMAs were obtained from US BioMax.

FFPE tumor array slides were deparaffinized and subjected to antigen retrieval (pH 6) and endogenous peroxidase was exhausted by incubation with 0.1% H2O2 in citrate/phosphate buffer. To detect AXL expression, the TMAs were incubated with rabbit-anti-AXL (Santa Cruz, cat nr: sc-20741) at a concentration of 1 µg/mL for 60 min (room temperature (RT)). To identify (tumor) cells of epithelial origin, TMAs were incubated with rabbit-anti-cytokeratin (Abcam, cat. Nr. ab9377) at a dilution of 1:50 for 60 min (RT). After a washing step, the TMAs were incubated with peroxidase conjugated, anti-rabbit IgG dextran polymer (ImmunoLogic, cat no: DPVR55HRP) to detect binding of rabbit Anti-AXL and rabbit anti-cytokeratin antibodies. Finally, binding of anti-rabbit IgG dextran polymer was visualized with di-amino-benzadine (DAB; brown color; DAKO, cat no: K346811). In the TMA with malignant melanoma tissue cores, binding of anti-rabbit IgG dextran polymer was visualized with amino-ethyl carbazole (AEC; red color; Vector, SK4200). Nuclei in TMAs were visualized with hematoxylin (blue color).

AXL and cytokeratin immunostained TMAs were digitized with an Aperio slide scanner at 20x magnification and immunostaining was quantified with tissue image analysis software (Definiens Tissue Studio software, version 3.6.1), using a cell-based algorithm. The algorithm was designed to identify and quantify the percentage of AXL- or cytokeratin-positive cells in the biopsies (range 0 - 100%) and to quantify AXL staining intensity in AXL-positive tumor cells (optical density (OD); range 0 - 3) in each tumor core. Tumor cells were scored AXL positive, when AXL OD was at least 0.1. The percentage of AXL positive tumor cells per tumor core (range 0 - 100%) was calculated by dividing the total number of AXL positive cells by the total number of cytokeratin-positive cells in sequential tumor cores. The average AXL staining intensity (OD) in each tumor core was calculated by dividing the sum of AXL OD of all AXL positive tumor cells by the number of AXL positive tumor cells.

Tumor array from patients with malignant melanoma were scored manually. AXL staining intensity was scored as either weak (1+), moderate (2+) or strong (3+) and the percentage AXL positive melanoma cells was scored in 10% intervals (range 0 - 100%).

**Figure 16** provides a graphical representation of AXL expression in tumor cores of thyroid, esophageal, ovarian, breast, lung, pancreatic, cervical and endometrial cancer. **Table 16** shows the percentage of tumor cores that showed AXL expression in more than 10% of tumor cells, for each indication. **Figure 17** shows a representative example of a tissue core immunostained for AXL, for each indication. The figures illustrate heterogeneous expression of AXL in the tumor issue.

**Table 16**

| **Tumor indication** | **Subtype** | **% tumor cores (patients) with >10% AXL-positive tumor cells** |
|---|---|---|
| Esophageal cancer | Adenocarcinoma (n=19) | 73 |
| | Squamous cell carcinoma (n=60) | 55 |
| Ovarian cancer | All subtypes (n=52) | 90 |
| Pancreatic cancer | All subtypes (n=58) | 60 |
| Lung cancer ( NSCLC) | Squamous cell carcinoma SSC (n=52) | 63 |
| | Adenocarcinoma (n=48) | 67 |
| Lung cancer (SCLC) | SCLC (n=5) | 60 |
| Thyroid cancer | All subtypes (n=48) | 92 |
| Uterine cancer | All subtypes (n=60) | 88 |
| Breast cancer | TNBC (n=54) | 24 |
| Cervical cancer | All subtypes (n=54) | 93 |
| Melanoma | Malignant melanoma (n=67) | 6 |

| | | |
|---|---|---|
| Abbreviations used: NSCLC, non small cell lung cancer; SLCL, small cell lung cancer;TNBC, triple negative breast cancer | | |

### Example 15 -AXL antibodies specifically bind AXL but not other TAM receptor family members.

### Expression of human AXL, MER, and TYRO3 in HEK-293F cells

The following codon-optimized constructs for expression of various full-length proteins were generated: human (Homo sapiens) AXL (Genbank accession no. NP_O68713.2), human MER (Genbank accession no. EAW52O96.1, and human TYRO3 (Genbank accession no. Q06418.1). The constructs contained suitable restriction sites for cloning and an optimal Kozak (GCCGCCACC) sequence (Kozak *et al.,* 1999). The constructs were cloned in the mammalian expression vector pcDNA3.3 (Invitrogen)

FreestyleTM 293-F (a HEK-293 subclone adapted to suspension growth and chemically defined Freestyle medium, (HEK-293F)) cells were obtained from Invitrogen and transfected with the expression plasmids using 293fectin (Invitrogen), according to the manufacturer's instructions and grown for 24-48 hours.

### Binding study of AXL antibodies to human AXL, human MER, or human TYRO3

HEK-293F cells transiently transfected with expression constructs for full length human AXL, MER, or TYRO3 were evaluated for binding of HuMab-AXL antibodies by flow cytometry. Transfected HEK-293F cells were incubated with serial dilutions of AXL-antibodies (4-fold dilutions; final concentration range 0.002-10 µg/mL) for 30 minutes at 4ºC. After washing three times in PBS/0.1% BSA/0.02% azide, cells were incubated with R-Phycoerythrin (PE)-conjugated goat-anti-human IgG F(ab')2 (Jackson ImmunoResearch Laboratories, Inc., West Grove, PA; cat. No. 109-116-098) diluted 1/100 in PBS/0.1% BSA/0.02% azide (final volume 100 µL). Next, cells were washed twice in PBS/0.1% BSA/0.02% azide, resuspended in 120 µL PBS/0.1% BSA/0.02% azide and analyzed on a FACS Cantoll (BD Biosciences). Staining with mouse anti-human Mer (R&D Systems, cat. Mab8912) and mouse anti-human Tyro3 (Dtk) (R&D Systems, cat. MAB859) were included as controls for expression, IgG1-b12 was included as a non-binding isotype control antibody. Binding curves were analyzed using non-linear regression (sigmoidal dose-response with variable slope) using GraphPad Prism V5.04 software (GraphPad Software, San Diego, CA, USA).

**Figure 18A** shows that Humab-AXL antibodies showed dose-dependent binding to the HEK293 cells expressing human AXL. In contrast, no binding of HuMab-AXL antibodies to cells expressing MER **(****Figure 18B****)** or TYRO3 **(****Figure 18C****)** or to untransfected HEK293 cells **(****Figure 18D****)** was observed. Staining with MER- and Tyro3-specific antibodies confirmed that transfected cells showed proper expression of MER **(****Figure 18F****)** or TYRO3 **(****Figure 18G****),** respectively.

### Example 16 - Internalization of cell surface bound AXL antibodies

### Internalization of cell surface bound HuMab-AXL evaluated by flow cytometry

Internalization of cell surface bound HuMab-AXL antibodies to MDA-MB-231 and Calu-1 cells (human lung carcinoma cell line; ATCC, catalognumber HTB-54) was evaluated by flow cytometry. 50,000 cells were seeded in 96-well tissue culture plates and allowed to attach for 6 hrs at 37°C. Plates were incubated at 4°C for 30 minutes before incubation with serial dilutions of AXL-antibodies (final concentration range 0.0032-10 µg/mL) at 4°C for 1 hour. Subsequently, the medium was replaced by tissue culture medium without antibody and cells were incubated overnight (16-18 hours) at 37°C or 4°C. Subsequently, the cells were detached with 40 µL warm trypsin solution, washed with ice-cold PBS/0.1% BSA/0.02% azide, and incubated for 30 minutes at 4°C with R-Phycoerythrin (PE)-conjugated goat-anti-human IgG F(ab')2 (Jackson ImmunoResearch Laboratories, Inc., West Grove, PA; cat. No. 109-116-098) diluted 1/100 in PBS/0.1% BSA/0.02% azide (final volume 100 µL), to detect AXL-antibodies on the cell surface. Finally, cells were washed twice in PBS/0.1% BSA/0.02% azide, resuspended in 120 µL PBS/0.1% BSA/0.02% azide and analyzed on a FACS Cantoll (BD Biosciences).

Binding curves were analyzed using non-linear regression (sigmoidal dose-response with variable slope) using GraphPad Prism V5.04 software (GraphPad Software, San Diego, CA, USA).

**Figure 19** shows that, for all AXL HuMab antibodies and at all concentrations tested, more antibody was detected on the plasma membrane of cells that had been incubated at 4°C after antibody binding, compared to cells that had been incubated at 37°C. This illustrates that, at 37°C, AXL antibodies are internalized upon binding to the plasma membrane.

### Fab-TAMRA/QSY7 internalization and intracellular degradation assay

Internalization of AXL antibodies was assessed in the Fab-TAMRA/QSY7 internalization assay. This assay uses a fluorophore (TAMRA) and quencher (QSY7) pair. In close proximity, for example, upon conjugation to the same protein, TAMRA fluorescence is quenched by QSY7. In this example, goat-anti-human IgG Fab-fragments (Jackson Immunoresearch) were conjugated with TAMRA/QSY7 (Fab-TAMRA/QSY7) as described (Ogawa et al., Mol Pharm 2009;6(2):386-395), and AXL HuMab (1.5 µg/mL) were preincubated with Fab-TAMRA/QSY7 (12 µg/mL; 30 min, 4ºC). The complex was subsequently added to LCLC-103H cells and incubated for 24 h incubation in the dark, under shaking conditions (200 rpm, 37ºC). Internalization of the HuMab-Fab-TAMRA/QSY7 complex and intracellular degradation in the endosomes and lysosomes causes dissociation of TAMRA/QSY7, resulting in dequenching of TAMRA. TAMRA fluorescence of LCLC-103H cells that had been incubated with AXL antibodies complexed with Fab-TAMRA/QSY7 was measured on a FACS Canto-II (BD Biosciences).

As shown in **Figure 20****,** the fluorescence intensity of LCLC-103H cells was enhanced upon incubation with AXL-antibody-Fab-TAMRA/QSY7 complex, compared to IgG1-b12-Fab-TAMRA/QSY7 or Fab-TAMRA/QSY7 alone. This illustrates that AXL antibodies are internalized upon binding to LCLC-103H cells.

### Example 17-Anti-tumor efficacy of AXL-ADCs in an esophageal cancer patient-derived xenograft (PDX) model

The anti-tumor activity of IgG1-AXL-107-vcMMAE (also referred to as "HuMax-AXL-ADC" herein) was evaluated in the subcutaneous esophageal PDX model ESO195 in BALB/c nude mice (experiments performed by Crown Bioscience. Taicang Jiangsu Province, China). Tumor fragments from donor mice bearing patient-derived esophageal xenografts (ESO195) were used for inoculation into BALB/c nude mice. Each mouse was inoculated subcutaneously at the right flank with one tumor fragment (2-3 mm in diameter) and tumors were allowed to grow until the tumor volume was about 150 mm³. Randomization of animals was performed as follows: animals bearing a tumor with a volume of about 150 mm³ were distributed in 5 experimental groups (8 animals per group), considering a comparable median and mean of group tumor volume. The treatment groups were: IgG1-b12, IgG1-b12-vcMMAE, IgG1-AXL-107, IgG1- AXL-107-vcMMAE, and paclitaxel. The antibodies and ADCs were dosed intravenously (i.v.) at 4 mg/kg at day of randomization (day 0) and day 7. Paclitaxel was dosed intra-peritoneally (i.p.) at 20 mg/kg at day 0, 7, and 14. Tumor volumes (mm³) were monitored twice weekly and were calculated from caliper (PLEXX) measurements as: 0.52 x (length) x (width)².

**Figure 21** shows that treatment of mice with IgG1-AXL-107-vcMMAE induced tumor regression of ES0195 tumors compared to the IgG1-b12 and IgG1-b12-MMAE control groups (p<0.001 at day 23, one-way ANOVA test). Treatment of mice with the untargeted ADC IgG1-b12-vcMMAE did not show anti-tumor activity in this model, illustrating that the therapeutic capacity of AXL-ADCs depends on specific target binding. Mice that were treated with paclitaxel showed tumor growth inhibition, but this was less effective compared to treatment with IgG1-AXL-107-vcMMAE (p<0.05 at day 23, one-way ANOVA test).

### Example 18 - Anti-tumor efficacy of AXL-ADC in a cervical cancer patient-derived xenograft (PDX) model

The anti-tumor activity of IgG1-AXL-183-vcMMAE and IgG1-AXL-726-vcMMAE was evaluated in the patient derived cervix carcinoma xenograft CEXF 773 model in NMRI nu/nu mice (Harlan, Netherlands). Experiments were performed by Oncotest (Freiburg, Germany).

Tumor fragments were obtained from xenografts in serial passage in nude mice. After removal from donor mice, tumors were cut into fragments (4-5 mm diameter) and placed in PBS (with 10% penicillin/streptomycin) until subcutaneous implantation. Mice under isofluorane anesthesia received unilateral, subcutaneous tumor implants in the flank. Tumors were allowed to grow until the tumor volume was 50-250 mm³.

Randomization of animals was performed as follows: animals bearing a tumor with a volume of 50-250 mm³ were distributed in 4 experimental groups (8 animals per group), considering a comparable median and mean of group tumor volume. The treatment groups were: IgG1-b12, IgG1-b12-vcMMAE, IgG1-AXL-183-vcMMAE and IgG1-AXL-726-vcMMAE. The antibodies and ADCs were dosed intravenously (i.v.) at 4 mg/kg on the day of randomization (day 0) and on day 7. Tumor volumes (mm³) were monitored twice weekly and were calculated from caliper (PLEXX) measurements as: 0.52 x (length) x (width)².

**Figure 22** shows that treatment of mice with IgG1-AXL-183-vcMMAE or IgG1-AXL-726-vcMMAE induced tumor regression of CEXF 773 tumors compared to the IgG1-b12 and IgG1-b12-MMAE control groups. Treatment of mice with the untargeted ADC IgG1-b12-vcMMAE did not show anti-tumor activity in this model, illustrating that the therapeutic capacity of AXL-ADCs depends on specific target binding. Statistical analysis of tumor size at day 28 (Kruskal-Wallis and Mantel-Cox using a tumor size cut-off 500 mm³), showed that the average tumor size in mice treated with IgG1-AXL-183-vcMMAE or IgG1-AXL-726-vcMMAE was significantly smaller than in mice that had been treated with IgG1-b12 and IgG1-b12-vcMMAE (p<0.001). IgG1-AXL-183-vcMMAE and IgG1-AXL-726-vcMMAE were equally effective.

### Example 19 - Anti-tumor efficacy of AXL-ADCs in an orthotopic breast cancer xenograft model

The anti-tumor activity of IgG1-AXL-183-vcMMAE and IgG1-AXL-726-vcMMAE was evaluated in in an orthotopic MDA-MB-231 D3H2LN xenograft model.

MDA-MB-231-luc D3H2LN Bioware cells (mammary gland adenocarcinoma; Perkin Elmer, Waltham, MA) were implanted in the mammary fat pad of 6-11 week old, female SCID (C.B-17/IcrPrkdc-scid/CRL) mice (Charles-River) under isofluorane anesthesia. Tumors were allowed to grow and mice were randomized when tumors reached a volume of ^{~}325 mm³. Therefore, mice were distributed in 4 experimental groups (6-7 animals per group), considering a comparable median and mean of group tumor volume. The treatment groups were: IgG1-b12, IgG1-b12-vcMMAE, IgG1-AXL-183-vcMMAE and IgG1-AXL-726-vcMMAE. The animals received a total of 4 biweekly doses of 3 mg/kg antibody or ADC starting at the day of randomization. Tumor volumes (mm³) were monitored twice weekly and were calculated from caliper (PLEXX) measurements as: 0.52 x (length) x (width)².

**Figure 23** shows that treatment of mice with IgG1-AXL-183-vcMMAE or IgG1-AXL-726-vcMMAE induced tumor regression of MDA-MB-231 tumors compared to the IgG1-b12 and IgG1-b12-MMAE control groups. Treatment of mice with the untargeted ADC IgG1-b12-vcMMAE did not show anti-tumor activity in this model, showing that the therapeutic capacity of AXL-ADCs depends on specific target binding. Statistical analysis of tumor size at day 32 (One Way Anova test), showed that the average tumor size in mice that had been treated with IgG1-AXL-183-vcMMAE or IgG1-AXL-726-vcMMAE was significantly smaller than in mice that had been treated with IgG1-b12 and IgG1-b12-vcMMAE (P<0.001). No differences were observed between the IgG1-AXL-183-vcMMAE and IgG1-AXL-726-vcMMAE treatment groups, illustrating that these induced equally effective anti-tumor activity.

### Example 20 - In vitro cytotoxicity induced by AXL-specific antibody drug conjugates is dependent on target expression

The *in vitro* cytotoxicity of IgG1-AXL-107-vcMMAE was tested in human tumor cell lines with different levels of AXL expression.

### Cell culture

LS174T cells (human colorectal adenocarcinoma cell line; ATCC, cat no CL-188) were cultured in Minimum Essential Medium (MEM) with Glutamax, Hepes and Phenol Red (Life Technologies, cat no 42360-024). Components are 10% Donor Bovine Serium with Iron (DBSI) (Life Technologies, cat no 10371-029) and 1% Sodium Pyruvate (100 mM; Lonza, cat no BE13-115E) and 1% Penicillin/Streptomycin (Lonza, cat no DE17-603E).

NCI-H226 cells (human lung squamous cell carcinoma; ATCC, cat no CRL-5826), NCI-H661 cells (human large cell lung cancer; ATCC, cat no HTB-183), and NCI-H1299 cells (human non-small cell lung cancer; ATCC, cat no CRL-5803) were cultured in RPMI 1640 Medium (ATCC Modification; Life Technologies, cat no A10491-01). Components are 10% Donor Bovine Serium with Iron (DBSI; Life Technologies, cat no 10371-029) and 1% Penicillin/Streptomycin (Lonza, cat no DE17-603E).

SKOV-3 cells (human ovarian adenocarcinoma; ATCC, cat no HTB-77) were cultured in McCoy's 5A Medium with L-glutamine and HEPES (Lonza, cat no BE12-168F). Components are 10% Donor Bovine Serium with Iron (DBSI; Life Technologies, cat no 10371-029) and 1% Penicillin/Streptomycin (Lonza, cat no DE17-603E).

Calu-1 cells (human lung epidermoid carcinoma; ATCC, cat no HTB-54) were cultured in McCoy's 5A Medium with Catopeptone, without HEPES (Life Technologies, cat no 26600-023). Components are 10% Donor Bovine Serium with Iron (DBSI; Life Technologies, cat no 10371-029) and 1% L-glutamine (200 nM) in 0.85% NaCl solution (Lonza, cat no BE17-605F) and 1% Penicillin/Streptomycin (Lonza, cat no DE17-603E). Calu-1 cells are resistant to EGFR targeted therapy.

LCLC-103H cells (human large cell lung cancer), A431 cells (human epidermoid adenocarcinoma) and MDA-MB-231 cells (human breast cancer) were cultured as described in Example 8.

### Quantification of AXL expression on the plasma membrane of human tumor cell lines

AXL expression on the plasma membrane of human tumor cell lines was assessed by indirect immunofluorescence using QIFIKIT (DAKO, Cat nr K0078) with mouse monoclonal antibody Z49M (Santa Cruz biotechnology, Cat nr sc-73719). Adherent cells were trypsinized and passed through a cell strainer to obtain single cell suspensions. Cells were pelleted by centrifugation for 5 minutes at 1,200 rpm, washed with PBS and resuspended at a concentration of 1x10⁶ cells/mL. The next steps were performed on ice. 100 µL of the single cell suspensions (100,000 cells per well) were seeded in polystyrene 96-well round-bottom plates (Greiner Bio-One, Cat nr 650101). Cells were pelleted by centrifugation for 3 minutes at 300xg and resuspended in 50 µL antibody sample or mouse IgG1 isotype control sample (BD/Pharmingen, Cat nr 555746) at a concentration of 10 µg/mL. After an incubation of 30 minutes at 4ºC, cells were pelleted and resuspended in 150 µL FACS buffer. Set-up and calibration beads were added to the plate according to the manufacturer's instructions. Cells and beads in parallel were washed two more times with 150 µL FACS buffer and resuspended in 50 µL FITC-conjugated goat-anti-mouse IgG (1/50; DAKO, Cat nr K0078). Secondary antibody was incubated for 30 minutes at 4ºC in the dark. Cells and beads were washed twice with 150 µL FACS buffer and resuspended in 100 µL FACS buffer. Immunofluorescence was measured on a FACS Canto II (BD Biosciences) by recording 10,000 events within the gate of viable cells. The mean fluorescence intensity of the calibration beads was used to calculate the calibration curve using GraphPad Prism software (GraphPad Software, San Diego, CA, USA). For each cell line, the antibody binding capacity (ABC), an estimate for the number of AXL molecules expressed on the plasma membrane, was calculated using the mean fluorescence intensity of the AXL antibody-stained cells, based on the equation of the calibration curve (interpolation of unknowns from the standard curve, using GraphPad Software).

### Cytotoxicity assay

For LCLC-103H, A431, MDA-MB-231, NCI-H226, NCI-H661, NCI-H1299, LS174T and SKOV-3 cells, the *in vitro* cytotoxicity assay was performed as described in Example 8. For Calu-1, the cytotoxicity assay was performed as described in Example 8, with the adaptation that the cell cultures were incubated for 11 instead of 5 days. Dose-response curves were generated using Graphpad Prism software, using non-linear regression analysis. The percentage of viable cells at an IgG1-AXL-107-vcMMAE concentration of 1 µg/mL was interpolated from the dose-response curves.

As shown in **Figure 24****,** IgG1-AXL-107-vcMMAE induced the most potent cytotoxicity in cell lines with high AXL expression, whereas cytotoxicity was low or absent in cell lines with low AXL expression. The figure also illustrates that IgG1-AXL-107-vcMMAE is effective in induction of cytotoxicity in cells resistant to EGFR targeted therapy, such as Calu-1.

### Example 21 - Improved anti-tumor efficacy of IgG1-AXL-107-vcMMAE in combination with erlotinib in a NSCLC patient-derived xenograft (PDX) model.

### LU2511 PDX model

The anti-tumor activity of IgG1-AXL-107-vcMMAE was evaluated in the subcutaneous erlotinib-resistant NSCLC PDX model LU2511 in BALB/c nude mice (experiments performed by Crown Bioscience, Changping District, Beijing, China). Tumor fragments from donor mice bearing patient-derived NSCLC xenografts (LU2511) were used for inoculation into BALB/c nude mice. Each mouse was inoculated subcutaneously at the right flank with one tumor fragment (2-3 mm in diameter) and tumors were allowed to grow until the tumor volume was about 200 mm³. Randomization of animals was performed as follows: animals bearing a tumor with a volume of about 200 mm³ were distributed in 5 experimental groups (8 animals per group), considering a comparable median and mean of group tumor volume. The treatment groups were: IgG1-b12, IgG1-b12-vcMMAE, IgG1-AXL-107-vcMMAE, erlotinib, and erlotinib plus IgG1-AXL-107-vcMMAE. The antibodies and ADCs were dosed intravenously (i.v.) at 4 mg/kg on the day of randomization (day 0) and on day 7. Erlotinib was dosed orally (per os) at 50 mg/kg daily for 2 weeks. Tumor volumes (mm³) were monitored twice weekly and were calculated from caliper (PLEXX) measurements as: 0.5 x (length) x (width)².

**Figure 25** shows that treatment of mice with erlotinib did not induce anti-tumor activity, which was expected. IgG1-AXL-107-vcMMAE induced tumor growth inhibition of LU2511 tumors compared to the IgG1-b12 (p<0.01 at day 10, one-way ANOVA test; **Figure 25B**) and IgG1-b12-MMAE (p<0.05 at day 10, one-way ANOVA test; **Figure 25B**) control groups. Treatment of mice with the combination of IgG1-AXL-107-vcMMAE and erlotinib induced more potent anti-tumor activity than IgG1-AXL-107-vcMMAE alone in this model (p<0.05 at day 17, one-way ANOVA test; **Figure 25C**).

### LU0858 PDX model

The anti-tumor activity of IgG1-AXL-107-vcMMAE was evaluated in the subcutaneous erlotinib-resistant NSCLC PDX model LU0858 in BALB/c nude mice (experiments performed by CrownBioscience, Changping District, Beijing, China). Inoculation of tumor fragments into BALB/c nude mice and randomization was performed as described above.

Treatment with IgG1-AXL-107-vcMMAE (2 or 4 mg/kg) was performed at day 0 and 7 after randomization of the groups **(****Figure 32****).** IgG1-AXL-107-vcMMAE treatment in combination with EGFR inhibitor erlotinib was also tested. Erlotinib was given daily for 14 days at a dose of 50 mg/kg. Erlotinib alone, IgG1-b12-vcMMAE and IgG1-b12 were used as controls. Erlotinib alone had no effect on tumor growth. At 2 mg/kg, IgG1-AXL-107-vcMMAE alone had no effect on tumor growth. At 4 mg/kg, IgG1-AXL-107-vcMMAE alone induced tumor growth inhibition compared to the IgG1-b12-vcMMAE control. The combination of 4 mg/kg IgG1-AXL-107-vcMMAE with erlotinib did not improve the outcome versus IgG1-AXL-107-vcMMAE alone **(****Figure 32****).** Addition of erlotinib to the 2 mg/kg IgG1-AXL-107-vcMMAE treatment led to similar growth inhibition as the group that received 4 mg/kg IgG1-AXL-107-vcMMAE.

### LU1868 PDX model

The anti-tumor activity of IgG1-AXL-107-vcMMAE was evaluated in the subcutaneous erlotinib-resistant NSCLC PDX model LU1858 in BALB/c nude mice (experiments performed by CrownBioscience, Changping District, Beijing, China). Inoculation of tumor fragments into BALB/c nude mice and randomization was performed as described above.

Treatment with IgG1-AXL-107-vcMMAE (2 or 4 mg/kg) was performed at day 0 and 7 after randomization of the groups. IgG1-AXL-107-vcMMAE treatment in combination with EGFR inhibitor erlotinib was also tested. Erlotinib was given daily for 14 days at a dose of 50 mg/kg. Treatments with erlotinib alone, IgG1-b12-vcMMAE or IgG1-b12 were included as controls **(****Figure 33****).**

Analysis by Mann-Whitney test was done on day 21 to compare treatment effects versus IgG1-b12 or IgG1-b12-vcMMAE, on day 28 to compare the effects of IgG1-AXL-107-vcMMAE 2 mg/kg alone versus IgG1-AXL-107-vcMMAE 2 mg/kg in combination with erlotinib, and on day 31 to compare the effects of IgG1-AXL-107-vcMMAE 4 mg/kg alone versus IgG1-AXL-107-vcMMAE 4 mg/kg in combination with erlotinib. Erlotinib alone had no effect on tumor growth. At 2 mg/kg and 4 mg/kg, IgG1-AXL-107-vcMMAE alone induced tumor growth inhibition, while the combination of IgG1-AXL-107-vcMMAE with erlotinib did not improve the outcome versus IgG1-AXL-107-vcMMAE alone **(****Figure 33****).**

### LXFA 526 PDX model

The anti-tumor activity of IgG1-AXL-107-vcMMAE was evaluated in the subcutaneous erlotinib-resistant NSCLC PDX model LXFA 526 (experiments performed by Oncotest, Freiburg, Germany). Inoculation of tumor fragments into 4-6 weeks old male NMRI nu/nu mice and randomization was performed as described above.

Treatment with IgG1-AXL-107-vcMMAE (2 or 4 mg/kg) was performed at day 0 and 7 after randomization of the groups **(****Figure 34****).** IgG1-AXL-107-vcMMAE treatment in combination with EGFR inhibitor erlotinib was also tested. Erlotinib was given daily for 14 days at a dose of 50 mg/kg. Erlotinib alone, IgG1-b12-vcMMAE and IgG1-b12 were used as control. Erlotinib alone had no effect on tumor growth. IgG1-AXL-107-vcMMAE induced tumor growth inhibition at a dose of 2 mg//kg, while at a dose of 4 mg/kg, IgG1-AXL-107-vcMMAE induced complete tumor regression in all mice at least until day 76. Combination treatment of IgG1-AXL-107-vcMMAE at dose levels of 2 mg/kg or 4 mg/kg with erlotinib showed similar antitumor activity compared to IgG1-AXL-107-vcMMAE alone **(****Figure 34****).**

### LXFA 677 and LXFA 677 3 PDX models

The anti-tumor activity of IgG1-AXL-107-vcMMAE was evaluated in the subcutaneous NSCLC PDX model LXFA 677 and the LXFA 677_3 model, which is derived from the LXFA 677 model and has acquired resistance to erlotinib (experiments performed by Oncotest, Freiburg, Germany). Inoculation of tumor fragments into 4-6 weeks old male NMRI nu/nu mice and randomization was performed as described above.

Treatment with IgG1-AXL-107-vcMMAE (2 or 4 mg/kg) was performed at day 0 and 7 after randomization of the groups. IgG1-AXL-107-vcMMAE treatment in combination with the EGFR inhibitor erlotinib was also tested. Erlotinib was given daily for 14 days at a dose of 50 mg/kg. Erlotinib alone, IgG1-b12-vcMMAE and IgG1-b12 were used as controls. Erlotinib induced partial tumor regression in the LXFA 677 model but had no effect on tumor growth in the erlotinib-resistant LXFA 677_3 model, as expected **(****Figure 35****).** IgG1-AXL-107-vcMMAE induced tumor growth inhibition at a dose of 2 mg/kg, while at a dose of 4 mg/kg, IgG1-AXL-107-vcMMAE induced partial tumor regression in the LXFA 677 model. In the erlotinib-resistant LXFA 677_3 model, IgG1-AXL-107-vcMMAE induced complete tumor regression at both dose levels, which lasted at least until day 41. In the two models, combination treatment of IgG1-AXL-107-vcMMAE at 4 mg/kg as well as 2 mg/kg with erlotinib induced similar antitumor activity compared to IgG1-AXL-107-vcMMAE alone **(****Figure 35****).**

**Table 17. Overview of Lung PDX models, EGFR mutational status and response to erlotinib and AXL-ADC.**

| **Model** | **EGFR status** | **Erlotinib resistance** |
|---|---|---|
| LU2511^{a} | WT | R |
| LU0858^{b} | L858R | R |
| LU1868^{b} | T790M/L858R | R |
| LXFA526 | WT | R |
| LXFA677^{c} | WT | sensitive |
| LXFA677_res3^{c} | WT | R |

| | | |
|---|---|---|
| ^{a} Yang et al. EORTC meeting 2013, Poster 493 (2013) ^{b} Yang et al. Int. J. Cancer: 132, E74-E84 (2013) ^{c} Tschuch et al. AACR-EORTC meeting 2015, Poster A10 (2015) | | |

### Example 22 - NSCLC cell lines that are resistant to the EGFR inhibitors erlotinib, gefitinib, and afatinib show enhanced Axl protein expression and enhanced sensitivity to IgG1-AXL-107-vcMMAE in vitro

The influence of acquired resistance to erlotinib on Axl protein expression in a panel of NSCLC cell lines was evaluated by Western blot analysis. Furthermore, the NSCLC cell lines were evaluated for their sensitivity to IgG1-AXL-107-vcMMAE *in vitro.*

### Cell culture and anticancer agents

All tissue culture materials were obtained from Gibco Life Technologies (Carlsbad, CA). The erlotinib-sensitive NSCLC adenocarcinoma cell line HCC827 was purchased from the ATCC. HCC827 cells are KRAS wildtype and harbor the exon19del mutation in EGFR (deletion of E746 - A750), which is associated with sensitivity to EGFR-TKIs. Cells were cultured in RPMI-1640 Glutamax medium supplemented with 10% fetal bovine serum (FBS) and 50 µg/mL penicillin-streptomycin and maintained in a humidified atmosphere with 5% CO₂ at 37ºC. EGFR inhibitors (erlotinib, gefitinib, and afatinib) were purchased from Selleck Chemicals (Houston, TX). Erlotinib and gefitinib were dissolved in DMSO, aliquoted and stored at -20°C.

### Short tandem repeat analysis

To confirm cell line authenticity, short tandem repeat (STR) analysis was performed using the Cell ID™ System (cat. G9500, Promega, Madison, USA) as described by the manufacturer. In brief, ten specific loci of the human genome were PCR amplified and analyzed by capillary electrophoresis. We found that ER10, ER20 and ER30 had the same allelic sizes at all ten loci as the parental HCC827 clone. We also found the allelic loci sizes to be identical to those published by ATCC.

### DNA purification and EGFR/KRAS mutation testing

DNA was extracted from the cells using the QIAamp DNA Mini Kit (Qiagen, Hilden, Germany), and EGFR and KRAS mutation status examined using the TheraScreen EGFR RGQ PCR kit and the TheraScreen KRAS RGQ PCR kits (Qiagen, Hilden, Germany) as described by the manufacturer.

### In vitro cytotoxicity assay to test cell line sensitivity to erlotinib or AXL-ADC

2000 cells/well (5000 cells in the case of ER20) were seeded in 96 well plates and allowed to adhere for 6-8h before adding erlotinib, gefitinib, afatinib, IgG1-AXL-107-vcMMAE or the isotype control ADC IgG1-b12-vcMMAE; then incubated at 37°C and 5% CO₂ for 5 days and quantified by Cell Titer Glo Assay (as described in Example 8). Untreated cells were used as reference for 100% cell growth. Plates were incubated for 4 or 5 days at 37°C, 5% CO₂. Crystal violet assay was performed by adding staining solution for 5 min at RT, washing cells twice in H₂O, redissolving in Na-citrate buffer (29.41g Na-citrate in 50% EtOH) and measuring the absorbance at 570 nm.

### Generation of erlotinib- or gefitinib-resistant NSCLC cell lines

Three isogenic erlotinib-resistant cell lines were generated from the HCC827 cell line, by continuous exposure to erlotinib. Cells were initially exposed to 1 µM erlotinib, and the erlotinib concentration was gradually increased to 20 µM or 30 µM, respectively, over a course of six months. Once cell lines had acquired resistance to erlotinib, they were cultured in culture medium as described above, supplemented with 20 µM or 30 µM erlotinib.

Similarly, one isogenic erlotinib-resistant cell line and 5 gefitinib-resistant cell lines were generated from the PC9 cell line, by continuous exposure to erlotinib or gefitinib. Cells were initially exposed to 1 µM erlotinib or gefitinib, and the TKI concentration was gradually increased to up to 30 µM over a course of six months.

### Western blotting

Expression of Axl was determined by Western blot analysis. Axl activation was determined by measuring the phosphorylation using phospho-specific antibodies. Cells were washed in ice cold TBS, spun down and lysed in RIPA buffer (10 mM Tris HCl pH 8, 5 mM Na2EDTA pH 8, 1% NP-40, 0,5% sodium dioxycholate, 0,1% SDS), containing both protease and phosphatase inhibitors (Complete Mini PhosphoSTOP, Roche, Basel, Switzerland). Protein concentrations were determined by Pierce BCA Protein Assay (Thermo Fisher Scientific, USA) according to the manufacturer's protocol. 5-40 µg protein was resolved on 4-12% RunBlue SDS-PAGE gels (Expedeon, San Diego, CA), transferred onto PVDF membrane (GE Healthcare Life Sciences, Denmark), blocked and then incubated with primary antibodies O/N at 4°C. The anti-actin antibody was purchased from Abcam (cat.no. ab8226) and the antibody against total AXL was purchased from R&D Systems (cat.no. AF154). Next, the membranes were incubated with goat anti-rabbit, goat anti-mouse (Dako, Denmark) or donkey anti-goat (Santa Cruz) HRP-conjugated secondary antibodies in 1:5000 dilution for 1h at room temperature. The immune reactive bands were visualized by Amersham ECL Prime Western Blotting Detecting Reagent (GE Healthcare Life Sciences, Buckinghamshire, UK) and exposed to CL-Xposure film (Thermo Fisher Scientific, USA).

### Results

The HCC827 wildtype cell line was highly sensitive to erlotinib treatment, with an IC₅₀ of approximately 0.005 µM. The erlotinib-resistant cell lines ER10, ER20 and ER30, which were generated by exposure to increasing concentrations of erlotinib for six months, were not sensitive to erlotinib (IC₅₀ >50 µM) **(Table 18).** The stability of the erlotinib-resistant phenotype was confirmed by culturing the ER10, ER20 and ER30 cell lines in absence of erlotinib for six weeks. After the six weeks, cell lines showed the same level of resistance to erlotinib. The mutational status of EGFR and KRAS of the erlotinib-resistant cell lines remained unchanged compared to the parental cell line **(Table 18).** The expression of Axl protein was upregulated in the HCC827-derived cell lines that had acquired resistance to erlotinib **(****Figure 26A****).** Axl upregulation was preserved when the cell lines were cultured in absence of erlotinib **(****Figure 26A****).**

Similarly, expression of Axl protein was upregulated in the PC9-derived cell lines that had acquired resistance to erlotinib or gefitinib **(****Figure 26B****).**

**Table 18. Characteristics of the parental HCC827 cell line and the derived erlotinib-resistant cell lines.**

| | **HCC827-wt** | **HCC827-ER10** | **HCC827-ER20** | **HCC827-ER30** |
|---|---|---|---|---|
| Erlotinib sensitivity | Sensitive | Resistant | Resistant | Resistant |
| IC₅₀ | 0.005 µM | >50 µM | >50 µM | >50 µM |
| Exposed to conc. of erlotinib | 0 µM | 10 µM | 20 µM | 30 µM |
| EGFR status | Exon19del | Exon19del | Exon19del | Exon19del |
| KRAS status | wt | Wt | wt | Wt |

The sensitivity of the wild type and erlotinib/gefitinib resistant HCC827 and PC9 cells to IgG1-AXL-107-vcMMAE was evaluated. Therefore, cells were exposed to increasing concentrations of IgG1-AXL-107-vcMMAE (range 10 µg/mL - 3.8 x 10⁻⁵ µg/mL) for 5 days after which the cell viability was determined. **Figure 27A and B** show that wild type HCC827 and PC9 cells are insensitive to treatment with IgG1-AXL-107-vcMMAE **(****Figure 27F** and **J)****,** but show strong reduced viability upon treatment with EGFR inhibitors **(****Figure 27C** and **I**). The HCC827-ER20 and HCC827-ER30 cell lines, with acquired resistance to the EGFR-TKI erlotinib, were also resistant to the EGFR-TKIs gefitinib and afatinib **(****Figure 27D** and **E****)** but showed reduced viability upon treatment with IgG1-AXL-107-vcMMAE **(****Figure 27A****).** The PC9-ER cell line with acquired resistance to the EGFR-TKI erlotinib **(****Figure 27I****)** also showed reduced viability upon treatment with IgG1-AXL-107-vcMMAE **(****Figure 27B** and **K****).** Treatment with the control ADC, IgG1-b12-vcMMAE, did not affect cell viability up to concentrations of 10 µg/mL in any of the cell lines tested **(****Figure 27F****, G, H, J, and K).**

### Example 23 - Resistance to the BRAF inhibitor PLX4720 is associated with upregulated Axl protein expression and enhanced sensitivity to IgG1-AXL-107-vcMMAE

In a panel of established human melanoma cell lines (CDX) and patient derived low passage melanoma cell lines (PDX), Axl protein expression and sensitivity to IgG1-AXL-107-vcMMAE were evaluated in relation to their intrinsic or acquired resistance to growth inhibition by treatment with the BRAF inhibitor PLX4720, an analogue to the clinically approved BRAF inhibitor vemurafenib.

### Cell culture

SKMEL147 was obtained from the Laboratory of Reuven Agami at the Netherlands Cancer Institute. A875 was obtained from Thermo Fischer, COLO679 from Sigma, SKMEL28 and A375 cells from ATCC. Melanoma cell lines were cultured in DMEM supplemented with 10% fetal bovine serum (Sigma), 100 U/ml penicillin and 0.1 mg/ml streptomycin (all Gibco). The cell lines were maintained at 37°C in a 5% (vol/vol) CO₂ humidified incubator.

### Generation of PLX4720 resistant cell lines

BRAF inhibitor sensitive cell lines (SKMEL28, and A375) were cultured in the presence of increasing concentrations of the BRAF inhibitor PLX4720 (Selleck Chemicals, Houston, TX, USA, Company: Selleck Chemicals, Houston, TX, USA, Catalog number: S1152,) up to 3 µM to establish the corresponding PLX4720 resistant SKMEL28R, and A375R. All drug-resistant cell lines were permanently cultured in the presence of 3 µM of PLX4720.

### Generation of patient derived low passage (PDX) melanoma cell lines

The Medical Ethical Board of the Antoni van Leeuwenhoek hospital, Netherlands Cancer Institute has approved the collection and use of human tissue. Animal experiments were approved by the animal experimental committee of the institute and performed according to applicable rules and regulations. Human tumor material was obtained during surgery, or by taking tumor biopsies from malignant melanoma patients using a 14-gauge needle. Tumor fragments of ^{~}5mm³ were used for subcutaneous implantation in NOD.Cg*-PrkdcS^{cid} Il2rg^{tm1Wjl}*/SzJ mice, which was performed under anesthesia. Tumor outgrowth was measured twice per week with a caliper. Before reaching the a tumor size of 1000 mm³, mice were sacrificed, tumors were removed and tumor pieces were dissociated into single cells suspensions, plated on 10-cm dishes and grown as primary cell cultures in DMEM + 10% FBS (Sigma) + 100 U/ml penicillin and 0.1 mg/ml streptomycin (all Gibco).

### Western blot analysis

Expression of Axl and MITF was determined using Western blot analysis. The proteins in the cell lysate were separated on a 4-12% SDS-PAGE gel and transferred to PVDF membrane that was subsequently stained with antibody specific for Axl (sc-1096 Santa Cruz) in 5% BSA in PBS-Tween, or to a nitrocellulose membrane stained with MITF (ab12039 Abcam) in 5% non-fat dry milk in PBS-Tween. To control for gel loading, antibodies against vinculin or beta-actin were used.

### Quantification of AXL expression on the plasma membrane of melanoma cell lines

AXL expression on the plasma membrane of human tumor cell lines was quantified by indirect immunofluorescence using QIFIKIT analysis (DAKO, Cat nr K0078). Axl was detected using the mouse monoclonal antibody ab89224 (Abcam, Cambridge, UK). Adherent cells were trypsinized and passed through a cell strainer to obtain single cell suspensions. Cells were pelleted by centrifugation for 5 minutes at 1,200 rpm, washed with PBS and resuspended at a concentration of 1x10⁶ cells/mL. The next steps were performed on ice. 100 µL of the single cell suspensions (100,000 cells per well) were seeded in polystyrene 96-well round-bottom plates (Greiner Bio-One, Cat nr 650101). Cells were pelleted by centrifugation for 3 minutes at 300xg and resuspended in 50 µL antibody sample or mouse IgG1 isotype control sample (cat number QF2040741, lot number MA1-10406, Pierce) at a concentration of 10 µg/mL. After an incubation of 30 minutes at 4ºC, cells were pelleted and resuspended in 150 µL FACS buffer (PBS containing 0.1 % BSA). Set-up and calibration beads were added to the plate according to the manufacturer's instructions. Cells and beads in parallel were washed two more times with 150 µL FACS buffer and resuspended in 50 µL FITC-conjugated goat-anti-mouse IgG (1/50; DAKO, cat.no. K0078). Secondary antibody was incubated for 30 minutes at 4ºC in the dark. Cells and beads were washed twice with 150 µL FACS buffer and resuspended in 100 µL FACS buffer. Immunofluorescence was measured on a FACS Calibur (BD Biosciences) by recording 10,000 events within the gate of viable cells. The mean fluorescence intensity of the calibration beads was used to calculate the calibration curve using GraphPad Prism software (GraphPad Software, San Diego, CA, USA). For each cell line, the antibody binding capacity (ABC), an estimate for the number of AXL molecules expressed on the plasma membrane, was calculated using the mean fluorescence intensity of the AXL antibody-stained cells, based on the equation of the calibration curve (interpolation of unknowns from the standard curve, using GraphPad Software).

### In vitro cytotoxicity

Cells were cultured to near confluency, after which cells were trypsinized, resuspended in culture medium and passed through a cell strainer (BD Falcon, cat.no. 352340) to obtain single cell suspensions. Cells were plated in a 96-well format using the following seeding densities: 2000 cells/well for established cell lines, 4000 cells/well for PDX-derived cell lines. IgG1-AXL-107-vcMMAE was added 4 hours after seeding. Serial dilutions (10-fold; final concentrations ranging from 0.0001 to 10 µg/mL) of IgG1-AXL-107-vcMMAE were prepared in culture medium and added to the plates. After 5 days (for CD samples) or 8 (PDX samples) days of incubation at 37ºC, 5% CO₂, CellTiter-Glo Reagent (Promega; cat.no. G7571) was added to the wells and the Luminescent Cell Viability Assay (Promega, Madison, WI) was performed according to the manufacturer's protocol. Luminescence was measured by the Infinite M200 microplate reader (Tecan) and viability was calculated as follows: % viability = (luminescence sample of interest - luminescence PAO)/(average luminescence of control vehicle treated - luminescence PAO), with PAO representing 5 µM phenyl arsine oxide for 100% cell killing.

### SKMEL147 melanoma xenograft model

The anti-tumor activity of IgG1-AXL-107-vcMMAE was evaluated in the subcutaneous melanoma model SKMEL147 in NMRI nude mice. Mice were subcutaneously injected in the left flank with 2.5x105 SKMEL147 melanoma cells, which express high levels of Axl (see Figure 28 and Table 15), that were resuspended 1:1 in matrigel in a total volume of 100 µL. Tumors were measured three times weekly with a caliper, and when tumors were 100 mm3 the animals were randomized over the following treatment groups: IgG1-b12 (4 mg/kg), IgG1-b12-vcMMAE (4 mg/kg), IgG1-107 (4 mg/kg), IgG1-107-vcMMAE (2 mg/kg), and IgG1-107-vcMMAE (4 mg/kg).

On day 12 and day 19 after tumor cell injection (day 1 and day 8 of randomization) the test compounds were injected into the tail vein of the animals in a total volume of 100 µL. Animals were sacrificed when the size of the tumor exceeded 1000 mm3.

### Treatment of a mixed population of SKMEL28 wild type cells and SKMEL28 cells resistant to PLX4720

SKMEL28 wild-type cells and SKMEL28 cells resistant to PLX4720 (SKMEL28-R) were transfected with expression vectors of the fluorophores mCherry (red) or GFP (green), respectively. Subsequently, cells were seeded in a 1:1 ratio, with 50.000 cells of each cell line in a 6-well plate (in total 100.000 cells/well). After 3 hours, the following compounds were added to the wells: IgG1-AXL-107-vcMMAE (1 µg/mL), IgG1-b12-MMAE (1 µg/mL; isotype control ADC), PLX4720 (10 µM; BRAF inhibitor), dabrafenib (1 µM; BRAF inhibitor), or trametinib (0.1 µM; MEK inhibitor). After 4 days, cells were trypsinized, washed once in PBS + 1% BSA and analyzed by flow cytometry.

### Immunohistochemistry

Expression of AXL was evaluated in freshly cut paraffin embedded and formalin fixated (FFPE) whole tissues (WT) with malignant melanoma. Staining was performed manually in Sequenza Slide Racks (Ted Pella Inc., Redding, CA, USA; cat. no. 36105).

Prior to staining, FFPE tissue slides were deparaffinized in 100% xylene (Sigma-Aldrich, cat. no. 16446; three times, 5 min.) and dehydrated in 96% ethanol (Sigma Aldrich, cat. no. 32294; two times, 5 min.) at RT. Thereafter, antigen retrieval was performed. IHC slides were incubated in citrate buffer (pH6; DAKO; cat. no. S2369) for 5 min. and blocked for endogenous peroxidase in citrate/phosphate buffer (0.43 M citric acid, 0.35 M Na₂HPO₄.2H₂O; pH5.8) at RT for 15 min. Slides were incubated in 10% normal human serum (CLB/Sanquin, cat. no. K1146) in PBS, prior to incubation with primary antibodies. Axl expression was determined by incubation with 3 µg/mL rabbit polyclonal anti-human Axl antibody H-124 in PBS supplemented with 2% normal human serum at RT for 60 min. Slides were washed in PBS supplemented with 0.1% Tween-20 (twice, 3 min.) and binding of rabbit antibodies specific for Axl were detected with undiluted Bright Vision poly-HRP-anti-rabbit IgG. HRP was visualized with 3-amino-9-ethylcarbazole (AEC) chromophore (red color; Sigma, cat. no. A6926-100TAB); nuclei were counterstained with hematoxylin (DAKO, cat. no. S3309). Slides were analyzed by a certified pathologist at the Netherlands Cancer Institute (NKI, Amsterdam, The Netherlands), who scored the intensity and localization of Axl staining in each sample. Examples are shown in Figure 39.

### Results

AXL expression was evaluated in a panel of established melanoma cell lines **(Table 19)** and low passage primary melanoma lines (PDX, **Table 20**). AXL expression, as determined by western blot **(****Figure 28****),** was inversely correlated with MITF expression in established cell lines **(****Figure 28A****)** as well as clinical patient-derived samples **(****Figure 28B****).** In the established cell line panel, Axl expression was also determined by quantitative flow cytometry. An example of an AXL negative and positive cell line is shown in **Figure 29****.** Axl expression levels (expressed as ABC) for all cell lines are listed in **Table 19,** along with the BRAF mutation status of the cell lines.

Next, sensitivity of the established melanoma cell lines and PDX panel to IgG1-AXL-107-vcMMAE was evaluated in viability assays. Cells were exposed to increasing concentrations of IgG1-AXL-107-vcMMAE (range 1 x 10⁻⁴ to 10 µg/mL) for 5 days after which the cell viability was determined. Results are summarized in **Table 19 and 20,** dose-response curves are shown in **Figure 30** **and** **31****.** **Figure 30** shows that all 4 AXL expressing cell lines (SKMEL147, A875, A375R, SKMEL28R), three of which were resistant to PLX4720, are sensitive to treatment with IgG1-AXL-107-vcMMAE. The two AXL negative cell lines COLO679 and SKMEL28 did not show changes in viability upon treatment with IgG1-AXL-107-vcMMAE. Three PLX4720-resistant PDX samples were tested in viability assays with IgG1-AXL-107-vcMMAE. **Figure 31** shows that the two AXL high expressing PDX cultures, MO16 and MO19R, were sensitive to treatment with IgG1-AXL-107-vcMMAE, whereas the AXL low expressing PDX culture M082 did not show a different response from that seen with the IgG1-b12-vcMMAE control treatment.

**Table 19. Characteristics of the melanoma cell line panel.**

| **Cell line** | **AXL expression (western blot)** | **AXL expression (FACS)** | **BRAF** | **NRAS** | **PLX4720 sensitivity** | **HuMax-AXL-ADC sensitivity** |
|---|---|---|---|---|---|---|
| | | **Receptor number (ABC)** | | | | |
| SKMEL147 | + | 34981 | wt | Q61R | resistant | Sensitive |
| A875 | + | 37079 | V600E | wildtype | sensitive | Sensitive |
| COLO679 | - | BLQ | V600E | Wt | untested | Resistant |
| A375R | + | 14228 | V600E | Wt | resistant | Sensitive |
| SKMEL28 | - | BLQ | V600E | Wt | sensitive | Resistant |
| SKMEL28R | + | 63809 | V600E | Wt | resistant | Sensitive |

| | | | | | | |
|---|---|---|---|---|---|---|
| *BLQ = Below Limit of Quantitation (<3300, lowest ABC value of calibration beads) | | | | | | |

**Table 20. Characteristics of the patient-derived melanoma cultures**

| **Name** | **BRAF/NRAS status** | **AXL expression (western Blot)** | **AXL expression Receptor number (ABC, FACS)** | **PLX4720 sensitivity** | **HuMax-AXL-ADC sensitivity** |
|---|---|---|---|---|---|
| M016 | NRAS^{Q61R} | + | 13688 | resistant | Sensitive |
| M019R | BRAF^{V600E} | ++ | 25988 | resistant | Sensitive |
| M082 | BRAF^{V600E} | (low) | 3376 | resistant | Insensitive |

In the SKMEL147 melanoma xenograft model, mice treated with IgG1-b12, IgG1-b12-vcMMAE, or IgG1-AXL-107 did not show tumor growth inhibition. IgG1-AXL-107-vcMMAE induced tumor growth inhibition at 2 mg/kg, and at a dose of 4 mg/kg IgG1-AXL-107-vcMMAE induced strong tumor regression, which lasted until around day 50 **(****Figure 36A****).**

HuMax-AXL-ADC at a dose of 4 mg/kg thus showed a profound anti-tumor effect, but tumors started to grow out again after day 50. Four mice that showed tumor regrowth upon initial tumor regression with 4 mg/kg IgG1-AXL-107-vcMMAE were retreated with a single dose of 4 mg/kg IgG1-AXL-107-vcMMAE on days 55, while for comparison two other mice were observed.

Retreatment with 4 mg/kg IgG1-AXL-107-vcMMAE resulted in tumor regression in all four mice, whereas the 2 mice that were observed, showed tumor growth **(****Figure 36B****).** Two of the four retreated mice showed tumor regression that remained at least until day 80, while tumor regrowth was observed around day 70 in the two other retreated mice **(****Figure 36B****).**

In the mixed population of SKMEL28 wt cells and SKMEL28 PLX4720-resistant cells, compared to the untreated control, total cell numbers were reduced with 74-62 % when cell mixtures were treated with IgG1-AXL-107-vcMMAE, PLX4720, or dabrafenib **(****Figure 37A****).** Treatment of cell mixtures with the combinations of IgG1-AXL-107-vcMMAE and PLX4720, IgG1-AXL-107-vcMMAE and dabrafenib, dabrafenib and trametinib, or dabrafenib, trametinib and IgG1-AXL-107-vcMMAE induced 81-92 % reduction of total cell numbers compared to untreated cells **(****Figure 37A****).**

To evaluate if specific cell populations were eradicated, the ratio of green (GFP-positive SKMEL28-R cells) and red (mCherry-positive SKMEL28 cells) was determined. As expected, untreated and IgG1-b12-vcMMAE treatment did not affect the GFP/mCherry ratio, as total cell numbers were also unaffected **(****Figure 37B****).** Treatment with IgG1-AXL-107-vcMMAE resulted in a strongly reduced GFP/mCherry ratio **(****Figure 37B****),** indicating specific killing of SKMEL28-R cells. Conversely, treatment with BRAF inhibitors PLX4720 or dabrafenib increased the GFP/mCherry ratio **(****Figure 37B****),** indicating specific killing of SKMEL28 cells. Combinations of IgG1-AXL-107-vcMMAE and PLX4720, dabrafenib and trametinib, or dabrafenib, trametinib and IgG1-AXL-107-vcMMAE showed ratios closer to 1 **(****Figure 37B****),** indicating that both cell types were killed with similar efficacy. Treatment with the combination of IgG1-AXL-107-vcMMAE and dabrafenib resulted in a strongly reduced GFP/mCherry ratio **(****Figure 37B****),** indicating more efficient killing of SKMEL28-R cells at the concentrations used.

### Results IHC

In total 45 samples were analyzed, of which 3 did not contain any tumor material and were thus excluded from analysis. In addition, 7 matched pre - and post vemurafenib samples from the same patients were included, and 1 matched pre - and post dabrafenib/trametinib sample.

In 41/42 samples Axl expression was detected in subsets of the melanoma region. Staining intensity differed per patient tumor (Table 21).

Furthermore, up regulation of Axl expression (as measured by increase of staining intensity by pathologist) was observed in 4/7 matched pre- and post vemurafenib samples (Table 21).

**Table 21. Axl staining in tumor tissue from melanoma patients.**

| **Case nr.** | **Treatment** | **Pre-/post-treatment** | **Matched sample** | **Axl staining tumor cells^{a}** | **Comments** |
|---|---|---|---|---|---|
| 1 | vemurafenib | post | NA | Partially + | |
| 2 | vemurafenib | post | 17 | Weakly + to + | |
| 3 | dabr/tram | post | NA | ++ to +++ | |
| 4 | vemurafenib | post | NA | Focally + | |
| 5 | vemurafenib | post | NA | Partially weakly + | |
| 6 | dabr/tram | post | 40 | NA | very necrotic |
| 7 | dabr/tram | pre | 16 | Sporadic + | |
| 8 | vemurafenib | post | 38 | Sporadic + | the weakly positive cells at the edge of the tumor could be the result of staining artefact |
| 9 | vemurafenib | post | NA | - | |
| 10 | vemurafenib | post | NA | Partially weakly + | |
| 11 | vemurafenib | post | NA | Weakly + | many melanophages + |
| 12 | vemurafenib | post | NA | Locally weakly + | some melanophages + |
| 13 | vemurafenib | post | NA | ++ to +++ | |
| 14 | vemurafenib | post | 39 | Weakly + | many melanophages + |
| 15 | vemurafenib | post | 24 | Weakly + | |
| 16 | dabr/tram | post | 7 | Weakly + | |
| 17 | vemurafenib | pre | 2 | Partially + | |
| 18 | vemurafenib | 18 stable disease post | NA | Weakly + | |
| 19 | vemurafenib | post | NA | Locally + to ++ | |
| 20 | vemurafenib | 20 stable disease post | NA | Weakly + | |
| 21 | vemurafenib | post | NA | Weakly + | |
| 22 | vemurafenib | post | NA | Partially + | many melanophaqes + |
| 23 | vemurafenib | post | NA | + to ++ | |
| 24 | vemurafenib | pre | 15 | Sporadic + | |
| 25 | vemurafenib | post | NA | Sporadic + | |
| 26 | vemurafenib | pre | 44 | Weakly + | many melanophaqes + |
| 27 | vemurafenib | post | NA | Partially and weakly + | |
| 28 | vemurafenib | 28 stable disease post | NA | Weakly + | limited amount of tumor cells are present |
| 29 | vemurafenib | post | NA | Partially and weakly + | |
| 30 | vemurafenib | post | NA | Partially + | |
| 31 | vemurafenib | post | NA | Partially + | |
| 32 | vemurafenib | post | NA | + | small amount of tumor cells/melanophages with melanin |
| 33 | vemurafenib | post | NA | Locally weakly + | |
| 34 | vemurafenib | post | NA | Weakly + to + | |
| 35 | vemurafenib | post | NA | Weakly + | |
| 36 | vemurafenib | post | NA | weakly + | many melanophaqes + |
| 37 | vemurafenib | post | NA | Partially weakly + | |
| 38 | vemurafenib | pre | 8 | Weakly + to + | |
| 39 | vemurafenib | pre | 14 | + | the positive cells are present in the sinuses of the lymph nodes. It is not certain whether they are tumor cells or macrophage since these cells contain rather rich cytoplasm |
| 40 | dabr/tram | pre | 6 | NA | no neoplastic lesions are encountered |
| 41 | vemurafenib | post | NA | NA | no neoplastic lesions are encountered |
| 42 | vemurafenib | post | NA | Partially + | partial negative areas could be due to staining artefact |
| 43 | vemurafenib | post | NA | Weakly + to + | |
| 44 | vemurafenib | post | 26 | + to ++ | |
| 45 | vemurafenib | post | NA | Partially weakly + | |

| | | | | | |
|---|---|---|---|---|---|
| ^{a}-: negative; positive staining intensity: weakly +< + < ++ < +++; positive staining area: sporadic < focal < local < partial; NA: not available | | | | | |

### Example 24 - CV1664 PDX model

The anti-tumor activity of IgG1-AXL-107-vcMMAE was evaluated in the subcutaneous cervical cancer PDX model CV1664 in BALB/c nude mice (experiments performed by CrownBioscience, Changping District, Beijing, China). Inoculation of tumor fragments into BALB/c nude mice and randomization was performed as described in Example 21.

Treatment with IgG1-AXL-107-vcMMAE (2 or 4 mg/kg) was performed at day 0 and 7 after randomization of the groups **(****Figure 38****).** Treatment on the same days with paclitaxel (20 mg/kg; intraperitoneally), unconjugated IgG1-AXL-107 (4 mg/kg), IgG1-b12-vcMMAE (4 mg/kg) and IgG1-b12 (4 mg/kg) were used as controls.

IgG1-AXL-107-vcMMAE induced strong tumor regression at both dose levels, which lasted at least until day 49 **(****Figure 38A****, B).** Treatment with unconjugated IgG1-AXL-107 and IgG1-b12-vcMMAE only induced minor inhibition of tumor growth compared to the IgG1-b12 control group. Paclitaxel induced partial tumor regression.

Two mice that showed tumor regrowth upon initial tumor regression with 4 mg/kg IgG1-AXL-107-vcMMAE were retreated with 2 doses of 4 mg/kg IgG1-AXL-107-vcMMAE on days 55 and 62. This resulted in partial tumor regression in both mice **(****Figure 38C****).** Upon regrowth of the tumors, these mice were retreated again with 2 doses of 4 mg/kg IgG1-AXL-107-vcMMAE on days 105 and 112, which again resulted in partial tumor regression in both animals **(****Figure 38C****).**

Three mice that showed tumor regrowth upon initial tumor regression with paclitaxel were retreated with 2 doses of 4 mg/kg IgG1-AXL-107-vcMMAE on days 55 and 62. Two of the three mice showed complete tumor regression upon retreatment with IgG1-AXL-107-vcMMAE **(****Figure 38D****).** The other mouse showed partial tumor regression. Upon regrowth of the tumor, this mouse was retreated again with 2 doses of 4 mg/kg IgG1-AXL-107-vcMMAE on days 98 and 105, which again resulted in partial tumor regression **(****Figure 38D****).**

### LIST OF REFERENCES

Bahadoran et al., J Clin Oncol; 2013 Jul 1; 31(19): e324-e326
Bansal et al., Oncotarget. 2015 Jun 20;6(17):15321-31
Blakely et al., Cancer Discov. 2012 Oct;2(10):872-5
Bleeker et al., J Immunol. 2004 Oct 1;173(7):4699-707.
Bollag et al., Nat Rev Drug Discov 2012 Nov;11(11):873-86
Brand et al., Clin Cancer Res. 2015 Jun 1;21(11):2601-12
Dahlman et al., Cancer Discov. 2012 Sep;2(9):791-7. Epub 2012 Jul 13.
Debruyne et al., Oncogene. 2015 Nov 30. doi: 10.1038/onc.2015.434
Dufies et al., Oncotarget. 2011 Nov;2(11):874-85
Elkabets et al., Cancer Cell. 2015 Apr 13;27(4):533-46
Greig et al., Drugs, 2016 Feb;76(2):263-73.
Herbst et al., Expert Opin Investig Drugs. 2007 Feb;16(2):239-49
Hilger et al., Int J Clin Pharmacol Ther. 2002 Dec;40(12):567-8.
Hong et al., Cancer Lett. 2008 Sep 18;268(2):314-24.
Hong et al., Cancer Res. 2013 Jan 1;73(1):331-40.
Hong et al., Clin Cancer Res. 2012 Apr 15;18(8):2326-35.
Huang et al., Cancer Res. 2010 Sep 15;70(18):7221-31. doi: 10.1158/0008-5472.CAN-10-0391
Kim et al., Curr Opin Mol Ther. 2004 Feb;6(1):96-103.
Kim et al., Mol Oncol. 2013 Dec;7(6):1093-102.
Konieczkowski et al., 2014, Cancer Discov 4: 816-827.
Li et al., Oncogene. 2008 Aug 7;27(34):4702-11.
Li et al., Cancer Lett. 2016 Jan 28;370(2):332-44.
Liu et al., Cancer Res. 2009 Sep 1;69(17):6871-8
Mahadevan et al., Oncotarget. 2015 Feb 10;6(4):1954-66
Mordant et al., Mol Cancer Ther. 2010 Feb;9(2):358-68
Müller et al., Nat Commun. 2014 Dec 15;5:5712
Park et al., Leukemia. 2015 Dec;29(12):2382-9
Pettazzoni et al., Cancer Research 2015;75: 1091-1101.
Pollack et al., J Pharmacol Exp Ther. 1999 Nov;291(2):739-48
Prewett et al., J Immunother Emphasis Tumor Immunol. 1996 Nov;19(6):419-27.
Sirotnak et al., Clin Cancer Res. 2000 Dec;6(12):4885-92.
Talavera et al., Cancer Res. 2009 Jul 15;69(14):5851-9
Tan et al., Lung Cancer. 2012 May;76(2):177-82.
Wilson et al., Cancer Res. 2014 Oct 15;74(20):5878-90
Wong et al., J Pharmacol Exp Ther. 2009 Apr;329(1):360-7.
Xia et al., Oncogene. 2002 Sep 12;21(41):6255-63.
Yang et al., Crit Rev Oncol Hematol. 2001 Apr;38(1):17-23.
Zhang et al., Nat Genet. 2012 Jul 1;44(8):852-60
Zhou et al., Oncogene. 2016 May;35(21):2687-97
WO 2014/174111; Pierre Fabré Medicament and Spirogen Sarl
WO 09/062690; U3 Pharma
WO 2010/130751; U3 Pharma
WO 2014/093707; Stanford University
EP 2 228 392 A1; Chugai
Yang et al., EORTC meeting 2013, Poster 493 (2013a)
Yang et al., Int. J. Cancer: 132, E74-E84 (2013b)
Paccez et al., Int. J. Cancer: 134, 1024-1033 (2014) (Epub 2013 Jun 4)
Leconet et al., Oncogene, 1-10 (2013)
Linger et al., Expert Opin. Ther. Targets, 14(10):1073-1090 (2010)
Li et al., Oncogene, 28, 3442-3455 (2009)
Ye et al., Oncogene, 1-11 (2010)
Alley et al., Current Opinion in Chem. Bio., 4, 529-537 (2010)
lida et al., Anticancer Research, 34:1821-1828 (2014)
Tschuch et al., AACR-EORTC meeting 2015, Poster A10 (2015)
King et al., Cancer Res. 2006 Dec 1;66(23):11100-5.
Montagut et al., J.Cancer Res. 2008 Jun 15;68(12):4853-61.
Sequist et al., N Engl J Med. 2015 Aug 6;373(6):578-9
Li et al., Structure. 2008 Feb;16(2):216-27
Pedersen et al., Cancer Res. 2010 Jan 15;70(2):588-97.
Mishima et al., Cancer Res. 2001 Jul 15;61(14):5349-54
WO 2012/175691; INSERM
WO 2012/175692; INSERM
WO 2013/064685; PF Medicament
WO 2013/090776; INSERM
WO 2009/063965; Chugai Pharmaceuticals
WO 2010/131733
Hfizi et al. et al., 2006, FEBS Journal, 273; 5231-5244
WO 2007/059782; Genmab A/S
Ward et al., Nature 341, 544-546 (1989)
Holt et al.; Trends Biotechnol. 2003 Nov;21(11):484-90
Revets et al.; Expert Opin Biol Ther. 2005 Jan;5(1):111-24
Bird et al., Science 242, 423-426 (1988)
Huston et a/., PNAS USA 85, 5879-5883 (1988)
Fundamental Immunology Ch. 7 (Paul, W., ed., 2nd ed. Raven Press, N.Y. (1989)
Lefranc MP. et al., Nucleic Acids Research, 27 et al., 209-212, 1999
Brochet X. Nucl. Acids Res. 36, W503-508 (2008)
Korshunov et al, Clin Sci (Lond). 2012 Apr;122(8):361-8.
Sambrook et al, Molecular Cloning: A laboratory Manual, New York: Cold Spring Harbor Laboratory Press, 1989, Ch. 15
Kabat, E.A. et al., Sequences of proteins of immunological interest. 5th Edition - US Department of Health and Human Services, NIH publication No. 91-3242, pp 662,680,689 (1991)
WO 2004/010957; Seattle Genetics, Inc.
US 7,659,241; Seattle Genetics, Inc.
Wu et al., Generation and Characterization of a Dual Variable Domain Immunoglobulin (DVD-Ig™) Molecule, In: Antibody Engineering, Springer Berlin Heidelberg (2010)
WO 2011/131746; Genmab A/S
WO/2002/020039; Trion Pharma/Fresenius Biotech
WO9850431; Genetech
WO2011117329; Roche
EP1870459; Amgen
WO2009089004; Amgen
US 2010/00155133; Chugai
WO 2010/129304; Oncomed
WO2007/110205; EMD Serono
WO 2010/015792; Regeneron
WO 11/143545; Pfizer/Rinat
WO 2012/058768: Zymeworks/Merck
WO 2011/028952; Xencor
WO 2009/080254; Roche
WO 2008/003116; F-Star
US 7,262,028; Crucell/Merus
US 7,612,181; Abbott
WO 2010/0226923; Unilever, Sanofi Aventis
US 7,951,918; Biogen Idec
CN 102250246; Changzhou Adam Biotech Inc
WO 2012/025525; Roche
WO 2012/025530; Roche
WO 2008/157379; Macrogenics
WO 2010/080538; Macrogenics
Goodman *et al.,* Goodman and Gilman's The Pharmacological Basis Of Therapeutics, 8th Ed., Macmillan Publishing Co., 1990
Vitetta, Immunol. Today 14, 252 (1993)
US 5,194,594
US 2005/0238649
WO 2013/173391; Concortis Biosystems, Corp.
Junghans et al., in Cancer Chemotherapy and Biotherapy 655-686 (2d edition, Chafner and Longo, eds., Lippincott Raven (1996))
US 4,681,581
US 4,735,210
US 5,101,827
US 5,102,990
US 5,648,471
US 5,697,902
US 4,766,106
US 4,179,337
US 4,495,285
US 4,609,546
Hunter et al., Nature 144, 945 (1962), David et al., Biochemistry 13, 1014 (1974)
Pain et al., J. Immunol. Meth. 40, 219 (1981)
Nygren, J. Histochem. and Cytochem. 30, 407 (1982)
Antibody Engineering Handbook, edited by Osamu Kanemitsu, published by Chijin Shokan (1994)
WO 2002/083180; Syngenta BV
WO 2004/043493; Syngenta BV
WO 2007/018431; Syngenta BV
WO 2007/089149; Syngenta BV
WO 2009/017394; Syngenta BV
WO 2010/62171; Syngenta BV
US 6,989,452; Medarex
Remington: The Science and Practice of Pharmacy, 19th Edition, Gennaro, Ed., Mack Publishing Co., Easton, PA, 1995
Sustained and Controlled Release Drug Delivery Systems, J.R. Robinson, ed., Marcel Dekker, Inc., New York, 1978
Sykes and Johnston, Nat Biotech 17, 355-59 (1997)
US 6,077, 835
WO 00/70087
Schakowski et al., Mol Ther 3, 793-800 (2001)
WO 00/46147
Benvenisty and Reshef, PNAS USA 83, 9551-55 (1986)
Wigler et al., Cell 14, 725 (1978)
Coraro and Pearson, Somatic Cell Genetics 7, 603 (1981)
US 5,589,466
US 5,973,972
Van Heeke & Schuster, J Biol Chem 264, 5503-5509 (1989)
Ausubel et al., ed. Current Protocols in Molecular Biology, Greene Publishing and Wiley InterScience New York (1987)
Grant et al., Methods in Enzymol 153, 516-544 (1987)
Lonberg, N. et al., Nature 368, 856 859 (1994a)
Lonberg, N. Handbook of Experimental Pharmacology 113, 49 101 (1994b)
Lonberg, N. and Huszar, D., Intern. Rev. Immunol. Vol. 13 65 93 (1995)
Harding, F. and Lonberg, N. Ann. N.Y. Acad. Sci 764 536 546 (1995)
Taylor, L. et al., Nucleic Acids Research 20, 6287 6295 (1992)
Chen, J. et al., International Immunology 5, 647 656 (1993)
Tuaillon et al., J. Immunol. 152, 2912 2920 (1994)
Taylor, L. et al., International Immunology 6, 579 591 (1994)
Fishwild, D. et al., Nature Biotechnology 14, 845 851 (1996)
US 5,545,806
US 5,569,825
US 5,625,126
US 5,633,425
US 5,789,650
US 5,877,397
US 5,661,016
US 5,814,318
US 5,874,299
US 5,770,429
US 5,545,807
WO 98/024884
WO 94/025585
WO 93/001227
WO 92/022645
WO 92/003918
WO 01/009187
Shieh, Neoplasia 2005
Koorstra, Cancer Biol Ther 2009
Hector, Cancer Biol Ther 2010
Sun, Ann Oncol 2003
Srivastava (ed.), Radiolabeled Monoclonal Antibodies For Imaging And Therapy (Plenum Press 1988), Chase
"Medical Applications of Radioisotopes," in Remington's Pharmaceutical Sciences, 18th Edition, Gennaro et al., (eds.), pp. 624-652 (Mack Publishing Co., 1990)
Brown, "Clinical Use of Monoclonal Antibodies," in Biotechnology And Pharmacy 227-49, Pezzuto et al., (eds.) (Chapman & Hall 1993)
US 5,057,313
US 6,331,175
US 5,635,483
US 5,780,588
Woyke et al (2001) Antimicrob. Agents and Chemother. 45(12): 3580-3584
US5663149
Pettit et al., (1998) Antimicrob. Agents and Chemother. 42:2961-2965
Senter et al., Proceedings of the American Association for Cancer Research. Volume 45, abstract number 623, presented March 28, 2004
US 2005/0238649
US 7,498,298; Seattle Genetics, Inc.
US 7,994,135; Seattle Genetics, Inc.
WO 2005/081711; Seattle Genetics, Inc.
Kozak et al. (1999) Gene 234: 187-208
EP 2 220 131; U3 Pharma
WO 2011/159980; Genentech
Barbas, CF. J Mol Biol. 1993 Apr 5;230(3):812-23
US 7,829,531; Seattle Genetics, Inc.
US 7,851,437; Seattle Genetics, Inc.
WO 2013/173392; Concortis Biosystems, Corp.
WO 2013/173393; Concortis Biosystems, Corp.
Sun et al. (2005) Bioconjugate Chem. 16: 1282-1290
McDonagh et al., (2006) Protein Eng. Design Sel. 19: 299-307
Alley et al., (2008) Bioconjugate Chem. 19: 759-765

## Claims

1. An antibody-drug conjugate (ADC) comprising an antibody binding to human AXL for use in treating cancer resistant to at least one therapeutic agent selected from the group consisting of a tyrosine kinase inhibitor, a serine/threonine kinase inhibitor and a chemotherapeutic agent, wherein the ADC comprises least one binding region that comprises a VH region comprising the CDR1, CDR2, and CDR3 sequences of SEQ ID Nos.: 36, 37, and 38, respectively; and a VL region comprising the CDR1, CDR2, and CDR3 sequences of SEQ ID Nos.: 39, GAS, and 40, respectively.

2. The ADC for the use of claim 1, wherein the tyrosine kinase inhibitor is selected from the group consisting of erlotinib, afatinib, gefitinib, lapatinib, osimertinib, rociletinib, imatinib, sunitinib, crizotinib, midostaurin (PKC412) and quizartinib (AC220) .

3. The ADC for the use of claim 1, wherein the serine/threonine kinase inhibitor is a BRAF-inhibitor or a MEK-inhibitor.

4. The ADC for the use of claim 3, wherein
(a) the BRAF inhibitor is selected from the group consisting of vemurafenib (PLX4032), dabrafenib, and a therapeutically effective analog or derivative of any thereof, such as PLX4720;
(b) the MEK-inhibitor selected from trametinib and selumetinib (AZD6244),and a therapeutically effective analog or derivative of any thereof.

5. The ADC for the use of claim 1, wherein the chemotherapeutic agent is selected from the group consisting of paclitaxel, docetaxel, cisplatin, metformin, doxorubicin, etoposide, carboplatin, or a combination thereof.

6. The ADC for the use of any one of the preceding claims, wherein
(a) the tyrosine kinase inhibitor is selected from an Epidermal Growth Factor Receptor (EGFR) antagonist, HER2 antagonist, ALK-inhibitor and a FLT3 inhibitor, or a combination of any thereof;
(b) the serine/threonine kinase inhibitor is selected from a BRAF inhibitor and a MEK inhibitor, or a combination thereof;
(c) the chemotherapeutic agent is selected from paclitaxel, docetaxel, cisplatin, metformin, doxorubicin, etoposide, carboplatin, or a combination thereof.

7. The ADC for the use of claim 6, wherein
(a) the tyrosine kinase inhibitor is an EGFR inhibitor;
(b) the serine/threonine kinase inhibitor is a BRAF inhibitor; and
(c) the chemotherapeutic agent is a taxane.

8. The ADC for the use of any one of the preceding claims, wherein the cancer is an AXL-expressing cancer.

9. The ADC for the use of any one of the preceding claims, wherein the cancer is selected from a melanoma, a non-small cell lung cancer (NSCLC), a cervical cancer, a squamous cell carcinoma of the head and neck (SCCHN), a breast cancer, a gastrointestinal stromal tumor (GIST), a renal cancer, a prostate cancer, a neuroblastoma, a pancreatic cancer, an oesophageal cancer, a rhabdomyosarcoma, an acute myeloid leukaemia (AML), or a chronic myeloid leukaemia (CML).

10. The ADC for the use of any one of the preceding claims, which is for use in combination with the therapeutic agent, wherein the ADC and the therapeutic agent are administered simultaneously, separately or sequentially.

11. The ADC for the use according to any of the preceding claims for use in treating an NSCLC resistant to an EGFR-inhibiting agent, such as erlotinib.

12. The ADC for the use of any one of the preceding claims, which is for use in treating a melanoma resistant to vemurafenib.

13. The ADC for the use of any one of the preceding claims, which is for use in treating a cervical cancer resistant to paclitaxel or a therapeutically effective analog or derivative thereof, such as docetaxel.

14. The ADC for the use of any one of the preceding claims, wherein the ADC comprises a cytotoxic agent, a chemotherapeutic drug or a radioisotope linked to the antibody.

15. The ADC for the use of any one of the preceding claims, wherein the therapeutic moiety is a cytotoxic agent, optionally linked to the ADC with a linker.

16. The ADC for the use of claim 15, wherein the cytotoxic agent is linked to the antibody with a cleavable linker, such as *N*-succinimydyl 4-(2-pyridyldithio)-pentanoate (SSP), maleimidocaproyl-valine-citrulline-p-aminobenzyloxycarbonyl (mc-vc-PAB) or AV-1 K-lock valine-citrulline.

17. The ADC for the use of any one of claims 15 to 16, wherein the cytotoxic agent is linked to the antibody with a non-cleavable linker, such as succinimidyl-4(*N*-maleimidomethyl)cyclohexane-1-carboxylate (MCC) or maleimidocaproyl (MC).

18. The ADC for the use of any one of claims 15 to 17, wherein the cytotoxic agent is selected from the group consisting of DNA-targeting agents, e.g. DNA alkylators and cross-linkers, such as calicheamicin, duocarmycin, rachelmycin (CC-1065), pyrrolo[2,1-c][1,4] benzodiazepines (PBDs), and indolinobenzodiazepine (IGN); microtubule-targeting agents, such as duostatin, such as duostatin-3, auristatin, such as monomethylauristatin E (MMAE) and monomethylauristatin F (MMAF), dolastatin, maytansine, *N*(2')-deacetyl-*N*(2')-(3-marcapto-1-oxopropyl)-maytansine (DM1), and tubulysin; and nucleoside analogs; or an analogs, derivatives, or prodrugs thereof.

19. The ADC for the use of any one of claims 15 to 18, wherein
(a) the linker is cleavable and the cytotoxic agent has bystander kill capacity;
(b) the linker is cleavable and the cytotoxic agent does not have bystander kill capacity;
(c) the linker is non-cleavable and the cytotoxic agent has bystander kill capacity; or
(d) the linker is non-cleavable and the cytotoxic agent does not have bystander kill capacity.

20. The ADC for the use of any one of claims 15 to 19, wherein the linker is mc-vc-PAB and the cytotoxic agent is MMAE.

21. The ADC for the use of any one of claims 15 to 19, wherein the linker is SSP and the cytotoxic agent is DM1.

22. The ADC for the use of any one of claims 15 to 19, wherein the drug is duostatin3.

23. The ADC for the use of any one of the preceding claims, wherein the amino acid sequence of the human AXL is as specified in SEQ ID NO: 130.

24. The ADC for the use of any one of the preceding claims, which binds to cynomolgus monkey AXL as specified in SEQ ID NO: 147.

25. The ADC for the use of any one of the preceding claims, wherein the antibody comprises at least one binding region comprising a VH region which is at least 90%, such as at least 95%, such as at least 97%, such as at least 99% identical to SEQ ID No: 1 and a VL region which is at least 90%, such as at least 95%, such as at least 97%, such as at least 99% identical to SEQ ID No: 2 [**107**].

26. The ADC for the use of any one of the preceding claims, wherein the at least one binding region comprises a VH region comprising SEQ ID No: 1 and a VL region comprising SEQ ID No: 2 [**107**].

27. The ADC for the use of any one of the preceding claims, wherein
the antibody comprises at least one binding region comprising a VH region comprising the CDR1, CDR2, and CDR3 sequences of SEQ ID Nos.: 36, 37, and 38, respectively; and a VL region comprising the CDR1, CDR2, and CDR3 sequences of SEQ ID Nos.: 39, GAS, and 40, respectively, [**107**],
the linker is mc-vc-PAB, and
the cytotoxic agent is MMAE.

28. The ADC for the use of any one of the preceding claims, wherein the antibody binds to an epitope on AXL wherein the epitope is recognized by any of the antibodies defined in claim 19.

29. The ADC for the use of any one of the preceding claims, wherein the antibody binds to an epitope within the Ig1 domain of AXL, the epitope comprising or requiring one or more amino acids corresponding to positions L121 to Q129 or T112 to Q124 of human AXL.

30. The ADC for the use of any of the preceding claims, wherein the antibody comprises a heavy chain of an isotype selected from the group consisting of IgG1, IgG2, IgG3, and IgG4.

31. The ADC for the use of claim 30, wherein the isotype is IgG1, optionally allotype IgGlm(f).

32. The ADC of any one of the preceding claims, which is a full-length monoclonal antibody, such as a full-length monoclonal IgG1,κ antibody.

33. The ADC for the use of any one of claims 1 to 32, wherein the antibody is an effector-function-deficient antibody, a stabilized IgG4 antibody or a monovalent antibody.

34. The ADC for the use of claim 33, wherein the heavy chain has been modified such that the entire hinge region has been deleted.

35. The ADC for the use of any one of claims 33 and 34, wherein the sequence of the antibody has been modified so that it does not comprise any acceptor sites for N-linked glycosylation.

36. The ADC for the use of any one of the preceding claims, wherein the antibody is a single-chain antibody.

37. The ADC for the use of any one of the preceding claims, wherein the antibody is a bispecific antibody comprising a first binding region of an antibody according to any one of the preceding claims, and a second binding region which binds a different target or epitope than the first binding region.

## Patentansprüche

1. Antikörper-Arzneimittel-Konjugat (antibody-drugconjugate, ADC), umfassend einen Antikörper, der an humanes AXL bindet, zur Verwendung in der Behandlung von Krebs, der resistent gegenüber wenigstens einem therapeutischen Mittel ist, ausgewählt aus der Gruppe, bestehend aus einem Tyrosinkinase-Inhibitor, einem Serin/Threonin-Kinase-Inhibitor und einem chemotherapeutischen Mittel, wobei das ADC wenigstens eine Bindungsregion umfasst, die eine VH-Region, umfassend die CDR1-, CDR2- und CDR3-Sequenzen von SEQ ID Nos.: 36, 37 bzw. 38; und eine VL-Region, umfassend die CDR1-, CDR2- und CDR3-Sequenzen von SEQ ID Nos.: 39, GAS bzw. 40, umfasst.

2. ADC zur Verwendung gemäß Anspruch 1, wobei der Tyrosinkinase-Inhibitor ausgewählt ist aus der Gruppe, bestehend aus Erlotinib, Afatinib, Gefitinib, Lapatinib, Osimertinib, Rociletinib, Imatinib, Sunitinib, Crizotinib, Midostaurin (PKC412) und Quizartinib (AC220).

3. ADC zur Verwendung gemäß Anspruch 1, wobei der Serin/Theronin-Kinase-Inhibitor ein BRAF-Inhibitor oder ein MEK-Inhibitor ist.

4. ADC zur Verwendung gemäß Anspruch 3, wobei
(a) der BRAF-Inhibitor ausgewählt ist aus der Gruppe, bestehend aus Vemurafenib (PLX4032), Dabrafenib und einem therapeutisch wirksamen Analog oder Derivat eines davon, beispielsweise PLX4720;
(b) der MEK-Inhibitor ausgewählt ist aus Trametinib und Selumetinib (AZD6244) und einem therapeutisch wirksamen Analog oder Derivat eines davon.

5. ADC zur Verwendung gemäß Anspruch 1, wobei das chemotherapeutische Mittel ausgewählt ist aus der Gruppe, bestehend aus Paclitaxel, Docetaxel, Cisplatin, Metformin, Doxorubicin, Etoposid, Carboplatin oder einer Kombination davon.

6. ADC zur Verwendung gemäß einem der vorangehenden Ansprüche, wobei
(a) der Tyrosinkinase-Inhibitor ausgewählt ist aus einem epidermalen Wachstumsfaktor-Rezeptor (EGFR)-Antagonisten, HER2-Antagonisten, ALK-Inhibitor und einem FLT3-Inhibitor oder einer Kombination beliebiger davon;
(b) der Serin/Threonin-Kinase-Inhibitor ausgewählt ist aus einem BRAF-Inhibitor und einem MEK-Inhibitor oder einer Kombination davon;
(c) das chemotherapeutische Mittel ausgewählt ist aus Paclitaxel, Docetaxel, Cisplatin, Metformin, Doxorubicin, Etoposid, Carboplatin oder einer Kombination davon.

7. ADC zur Verwendung gemäß Anspruch 6, wobei
(a) der Tyrosinkinase-Inhibitor ein EGFR-Inhibitor ist;
(b) der Serin/Threonin-Kinase-Inhibitor ein BRAF-Inhibitor ist; und
(c) das chemotherapeutische Mittel ein Taxan ist.

8. ADC zur Verwendung gemäß einem der vorangehenden Ansprüche, wobei der Krebs ein AXL-exprimierender Krebs ist.

9. ADC zur Verwendung gemäß einem der vorangehenden Ansprüche, wobei der Krebs ausgewählt ist aus einem Melanom, einem nichtkleinzelligen Lungenkrebs (NSCLC), einem Gebärmutterhalskrebs, einem Plattenepithelkarzinom von Kopf und Hals (SCCHN), einem Brustkrebs, einem gastrointestinalen Stromatumor (GIST), einem Nierenkrebs, einem Prostatakrebs, einem Neuroblastom, einem Bauchspeicheldrüsenkrebs, einem Speiseröhrenkrebs, einem Rhabdomyosarkom, einer akuten myeloischen Leukämie (AML) oder einer chronischen myeloischen Leukämie (CML.

10. ADC zur Verwendung gemäß einem der vorangehenden Ansprüche, welches zur Verwendung in Kombination mit dem therapeutischen Mittel bestimmt ist, wobei das ADC und das therapeutische Mittel gleichzeitig, getrennt oder nacheinander verabreicht werden.

11. ADC zur Verwendung gemäß einem der vorangehenden Ansprüche zur Verwendung in der Behandlung eines NSCLC, der gegenüber einem EGFR-inhibierenden Mittel, beispielsweise Erlotinib, resistent ist.

12. ADC zur Verwendung gemäß einem der vorangehenden Ansprüche, welches zur Verwendung in der Behandlung eines Melanoms bestimmt ist, das gegenüber Vemurafenib resistent ist.

13. ADC zur Verwendung gemäß einem der vorangehenden Ansprüche, welches zur Verwendung in der Behandlung eines Gebärmutterhalskrebses bestimmt ist, der resistent gegenüber Paclitaxel oder einem therapeutisch wirksamen Analog oder Derivat davon ist, beispielsweise Docetaxel.

14. ADC zur Verwendung gemäß einem der vorangehenden Ansprüche, wobei das ADC ein cytotoxisches Mittel, ein chemotherapeutisches Arzneimittel oder ein Radioisotop, der mit dem Antikörper verknüpft ist, umfasst.

15. ADC zur Verwendung gemäß einem der vorangehenden Ansprüche, wobei die therapeutische Gruppierung ein cytotoxisches Mittel ist, das gegebenenfalls an das ADC über einen Linker verknüpft ist.

16. ADC zur Verwendung gemäß Anspruch 15, wobei das cytotoxische Mittel mit dem Antikörper über einen spaltbaren Linker, beispielsweise N-Succinimidyl-4-(2-pyridyldithio)-pentanoat (SSP), Maleimidocaproyl-valincitrullin-p-aminobenzyloxycarbonyl (mc-vc-PAB) oder AV-1K-lock-valin-citrullin, verknüpft ist.

17. ADC zur Verwendung gemäß einem der Ansprüche 15 bis 16, wobei das cytotoxische Mittel mit dem Antikörper über einen nicht-spaltbaren Linker, beispielsweise Succinimidyl-4-(*N*-maleimidornethyl)cyclohexan-1-carboxylat (MCC) oder Maleimidocaproyl (MC), verknüpft ist.

18. ADC zur Verwendung gemäß einem der Ansprüche 15 bis 17, wobei das cytotoxische Mittel ausgewählt ist aus der Gruppe, bestehend aus DNA-targetierenden Mitteln, z. B. DNA-Alkylatoren und Vernetzungsmitteln, beispielsweise Calicheamicin, Duocarmycin, Rachelmycin (CC-1065), Pyrrolo[2,1-c][1,4]-benzodiazepinen (PBDs) und Indolinobenzodiazepin (IGN); Mikrotubuli-targetierenden Mitteln, beispielsweise Duostatin, beispielsweise Duostatin-3, Auristatin, beispielsweise Monomethylauristatin E (MMAE) und Monomethylauristatin F (MMAF), Dolastatin, Maytansin, *N*(2')-Deacetyl-*N*(2')-(3-mercapto-l-oxopropyl)-maytansin (DM-1) und Tubulysin; und Nucleosidanalogen; oder Analogen, Derivaten oder Prodrugs davon.

19. ADC zur Verwendung gemäß einem der Ansprüche 15 bis 18, wobei
(a) der Linker spaltbar ist und das cytotoxische Mittel Bystander-Tötungs-Fähigkeit (bystander kill capacity) hat;
(b) der Linker spaltbar ist und das cytotoxische Mittel keine Bystander-Tötungs-Fähigkeit hat;
(c) der Linker nicht spaltbar ist und das cytotoxische Mittel Bystander-Tötungs-Fähigkeit hat; oder
(d) der Linker nicht spaltbar ist und das cytotoxische Mittel keine Bystander-Tötungs-Fähigkeit hat.

20. ADC zur Verwendung gemäß einem der Ansprüche 15 bis 19, wobei der Linker mc-vc-PAB ist und das cytotoxische Mittel MMAE ist.

21. ADC zur Verwendung gemäß einem der Ansprüche 15 bis 19, wobei der Linker SSP ist und das cytotoxische Mittel DM1 ist.

22. ADC zur Verwendung gemäß einem der Ansprüche 15 bis 19, wobei das Arzneimittel Duostatin-3 ist.

23. ADC zur Verwendung gemäß einem der vorangehenden Ansprüche, wobei die Aminosäuresequenz vom humanen AXL in SEQ ID No.: 130 angegeben ist.

24. ADC zur Verwendung gemäß einem der vorangehenden Ansprüche, welches an Cynomolgus-Affen-AXL bindet, wie in SEQ ID No.: 147 angegeben.

25. ADC zur Verwendung gemäß einem der vorangehenden Ansprüche, wobei der Antikörper wenigstens eine Bindungsregion, umfassend eine VH-Region, die wenigstens 90%, beispielsweise wenigstens 95%, beispielsweise wenigstens 97%, beispielsweise wenigstens 99% identisch ist mit SEQ ID No.: 1, und eine VL-Region, die wenigstens 90%, beispielsweise wenigstens 95%, beispielsweise wenigstens 97%, beispielsweise wenigstens 99% identisch ist mit SEQ ID No.: 2 [**107**], umfasst.

26. ADC zur Verwendung gemäß einem der vorangehenden Ansprüche, wobei die wenigstens eine Bindungsregion eine VH-Region, umfassend SEQ ID No.: 1, und eine VL-Region, umfassend SEQ ID No.: 2 [**107**], umfasst.

27. ADC zur Verwendung gemäß einem der vorangehenden Ansprüche, wobei
der Antikörper wenigstens eine Bindungsregion, umfassend eine VH-Region, umfassend die CDR1-, CDR2- und CDR3-Sequenzen von SEQ ID Nos.: 36, 37 bzw. 38; und eine VL-Region, umfassend die CDR1-, CDR2- und CDR3-Sequenzen von SEQ ID Nos.: 39, GAS bzw. 40 [**107**], umfasst,
der Linker mc-vc-PAB ist, und
das cytotoxische Mittel MMAE ist.

28. ADC zur Verwendung gemäß einem der vorangehenden Ansprüche, wobei der Antikörper an ein Epitop an AXL bindet, wobei das Epitop von einem der in Anspruch 19 definierten Antikörper erkannt wird.

29. ADC zur Verwendung gemäß einem der vorangehenden Ansprüche, wobei der Antikörper an ein Epitop innerhalb der Ig1-Domäne von AXL bindet, wobei das Epitop eine oder mehrere Aminosäure(n) entsprechend Positionen L121 bis Q129 oder T112 bis Q124 vom humanen AXL umfasst oder diese benötigt.

30. ADC zur Verwendung gemäß einem der vorangehenden Ansprüche, wobei der Antikörper eine schwere Kette eines Isotyps, ausgewählt aus der Gruppe, bestehend aus IgG1, IgG2, IgG3 und IgG4 umfasst.

31. ADC zur Verwendung gemäß Anspruch 30, wobei der Isotyp IgG1 ist, gegebenenfalls Allotyp IgGlm(f).

32. ADC gemäß einem der vorangehenden Ansprüche, welches ein monoklonaler Volllängen-Antikörper, beispielsweise ein monoklonaler IgG1, κ-Volllängen-Antikörper, ist.

33. ADC zur Verwendung gemäß einem der Ansprüche 1 bis 32, wobei der Antikörper ein effektorfunktionsdefizienter Antikörper, ein stabilisierter IgG4-Antikörper oder ein monovalenter Antikörper ist.

34. ADC zur Verwendung gemäß Anspruch 33, wobei die schwere Kette derart modifiziert wurde, dass die gesamte Hinge-Region entfernt wurde.

35. ADC zur Verwendung gemäß einem der Ansprüche 33 und 34, wobei die Sequenz des Antikörpers derart modifiziert wurde, dass sie keine Akzeptorstellen für N-verknüpfte Glycosylierung umfasst.

36. ADC zur Verwendung gemäß einem der vorangehenden Ansprüche, wobei der Antikörper ein Einzelketten-Antikörper ist.

37. ADC zur Verwendung gemäß einem der vorangehenden Ansprüche, wobei der Antikörper ein bispezifischer Antikörper ist, umfassend eine erste Bindungsregion eines Antikörpers gemäß einem der vorangehenden Ansprüche, und eine zweite Bindungsregion, welche an ein anderes Target oder Epitop als die erste Bindungsregion bindet.

## Revendications

1. Conjugué anticorps-médicament (CAM) comprenant un anticorps se liant à AXL humaine, destiné à être utilisé dans le traitement d'un cancer résistant à au moins un agent thérapeutique choisi dans l'ensemble constitué par un inhibiteur de tyrosine kinase, un inhibiteur de sérine/thréonine kinase et un agent chimiothérapeutique, où le CAM comprend au moins une région de liaison qui comprend une région VH comprenant les séquences de CDR1, CDR2, et CDR3 des séquences SEQ ID NO: 36, 37, et 38, respectivement ; et une région VL comprenant les séquences de CDR1, CDR2, et CDR3 des séquences SEQ ID NO: 39, GAS, et 40, respectivement.

2. CAM destiné à être utilisé de la revendication 1, dans lequel l'inhibiteur de tyrosine kinase est choisi dans l'ensemble constitué par l'erlotinib, l'afatinib, le géfitinib, le lapatinib, l'osimertinib, le rociletinib, l'imatinib, le sunitinib, le crizotinib, la midostaurine (PKC412) et le quizartinib (AC220).

3. CAM destiné à être utilisé de la revendication 1, dans lequel l'inhibiteur de sérine/thréonine kinase est un inhibiteur de BRAF ou un inhibiteur de MEK.

4. CAM destiné à être utilisé de la revendication 3, dans lequel
(a) l'inhibiteur de BRAF est choisi dans l'ensemble constitué par le vémurafénib (PLX4032), le dabrafénib, et un analogue ou dérivé thérapeutiquement efficace de l'un quelconque de ceux-ci, tel que PLX4720 ;
(b) l'inhibiteur de MEK est choisi parmi le tramétinib et sélumétinib (AZD6244), et un analogue ou dérivé thérapeutiquement efficace de l'un quelconque de ceux-ci.

5. CAM destiné à être utilisé de la revendication 1, dans lequel l'agent chimiothérapeutique est choisi dans l'ensemble constitué par le paclitaxel, le docétaxel, le cisplatine, la metformine, la doxorubicine, l'étoposide, le carboplatine, ou une association de tels agents.

6. CAM destiné à être utilisé de l'une quelconque des revendications précédentes, dans lequel
(a) l'inhibiteur de tyrosine kinase est choisi parmi un antagoniste de récepteur de facteur de croissance épidermique (EFGR), un antagoniste de HER2, un inhibiteur d'ALK et un inhibiteur de FLT3, ou une association de n'importe lesquels de ceux-ci ;
(b) l'inhibiteur de sérine/thréonine kinase est choisi parmi un inhibiteur de BRAF et un inhibiteur de MEK ou une association de tels inhibiteurs ;
(c) l'agent chimiothérapeutique est choisi parmi le paclitaxel, le docétaxel, le cisplatine, la metformine, la doxorubicine, l'étoposide, le carboplatine, ou une association de tels agents.

7. CAM destiné à être utilisé de la revendication 6, dans lequel
(a) l'inhibiteur de tyrosine kinase est un inhibiteur d'EGFR ;
(b) l'inhibiteur de sérine/thréonine kinase est un inhibiteur de BRAF ; et
(c) l'agent chimiothérapeutique est un taxane.

8. CAM destiné à être utilisé de l'une quelconque des revendications précédentes, dans lequel le cancer est un cancer exprimant AXL.

9. CAM destiné à être utilisé de l'une quelconque des revendications précédentes, dans lequel le cancer est choisi parmi un mélanome, un cancer du poumon non à petites cellules (CPNPC), un cancer du col utérin, un carcinome spinocellulaire de la tête et du cou (HNSCC), un cancer du sein, une tumeur stromale gastro-intestinale (TSGI), un cancer rénal, un cancer de la prostate, un neuroblastome, un cancer du pancréas, un cancer de l'œsophage, un rhabdomyosarcome, une leucémie myéloïde aiguë (LMA), ou une leucémie myéloïde chronique (LMC).

10. CAM destiné à être utilisé de l'une quelconque des revendications précédentes, qui est destiné à être utilisé en association avec l'agent thérapeutique, le CAM et l'agent thérapeutique étant administrés simultanément, séparément ou séquentiellement.

11. CAM destiné à être utilisé selon l'une quelconque des revendications précédentes, destiné à être utilisé dans le traitement d'un CPNPC résistant à un agent inhibiteur d'EGFR, tel que l'erlotinib.

12. CAM destiné à être utilisé de l'une quelconque des revendications précédentes, qui est destiné à être utilisé dans le traitement d'un mélanome résistant au vémurafénib.

13. CAM destiné à être utilisé de l'une quelconque des revendications précédentes, qui est destiné à être utilisé dans le traitement d'un cancer du col utérin résistant au paclitaxel ou à un analogue ou dérivé thérapeutiquement efficace de celui-ci, tel que le docétaxel.

14. CAM destiné à être utilisé de l'une quelconque des revendications précédentes, où le CAM comprend un agent cytotoxique, un médicament chimiothérapeutique ou un radio-isotope lié à l'anticorps.

15. CAM destiné à être utilisé de l'une quelconque des revendications précédentes, dans lequel le fragment thérapeutique est un agent cytotoxique, en option lié au CAM par un agent de liaison.

16. CAM destiné à être utilisé de la revendication 15, dans lequel l'agent cytotoxique est lié à l'anticorps par un agent de liaison clivable, tel que le 4-(2-pyridyldithio)-pentanoate de *N*-succinimidyle (SSP), le maléimidocaproyl-valine-citrulline-*p*-aminobenzyloxycarbonyle (mc-vc-PAB) ou l'AV-1 K-lock valine-citrulline.

17. CAM destiné à être utilisé de l'une quelconque des revendications 15 et 16, dans lequel l'agent cytotoxique est lié à l'anticorps par un agent de liaison non clivable, tel que le 4(*N*-maléimidométhyl)cyclohexane-1-carboxylate de succinimidyle (MCC) or le maléimidocaproyle (MC).

18. CAM destiné à être utilisé de l'une quelconque des revendications 15 à 17, dans lequel l'agent cytotoxique est choisi dans l'ensemble constitué par les agents ciblant l'ADN, par exemple les agents pontant et alkylant l'ADN, tels que la calicheamicine, la duocarmycine, la rachelmycine (CC-1065), les pyrrolo[2,1-c][1,4] benzodiazépines (PBD), et l'indolinobenzodiazépine (IGN) ; les agents ciblant les microtubules, tels qu'une duostatine, telle que la duostatine 3, une auristatine, telle que la monométhylauristatine E (MMAE) et la monométhylauristatine F (MMAF), la dolastatine, la maytansine, la *N*(2')-déacétyl-*N*(2')-(3-mercapto-1-oxopropyl)-maytansine (DM1), et la tubulysine ; et les analogues de nucléosides ; ou leurs analogues, dérivés ou précurseurs de principes actifs.

19. CAM destiné à être utilisé de l'une quelconque des revendications 15 à 18, dans lequel
(a) l'agent de liaison est clivable et l'agent cytotoxique a une capacité de destruction non spécifique ;
(b) l'agent de liaison est clivable et l'agent cytotoxique n'a pas une capacité de destruction non spécifique ;
(c) l'agent de liaison est non clivable et l'agent cytotoxique a une capacité de destruction non spécifique ; ou
(d) l'agent de liaison est non clivable et l'agent cytotoxique n'a pas une capacité de destruction non spécifique.

20. CAM destiné à être utilisé de l'une quelconque des revendications 15 à 19, dans lequel l'agent de liaison est mc-vc-PAB et l'agent cytotoxique est MMAE.

21. CAM destiné à être utilisé de l'une quelconque des revendications 15 à 19, dans lequel l'agent de liaison est SSP et l'agent cytotoxique est DM1.

22. CAM destiné à être utilisé de l'une quelconque des revendications 15 à 19, dans lequel le médicament est la duostatine 3.

23. CAM destiné à être utilisé de l'une quelconque des revendications précédentes, dans lequel la séquence d'acides aminés de l'AXL humaine est telle que spécifiée dans la séquence SEQ ID NO: 130.

24. CAM destiné à être utilisé de l'une quelconque des revendications précédentes, qui se lie à AXL de macaque crabier, telle que spécifiée dans la séquence SEQ ID NO: 147.

25. CAM destiné à être utilisé de l'une quelconque des revendications précédentes, dans lequel l'anticorps comprend au moins une région de liaison comprenant une région VH qui a un degré d'identité d'au moins 90 %, tel que d'au moins 95 %, tel que d'au moins 97 %, tel qu'un degré d'identité d'au moins 99 % avec la séquence SEQ ID NO: 1 et une région VL qui a un degré d'identité d'au moins 90 %, tel que d'au moins 95 %, tel que d'au moins 97 %, tel qu'un degré d'identité d'au moins 99 % avec la séquence SEQ ID NO: 2 [**107**].

26. CAM destiné à être utilisé de l'une quelconque des revendications précédentes, dans lequel ladite au moins une région de liaison comprend une région VH comprenant la séquence SEQ ID NO: 1 et une région VL comprenant la séquence SEQ ID NO: 2 [**107**]

27. CAM destiné à être utilisé de l'une quelconque des revendications précédentes, dans lequel
l'anticorps comprend au moins une région de liaison comprenant une région VH comprenant les séquences de CDR1, CDR2, et CDR3 des séquences SEQ ID NO: 36, 37, et 38, respectivement, et une région VL comprenant les séquences de CDR1, CDR2, et CDR3 des séquences SEQ ID NO: 39, GAS, et 40, respectivement, [**107**],
l'agent de liaison est mc-vc-PAB, et
l'agent cytotoxique est MMAE.

28. CAM destiné à être utilisé de l'une quelconque des revendications précédentes, dans lequel l'anticorps se lie à un épitope sur l'AXL, où l'épitope est reconnu par l'un quelconque des anticorps définis dans la revendication 19.

29. CAM destiné à être utilisé de l'une quelconque des revendications précédentes, dans lequel l'anticorps se lie à un épitope à l'intérieur du domaine Ig1 d'AXL, l'épitope comprenant ou mobilisant un ou plusieurs acide(s) aminé(s) correspondant aux positions L121 à Q129 ou T112 à Q124 de l'AXL humaine.

30. CAM destiné à être utilisé de l'une quelconque des revendications précédentes, dans lequel l'anticorps comprend une chaîne lourde d'un isotype choisi dans l'ensemble constitué par IgG1, IgG2, IgG3, et IgG4.

31. CAM destiné à être utilisé de la revendication 30, dans lequel l'isotype est IgG1, en option l'allotype IgGlm(f).

32. CAM destiné à être utilisé de l'une quelconque des revendications précédentes, qui est un anticorps monoclonal de longueur complète, tel qu'un anticorps monoclonal IgG1,k de longueur complète.

33. CAM destiné à être utilisé de l'une quelconque des revendications 1 à 32, dans lequel l'anticorps est un anticorps déficient en fonction d'effecteur, un anticorps IgG4 stabilisé ou un anticorps monovalent.

34. CAM destiné à être utilisé de la revendication 33, dans lequel la chaîne lourde a été modifiée de sorte que la région de charnière complète a été supprimée.

35. CAM destiné à être utilisé de l'une quelconque des revendications 33 et 34, dans lequel la séquence de l'anticorps a été modifiée de telle façon qu'elle ne comprend aucun site accepteur pour glycosylation liée à N.

36. CAM destiné à être utilisé de l'une quelconque des revendications précédentes, dans lequel l'anticorps est un anticorps monocaténaire.

37. CAM destiné à être utilisé de l'une quelconque des revendications précédentes, dans lequel l'anticorps est un anticorps bispécifique comprenant une première région de liaison d'un anticorps selon l'une quelconque des revendications précédentes, et une seconde région de liaison qui se lie à une cible différente ou un épitope différent de celle/celui de la première région de liaison.


### SEQUENCE LISTING

<110> Genmab A/S
<120> AXL-SPECIFIC ANTIBODY-DRUG CONJUGATES FOR CANCER TREATMENT
<130> P/0097-WO
<160> 162
<170> PatentIn version 3.5
<210> 1
   <211> 116
   <212> PRT
   <213> homo sapiens
<400> 1
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Ser Tyr
               20                  25                  30
   Ala Met Asn Trp Val Arg Gln Ala Pro Gly Lys Gly Leu Glu Trp Val
           35                  40                  45
   Ser Thr Thr Ser Gly Ser Gly Ala Ser Thr Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Lys Ile Trp Ile Ala Phe Asp Ile Trp Gly Gln Gly Thr Met Val
               100                 105                 110
   Thr Val Ser Ser
           115
<210> 2
   <211> 108
   <212> PRT
   <213> homo sapiens
<400> 2
   Glu Ile Val Leu Thr Gln Ser Pro Gly Thr Leu Ser Leu Ser Pro Gly
   1               5                   10                  15
   Glu Arg Ala Thr Leu Ser Cys Arg Ala Ser Gln Ser Val Ser Ser Ser
               20                  25                  30
   Tyr Leu Ala Trp Tyr Gln Gln Lys Pro Gly Gln Ala Pro Arg Leu Leu
           35                  40                  45
   Ile Tyr Gly Ala Ser Ser Arg Ala Thr Gly Ile Pro Asp Arg Phe Ser
       50                  55                  60
   Gly Ser Gly Ser Gly Thr Asp Phe Thr Leu Thr Ile Ser Arg Leu Glu
   65                  70                  75                  80
   Pro Glu Asp Phe Ala Val Tyr Tyr Cys Gln Gln Tyr Gly Ser Ser Pro
                   85                  90                  95
   Tyr Thr Phe Gly Gln Gly Thr Lys Leu Glu Ile Lys
               100                 105
<210> 3
   <211> 120
   <212> PRT
   <213> homo sapiens
<400> 3
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Ser Tyr
               20                  25                  30
   Ala Met Thr Trp Val Arg Gln Ala Pro Gly Lys Gly Leu Glu Trp Val
           35                  40                  45
   Ser Ala Ile Ser Ile Ser Gly Ala Ser Thr Phe Tyr Ala Asp Ser Val
       50                  55                  60
   Lys Gly Arg Phe Thr Ile Ser Arg Asp Asn Ser Lys Asn Thr Leu Ser
   65                  70                  75                  80
   Leu Gln Met Asn Ser Leu Arg Ala Glu Asp Thr Ala Val Tyr Phe Cys
                   85                  90                  95
   Arg Gly Tyr Ser Gly Tyr Val Tyr Asp Ala Phe Asp Ile Trp Gly Gln
               100                 105                 110
   Gly Thr Met Val Thr Val Ser Ser
           115                 120
<210> 4
   <211> 107
   <212> PRT
   <213> homo sapiens
<400> 4
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Gly Ile Ser Asn Trp
               20                  25                  30
   Leu Ala Trp Tyr Gln Gln Lys Pro Glu Lys Ala Pro Lys Ser Leu Ile
           35                  40                  45
   Tyr Ala Ala Ser Ser Leu Gln Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gln Gln Tyr Asn Ser Tyr Pro Leu
                   85                  90                  95
   Thr Phe Gly Gly Gly Thr Lys Val Glu Ile Lys
               100                 105
<210> 5
   <211> 120
   <212> PRT
   <213> homo sapiens
<400> 5
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Ser Tyr
               20                  25                  30
   Ala Met Thr Trp Val Arg Gln Ala Pro Gly Lys Gly Leu Glu Trp Val
           35                  40                  45
   Ser Ala Ile Ser Ile Ser Gly Gly Ser Thr Phe Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Arg Gly Tyr Ser Gly Tyr Val Tyr Asp Ala Phe Asp Phe Trp Gly Gln
   100 105 110
   Gly Thr Met Val Thr Val Ser Ser
   115 120
<210> 6
   <211> 107
   <212> PRT
   <213> homo sapiens
<400> 6
   Asp Ile Gln Met Thr Gln Ser Pro Ser Ser Leu Ser Ala Ser Val Gly
   1 5 10 15
   Asp Arg Val Thr Ile Thr Cys Arg Ala Ser Gln Gly Ile Ser Asn Trp
   20 25 30
   Leu Ala Trp Tyr Gln Gln Lys Pro Glu Lys Ala Pro Lys Ser Leu Ile
   35 40 45
   Tyr Ala Ala Ser Ser Leu Gln Ser Gly Val Pro Ser Arg Phe Ser Gly
   50 55 60
   Ser Gly Ser Gly Thr Asp Phe Thr Leu Thr Ile Ser Ser Leu Gln Pro
   65 70 75 80
   Glu Asp Phe Ala Thr Tyr Tyr Cys Gln Gln Tyr Asn Ser Tyr Pro Leu
   85 90 95
   Thr Phe Gly Gly Gly Thr Lys Val Glu Ile Lys
   100 105
<210> 7
   <211> 121
   <212> PRT
   <213> homo sapiens
<400> 7
   Glu Val Gln Leu Leu Asp Ser Gly Gly Gly Leu Val Gln Pro Gly Gly
   1 5 10 15
   Ser Leu Arg Leu Ser Cys Ala Ala Ser Gly Phe Thr Phe Ser Ser Tyr
   20 25 30
   Ala Met Ser Trp Val Arg Gln Ala Pro Gly Lys Gly Leu Glu Trp Val
   35 40 45
   Ser Ala Ile Ser Ile Gly Gly Gly Asn Ala Tyr Tyr Ala Asp Ser Val
   50 55 60
   Lys Gly Arg Phe Thr Ile Ser Arg Asp Asn Ser Lys Asn Thr Leu Tyr
   65 70 75 80
   Leu Gln Met Asn Ser Leu Arg Ala Ala Asp Thr Ala Val Tyr Tyr Cys
   85 90 95
   Ala Lys Pro Gly Phe Ile Met Val Arg Gly Pro Leu Asp Tyr Trp Gly
   100 105 110
   Gln Gly Ala Leu Val Thr Val Ser Ser
   115 120
<210> 8
   <211> 121
   <212> PRT
   <213> homo sapiens
<400> 8
   Glu Val Gln Leu Leu Asp Ser Gly Gly Gly Leu Val Gln Pro Gly Gly
   1 5 10 15
   Ser Leu Arg Leu Ser Cys Ala Ala Ser Gly Phe Thr Phe Ser Ser Tyr
   20 25 30
   Ala Met Ser Trp Val Arg Gln Ala Pro Gly Lys Gly Leu Glu Trp Val
   35 40 45
   Ser Ala Ile Ser Ile Gly Gly Gly Asn Ala Tyr Tyr Ala Asp Ser Val
   50 55 60
   Lys Gly Arg Phe Thr Ile Ser Arg Asp Asn Ser Lys Asn Thr Leu Tyr
   65 70 75 80
   Leu Gln Met Asn Ser Leu Arg Ala Ala Asp Thr Ala Val Tyr Tyr Cys
   85 90 95
   Ala Lys Pro Gly Phe Ile Leu Val Arg Gly Pro Leu Asp Tyr Trp Gly
   100 105 110
   Gln Gly Ala Leu Val Thr Val Ser Ser
   115 120
<210> 9
   <211> 108
   <212> PRT
   <213> homo sapiens
<400> 9
   Glu Ile Val Leu Thr Gln Ser Pro Gly Thr Leu Ser Leu Ser Pro Gly
   1 5 10 15
   Glu Arg Ala Thr Leu Ser Cys Arg Ala Ser Gln Ser Val Ser Asn Ser
   20 25 30
   Tyr Leu Ala Trp Tyr Gln Gln Lys Pro Gly Gln Ala Pro Arg Leu Leu
   35 40 45
   Ile Tyr Gly Ala Ser Ser Arg Ala Thr Gly Ile Pro Asp Arg Phe Ser
   50 55 60
   Gly Ser Gly Ser Gly Thr Asp Phe Thr Leu Thr Ile Ser Arg Leu Glu
   65 70 75 80
   Pro Glu Asp Phe Ala Val Tyr Tyr Cys Gln Gln Tyr Gly Ser Ser Pro
   85 90 95
   Tyr Thr Phe Gly Gln Gly Thr Lys Leu Glu Ile Lys
   100 105
<210> 10
   <211> 125
   <212> PRT
   <213> homo sapiens
<400> 10
   Glu Val Gln Leu Leu Glu Ser Gly Gly Gly Leu Val Gln Pro Gly Gly
   1 5 10 15
   Ser Leu Arg Leu Ser Cys Ala Ala Ser Gly Phe Thr Phe Ser Ser Tyr
   20 25 30
   Ala Met Ser Trp Val Arg Gln Ala Pro Gly Lys Gly Leu Glu Trp Val
   35 40 45
   Ser Asp Ile Ser Val Ser Gly Gly Ser Thr Tyr Tyr Ala Asp Ser Val
   50 55 60
   Lys Gly Arg Phe Thr Ile Ser Arg Asp Asn Ser Lys Asn Thr Leu Tyr
   65 70 75 80
   Leu Gln Met Asn Ser Leu Arg Ala Glu Asp Thr Ala Val Tyr Tyr Cys
   85 90 95
   Ala Lys Glu Gly Tyr Ile Trp Phe Gly Glu Ser Leu Ser Tyr Ala Phe
   100 105 110
   Asp Ile Trp Gly Gln Gly Thr Met Val Thr Val Ser Ser
   115 120 125
<210> 11
   <211> 107
   <212> PRT
   <213> homo sapiens
<400> 11
   Glu Ile Val Leu Thr Gln Ser Pro Gly Thr Leu Ser Leu Ser Pro Gly
   1 5 10 15
   Glu Arg Ala Thr Leu Ser Cys Arg Ala Ser Gln Ser Val Ser Ser Ser
   20 25 30
   Tyr Leu Ala Trp Tyr Gln Gln Lys Pro Gly Gln Ala Pro Arg Leu Leu
   35 40 45
   Ile Tyr Gly Ala Ser Ser Arg Ala Thr Gly Ile Pro Asp Arg Phe Ser
   50 55 60
   Gly Ser Gly Ser Gly Thr Asp Phe Thr Leu Thr Ile Ser Arg Leu Glu
   65 70 75 80
   Pro Glu Asp Phe Ala Val Tyr Tyr Cys Gln Gln Tyr Gly Arg Ser Phe
   85 90 95
   Thr Phe Gly Pro Gly Thr Lys Val Asp Ile Lys
   100 105
<210> 12
   <211> 125
   <212> PRT
   <213> homo sapiens
<400> 12
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Asn Tyr
               20                  25                  30
   Ala Met Ser Trp Val Arg Gln Ala Pro Gly Lys Gly Leu Glu Trp Val
           35                  40                  45
   Ser Asp Ile Ser Val Ser Gly Gly Ser Thr Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Lys Glu Gly Tyr Ile Trp Phe Gly Glu Ser Leu Ser Tyr Ala Phe
               100                 105                 110
   Asp Ile Trp Gly Gln Gly Thr Met Val Thr Val Ser Ser
           115                 120                 125
<210> 13
   <211> 107
   <212> PRT
   <213> homo sapiens
<400> 13
   Glu Ile Val Leu Thr Gln Ser Pro Gly Thr Leu Ser Leu Ser Pro Gly
   1               5                   10                  15
   Glu Arg Ala Thr Leu Ser Cys Arg Ala Ser Gln Ser Val Ser Ser Ser
               20                  25                  30
   Tyr Leu Ala Trp Tyr Gln Gln Lys Pro Gly Gln Ala Pro Arg Leu Leu
           35                  40                  45
   Ile Tyr Gly Ala Ser Ser Arg Ala Thr Gly Ile Pro Asp Arg Phe Ser
       50                  55                  60
   Gly Ser Gly Ser Gly Thr Asp Phe Thr Leu Thr Ile Ser Arg Leu Glu
   65                  70                  75                  80
   Pro Glu Asp Phe Ala Val Tyr Tyr Cys Gln Gln Tyr Gly Arg Ser Phe
                   85                  90                  95
   Thr Phe Gly Pro Gly Thr Lys Val Asp Ile Lys
               100                 105
<210> 14
   <211> 125
   <212> PRT
   <213> homo sapiens
<400> 14
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Ser Tyr
               20                  25                  30
   Ala Met Ser Trp Val Arg Gln Ala Pro Gly Lys Gly Leu Glu Trp Val
           35                  40                  45
   Ser Asp Ile Ser Val Ser Gly Gly Ser Thr Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu His Met Asn Ser Leu Arg Ala Glu Asp Thr Ala Val Tyr Tyr Cys
                   85                  90                  95
   Ala Lys Glu Gly Tyr Ile Trp Phe Gly Glu Ser Leu Ser Tyr Ala Phe
               100                 105                 110
   Asp Ile Trp Gly Gln Gly Thr Met Val Thr Val Ser Ser
           115                 120                 125
<210> 15
   <211> 107
   <212> PRT
   <213> homo sapiens
<400> 15
   Glu Ile Val Leu Thr Gln Ser Pro Gly Thr Leu Ser Leu Ser Pro Gly
   1               5                   10                  15
   Glu Arg Ala Thr Leu Ser Cys Arg Ala Ser Gln Ser Val Ser Ser Ser
               20                  25                  30
   Tyr Leu Ala Trp Tyr Gln Gln Lys Pro Gly Gln Ala Pro Arg Leu Leu
           35                  40                  45
   Ile Tyr Gly Ala Ser Ser Arg Ala Thr Gly Ile Pro Asp Arg Phe Ser
       50                  55                  60
   Gly Ser Gly Ser Gly Thr Asp Phe Thr Leu Thr Ile Ser Arg Leu Glu
   65                  70                  75                  80
   Pro Glu Asp Phe Ala Val Tyr Tyr Cys Gln Gln Tyr Gly Arg Ser Phe
                   85                  90                  95
   Thr Phe Gly Pro Gly Thr Lys Val Asp Ile Lys
               100                 105
<210> 16
   <211> 117
   <212> PRT
   <213> homo sapiens
<400> 16
   Gln Val Gln Leu Gln Gln Trp Gly Ala Gly Leu Leu Lys Pro Ser Glu
   1               5                   10                  15
   Thr Leu Ser Leu Thr Cys Ala Val Tyr Gly Gly Ser Phe Ser Gly Tyr
               20                  25                  30
   Tyr Trp Ser Trp Ile Arg Gln Pro Pro Gly Lys Gly Leu Glu Trp Ile
           35                  40                  45
   Gly Glu Ile Asn Gln Ser Gly Ser Thr Asn Tyr Asn Pro Ser Leu Lys
       50                  55                  60
   Ser Arg Val Thr Ile Ser Val Asp Thr Ser Lys Asn Gln Phe Ser Leu
   65                  70                  75                  80
   Lys Leu Ser Ser Val Thr Ala Ala Asp Thr Ser Val Tyr Tyr Cys Ala
                   85                  90                  95
   Ser Gly Asn Trp Asp His Phe Phe Asp Tyr Trp Gly Gln Gly Thr Leu
               100                 105                 110
   Val Thr Val Ser Ser
           115
<210> 17
   <211> 117
   <212> PRT
   <213> homo sapiens
<400> 17
   Gln Val Gln Leu Gln Gln Trp Gly Ala Gly Leu Leu Lys Pro Ser Glu
   1               5                   10                  15
   Thr Leu Ser Leu Thr Cys Ala Val Tyr Gly Gly Ser Phe Ser Gly Tyr
               20                  25                  30
   Tyr Trp Ser Trp Ile Arg Gln Pro Pro Gly Lys Gly Leu Glu Trp Ile
           35                  40                  45
   Gly Glu Ile Gln Gln Ser Gly Ser Thr Asn Tyr Asn Pro Ser Leu Lys
       50                  55                  60
   Ser Arg Val Thr Ile Ser Val Asp Thr Ser Lys Asn Gln Phe Ser Leu
   65 70 75 80
   Lys Leu Ser Ser Val Thr Ala Ala Asp Thr Ser Val Tyr Tyr Cys Ala
   85 90 95
   Ser Gly Asn Trp Asp His Phe Phe Asp Tyr Trp Gly Gln Gly Thr Leu
   100 105 110
   Val Thr Val Ser Ser
   115
<210> 18
   <211> 107
   <212> PRT
   <213> homo sapiens
<400> 18
   Asp Ile Gln Met Thr Gln Ser Pro Ser Ser Val Ser Ala Ser Val Gly
   1 5 10 15
   Asp Arg Val Thr Ile Thr Cys Arg Ala Ser Gln Gly Ile Ser Ser Trp
   20 25 30
   Leu Ala Trp Tyr Gln His Lys Pro Gly Lys Ala Pro Lys Leu Leu Ile
   35 40 45
   Tyr Ala Thr Ser Ser Leu Gln Ser Gly Val Thr Ser Arg Phe Ser Gly
   50 55 60
   Ser Gly Ser Gly Thr Asp Phe Thr Leu Thr Ile Ser Ser Leu Gln Pro
   65 70 75 80
   Glu Asp Phe Ala Thr Tyr Tyr Cys Gln Gln Ala Lys Ser Phe Pro Trp
   85 90 95
   Thr Phe Gly Gln Gly Thr Lys Val Glu Ile Lys
   100 105
<210> 19
   <211> 123
   <212> PRT
   <213> homo sapiens
<400> 19
   Gln Val Pro Leu Gln Gln Trp Gly Ala Gly Leu Leu Lys Pro Ser Glu
   1 5 10 15
   Thr Leu Ser Leu Thr Cys Ala Val Tyr Gly Gly Ser Phe Ser Gly Tyr
   20 25 30
   His Trp Ser Trp Ile Arg Gln Pro Pro Gly Lys Gly Leu Glu Trp Ile
   35 40 45
   Gly Glu Ile Ser His Ser Gly Arg Thr Asn Tyr Asn Pro Ser Leu Lys
   50 55 60
   Ser Arg Val Thr Ile Ser Ile Asp Thr Ser Lys Asn Gln Phe Ser Leu
   65 70 75 80
   Lys Leu Ser Ser Val Thr Ala Ala Asp Thr Ala Val Tyr Tyr Cys Ala
   85 90 95
   Ser Phe Ile Thr Met Ile Arg Gly Thr Ile Ile Thr His Phe Asp Tyr
   100 105 110
   Trp Gly Gln Gly Thr Leu Val Thr Val Ser Ser
   115 120
<210> 20
   <211> 107
   <212> PRT
   <213> homo sapiens
<400> 20
   Asp Ile Gln Met Thr Gln Ser Pro Ser Ser Leu Ser Ala Ser Val Gly
   1 5 10 15
   Asp Arg Val Thr Ile Thr Cys Arg Ala Ser Gln Gly Ile Ser Ser Trp
   20 25 30
   Leu Ala Trp Tyr Gln Gln Lys Pro Glu Lys Ala Pro Lys Ser Leu Ile
   35 40 45
   Tyr Ala Ala Ser Ser Leu Gln Ser Gly Val Pro Ser Arg Phe Ser Gly
   50 55 60
   Ser Gly Ser Gly Thr Asp Phe Thr Leu Thr Ile Ser Ser Leu Gln Pro
   65 70 75 80
   Glu Asp Phe Ala Thr Tyr Tyr Cys Gln Gln Tyr His Ser Tyr Pro Tyr
   85 90 95
   Thr Phe Gly Gln Gly Thr Lys Leu Glu Ile Lys
   100 105
<210> 21
   <211> 124
   <212> PRT
   <213> homo sapiens
<400> 21
   Gln Val Gln Leu Val Gln Ser Gly Ala Glu Val Lys Lys Pro Gly Ser
   1               5                   10                  15
   Ser Val Lys Val Ser Cys Lys Ala Ser Gly Gly Thr Phe Ser Ser Tyr
               20                  25                  30
   Ala Ile Ser Trp Val Arg Gln Ala Pro Gly Gln Gly Leu Glu Trp Met
           35                  40                  45
   Gly Arg Ile Ile Pro Ile Phe Gly Ile Ala Asn Tyr Val Gln Lys Phe
       50                  55                  60
   Gln Gly Arg Val Thr Ile Thr Ala Asp Lys Ser Thr Ser Thr Ala Tyr
   65                  70                  75                  80
   Met Glu Leu Ser Ser Leu Arg Ala Glu Asp Thr Ala Val Tyr Tyr Cys
                   85                  90                  95
   Ala Arg Arg Gly Asp Tyr Tyr Gly Ser Gly Ser Pro Asp Val Phe Asp
               100                 105                 110
   Ile Trp Gly Gln Gly Thr Met Val Thr Val Ser Ser
           115                 120
<210> 22
   <211> 107
   <212> PRT
   <213> homo sapiens
<400> 22
   Glu Ile Val Leu Thr Gln Ser Pro Gly Thr Leu Ser Leu Ser Pro Gly
   1               5                   10                  15
   Glu Arg Ala Thr Leu Ser Cys Arg Ala Ser Gln Ser Val Ser Ser Ser
               20                  25                  30
   Tyr Leu Ala Trp Tyr Gln Gln Lys Pro Gly Gln Ala Pro Arg Leu Leu
           35                  40                  45
   Ile Tyr Gly Ala Ser Ser Arg Ala Thr Gly Ile Pro Asp Arg Phe Ser
       50                  55                  60
   Gly Ser Gly Ser Gly Thr Asp Phe Thr Leu Thr Ile Ser Arg Leu Glu
   65                  70                  75                  80
   Pro Glu Asp Phe Ala Val Tyr Tyr Cys Gln Gln Tyr Gly Ser Ser Tyr
                   85                  90                  95
   Thr Phe Gly Gln Gly Thr Lys Leu Glu Ile Lys
               100                 105
<210> 23
   <211> 124
   <212> PRT
   <213> homo sapiens
<400> 23
   Gln Val Gln Leu Val Gln Ser Gly Ala Glu Val Lys Lys Pro Gly Ser
   1 5 10 15
   Ser Val Lys Val Ser Cys Lys Ala Ser Gly Gly Thr Phe Ser Ser Tyr
   20 25 30
   Ala Ile Ser Trp Val Arg Gln Ala Pro Gly Gln Gly Leu Glu Trp Met
   35 40 45
   Gly Arg Ile Ile Pro Ile Phe Gly Ile Ala Asn Tyr Val Gln Lys Phe
   50 55 60
   Gln Gly Arg Val Thr Ile Thr Ala Asp Lys Ser Thr Ser Thr Ala Tyr
   65 70 75 80
   Met Glu Leu Ser Ser Leu Arg Ala Glu Asp Thr Ala Val Tyr Tyr Cys
   85 90 95
   Ala Arg Arg Gly Asn Tyr Tyr Gly Ser Gly Ser Pro Asp Val Phe Asp
   100 105 110
   Ile Trp Gly Gln Gly Thr Met Val Thr Val Ser Ser
   115 120
<210> 24
   <211> 107
   <212> PRT
   <213> homo sapiens
<400> 24
   Glu Ile Val Leu Thr Gln Ser Pro Gly Thr Leu Ser Leu Ser Pro Gly
   1 5 10 15
   Glu Arg Ala Thr Leu Ser Cys Arg Ala Ser Gln Ser Val Ser Ser Ser
   20 25 30
   Tyr Leu Ala Trp Tyr Gln Gln Lys Pro Gly Gln Ala Pro Arg Leu Leu
   35 40 45
   Ile Tyr Gly Ala Ser Ser Arg Ala Thr Gly Ile Pro Asp Arg Phe Ser
   50 55 60
   Gly Ser Gly Ser Gly Thr Asp Phe Thr Leu Thr Ile Ser Arg Leu Glu
   65 70 75 80
   Pro Glu Asp Phe Ala Val Tyr Tyr Cys Gln Gln Tyr Gly Ser Ser Tyr
   85 90 95
   Thr Phe Gly Gln Gly Thr Lys Leu Glu Ile Lys
   100 105
<210> 25
   <211> 124
   <212> PRT
   <213> homo sapiens
<400> 25
   Gln Val Gln Leu Val Gln Ser Gly Ala Glu Val Lys Lys Pro Gly Ser
   1 5 10 15
   Ser Val Lys Val Ser Cys Lys Ala Ser Gly Gly Thr Phe Ser Ser Tyr
   20 25 30
   Ala Ile Asn Trp Met Arg Gln Ala Pro Gly Gln Gly Leu Glu Trp Met
   35 40 45
   Gly Arg Ile Ile Pro Ile Phe Gly Ile Val Asn Tyr Ala Gln Lys Phe
   50 55 60
   Gln Gly Arg Val Thr Leu Thr Ala Asp Lys Ser Thr Ser Thr Ala Tyr
   65 70 75 80
   Met Glu Leu Ser Ser Leu Arg Ser Glu Asp Thr Ala Val Tyr Tyr Cys
   85 90 95
   Ala Arg Arg Gly Asn Tyr Tyr Gly Ser Gly Ser Pro Asp Val Phe Asp
   100 105 110
   Ile Trp Gly Gln Gly Thr Met Val Thr Val Ser Ser
   115 120
<210> 26
   <211> 107
   <212> PRT
   <213> homo sapiens
<400> 26
   Glu Ile Val Leu Thr Gln Ser Pro Gly Thr Leu Ser Leu Ser Pro Gly
   1               5                   10                  15
   Glu Arg Ala Thr Leu Ser Cys Arg Ala Ser Gln Ser Val Ser Ser Ser
               20                  25                  30
   Tyr Leu Ala Trp Tyr Gln Gln Lys Pro Gly Gln Ala Pro Arg Leu Leu
           35                  40                  45
   Ile Tyr Gly Ala Ser Ser Arg Ala Thr Gly Ile Pro Asp Arg Phe Ser
       50                  55                  60
   Gly Ser Gly Ser Gly Thr Asp Phe Thr Leu Thr Ile Ser Arg Leu Glu
   65                  70                  75                  80
   Pro Glu Asp Phe Ala Val Tyr Tyr Cys Gln Gln Tyr Gly Ser Ser Tyr
                   85                  90                  95
   Thr Phe Gly Gln Gly Thr Lys Leu Glu Ile Lys
               100                 105
<210> 27
   <211> 124
   <212> PRT
   <213> homo sapiens
<400> 27
   Gln Val Gln Leu Val Gln Ser Gly Ala Glu Val Lys Lys Pro Gly Ser
   1               5                   10                  15
   Ser Val Lys Val Ser Cys Lys Ala Ser Gly Gly Thr Phe Ser Ser Tyr
               20                  25                  30
   Ala Ile Asn Trp Met Arg Gln Ala Pro Gly Gln Gly Leu Glu Trp Met
           35                  40                  45
   Gly Arg Ile Ile Pro Ile Phe Gly Ile Val Asn Tyr Ala Gln Lys Phe
       50                  55                  60
   Gln Gly Arg Val Thr Leu Thr Ala Asp Lys Ser Thr Ser Thr Ala Tyr
   65                  70                  75                  80
   Met Glu Leu Ser Ser Leu Arg Ser Glu Asp Thr Ala Val Tyr Tyr Cys
                   85                  90                  95
   Ala Arg Arg Gly Asn Tyr Tyr Gly Ser Gly Ser Pro Asp Val Phe Asp
   100 105 110
   Ile Trp Gly Gln Gly Thr Met Val Thr Val Ser Ser
   115 120
<210> 28
   <211> 106
   <212> PRT
   <213> homo sapiens
<400> 28
   Glu Ile Val Leu Thr Gln Ser Pro Ala Thr Leu Ser Leu Ser Pro Gly
   1 5 10 15
   Glu Arg Ala Thr Leu Ser Cys Arg Ala Ser Gln Ser Val Ser Ser Tyr
   20 25 30
   Leu Ala Trp Tyr Gln Gln Lys Pro Gly Gln Ala Pro Arg Leu Leu Ile
   35 40 45
   Tyr Asp Ala Ser Asn Arg Ala Thr Gly Ile Pro Ala Arg Phe Ser Gly
   50 55 60
   Ser Gly Ser Gly Thr Asp Phe Thr Leu Thr Ile Ser Ser Leu Glu Pro
   65 70 75 80
   Glu Asp Phe Ala Val Tyr Tyr Cys Gln Gln Arg Ser Asn Trp Leu Thr
   85 90 95
   Phe Gly Gly Gly Thr Lys Val Glu Ile Lys
   100 105
<210> 29
   <211> 124
   <212> PRT
   <213> homo sapiens
<400> 29
   Gln Val Gln Leu Val Gln Ser Gly Ala Glu Val Lys Lys Pro Gly Ser
   1 5 10 15
   Ser Val Lys Val Ser Cys Lys Ala Ser Gly Gly Thr Phe Ser Ser Tyr
   20 25 30
   Ala Ile Ser Trp Val Arg Gln Ala Pro Gly Gln Gly Leu Glu Trp Met
   35 40 45
   Gly Arg Ile Ile Pro Ile Phe Gly Ile Ala Asn Tyr Ala Gln Lys Phe
   50 55 60
   Gln Gly Arg Val Thr Ile Thr Ala Asp Lys Ser Thr Ser Thr Ala Tyr
   65 70 75 80
   Met Glu Leu Ser Ser Leu Arg Ser Glu Asp Thr Ala Val Tyr Tyr Cys
   85 90 95
   Ala Arg Arg Gly Asn Tyr Tyr Gly Ser Gly Ser Pro Asp Val Phe Asp
   100 105 110
   Ile Trp Gly Gln Gly Thr Met Val Thr Val Ser Ser
   115 120
<210> 30
   <211> 107
   <212> PRT
   <213> homo sapiens
<400> 30
   Glu Ile Val Leu Thr Gln Ser Pro Gly Thr Leu Ser Leu Ser Pro Gly
   1 5 10 15
   Glu Arg Ala Thr Leu Ser Cys Arg Ala Ser Gln Ser Val Ser Ser Ser
   20 25 30
   Tyr Leu Ala Trp Tyr Gln Gln Lys Pro Gly Gln Ala Pro Arg Leu Leu
   35 40 45
   Ile Tyr Gly Ala Ser Ser Arg Ala Thr Gly Ile Pro Asp Arg Phe Ser
   50 55 60
   Gly Ser Gly Ser Gly Thr Asp Phe Thr Leu Thr Ile Ser Arg Leu Glu
   65 70 75 80
   Pro Glu Asp Phe Ala Val Tyr Tyr Cys Gln Gln Tyr Gly Ser Ser Tyr
   85 90 95
   Thr Phe Gly Gln Gly Thr Lys Leu Glu Ile Lys
   100 105
<210> 31
   <211> 123
   <212> PRT
   <213> homo sapiens
<400> 31
   Gln Val Gln Leu Gln Gln Trp Gly Ala Gly Leu Leu Lys Pro Ser Glu
   1 5 10 15
   Thr Leu Ser Leu Thr Cys Ala Ile Asp Gly Gly Ser Phe Ser Gly Tyr
   20 25 30
   Tyr Trp Ser Trp Ile Arg Gln Pro Pro Gly Lys Gly Leu Glu Trp Ile
   35 40 45
   Gly Glu Ile Ser His Ser Gly Arg Thr Asn Tyr Asn Pro Ser Leu Lys
   50 55 60
   Ser Arg Val Thr Ile Ser Ile Asp Thr Ser Lys Asn Gln Phe Ser Leu
   65 70 75 80
   Lys Leu Ser Ser Val Ala Ala Ala Asp Thr Ala Val Tyr Tyr Cys Ala
   85 90 95
   Arg Phe Ile Thr Met Ile Arg Gly Ala Ile Ile Thr His Phe Asp Tyr
   100 105 110
   Trp Gly Gln Gly Ala Leu Val Thr Val Ser Ser
   115 120
<210> 32
   <211> 123
   <212> PRT
   <213> homo sapiens
<400> 32
   Gln Val Gln Leu Gln Gln Trp Gly Ala Gly Leu Leu Lys Pro Ser Glu
   1               5                   10                  15
   Thr Leu Ser Leu Thr Cys Ala Ile Asp Gly Gly Ser Phe Ser Gly Tyr
               20                  25                  30
   Tyr Trp Ser Trp Ile Arg Gln Pro Pro Gly Lys Gly Leu Glu Trp Ile
           35                  40                  45
   Gly Glu Ile Ser His Ser Gly Arg Thr Asn Tyr Asn Pro Ser Leu Lys
       50                  55                  60
   Ser Arg Val Thr Ile Ser Ile Asp Thr Ser Lys Asn Gln Phe Ser Leu
   65                  70                  75                  80
   Lys Leu Ser Ser Val Ala Ala Ala Asp Thr Ala Val Tyr Tyr Cys Ala
                   85                  90                  95
   Arg Phe Ile Thr Leu Ile Arg Gly Ala Ile Ile Thr His Phe Asp Tyr
                   100                 105                 110
   Trp Gly Gln Gly Ala Leu Val Thr Val Ser Ser
           115                 120
<210> 33
   <211> 107
   <212> PRT
   <213> homo sapiens
<400> 33
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Gly Ile Ser Ser Trp
               20                  25                  30
   Leu Ala Trp Tyr Gln Gln Lys Pro Glu Lys Ala Pro Lys Ser Leu Ile
           35                  40                  45
   Tyr Ala Ala Ser Ser Leu Gln Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gln Gln Tyr His Ser Tyr Pro Tyr
                   85                  90                  95
   Thr Phe Gly Gln Gly Thr Lys Leu Glu Ile Lys
               100                 105
<210> 34
   <211> 120
   <212> PRT
   <213> homo sapiens
<400> 34
   Gln Val Gln Leu Val Glu Ser Gly Gly Gly Val Val Gln Pro Gly Arg
   1               5                   10                  15
   Ser Leu Arg Leu Ser Cys Ala Ala Ser Gly Phe Ser Phe Ser Thr Tyr
               20                  25                  30
   Ala Met His Trp Val Arg Gln Ala Pro Gly Lys Gly Leu Glu Trp Val
           35                  40                  45
   Ala Val Ile Ser Tyr Asp Gly Asp Asn Lys Tyr Ser Ala Asp Ser Val
       50                  55                  60
   Lys Gly Arg Phe Thr Ile Ser Arg Asp Asn Ser Lys Asn Thr Leu Tyr
   65 70 75 80
   Leu Gln Met Asn Ser Leu Arg Ala Glu Asp Thr Ala Val Tyr Tyr Cys
   85 90 95
   Ala Arg Gly Arg Lys Leu Gly Ile Asp Ala Phe Asp Ile Trp Gly Gln
   100 105 110
   Gly Thr Met Val Thr Val Ser Ser
   115 120
<210> 35
   <211> 107
   <212> PRT
   <213> homo sapiens
<400> 35
   Ala Ile Gln Leu Thr Gln Ser Pro Ser Ser Leu Ser Ala Ser Val Gly
   1 5 10 15
   Asp Arg Val Thr Ile Thr Cys Arg Ala Ser Gln Gly Ile Ser Ser Ala
   20 25 30
   Leu Ala Trp Tyr Gln Gln Lys Pro Gly Lys Ala Pro Lys Leu Leu Ile
   35 40 45
   Tyr Asp Ala Ser Ser Leu Glu Ser Gly Val Pro Ser Arg Phe Ser Gly
   50 55 60
   Ser Gly Ser Gly Thr Asp Phe Thr Leu Thr Ile Ser Gly Leu Gln Pro
   65 70 75 80
   Glu Asp Phe Ala Thr Tyr Tyr Cys Gln Gln Phe Asn Ser Tyr Pro Phe
   85 90 95
   Thr Phe Gly Pro Gly Thr Lys Val Asp Ile Lys
   100 105
<210> 36
   <211> 8
   <212> PRT
   <213> homo sapiens
<400> 36
   Gly Phe Thr Phe Ser Ser Tyr Ala
   1 5
<210> 37
   <211> 8
   <212> PRT
   <213> homo sapiens
<400> 37
   Thr Ser Gly Ser Gly Ala Ser Thr
   1 5
<210> 38
   <211> 9
   <212> PRT
   <213> homo sapiens
<400> 38
   Ala Lys Ile Trp Ile Ala Phe Asp Ile
   1 5
<210> 39
   <211> 7
   <212> PRT
   <213> homo sapiens
<400> 39
   Gln Ser Val Ser Ser Ser Tyr
   1 5
<210> 40
   <211> 9
   <212> PRT
   <213> homo sapiens
<400> 40
   Gln Gln Tyr Gly Ser Ser Pro Tyr Thr
   1 5
<210> 41
   <211> 8
   <212> PRT
   <213> homo sapiens
<400> 41
   Gly Phe Thr Phe Ser Ser Tyr Ala
   1 5
<210> 42
   <211> 8
   <212> PRT
   <213> homo sapiens
<400> 42
   Ile Ser Ile Ser Gly Ala Ser Thr
   1 5
<210> 43
   <211> 13
   <212> PRT
   <213> homo sapiens
<400> 43
   Arg Gly Tyr Ser Gly Tyr Val Tyr Asp Ala Phe Asp Ile
   1 5 10
<210> 44
   <211> 6
   <212> PRT
   <213> homo sapiens
<400> 44
   Gln Gly Ile Ser Asn Trp
   1 5
<210> 45
   <211> 9
   <212> PRT
   <213> homo sapiens
<400> 45
   Gln Gln Tyr Asn Ser Tyr Pro Leu Thr
   1 5
<210> 46
   <211> 8
   <212> PRT
   <213> homo sapiens
<400> 46
   Gly Phe Thr Phe Ser Ser Tyr Ala
   1 5
<210> 47
   <211> 8
   <212> PRT
   <213> homo sapiens
<400> 47
   Ile Ser Ile Ser Gly Gly Ser Thr
   1 5
<210> 48
   <211> 13
   <212> PRT
   <213> homo sapiens
<400> 48
   Arg Gly Tyr Ser Gly Tyr Val Tyr Asp Ala Phe Asp Phe
   1 5 10
<210> 49
   <211> 6
   <212> PRT
   <213> homo sapiens
<400> 49
   Gln Gly Ile Ser Asn Trp
   1 5
<210> 50
   <211> 9
   <212> PRT
   <213> homo sapiens
<400> 50
   Gln Gln Tyr Asn Ser Tyr Pro Leu Thr
   1 5
<210> 51
   <211> 8
   <212> PRT
   <213> homo sapiens
<400> 51
   Gly Phe Thr Phe Ser Ser Tyr Ala
   1 5
<210> 52
   <211> 8
   <212> PRT
   <213> homo sapiens
<400> 52
   Ile Ser Ile Gly Gly Gly Asn Ala
   1 5
<210> 53
   <211> 14
   <212> PRT
   <213> homo sapiens
<400> 53
   Ala Lys Pro Gly Phe Ile Met Val Arg Gly Pro Leu Asp Tyr
   1 5 10
<210> 54
   <211> 14
   <212> PRT
   <213> homo sapiens
<400> 54
   Ala Lys Pro Gly Phe Ile Leu Val Arg Gly Pro Leu Asp Tyr
   1 5 10
<210> 55
   <211> 7
   <212> PRT
   <213> homo sapiens
<400> 55
   Gln Ser Val Ser Asn Ser Tyr
   1 5
<210> 56
   <211> 9
   <212> PRT
   <213> homo sapiens
<400> 56
   Gln Gln Tyr Gly Ser Ser Pro Tyr Thr
   1 5
<210> 57
   <211> 8
   <212> PRT
   <213> homo sapiens
<400> 57
   Gly Phe Thr Phe Ser Ser Tyr Ala
   1 5
<210> 58
   <211> 8
   <212> PRT
   <213> homo sapiens
<400> 58
   Ile Ser Val Ser Gly Gly Ser Thr
   1 5
<210> 59
   <211> 18
   <212> PRT
   <213> homo sapiens
<400> 59
   Ala Lys Glu Gly Tyr Ile Trp Phe Gly Glu Ser Leu Ser Tyr Ala Phe
   1 5 10 15
   Asp Ile
<210> 60
   <211> 7
   <212> PRT
   <213> homo sapiens
<400> 60
   Gln Ser Val Ser Ser Ser Tyr
   1               5
<210> 61
   <211> 8
   <212> PRT
   <213> homo sapiens
<400> 61
   Gln Gln Tyr Gly Arg Ser Phe Thr
   1               5
<210> 62
   <211> 8
   <212> PRT
   <213> homo sapiens
<400> 62
   Gly Phe Thr Phe Ser Asn Tyr Ala
   1               5
<210> 63
   <211> 8
   <212> PRT
   <213> homo sapiens
<400> 63
   Ile Ser Val Ser Gly Gly Ser Thr
   1               5
<210> 64
   <211> 18
   <212> PRT
   <213> homo sapiens
<400> 64
   Ala Lys Glu Gly Tyr Ile Trp Phe Gly Glu Ser Leu Ser Tyr Ala Phe
   1               5                   10                  15
   Asp Ile
<210> 65
   <211> 7
   <212> PRT
   <213> homo sapiens
<400> 65
   Gln Ser Val Ser Ser Ser Tyr
   1 5
<210> 66
   <211> 8
   <212> PRT
   <213> homo sapiens
<400> 66
   Gln Gln Tyr Gly Arg Ser Phe Thr
   1 5
<210> 67
   <211> 8
   <212> PRT
   <213> homo sapiens
<400> 67
   Gly Phe Thr Phe Ser Ser Tyr Ala
   1 5
<210> 68
   <211> 8
   <212> PRT
   <213> homo sapiens
<400> 68
   Ile Ser Val Ser Gly Gly Ser Thr
   1 5
<210> 69
   <211> 18
   <212> PRT
   <213> homo sapiens
<400> 69
   Ala Lys Glu Gly Tyr Ile Trp Phe Gly Glu Ser Leu Ser Tyr Ala Phe
   1 5 10 15
   Asp Ile
<210> 70
   <211> 7
   <212> PRT
   <213> homo sapiens
<400> 70
   Gln Ser Val Ser Ser Ser Tyr
   1 5
<210> 71
   <211> 8
   <212> PRT
   <213> homo sapiens
<400> 71
   Gln Gln Tyr Gly Arg Ser Phe Thr
   1 5
<210> 72
   <211> 8
   <212> PRT
   <213> homo sapiens
<400> 72
   Gly Gly Ser Phe Ser Gly Tyr Tyr
   1 5
<210> 73
   <211> 5
   <212> PRT
   <213> homo sapiens
<400> 73
   Ile Asn Gln Ser Gly
   1 5
<210> 74
   <211> 7
   <212> PRT
   <213> homo sapiens
<400> 74
   Ile Gln Gln Ser Gly Ser Thr
   1 5
<210> 75
   <211> 11
   <212> PRT
   <213> homo sapiens
<400> 75
   Ala Ser Gly Asn Trp Asp His Phe Phe Asp Tyr
   1 5 10
<210> 76
   <211> 6
   <212> PRT
   <213> homo sapiens
<400> 76
   Gln Gly Ile Ser Ser Trp
   1 5
<210> 77
   <211> 9
   <212> PRT
   <213> homo sapiens
<400> 77
   Gln Gln Ala Lys Ser Phe Pro Trp Thr
   1 5
<210> 78
   <211> 8
   <212> PRT
   <213> homo sapiens
<400> 78
   Gly Gly Ser Phe Ser Gly Tyr His
   1 5
<210> 79
   <211> 7
   <212> PRT
   <213> homo sapiens
<400> 79
   Ile Ser His Ser Gly Arg Thr
   1 5
<210> 80
   <211> 17
   <212> PRT
   <213> homo sapiens
<400> 80
   Ala Ser Phe Ile Thr Met Ile Arg Gly Thr Ile Ile Thr His Phe Asp
   1 5 10 15
   Tyr
<210> 81
   <211> 6
   <212> **PRT**
   <213> homo sapiens
<400> 81
   Gln Gly Ile Ser Ser Trp
   1 5
<210> 82
   <211> 9
   <212> PRT
   <213> homo sapiens
<400> 82
   Gln Gln Tyr His Ser Tyr Pro Tyr Thr
   1 5
<210> 83
   <211> 8
   <212> PRT
   <213> homo sapiens
<400> 83
   Gly Gly Thr Phe Ser Ser Tyr Ala
   1 5
<210> 84
   <211> 8
   <212> PRT
   <213> homo sapiens
<400> 84
   Ile Ile Pro Ile Phe Gly Ile Ala
   1 5
<210> 85
   <211> 17
   <212> PRT
   <213> homo sapiens
<400> 85
   Ala Arg Arg Gly Asp Tyr Tyr Gly Ser Gly Ser Pro Asp Val Phe Asp
   1 5 10 15
   Ile
<210> 86
   <211> 7
   <212> PRT
   <213> homo sapiens
<400> 86
   Gln Ser Val Ser Ser Ser Tyr
   1 5
<210> 87
   <211> 8
   <212> PRT
   <213> homo sapiens
<400> 87
   Gln Gln Tyr Gly Ser Ser Tyr Thr
   1 5
<210> 88
   <211> 8
   <212> PRT
   <213> homo sapiens
<400> 88
   Gly Gly Thr Phe Ser Ser Tyr Ala
   1 5
<210> 89
   <211> 8
   <212> PRT
   <213> homo sapiens
<400> 89
   Ile Ile Pro Ile Phe Gly Ile Ala
   1 5
<210> 90
   <211> 17
   <212> PRT
   <213> homo sapiens
<400> 90
   Ala Arg Arg Gly Asn Tyr Tyr Gly Ser Gly Ser Pro Asp Val Phe Asp
   1 5 10 15
   Ile
<210> 91
   <211> 7
   <212> PRT
   <213> homo sapiens
<400> 91
   Gln Ser Val Ser Ser Ser Tyr
   1 5
<210> 92
   <211> 8
   <212> PRT
   <213> homo sapiens
<400> 92
   Gln Gln Tyr Gly Ser Ser Tyr Thr
   1 5
<210> 93
   <211> 8
   <212> PRT
   <213> homo sapiens
<400> 93
   Gly Gly Thr Phe Ser Ser Tyr Ala
   1               5
<210> 94
   <211> 8
   <212> PRT
   <213> homo sapiens
<400> 94
   Ile Ile Pro Ile Phe Gly Ile Val
   1               5
<210> 95
   <211> 17
   <212> PRT
   <213> homo sapiens
<400> 95
   Ala Arg Arg Gly Asn Tyr Tyr Gly Ser Gly Ser Pro Asp Val Phe Asp
   1               5                   10                  15
   Ile
<210> 96
   <211> 7
   <212> PRT
   <213> homo sapiens
<400> 96
   Gln Ser Val Ser Ser Ser Tyr
   1               5
<210> 97
   <211> 8
   <212> PRT
   <213> homo sapiens
<400> 97
   Gln Gln Tyr Gly Ser Ser Tyr Thr
   1               5
<210> 98
   <211> 8
   <212> PRT
   <213> homo sapiens
<400> 98
   Gly Gly Thr Phe Ser Ser Tyr Ala
   1 5
<210> 99
   <211> 8
   <212> PRT
   <213> homo sapiens
<400> 99
   Ile Ile Pro Ile Phe Gly Ile Val
   1 5
<210> 100
   <211> 17
   <212> PRT
   <213> homo sapiens
<400> 100
   Ala Arg Arg Gly Asn Tyr Tyr Gly Ser Gly Ser Pro Asp Val Phe Asp
   1 5 10 15
   Ile
<210> 101
   <211> 6
   <212> PRT
   <213> homo sapiens
<400> 101
   Gln Ser Val Ser Ser Tyr
   1 5
<210> 102
   <211> 8
   <212> PRT
   <213> homo sapiens
<400> 102
   Gln Gln Arg Ser Asn Trp Leu Thr
   1 5
<210> 103
   <211> 8
   <212> PRT
   <213> homo sapiens
<400> 103
   Gly Gly Thr Phe Ser Ser Tyr Ala
   1               5
<210> 104
   <211> 8
   <212> PRT
   <213> homo sapiens
<400> 104
   Ile Ile Pro Ile Phe Gly Ile Ala
   1               5
<210> 105
   <211> 18
   <212> PRT
   <213> homo sapiens
<400> 105
   Ala Arg Arg Gly Asn Tyr Tyr Gly Ser Gly Ser Pro Asp Val Phe Asp
   1               5                   10                  15
   Ile Ser
<210> 106
   <211> 7
   <212> PRT
   <213> homo sapiens
<400> 106
   Gln Ser Val Ser Ser Ser Tyr
   1               5
<210> 107
   <211> 8
   <212> PRT
   <213> homo sapiens
<400> 107
   Gln Gln Tyr Gly Ser Ser Tyr Thr
   1               5
<210> 108
   <211> 8
   <212> PRT
   <213> homo sapiens
<400> 108
   Gly Gly Ser Phe Ser Gly Tyr Tyr
   1               5
<210> 109
   <211> 7
   <212> PRT
   <213> homo sapiens
<400> 109
   Ile Ser His Ser Gly Arg Thr
   1               5
<210> 110
   <211> 17
   <212> PRT
   <213> homo sapiens
<400> 110
   Ala Arg Phe Ile Thr Met Ile Arg Gly Ala Ile Ile Thr His Phe Asp
   1               5                   10                  15
   Tyr
<210> 111
   <211> 17
   <212> PRT
   <213> homo sapiens
<400> 111
   Ala Arg Phe Ile Thr Leu Ile Arg Gly Ala Ile Ile Thr His Phe Asp
   1               5                   10                  15
   Tyr
<210> 112
   <211> 6
   <212> PRT
   <213> homo sapiens
<400> 112
   Gln Gly Ile Ser Ser Trp
   1               5
<210> 113
   <211> 9
   <212> PRT
   <213> homo sapiens
<400> 113
   Gln Gln Tyr His Ser Tyr Pro Tyr Thr
   1               5
<210> 114
   <211> 8
   <212> PRT
   <213> homo sapiens
<400> 114
   Gly Phe Ser Phe Ser Thr Tyr Ala
   1 5
<210> 115
   <211> 8
   <212> PRT
   <213> homo sapiens
<400> 115
   Ile Ser Tyr Asp Gly Asp Asn Lys
   1 5
<210> 116
   <211> 13
   <212> PRT
   <213> homo sapiens
<400> 116
   Ala Arg Gly Arg Lys Leu Gly Ile Asp Ala Phe Asp Ile
   1 5 10
<210> 117
   <211> 6
   <212> PRT
   <213> homo sapiens
<400> 117
   Gln Gly Ile Ser Ser Ala
   1 5
<210> 118
   <211> 9
   <212> PRT
   <213> homo sapiens
<400> 118
   Gln Gln Phe Asn Ser Tyr Pro Phe Thr
   1 5
<210> 119
   <211> 8
   <212> PRT
   <213> homo sapiens
<220>
   <221> MISC_FEATURE
   <222> (6)..(6)
   <223> Wherein X is A or G
<400> 119
   Ile Ser Ile Ser Gly Xaa Ser Thr
   1 5
<210> 120
   <211> 13
   <212> PRT
   <213> homo sapiens
<220>
   <221> Misc
   <222> (13)..(13)
   <223> Wherein X is I of F
<400> 120
   Arg Gly Tyr Ser Gly Tyr Val Tyr Asp Ala Phe Asp Xaa
   1 5 10
<210> 121
   <211> 8
   <212> PRT
   <213> homo sapiens
<220>
   <221> MISC_FEATURE
   <222> (8)..(8)
   <223> Wherein X is I or F
<400> 121
   Gly Gly Ser Phe Ser Gly Tyr Xaa
   1 5
<210> 122
   <211> 17
   <212> PRT
   <213> homo sapiens
<220>
   <221> MISC_FEATURE
   <222> (2)..(2)
   <223> Wherein X is S or R
<220>
   <221> MISC_FEATURE
   <222> (10)..(10)
   <223> Wherein X is T or A
<400> 122
   Ala Xaa Phe Ile Thr Met Ile Arg Gly Xaa Ile Ile Thr His Phe Asp
   1 5 10 15
   Tyr
<210> 123
   <211> 8
   <212> PRT
   <213> homo sapiens
<220>
   <221> MISC_FEATURE
   <222> (6)..(6)
   <223> Wherein X is S or N
<400> 123
   Gly Phe Thr Phe Ser Xaa Tyr Ala
   1 5
<210> 124
   <211> 8
   <212> PRT
   <213> homo sapiens
<400> 124
   Ile Ser Val Ser Gly Gly Ser Thr
   1 5
<210> 125
   <211> 18
   <212> PRT
   <213> homo sapiens
<400> 125
   Ala Lys Glu Gly Tyr Ile Trp Phe Gly Glu Ser Leu Ser Tyr Ala Phe
   1 5 10 15
   Asp Ile
<210> 126
   <211> 8
   <212> PRT
   <213> homo sapiens
<220>
   <221> MISC_FEATURE
   <222> (8)..(8)
   <223> Wherein X is A or V
<400> 126
   Ile Ile Pro Ile Phe Gly Ile Xaa
   1 5
<210> 127
   <211> 17
   <212> PRT
   <213> homo sapiens
<220>
   <221> MISC_FEATURE
   <222> (5)..(5)
   <223> Wherein X is D or N
<400> 127
   Ala Arg Arg Gly Xaa Tyr Tyr Gly Ser Gly Ser Pro Asp Val Phe Asp
   1               5                   10                  15
   Ile
<210> 128
   <211> 7
   <212> PRT
   <213> homo sapiens
<220>
   <221> MISC_FEATURE
   <222> (4)..(4)
   <223> Wherein X is S or deleted
<400> 128
   Gln Ser Val Xaa Ser Ser Tyr
   1               5
<210> 129
   <211> 8
   <212> PRT
   <213> homo sapiens
<220>
   <221> MISC_FEATURE
   <222> (3)..(3)
   <223> Wherein X is R or Y
<220>
   <221> MISC_FEATURE
   <222> (4)..(4)
   <223> Wherein X is Sor G
<220>
   <221> MISC_FEATURE
   <222> (4)..(4)
   <223> Wherein X is Sor G
<220>
   <221> MISC_FEATURE
   <222> (5)..(5)
   <223> Wherein X is N or S
<220>
   <221> MISC_FEATURE
   <222> (6)..(6)
   <223> Wherein X is W or S
<220>
   <221> MISC_FEATURE
   <222> (6)..(6)
   <223> Wherein X is W or S
<220>
   <221> MISC_FEATURE
   <222> (7)..(7)
   <223> Wherein X is L or Y
<400> 129
   Gln Gln Xaa Xaa Xaa Xaa Xaa Thr
   1               5
<210> 130
   <211> 894
   <212> PRT
   <213> homo sapiens
<400> 130
   Met Ala Trp Arg Cys Pro Arg Met Gly Arg Val Pro Leu Ala Trp Cys
   1               5                   10                  15
   Leu Ala Leu Cys Gly Trp Ala Cys Met Ala Pro Arg Gly Thr Gln Ala
               20                  25                  30
   Glu Glu Ser Pro Phe Val Gly Asn Pro Gly Asn Ile Thr Gly Ala Arg
           35                  40                  45
   Gly Leu Thr Gly Thr Leu Arg Cys Gln Leu Gln Val Gln Gly Glu Pro
       50                  55                  60
   Pro Glu Val His Trp Leu Arg Asp Gly Gln Ile Leu Glu Leu Ala Asp
   65                  70                  75                  80
   Ser Thr Gln Thr Gln Val Pro Leu Gly Glu Asp Glu Gln Asp Asp Trp
   85 90 95
   Ile Val Val Ser Gln Leu Arg Ile Thr Ser Leu Gln Leu Ser Asp Thr
   100 105 110
   Gly Gln Tyr Gln Cys Leu Val Phe Leu Gly His Gln Thr Phe Val Ser
   115 120 125
   Gln Pro Gly Tyr Val Gly Leu Glu Gly Leu Pro Tyr Phe Leu Glu Glu
   130 135 140
   Pro Glu Asp Arg Thr Val Ala Ala Asn Thr Pro Phe Asn Leu Ser Cys
   145 150 155 160
   Gln Ala Gln Gly Pro Pro Glu Pro Val Asp Leu Leu Trp Leu Gln Asp
   165 170 175
   Ala Val Pro Leu Ala Thr Ala Pro Gly His Gly Pro Gln Arg Ser Leu
   180 185 190
   His Val Pro Gly Leu Asn Lys Thr Ser Ser Phe Ser Cys Glu Ala His
   195 200 205
   Asn Ala Lys Gly Val Thr Thr Ser Arg Thr Ala Thr Ile Thr Val Leu
   210 215 220
   Pro Gln Gln Pro Arg Asn Leu His Leu Val Ser Arg Gln Pro Thr Glu
   225 230 235 240
   Leu Glu Val Ala Trp Thr Pro Gly Leu Ser Gly Ile Tyr Pro Leu Thr
   245 250 255
   His Cys Thr Leu Gln Ala Val Leu Ser Asp Asp Gly Met Gly Ile Gln
   260 265 270
   Ala Gly Glu Pro Asp Pro Pro Glu Glu Pro Leu Thr Ser Gln Ala Ser
   275 280 285
   Val Pro Pro His Gln Leu Arg Leu Gly Ser Leu His Pro His Thr Pro
   290 295 300
   Tyr His Ile Arg Val Ala Cys Thr Ser Ser Gln Gly Pro Ser Ser Trp
   305 310 315 320
   Thr His Trp Leu Pro Val Glu Thr Pro Glu Gly Val Pro Leu Gly Pro
   325 330 335
   Pro Glu Asn Ile Ser Ala Thr Arg Asn Gly Ser Gln Ala Phe Val His
               340                 345                 350
   Trp Gln Glu Pro Arg Ala Pro Leu Gln Gly Thr Leu Leu Gly Tyr Arg
           355                 360                 365
   Leu Ala Tyr Gln Gly Gln Asp Thr Pro Glu Val Leu Met Asp Ile Gly
       370                 375                 380
   Leu Arg Gln Glu Val Thr Leu Glu Leu Gln Gly Asp Gly Ser Val Ser
   385                 390                 395                 400
   Asn Leu Thr Val Cys Val Ala Ala Tyr Thr Ala Ala Gly Asp Gly Pro
                   405                 410                 415
   Trp Ser Leu Pro Val Pro Leu Glu Ala Trp Arg Pro Gly Gln Ala Gln
               420                 425                 430
   Pro Val His Gln Leu Val Lys Glu Pro Ser Thr Pro Ala Phe Ser Trp
           435                 440                 445
   Pro Trp Trp Tyr Val Leu Leu Gly Ala Val Val Ala Ala Ala Cys Val
       450                 455                 460
   Leu Ile Leu Ala Leu Phe Leu Val His Arg Arg Lys Lys Glu Thr Arg
   465                 470                 475                 480
   Tyr Gly Glu Val Phe Glu Pro Thr Val Glu Arg Gly Glu Leu Val Val
                   485                 490                 495
   Arg Tyr Arg Val Arg Lys Ser Tyr Ser Arg Arg Thr Thr Glu Ala Thr
               500                 505                 510
   Leu Asn Ser Leu Gly Ile Ser Glu Glu Leu Lys Glu Lys Leu Arg Asp
           515                 520                 525
   Val Met Val Asp Arg His Lys Val Ala Leu Gly Lys Thr Leu Gly Glu
       530                 535                 540
   Gly Glu Phe Gly Ala Val Met Glu Gly Gln Leu Asn Gln Asp Asp Ser
   545                 550                 555                 560
   Ile Leu Lys Val Ala Val Lys Thr Met Lys Ile Ala Ile Cys Thr Arg
                   565                 570                 575
   Ser Glu Leu Glu Asp Phe Leu Ser Glu Ala Val Cys Met Lys Glu Phe
               580                 585                 590
   Asp His Pro Asn Val Met Arg Leu Ile Gly Val Cys Phe Gln Gly Ser
           595                 600                 605
   Glu Arg Glu Ser Phe Pro Ala Pro Val Val Ile Leu Pro Phe Met Lys
       610                 615                 620
   His Gly Asp Leu His Ser Phe Leu Leu Tyr Ser Arg Leu Gly Asp Gln
   625                 630                 635                 640
   Pro Val Tyr Leu Pro Thr Gln Met Leu Val Lys Phe Met Ala Asp Ile
                   645                 650                 655
   Ala Ser Gly Met Glu Tyr Leu Ser Thr Lys Arg Phe Ile His Arg Asp
               660                 665                 670
   Leu Ala Ala Arg Asn Cys Met Leu Asn Glu Asn Met Ser Val Cys Val
           675                 680                 685
   Ala Asp Phe Gly Leu Ser Lys Lys Ile Tyr Asn Gly Asp Tyr Tyr Arg
       690                 695                 700
   Gln Gly Arg Ile Ala Lys Met Pro Val Lys Trp Ile Ala Ile Glu Ser
   705                 710                 715                 720
   Leu Ala Asp Arg Val Tyr Thr Ser Lys Ser Asp Val Trp Ser Phe Gly
                   725                 730                 735
   Val Thr Met Trp Glu Ile Ala Thr Arg Gly Gln Thr Pro Tyr Pro Gly
               740                 745                 750
   Val Glu Asn Ser Glu Ile Tyr Asp Tyr Leu Arg Gln Gly Asn Arg Leu
           755                 760                 765
   Lys Gln Pro Ala Asp Cys Leu Asp Gly Leu Tyr Ala Leu Met Ser Arg
       770                 775                 780
   Cys Trp Glu Leu Asn Pro Gln Asp Arg Pro Ser Phe Thr Glu Leu Arg
   785                 790                 795                 800
   Glu Asp Leu Glu Asn Thr Leu Lys Ala Leu Pro Pro Ala Gln Glu Pro
                   805                 810                 815
   Asp Glu Ile Leu Tyr Val Asn Met Asp Glu Gly Gly Gly Tyr Pro Glu
               820                 825                 830
   Pro Pro Gly Ala Ala Gly Gly Ala Asp Pro Pro Thr Gln Pro Asp Pro
           835                 840                 845
   Lys Asp Ser Cys Ser Cys Leu Thr Ala Ala Glu Val His Pro Ala Gly
   850 855 860
   Arg Tyr Val Leu Cys Pro Ser Thr Thr Pro Ser Pro Ala Gln Pro Ala
   865 870 875 880
   Asp Arg Gly Ser Pro Ala Ala Pro Gly Gln Glu Asp Gly Ala
   885 890
<210> 131
   <211> 904
   <212> PRT
   <213> Mus Musculus
<400> 131
   Met Ala Trp Arg Cys Pro Arg Met Gly Arg Val Pro Leu Ala Trp Cys
   1 5 10 15
   Leu Ala Leu Cys Gly Trp Ala Cys Met Tyr Pro Tyr Asp Val Pro Asp
   20 25 30
   Tyr Ala Ala His Lys Asp Thr Gln Thr Glu Ala Gly Ser Pro Phe Val
   35 40 45
   Gly Asn Pro Gly Asn Ile Thr Gly Ala Arg Gly Leu Thr Gly Thr Leu
   50 55 60
   Arg Cys Glu Leu Gln Val Gln Gly Glu Pro Pro Glu Val Val Trp Leu
   65 70 75 80
   Arg Asp Gly Gln Ile Leu Glu Leu Ala Asp Asn Thr Gln Thr Gln Val
   85 90 95
   Pro Leu Gly Glu Asp Trp Gln Asp Glu Trp Lys Val Val Ser Gln Leu
   100 105 110
   Arg Ile Ser Ala Leu Gln Leu Ser Asp Ala Gly Glu Tyr Gln Cys Met
   115 120 125
   Val His Leu Glu Gly Arg Thr Phe Val Ser Gln Pro Gly Phe Val Gly
   130 135 140
   Leu Glu Gly Leu Pro Tyr Phe Leu Glu Glu Pro Glu Asp Lys Ala Val
   145 150 155 160
   Pro Ala Asn Thr Pro Phe Asn Leu Ser Cys Gln Ala Gln Gly Pro Pro
   165 170 175
   Glu Pro Val Thr Leu Leu Trp Leu Gln Asp Ala Val Pro Leu Ala Pro
               180                 185                 190
   Val Thr Gly His Ser Ser Gln His Ser Leu Gln Thr Pro Gly Leu Asn
           195                 200                 205
   Lys Thr Ser Ser Phe Ser Cys Glu Ala His Asn Ala Lys Gly Val Thr
       210                 215                 220
   Thr Ser Arg Thr Ala Thr Ile Thr Val Leu Pro Gln Arg Pro His His
   225                 230                 235                 240
   Leu His Val Val Ser Arg Gln Pro Thr Glu Leu Glu Val Ala Trp Thr
                   245                 250                 255
   Pro Gly Leu Ser Gly Ile Tyr Pro Leu Thr His Cys Asn Leu Gln Ala
               260                 265                 270
   Val Leu Ser Asp Asp Gly Val Gly Ile Trp Leu Gly Lys Ser Asp Pro
           275                 280                 285
   Pro Glu Asp Pro Leu Thr Leu Gln Val Ser Val Pro Pro His Gln Leu
       290                 295                 300
   Arg Leu Glu Lys Leu Leu Pro His Thr Pro Tyr His Ile Arg Ile Ser
   305                 310                 315                 320
   Cys Ser Ser Ser Gln Gly Pro Ser Pro Trp Thr His Trp Leu Pro Val
                   325                 330                 335
   Glu Thr Thr Glu Gly Val Pro Leu Gly Pro Pro Glu Asn Val Ser Ala
               340                 345                 350
   Met Arg Asn Gly Ser Gln Val Leu Val Arg Trp Gln Glu Pro Arg Val
           355                 360                 365
   Pro Leu Gln Gly Thr Leu Leu Gly Tyr Arg Leu Ala Tyr Arg Gly Gln
       370                 375                 380
   Asp Thr Pro Glu Val Leu Met Asp Ile Gly Leu Thr Arg Glu Val Thr
   385                 390                 395                 400
   Leu Glu Leu Arg Gly Asp Arg Pro Val Ala Asn Leu Thr Val Ser Val
                   405                 410                 415
   Thr Ala Tyr Thr Ser Ala Gly Asp Gly Pro Trp Ser Leu Pro Val Pro
   420 425 430
   Leu Glu Pro Trp Arg Pro Gly Gln Gly Gln Pro Leu His His Leu Val
   435 440 445
   Ser Glu Pro Pro Pro Arg Ala Phe Ser Trp Pro Trp Trp Tyr Val Leu
   450 455 460
   Leu Gly Ala Val Val Ala Ala Ala Cys Val Leu Ile Leu Ala Leu Phe
   465 470 475 480
   Leu Val His Arg Arg Lys Lys Glu Thr Arg Tyr Gly Glu Val Phe Glu
   485 490 495
   Pro Thr Val Glu Arg Gly Glu Leu Val Val Arg Tyr Arg Val Arg Lys
   500 505 510
   Ser Tyr Ser Arg Arg Thr Thr Glu Ala Thr Leu Asn Ser Leu Gly Ile
   515 520 525
   Ser Glu Glu Leu Lys Glu Lys Leu Arg Asp Val Met Val Asp Arg His
   530 535 540
   Lys Val Ala Leu Gly Lys Thr Leu Gly Glu Gly Glu Phe Gly Ala Val
   545 550 555 560
   Met Glu Gly Gln Leu Asn Gln Asp Asp Ser Ile Leu Lys Val Ala Val
   565 570 575
   Lys Thr Met Lys Ile Ala Ile Cys Thr Arg Ser Glu Leu Glu Asp Phe
   580 585 590
   Leu Ser Glu Ala Val Cys Met Lys Glu Phe Asp His Pro Asn Val Met
   595 600 605
   Arg Leu Ile Gly Val Cys Phe Gln Gly Ser Glu Arg Glu Ser Phe Pro
   610 615 620
   Ala Pro Val Val Ile Leu Pro Phe Met Lys His Gly Asp Leu His Ser
   625 630 635 640
   Phe Leu Leu Tyr Ser Arg Leu Gly Asp Gln Pro Val Tyr Leu Pro Thr
   645 650 655
   Gln Met Leu Val Lys Phe Met Ala Asp Ile Ala Ser Gly Met Glu Tyr
   660 665 670
   Leu Ser Thr Lys Arg Phe Ile His Arg Asp Leu Ala Ala Arg Asn Cys
           675                 680                 685
   Met Leu Asn Glu Asn Met Ser Val Cys Val Ala Asp Phe Gly Leu Ser
       690                 695                 700
   Lys Lys Ile Tyr Asn Gly Asp Tyr Tyr Arg Gln Gly Arg Ile Ala Lys
   705                 710                 715                 720
   Met Pro Val Lys Trp Ile Ala Ile Glu Ser Leu Ala Asp Arg Val Tyr
                   725                 730                 735
   Thr Ser Lys Ser Asp Val Trp Ser Phe Gly Val Thr Met Trp Glu Ile
               740                 745                 750
   Ala Thr Arg Gly Gln Thr Pro Tyr Pro Gly Val Glu Asn Ser Glu Ile
           755                 760                 765
   Tyr Asp Tyr Leu Arg Gln Gly Asn Arg Leu Lys Gln Pro Ala Asp Cys
       770                 775                 780
   Leu Asp Gly Leu Tyr Ala Leu Met Ser Arg Cys Trp Glu Leu Asn Pro
   785                 790                 795                 800
   Gln Asp Arg Pro Ser Phe Thr Glu Leu Arg Glu Asp Leu Glu Asn Thr
                   805                 810                 815
   Leu Lys Ala Leu Pro Pro Ala Gln Glu Pro Asp Glu Ile Leu Tyr Val
               820                 825                 830
   Asn Met Asp Glu Gly Gly Gly Tyr Pro Glu Pro Pro Gly Ala Ala Gly
           835                 840                 845
   Gly Ala Asp Pro Pro Thr Gln Pro Asp Pro Lys Asp Ser Cys Ser Cys
       850                 855                 860
   Leu Thr Ala Ala Glu Val His Pro Ala Gly Arg Tyr Val Leu Cys Pro
   865                 870                 875                 880
   Ser Thr Thr Pro Ser Pro Ala Gln Pro Ala Asp Arg Gly Ser Pro Ala
                   885                 890                 895
   Ala Pro Gly Gln Glu Asp Gly Ala
               900
<210> 132
   <211> 894
   <212> PRT
   <213> homo sapiens
<400> 132
   Met Ala Trp Arg Cys Pro Arg Met Gly Arg Val Pro Leu Ala Trp Cys
   1               5                   10                  15
   Leu Ala Leu Cys Gly Trp Ala Cys Met Ala Pro Arg Gly Thr Gln Ala
               20                  25                  30
   Glu Glu Ser Pro Phe Val Gly Asn Pro Gly Asn Ile Thr Gly Ala Arg
           35                  40                  45
   Gly Leu Thr Gly Thr Leu Arg Cys Gln Leu Gln Val Gln Gly Glu Pro
       50                  55                  60
   Pro Glu Val His Trp Leu Arg Asp Gly Gln Ile Leu Glu Leu Ala Asp
   65                  70                  75                  80
   Ser Thr Gln Thr Gln Val Pro Leu Gly Glu Asp Glu Gln Asp Asp Trp
                   85                  90                  95
   Ile Val Val Ser Gln Leu Arg Ile Thr Ser Leu Gln Leu Ser Asp Thr
               100                 105                 110
   Gly Gln Tyr Gln Cys Leu Val Phe Leu Gly His Gln Thr Phe Val Ser
           115                 120                 125
   Gln Pro Gly Tyr Val Gly Leu Glu Gly Leu Pro Tyr Phe Leu Glu Glu
       130                 135                 140
   Pro Glu Asp Lys Ala Val Pro Ala Asn Thr Pro Phe Asn Leu Ser Cys
   145                 150                 155                 160
   Gln Ala Gln Gly Pro Pro Glu Pro Val Thr Leu Leu Trp Leu Gln Asp
                   165                 170                 175
   Ala Val Pro Leu Ala Pro Val Thr Gly His Ser Ser Gln His Ser Leu
               180                 185                 190
   Gln Thr Pro Gly Leu Asn Lys Thr Ser Ser Phe Ser Cys Glu Ala His
           195                 200                 205
   Asn Ala Lys Gly Val Thr Thr Ser Arg Thr Ala Thr Ile Thr Val Leu
       210                 215                 220
   Pro Gln Gln Pro Arg Asn Leu His Leu Val Ser Arg Gln Pro Thr Glu
   225                 230                 235                 240
   Leu Glu Val Ala Trp Thr Pro Gly Leu Ser Gly Ile Tyr Pro Leu Thr
                   245                 250                 255
   His Cys Thr Leu Gln Ala Val Leu Ser Asp Asp Gly Met Gly Ile Gln
               260                 265                 270
   Ala Gly Glu Pro Asp Pro Pro Glu Glu Pro Leu Thr Ser Gln Ala Ser
           275                 280                 285
   Val Pro Pro His Gln Leu Arg Leu Gly Ser Leu His Pro His Thr Pro
       290                 295                 300
   Tyr His Ile Arg Val Ala Cys Thr Ser Ser Gln Gly Pro Ser Ser Trp
   305                 310                 315                 320
   Thr His Trp Leu Pro Val Glu Thr Pro Glu Gly Val Pro Leu Gly Pro
                   325                 330                 335
   Pro Glu Asn Ile Ser Ala Thr Arg Asn Gly Ser Gln Ala Phe Val His
               340                 345                 350
   Trp Gln Glu Pro Arg Ala Pro Leu Gln Gly Thr Leu Leu Gly Tyr Arg
           355                 360                 365
   Leu Ala Tyr Gln Gly Gln Asp Thr Pro Glu Val Leu Met Asp Ile Gly
       370                 375                 380
   Leu Arg Gln Glu Val Thr Leu Glu Leu Gln Gly Asp Gly Ser Val Ser
   385                 390                 395                 400
   Asn Leu Thr Val Cys Val Ala Ala Tyr Thr Ala Ala Gly Asp Gly Pro
                   405                 410                 415
   Trp Ser Leu Pro Val Pro Leu Glu Ala Trp Arg Pro Gly Gln Ala Gln
               420                 425                 430
   Pro Val His Gln Leu Val Lys Glu Pro Ser Thr Pro Ala Phe Ser Trp
           435                 440                 445
   Pro Trp Trp Tyr Val Leu Leu Gly Ala Val Val Ala Ala Ala Cys Val
       450                 455                 460
   Leu Ile Leu Ala Leu Phe Leu Val His Arg Arg Lys Lys Glu Thr Arg
   465                 470                 475                 480
   Tyr Gly Glu Val Phe Glu Pro Thr Val Glu Arg Gly Glu Leu Val Val
                   485                 490                 495
   Arg Tyr Arg Val Arg Lys Ser Tyr Ser Arg Arg Thr Thr Glu Ala Thr
   500 505 510
   Leu Asn Ser Leu Gly Ile Ser Glu Glu Leu Lys Glu Lys Leu Arg Asp
   515 520 525
   Val Met Val Asp Arg His Lys Val Ala Leu Gly Lys Thr Leu Gly Glu
   530 535 540
   Gly Glu Phe Gly Ala Val Met Glu Gly Gln Leu Asn Gln Asp Asp Ser
   545 550 555 560
   Ile Leu Lys Val Ala Val Lys Thr Met Lys Ile Ala Ile Cys Thr Arg
   565 570 575
   Ser Glu Leu Glu Asp Phe Leu Ser Glu Ala Val Cys Met Lys Glu Phe
   580 585 590
   Asp His Pro Asn Val Met Arg Leu Ile Gly Val Cys Phe Gln Gly Ser
   595 600 605
   Glu Arg Glu Ser Phe Pro Ala Pro Val Val Ile Leu Pro Phe Met Lys
   610 615 620
   His Gly Asp Leu His Ser Phe Leu Leu Tyr Ser Arg Leu Gly Asp Gln
   625 630 635 640
   Pro Val Tyr Leu Pro Thr Gln Met Leu Val Lys Phe Met Ala Asp Ile
   645 650 655
   Ala Ser Gly Met Glu Tyr Leu Ser Thr Lys Arg Phe Ile His Arg Asp
   660 665 670
   Leu Ala Ala Arg Asn Cys Met Leu Asn Glu Asn Met Ser Val Cys Val
   675 680 685
   Ala Asp Phe Gly Leu Ser Lys Lys Ile Tyr Asn Gly Asp Tyr Tyr Arg
   690 695 700
   Gln Gly Arg Ile Ala Lys Met Pro Val Lys Trp Ile Ala Ile Glu Ser
   705 710 715 720
   Leu Ala Asp Arg Val Tyr Thr Ser Lys Ser Asp Val Trp Ser Phe Gly
   725 730 735
   Val Thr Met Trp Glu Ile Ala Thr Arg Gly Gln Thr Pro Tyr Pro Gly
                   740                 745                 750
   Val Glu Asn Ser Glu Ile Tyr Asp Tyr Leu Arg Gln Gly Asn Arg Leu
               755                 760                 765
   Lys Gln Pro Ala Asp Cys Leu Asp Gly Leu Tyr Ala Leu Met Ser Arg
       770                 775                 780
   Cys Trp Glu Leu Asn Pro Gln Asp Arg Pro Ser Phe Thr Glu Leu Arg
   785                 790                 795                 800
   Glu Asp Leu Glu Asn Thr Leu Lys Ala Leu Pro Pro Ala Gln Glu Pro
                   805                 810                 815
   Asp Glu Ile Leu Tyr Val Asn Met Asp Glu Gly Gly Gly Tyr Pro Glu
               820                 825                 830
   Pro Pro Gly Ala Ala Gly Gly Ala Asp Pro Pro Thr Gln Pro Asp Pro
           835                 840                 845
   Lys Asp Ser Cys Ser Cys Leu Thr Ala Ala Glu Val His Pro Ala Gly
       850                 855                 860
   Arg Tyr Val Leu Cys Pro Ser Thr Thr Pro Ser Pro Ala Gln Pro Ala
   865                 870                 875                 880
   Asp Arg Gly Ser Pro Ala Ala Pro Gly Gln Glu Asp Gly Ala
                   885                 890
<210> 133
   <211> 894
   <212> PRT
   <213> homo sapiens
<400> 133
   Met Ala Trp Arg Cys Pro Arg Met Gly Arg Val Pro Leu Ala Trp Cys
   1               5                   10                  15
   Leu Ala Leu Cys Gly Trp Ala Cys Met Ala Pro Arg Gly Thr Gln Ala
               20                  25                  30
   Glu Glu Ser Pro Phe Val Gly Asn Pro Gly Asn Ile Thr Gly Ala Arg
           35                  40                  45
   Gly Leu Thr Gly Thr Leu Arg Cys Gln Leu Gln Val Gln Gly Glu Pro
       50                  55                  60
   Pro Glu Val His Trp Leu Arg Asp Gly Gln Ile Leu Glu Leu Ala Asp
   65                  70                  75                  80
   Ser Thr Gln Thr Gln Val Pro Leu Gly Glu Asp Glu Gln Asp Asp Trp
                   85                  90                  95
   Ile Val Val Ser Gln Leu Arg Ile Thr Ser Leu Gln Leu Ser Asp Thr
               100                 105                 110
   Gly Gln Tyr Gln Cys Leu Val Phe Leu Gly His Gln Thr Phe Val Ser
           115                 120                 125
   Gln Pro Gly Tyr Val Gly Leu Glu Gly Leu Pro Tyr Phe Leu Glu Glu
       130                 135                 140
   Pro Glu Asp Lys Ala Val Pro Ala Asn Thr Pro Phe Asn Leu Ser Cys
   145                 150                 155                 160
   Gln Ala Gln Gly Pro Pro Glu Pro Val Thr Leu Leu Trp Leu Gln Asp
                   165                 170                 175
   Ala Val Pro Leu Ala Pro Val Thr Gly His Ser Ser Gln His Ser Leu
               180                 185                 190
   Gln Thr Pro Gly Leu Asn Lys Thr Ser Ser Phe Ser Cys Glu Ala His
           195                 200                 205
   Asn Ala Lys Gly Val Thr Thr Ser Arg Thr Ala Thr Ile Thr Val Leu
       210                 215                 220
   Pro Gln Gln Pro Arg Asn Leu His Leu Val Ser Arg Gln Pro Thr Glu
   225                 230                 235                 240
   Leu Glu Val Ala Trp Thr Pro Gly Leu Ser Gly Ile Tyr Pro Leu Thr
                   245                 250                 255
   His Cys Thr Leu Gln Ala Val Leu Ser Asp Asp Gly Met Gly Ile Gln
               260                 265                 270
   Ala Gly Glu Pro Asp Pro Pro Glu Glu Pro Leu Thr Ser Gln Ala Ser
           275                 280                 285
   Val Pro Pro His Gln Leu Arg Leu Gly Ser Leu His Pro His Thr Pro
       290                 295                 300
   Tyr His Ile Arg Val Ala Cys Thr Ser Ser Gln Gly Pro Ser Ser Trp
   305                 310                 315                 320
   Thr His Trp Leu Pro Val Glu Thr Pro Glu Gly Val Pro Leu Gly Pro
                   325                 330                 335
   Pro Glu Asn Ile Ser Ala Thr Arg Asn Gly Ser Gln Ala Phe Val His
               340                 345                 350
   Trp Gln Glu Pro Arg Ala Pro Leu Gln Gly Thr Leu Leu Gly Tyr Arg
           355                 360                 365
   Leu Ala Tyr Gln Gly Gln Asp Thr Pro Glu Val Leu Met Asp Ile Gly
       370                 375                 380
   Leu Arg Gln Glu Val Thr Leu Glu Leu Gln Gly Asp Gly Ser Val Ser
   385                 390                 395                 400
   Asn Leu Thr Val Cys Val Ala Ala Tyr Thr Ala Ala Gly Asp Gly Pro
                   405                 410                 415
   Trp Ser Leu Pro Val Pro Leu Glu Ala Trp Arg Pro Gly Gln Ala Gln
               420                 425                 430
   Pro Val His Gln Leu Val Lys Glu Pro Ser Thr Pro Ala Phe Ser Trp
           435                 440                 445
   Pro Trp Trp Tyr Val Leu Leu Gly Ala Val Val Ala Ala Ala Cys Val
       450                 455                 460
   Leu Ile Leu Ala Leu Phe Leu Val His Arg Arg Lys Lys Glu Thr Arg
   465                 470                 475                 480
   Tyr Gly Glu Val Phe Glu Pro Thr Val Glu Arg Gly Glu Leu Val Val
                   485                 490                 495
   Arg Tyr Arg Val Arg Lys Ser Tyr Ser Arg Arg Thr Thr Glu Ala Thr
               500                 505                 510
   Leu Asn Ser Leu Gly Ile Ser Glu Glu Leu Lys Glu Lys Leu Arg Asp
           515                 520                 525
   Val Met Val Asp Arg His Lys Val Ala Leu Gly Lys Thr Leu Gly Glu
       530                 535                 540
   Gly Glu Phe Gly Ala Val Met Glu Gly Gln Leu Asn Gln Asp Asp Ser
   545                 550                 555                 560
   Ile Leu Lys Val Ala Val Lys Thr Met Lys Ile Ala Ile Cys Thr Arg
                   565                 570                 575
   Ser Glu Leu Glu Asp Phe Leu Ser Glu Ala Val Cys Met Lys Glu Phe
               580                 585                 590
   Asp His Pro Asn Val Met Arg Leu Ile Gly Val Cys Phe Gln Gly Ser
           595                 600                 605
   Glu Arg Glu Ser Phe Pro Ala Pro Val Val Ile Leu Pro Phe Met Lys
       610                 615                 620
   His Gly Asp Leu His Ser Phe Leu Leu Tyr Ser Arg Leu Gly Asp Gln
   625                 630                 635                 640
   Pro Val Tyr Leu Pro Thr Gln Met Leu Val Lys Phe Met Ala Asp Ile
                   645                 650                 655
   Ala Ser Gly Met Glu Tyr Leu Ser Thr Lys Arg Phe Ile His Arg Asp
               660                 665                 670
   Leu Ala Ala Arg Asn Cys Met Leu Asn Glu Asn Met Ser Val Cys Val
           675                 680                 685
   Ala Asp Phe Gly Leu Ser Lys Lys Ile Tyr Asn Gly Asp Tyr Tyr Arg
       690                 695                 700
   Gln Gly Arg Ile Ala Lys Met Pro Val Lys Trp Ile Ala Ile Glu Ser
   705                 710                 715                 720
   Leu Ala Asp Arg Val Tyr Thr Ser Lys Ser Asp Val Trp Ser Phe Gly
                   725                 730                 735
   Val Thr Met Trp Glu Ile Ala Thr Arg Gly Gln Thr Pro Tyr Pro Gly
               740                 745                 750
   Val Glu Asn Ser Glu Ile Tyr Asp Tyr Leu Arg Gln Gly Asn Arg Leu
           755                 760                 765
   Lys Gln Pro Ala Asp Cys Leu Asp Gly Leu Tyr Ala Leu Met Ser Arg
       770                 775                 780
   Cys Trp Glu Leu Asn Pro Gln Asp Arg Pro Ser Phe Thr Glu Leu Arg
   785                 790                 795                 800
   Glu Asp Leu Glu Asn Thr Leu Lys Ala Leu Pro Pro Ala Gln Glu Pro
                   805                 810                 815
   Asp Glu Ile Leu Tyr Val Asn Met Asp Glu Gly Gly Gly Tyr Pro Glu
               820                 825                 830
   Pro Pro Gly Ala Ala Gly Gly Ala Asp Pro Pro Thr Gln Pro Asp Pro
   835 840 845
   Lys Asp Ser Cys Ser Cys Leu Thr Ala Ala Glu Val His Pro Ala Gly
   850 855 860
   Arg Tyr Val Leu Cys Pro Ser Thr Thr Pro Ser Pro Ala Gln Pro Ala
   865 870 875 880
   Asp Arg Gly Ser Pro Ala Ala Pro Gly Gln Glu Asp Gly Ala
   885 890
<210> 134
   <211> 894
   <212> PRT
   <213> homo sapiens
<400> 134
   Met Ala Trp Arg Cys Pro Arg Met Gly Arg Val Pro Leu Ala Trp Cys
   1 5 10 15
   Leu Ala Leu Cys Gly Trp Ala Cys Met Ala Pro Arg Gly Thr Gln Ala
   20 25 30
   Glu Glu Ser Pro Phe Val Gly Asn Pro Gly Asn Ile Thr Gly Ala Arg
   35 40 45
   Gly Leu Thr Gly Thr Leu Arg Cys Gln Leu Gln Val Gln Gly Glu Pro
   50 55 60
   Pro Glu Val His Trp Leu Arg Asp Gly Gln Ile Leu Glu Leu Ala Asp
   65 70 75 80
   Ser Thr Gln Thr Gln Val Pro Leu Gly Glu Asp Glu Gln Asp Asp Trp
   85 90 95
   Ile Val Val Ser Gln Leu Arg Ile Thr Ser Leu Gln Leu Ser Asp Thr
   100 105 110
   Gly Gln Tyr Gln Cys Leu Val Phe Leu Gly His Gln Thr Phe Val Ser
   115 120 125
   Gln Pro Gly Tyr Val Gly Leu Glu Gly Leu Pro Tyr Phe Leu Glu Glu
   130 135 140
   Pro Glu Asp Arg Thr Val Ala Ala Asn Thr Pro Phe Asn Leu Ser Cys
   145 150 155 160
   Gln Ala Gln Gly Pro Pro Glu Pro Val Asp Leu Leu Trp Leu Gln Asp
                   165                 170                 175
   Ala Val Pro Leu Ala Thr Ala Pro Gly His Gly Pro Gln Arg Ser Leu
               180                 185                 190
   His Val Pro Gly Leu Asn Lys Thr Ser Ser Phe Ser Cys Glu Ala His
           195                 200                 205
   Asn Ala Lys Gly Val Thr Thr Ser Arg Thr Ala Thr Ile Thr Val Leu
       210                 215                 220
   Pro Gln Arg Pro His His Leu His Val Val Ser Arg Gln Pro Thr Glu
   225                 230                 235                 240
   Leu Glu Val Ala Trp Thr Pro Gly Leu Ser Gly Ile Tyr Pro Leu Thr
                   245                 250                 255
   His Cys Asn Leu Gln Ala Val Leu Ser Asp Asp Gly Val Gly Ile Trp
               260                 265                 270
   Leu Gly Lys Ser Asp Pro Pro Glu Asp Pro Leu Thr Leu Gln Val Ser
           275                 280                 285
   Val Pro Pro His Gln Leu Arg Leu Glu Lys Leu Leu Pro His Thr Pro
       290                 295                 300
   Tyr His Ile Arg Ile Ser Cys Ser Ser Ser Gln Gly Pro Ser Pro Trp
   305                 310                 315                 320
   Thr His Trp Leu Pro Val Glu Thr Thr Glu Gly Val Pro Leu Gly Pro
                   325                 330                 335
   Pro Glu Asn Ile Ser Ala Thr Arg Asn Gly Ser Gln Ala Phe Val His
               340                 345                 350
   Trp Gln Glu Pro Arg Ala Pro Leu Gln Gly Thr Leu Leu Gly Tyr Arg
           355                 360                 365
   Leu Ala Tyr Gln Gly Gln Asp Thr Pro Glu Val Leu Met Asp Ile Gly
       370                 375                 380
   Leu Arg Gln Glu Val Thr Leu Glu Leu Gln Gly Asp Gly Ser Val Ser
   385                 390                 395                 400
   Asn Leu Thr Val Cys Val Ala Ala Tyr Thr Ala Ala Gly Asp Gly Pro
                   405                 410                 415
   Trp Ser Leu Pro Val Pro Leu Glu Ala Trp Arg Pro Gly Gln Ala Gln
               420                 425                 430
   Pro Val His Gln Leu Val Lys Glu Pro Ser Thr Pro Ala Phe Ser Trp
           435                 440                 445
   Pro Trp Trp Tyr Val Leu Leu Gly Ala Val Val Ala Ala Ala Cys Val
       450                 455                 460
   Leu Ile Leu Ala Leu Phe Leu Val His Arg Arg Lys Lys Glu Thr Arg
   465                 470                 475                 480
   Tyr Gly Glu Val Phe Glu Pro Thr Val Glu Arg Gly Glu Leu Val Val
                   485                 490                 495
   Arg Tyr Arg Val Arg Lys Ser Tyr Ser Arg Arg Thr Thr Glu Ala Thr
               500                 505                 510
   Leu Asn Ser Leu Gly Ile Ser Glu Glu Leu Lys Glu Lys Leu Arg Asp
           515                 520                 525
   Val Met Val Asp Arg His Lys Val Ala Leu Gly Lys Thr Leu Gly Glu
       530                 535                 540
   Gly Glu Phe Gly Ala Val Met Glu Gly Gln Leu Asn Gln Asp Asp Ser
   545                 550                 555                 560
   Ile Leu Lys Val Ala Val Lys Thr Met Lys Ile Ala Ile Cys Thr Arg
                   565                 570                 575
   Ser Glu Leu Glu Asp Phe Leu Ser Glu Ala Val Cys Met Lys Glu Phe
               580                 585                 590
   Asp His Pro Asn Val Met Arg Leu Ile Gly Val Cys Phe Gln Gly Ser
           595                 600                 605
   Glu Arg Glu Ser Phe Pro Ala Pro Val Val Ile Leu Pro Phe Met Lys
       610                 615                 620
   His Gly Asp Leu His Ser Phe Leu Leu Tyr Ser Arg Leu Gly Asp Gln
   625                 630                 635                 640
   Pro Val Tyr Leu Pro Thr Gln Met Leu Val Lys Phe Met Ala Asp Ile
                   645                 650                 655
   Ala Ser Gly Met Glu Tyr Leu Ser Thr Lys Arg Phe Ile His Arg Asp
   660 665 670
   Leu Ala Ala Arg Asn Cys Met Leu Asn Glu Asn Met Ser Val Cys Val
   675 680 685
   Ala Asp Phe Gly Leu Ser Lys Lys Ile Tyr Asn Gly Asp Tyr Tyr Arg
   690 695 700
   Gln Gly Arg Ile Ala Lys Met Pro Val Lys Trp Ile Ala Ile Glu Ser
   705 710 715 720
   Leu Ala Asp Arg Val Tyr Thr Ser Lys Ser Asp Val Trp Ser Phe Gly
   725 730 735
   Val Thr Met Trp Glu Ile Ala Thr Arg Gly Gln Thr Pro Tyr Pro Gly
   740 745 750
   Val Glu Asn Ser Glu Ile Tyr Asp Tyr Leu Arg Gln Gly Asn Arg Leu
   755 760 765
   Lys Gln Pro Ala Asp Cys Leu Asp Gly Leu Tyr Ala Leu Met Ser Arg
   770 775 780
   Cys Trp Glu Leu Asn Pro Gln Asp Arg Pro Ser Phe Thr Glu Leu Arg
   785 790 795 800
   Glu Asp Leu Glu Asn Thr Leu Lys Ala Leu Pro Pro Ala Gln Glu Pro
   805 810 815
   Asp Glu Ile Leu Tyr Val Asn Met Asp Glu Gly Gly Gly Tyr Pro Glu
   820 825 830
   Pro Pro Gly Ala Ala Gly Gly Ala Asp Pro Pro Thr Gln Pro Asp Pro
   835 840 845
   Lys Asp Ser Cys Ser Cys Leu Thr Ala Ala Glu Val His Pro Ala Gly
   850 855 860
   Arg Tyr Val Leu Cys Pro Ser Thr Thr Pro Ser Pro Ala Gln Pro Ala
   865 870 875 880
   Asp Arg Gly Ser Pro Ala Ala Pro Gly Gln Glu Asp Gly Ala
   885 890
<210> 135
   <211> 894
   <212> PRT
   <213> homo sapiens
<400> 135
   Met Ala Trp Arg Cys Pro Arg Met Gly Arg Val Pro Leu Ala Trp Cys
   1               5                   10                  15
   Leu Ala Leu Cys Gly Trp Ala Cys Met Ala Pro Arg Gly Thr Gln Ala
               20                  25                  30
   Glu Glu Ser Pro Phe Val Gly Asn Pro Gly Asn Ile Thr Gly Ala Arg
           35                  40                  45
   Gly Leu Thr Gly Thr Leu Arg Cys Gln Leu Gln Val Gln Gly Glu Pro
       50                  55                  60
   Pro Glu Val His Trp Leu Arg Asp Gly Gln Ile Leu Glu Leu Ala Asp
   65                  70                  75                  80
   Ser Thr Gln Thr Gln Val Pro Leu Gly Glu Asp Glu Gln Asp Asp Trp
                   85                  90                  95
   Ile Val Val Ser Gln Leu Arg Ile Thr Ser Leu Gln Leu Ser Asp Thr
               100                 105                 110
   Gly Gln Tyr Gln Cys Leu Val Phe Leu Gly His Gln Thr Phe Val Ser
           115                 120                 125
   Gln Pro Gly Tyr Val Gly Leu Glu Gly Leu Pro Tyr Phe Leu Glu Glu
       130                 135                 140
   Pro Glu Asp Arg Thr Val Ala Ala Asn Thr Pro Phe Asn Leu Ser Cys
   145                 150                 155                 160
   Gln Ala Gln Gly Pro Pro Glu Pro Val Asp Leu Leu Trp Leu Gln Asp
                   165                 170                 175
   Ala Val Pro Leu Ala Thr Ala Pro Gly His Gly Pro Gln Arg Ser Leu
               180                 185                 190
   His Val Pro Gly Leu Asn Lys Thr Ser Ser Phe Ser Cys Glu Ala His
           195                 200                 205
   Asn Ala Lys Gly Val Thr Thr Ser Arg Thr Ala Thr Ile Thr Val Leu
       210                 215                 220
   Pro Gln Gln Pro Arg Asn Leu His Leu Val Ser Arg Gln Pro Thr Glu
   225                 230                 235                 240
   Leu Glu Val Ala Trp Thr Pro Gly Leu Ser Gly Ile Tyr Pro Leu Thr
                   245                 250                 255
   His Cys Thr Leu Gln Ala Val Leu Ser Asp Asp Gly Met Gly Ile Gln
               260                 265                 270
   Ala Gly Glu Pro Asp Pro Pro Glu Glu Pro Leu Thr Ser Gln Ala Ser
           275                 280                 285
   Val Pro Pro His Gln Leu Arg Leu Gly Ser Leu His Pro His Thr Pro
       290                 295                 300
   Tyr His Ile Arg Val Ala Cys Thr Ser Ser Gln Gly Pro Ser Ser Trp
   305                 310                 315                 320
   Thr His Trp Leu Pro Val Glu Thr Pro Glu Gly Val Pro Leu Gly Pro
                   325                 330                 335
   Pro Glu Asn Val Ser Ala Met Arg Asn Gly Ser Gln Val Leu Val Arg
               340                 345                 350
   Trp Gln Glu Pro Arg Val Pro Leu Gln Gly Thr Leu Leu Gly Tyr Arg
           355                 360                 365
   Leu Ala Tyr Arg Gly Gln Asp Thr Pro Glu Val Leu Met Asp Ile Gly
       370                 375                 380
   Leu Thr Arg Glu Val Thr Leu Glu Leu Arg Gly Asp Arg Pro Val Ala
   385                 390                 395                 400
   Asn Leu Thr Val Ser Val Thr Ala Tyr Thr Ser Ala Gly Asp Gly Pro
                   405                 410                 415
   Trp Ser Leu Pro Val Pro Leu Glu Pro Trp Arg Pro Gly Gln Gly Gln
               420                 425                 430
   Pro Leu His His Leu Val Ser Glu Pro Pro Pro Arg Ala Phe Ser Trp
           435                 440                 445
   Pro Trp Trp Tyr Val Leu Leu Gly Ala Val Val Ala Ala Ala Cys Val
       450                 455                 460
   Leu Ile Leu Ala Leu Phe Leu Val His Arg Arg Lys Lys Glu Thr Arg
   465                 470                 475                 480
   Tyr Gly Glu Val Phe Glu Pro Thr Val Glu Arg Gly Glu Leu Val Val
                   485                 490                 495
   Arg Tyr Arg Val Arg Lys Ser Tyr Ser Arg Arg Thr Thr Glu Ala Thr
               500                 505                 510
   Leu Asn Ser Leu Gly Ile Ser Glu Glu Leu Lys Glu Lys Leu Arg Asp
           515                 520                 525
   Val Met Val Asp Arg His Lys Val Ala Leu Gly Lys Thr Leu Gly Glu
       530                 535                 540
   Gly Glu Phe Gly Ala Val Met Glu Gly Gln Leu Asn Gln Asp Asp Ser
   545                 550                 555                 560
   Ile Leu Lys Val Ala Val Lys Thr Met Lys Ile Ala Ile Cys Thr Arg
                   565                 570                 575
   Ser Glu Leu Glu Asp Phe Leu Ser Glu Ala Val Cys Met Lys Glu Phe
               580                 585                 590
   Asp His Pro Asn Val Met Arg Leu Ile Gly Val Cys Phe Gln Gly Ser
           595                 600                 605
   Glu Arg Glu Ser Phe Pro Ala Pro Val Val Ile Leu Pro Phe Met Lys
       610                 615                 620
   His Gly Asp Leu His Ser Phe Leu Leu Tyr Ser Arg Leu Gly Asp Gln
   625                 630                 635                 640
   Pro Val Tyr Leu Pro Thr Gln Met Leu Val Lys Phe Met Ala Asp Ile
                   645                 650                 655
   Ala Ser Gly Met Glu Tyr Leu Ser Thr Lys Arg Phe Ile His Arg Asp
               660                 665                 670
   Leu Ala Ala Arg Asn Cys Met Leu Asn Glu Asn Met Ser Val Cys Val
           675                 680                 685
   Ala Asp Phe Gly Leu Ser Lys Lys Ile Tyr Asn Gly Asp Tyr Tyr Arg
       690                 695                 700
   Gln Gly Arg Ile Ala Lys Met Pro Val Lys Trp Ile Ala Ile Glu Ser
   705                 710                 715                 720
   Leu Ala Asp Arg Val Tyr Thr Ser Lys Ser Asp Val Trp Ser Phe Gly
                   725                 730                 735
   Val Thr Met Trp Glu Ile Ala Thr Arg Gly Gln Thr Pro Tyr Pro Gly
   740 745 750
   Val Glu Asn Ser Glu Ile Tyr Asp Tyr Leu Arg Gln Gly Asn Arg Leu
   755 760 765
   Lys Gln Pro Ala Asp Cys Leu Asp Gly Leu Tyr Ala Leu Met Ser Arg
   770 775 780
   Cys Trp Glu Leu Asn Pro Gln Asp Arg Pro Ser Phe Thr Glu Leu Arg
   785 790 795 800
   Glu Asp Leu Glu Asn Thr Leu Lys Ala Leu Pro Pro Ala Gln Glu Pro
   805 810 815
   Asp Glu Ile Leu Tyr Val Asn Met Asp Glu Gly Gly Gly Tyr Pro Glu
   820 825 830
   Pro Pro Gly Ala Ala Gly Gly Ala Asp Pro Pro Thr Gln Pro Asp Pro
   835 840 845
   Lys Asp Ser Cys Ser Cys Leu Thr Ala Ala Glu Val His Pro Ala Gly
   850 855 860
   Arg Tyr Val Leu Cys Pro Ser Thr Thr Pro Ser Pro Ala Gln Pro Ala
   865 870 875 880
   Asp Arg Gly Ser Pro Ala Ala Pro Gly Gln Glu Asp Gly Ala
   885 890
<210> 136
   <211> 124
   <212> PRT
   <213> homo sapiens
<400> 136
   Glu Val Gln Leu Leu Glu Ser Gly Gly Gly Leu Val Gln Pro Gly Gly
   1 5 10 15
   Ser Leu Arg Leu Ser Cys Ala Ala Ser Gly Phe Thr Phe Ser Ser Tyr
   20 25 30
   Ala Met Asn Trp Val Arg Gln Ala Pro Gly Lys Gly Leu Glu Trp Val
   35 40 45
   Ser Gly Ile Ser Gly Ser Gly Gly His Thr Tyr His Ala Asp Ser Val
   50 55 60
   Lys Gly Arg Phe Thr Ile Ser Arg Asp Asn Ser Lys Asn Thr Leu Tyr
   65 70 75 80
   Leu Gln Met Asn Ser Leu Arg Ala Glu Asp Thr Ala Val Tyr Tyr Cys
   85 90 95
   Ala Lys Asp Arg Tyr Asp Ile Leu Thr Gly Tyr Tyr Asn Leu Leu Asp
   100 105 110
   Tyr Trp Gly Gln Gly Thr Leu Val Thr Val Ser Ser
   115 120
<210> 137
   <211> 8
   <212> PRT
   <213> homo sapiens
<400> 137
   Gly Phe Thr Phe Ser Ser Tyr Ala
   1 5
<210> 138
   <211> 8
   <212> PRT
   <213> homo sapiens
<400> 138
   Ile Ser Gly Ser Gly Gly His Thr
   1 5
<210> 139
   <211> 17
   <212> PRT
   <213> homo sapiens
<400> 139
   Ala Lys Asp Arg Tyr Asp Ile Leu Thr Gly Tyr Tyr Asn Leu Leu Asp
   1 5 10 15
   Tyr
<210> 140
   <211> 107
   <212> PRT
   <213> homo sapiens
<400> 140
   Asp Ile Gln Met Thr Gln Ser Pro Ser Ser Leu Ser Ala Ser Val Gly
   1 5 10 15
   Asp Arg Val Thr Ile Thr Cys Arg Ala Ser Gln Gly Ile Ser Ser Trp
   20 25 30
   Leu Ala Trp Tyr Gln Gln Lys Pro Glu Glu Ala Pro Lys Ser Leu Ile
   35 40 45
   Tyr Ala Ala Ser Ser Leu Gln Ser Gly Val Pro Ser Arg Phe Ser Gly
   50 55 60
   Ser Gly Ser Gly Thr Asp Phe Thr Leu Thr Ile Ser Ser Leu Gln Pro
   65 70 75 80
   Glu Asp Phe Ala Thr Tyr Tyr Cys Gln Gln Tyr Asn Ser Tyr Pro Leu
   85 90 95
   Thr Phe Gly Gly Gly Ala Lys Val Glu Ile Lys
   100 105
<210> 141
   <211> 6
   <212> PRT
   <213> homo sapiens
<400> 141
   Gln Gly Ile Ser Ser Trp
   1 5
<210> 142
   <211> 9
   <212> PRT
   <213> homo sapiens
<400> 142
   Gln Gln Tyr Asn Ser Tyr Pro Leu Thr
   1 5
<210> 143
   <211> 123
   <212> PRT
   <213> homo sapiens
<400> 143
   Gln Val Gln Leu Val Gln Ser Gly Ala Glu Val Lys Lys Pro Gly Ala
   1               5                   10                  15
   Ser Val Lys Val Ser Cys Lys Ala Ser Gly Tyr Ala Phe Thr Gly Tyr
               20                  25                  30
   Gly Ile Ser Trp Val Arg Gln Ala Pro Gly Gln Gly Leu Glu Trp Ile
           35                  40                  45
   Gly Trp Ile Ser Ala Tyr Asn Gly Asn Thr Asn Tyr Val Gln Asn Leu
       50                  55                  60
   Gln Asp Arg Val Thr Met Thr Thr Asp Thr Ser Thr Ser Thr Ala Tyr
   65                  70                  75                  80
   Met Glu Leu Arg Ser Leu Arg Ser Asp Asp Thr Ala Val Tyr Tyr Cys
                   85                  90                  95
   Ala Arg Asp His Ile Ser Met Leu Arg Gly Ile Ile Ile Arg Asn Tyr
               100                 105                 110
   Trp Gly Gln Gly Thr Leu Val Thr Val Ser Ser
           115                 120
<210> 144
   <211> 108
   <212> PRT
   <213> homo sapiens
<400> 144
   Glu Ile Val Leu Thr Gln Ser Pro Ala Thr Leu Ser Leu Ser Pro Gly
   1               5                   10                  15
   Glu Arg Ala Thr Leu Ser Cys Arg Ala Ser Gln Ser Val Ser Ser Tyr
               20                  25                  30
   Leu Ala Trp Tyr Gln Gln Lys Pro Gly Gln Ala Pro Arg Leu Leu Ile
           35                  40                  45
   Tyr Asp Ala Ser Asn Arg Ala Thr Gly Ile Pro Ala Arg Phe Ser Gly
       50                  55                  60
   Ser Gly Ser Gly Thr Asp Phe Thr Leu Thr Ile Ser Ser Leu Glu Pro
   65                  70                  75                  80
   Glu Asp Phe Ala Val Tyr Tyr Cys Gln Gln Arg Ser Ser Trp Pro Arg
                   85                  90                  95
   Leu Thr Phe Gly Gly Gly Thr Lys Val Glu Ile Lys
               100                 105
<210> 145
   <211> 124
   <212> PRT
   <213> homo sapiens
<400> 145
   Gln Val Gln Leu Val Gln Ser Gly Ala Glu Val Lys Lys Pro Gly Ser
   1               5                   10                  15
   Ser Val Lys Val Ser Cys Lys Ala Ser Gly Gly Thr Phe Ser Arg Tyr
               20                  25                  30
   Ala Ile Ser Trp Val Arg Gln Ala Pro Gly Gln Gly Leu Glu Trp Met
           35                  40                  45
   Gly Arg Ile Ile Pro Ile Val Gly Ile Ala Asn Tyr Ala Gln Lys Phe
       50                  55                  60
   Gln Gly Arg Val Thr Leu Thr Ala Asp Lys Ser Thr Ser Thr Ala Tyr
   65                  70                  75                  80
   Met Glu Leu Ser Ser Leu Arg Ser Glu Asp Thr Ala Val Tyr Tyr Cys
                   85                  90                  95
   Ala Arg Glu Ala Gly Tyr Ser Ser Ser Trp Tyr Ala Glu Tyr Phe Gln
               100                 105                 110
   His Trp Gly Gln Gly Thr Leu Val Thr Val Ser Ser
           115                 120
<210> 146
   <211> 108
   <212> PRT
   <213> homo sapiens
<400> 146
   Glu Ile Val Leu Thr Gln Ser Pro Gly Thr Leu Ser Leu Ser Pro Gly
   1               5                   10                  15
   Glu Arg Ala Thr Leu Ser Cys Arg Ala Ser Gln Ser Val Ser Ser Asn
               20                  25                  30
   Tyr Leu Ala Trp Tyr Gln Gln Lys Pro Gly Gln Ala Pro Arg Leu Leu
           35                  40                  45
   Ile Tyr Gly Ala Ser Ser Arg Ala Thr Gly Phe Pro Asp Arg Phe Ser
       50                  55                  60
   Gly Ser Gly Ser Gly Thr Asp Phe Thr Leu Thr Ile Ser Arg Leu Glu
   65                  70                  75                  80
   Pro Glu Asp Phe Ala Val Tyr Tyr Cys Gln Gln Tyr Gly Ser Ser Pro
                   85                  90                  95
   Tyr Thr Phe Gly Gln Gly Thr Lys Leu Glu Ile Lys
   100 105
<210> 147
   <211> 893
   <212> PRT
   <213> Macaca fascicularis
<400> 147
   Ala Trp Arg Cys Pro Arg Met Gly Arg Val Pro Leu Ala Trp Cys Leu
   1 5 10 15
   Ala Leu Cys Gly Trp Val Cys Met Ala Pro Arg Gly Thr Gln Ala Glu
   20 25 30
   Glu Ser Pro Phe Val Gly Asn Pro Gly Asn Ile Thr Gly Ala Arg Gly
   35 40 45
   Leu Thr Gly Thr Leu Arg Cys Gln Leu Gln Val Gln Gly Glu Pro Pro
   50 55 60
   Glu Val His Trp Leu Arg Asp Gly Gln Ile Leu Glu Leu Ala Asp Ser
   65 70 75 80
   Thr Gln Thr Gln Val Pro Leu Gly Glu Asp Glu Gln Asp Asp Trp Ile
   85 90 95
   Val Val Ser Gln Leu Arg Ile Ala Ser Leu Gln Leu Ser Asp Ala Gly
   100 105 110
   Gln Tyr Gln Cys Leu Val Phe Leu Gly His Gln Asn Phe Val Ser Gln
   115 120 125
   Pro Gly Tyr Val Gly Leu Glu Gly Leu Pro Tyr Phe Leu Glu Glu Pro
   130 135 140
   Glu Asp Arg Thr Val Ala Ala Asn Thr Pro Phe Asn Leu Ser Cys Gln
   145 150 155 160
   Ala Gln Gly Pro Pro Glu Pro Val Asp Leu Leu Trp Leu Gln Asp Ala
   165 170 175
   Val Pro Leu Ala Thr Ala Pro Gly His Gly Pro Gln Arg Asn Leu His
   180 185 190
   Val Pro Gly Leu Asn Lys Thr Ser Ser Phe Ser Cys Glu Ala His Asn
   195 200 205
   Ala Lys Gly Val Thr Thr Ser Arg Thr Ala Thr Ile Thr Val Leu Pro
       210                 215                 220
   Gln Gln Pro Arg Asn Leu His Leu Val Ser Arg Gln Pro Thr Glu Leu
   225                 230                 235                 240
   Glu Val Ala Trp Thr Pro Gly Leu Ser Gly Ile Tyr Pro Leu Thr His
                   245                 250                 255
   Cys Thr Leu Gln Ala Val Leu Ser Asp Asp Gly Met Gly Ile Gln Ala
               260                 265                 270
   Gly Glu Pro Asp Pro Pro Glu Glu Pro Leu Thr Leu Gln Ala Ser Val
           275                 280                 285
   Pro Pro His Gln Leu Arg Leu Gly Ser Leu His Pro His Thr Pro Tyr
       290                 295                 300
   His Ile Arg Val Ala Cys Thr Ser Ser Gln Gly Pro Ser Ser Trp Thr
   305                 310                 315                 320
   His Trp Leu Pro Val Glu Thr Pro Glu Gly Val Pro Leu Gly Pro Pro
                   325                 330                 335
   Glu Asn Ile Ser Ala Thr Arg Asn Gly Ser Gln Ala Phe Val His Trp
               340                 345                 350
   Gln Glu Pro Arg Ala Pro Leu Gln Gly Thr Leu Leu Gly Tyr Arg Leu
           355                 360                 365
   Ala Tyr Gln Gly Gln Asp Thr Pro Glu Val Leu Met Asp Ile Gly Leu
       370                 375                 380
   Arg Gln Glu Val Thr Leu Glu Leu Gln Gly Asp Gly Ser Val Ser Asn
   385                 390                 395                 400
   Leu Thr Val Cys Val Ala Ala Tyr Thr Ala Ala Gly Asp Gly Pro Trp
                   405                 410                 415
   Ser Leu Pro Val Pro Leu Glu Ala Trp Arg Pro Gly Gln Ala Gln Pro
               420                 425                 430
   Val His Gln Leu Val Lys Glu Thr Ser Ala Pro Ala Phe Ser Trp Pro
           435                 440                 445
   Trp Trp Tyr Ile Leu Leu Gly Ala Val Val Ala Ala Ala Cys Val Leu
       450                 455                 460
   Ile Leu Ala Leu Phe Leu Val His Arg Arg Lys Lys Glu Thr Arg Tyr
   465                 470                 475                 480
   Gly Glu Val Phe Glu Pro Thr Val Glu Arg Gly Glu Leu Val Val Arg
                   485                 490                 495
   Tyr Arg Val Arg Lys Ser Tyr Ser Arg Arg Thr Thr Glu Ala Thr Leu
               500                 505                 510
   Asn Ser Leu Gly Ile Ser Glu Glu Leu Lys Glu Lys Leu Arg Asp Val
           515                 520                 525
   Met Val Asp Arg His Lys Val Ala Leu Gly Lys Thr Leu Gly Glu Gly
       530                 535                 540
   Glu Phe Gly Ala Val Met Glu Gly Gln Leu Asn Gln Asp Asp Ser Ile
   545                 550                 555                 560
   Leu Lys Val Ala Val Lys Thr Met Lys Ile Ala Ile Cys Thr Arg Ser
                   565                 570                 575
   Glu Leu Glu Asp Phe Leu Ser Glu Ala Val Cys Met Lys Glu Phe Asp
               580                 585                 590
   His Pro Asn Val Met Arg Leu Ile Gly Val Cys Phe Gln Gly Ser Glu
           595                 600                 605
   Arg Glu Ser Phe Pro Ala Pro Val Val Ile Leu Pro Phe Met Lys His
       610                 615                 620
   Gly Asp Leu His Ser Phe Leu Leu Tyr Ser Arg Leu Gly Asp Gln Pro
   625                 630                 635                 640
   Val Tyr Leu Pro Thr Gln Met Leu Val Lys Phe Met Ala Asp Ile Ala
                   645                 650                 655
   Ser Gly Met Glu Tyr Leu Ser Thr Lys Arg Phe Ile His Arg Asp Leu
               660                 665                 670
   Ala Ala Arg Asn Cys Met Leu Asn Glu Asn Met Ser Val Cys Val Ala
           675                 680                 685
   Asp Phe Gly Leu Ser Lys Lys Ile Tyr Asn Gly Asp Tyr Tyr Arg Gln
       690                 695                 700
   Gly Arg Ile Ala Lys Met Pro Val Lys Trp Ile Ala Ile Glu Ser Leu
   705                 710                 715                 720
   Ala Asp Arg Val Tyr Thr Ser Lys Ser Asp Val Trp Ser Phe Gly Val
                   725                 730                 735
   Thr Met Trp Glu Ile Ala Thr Arg Gly Gln Thr Pro Tyr Pro Gly Val
               740                 745                 750
   Glu Asn Ser Glu Ile Tyr Asp Tyr Leu Arg Gln Gly Asn Arg Leu Lys
           755                 760                 765
   Gln Pro Ala Asp Cys Leu Asp Gly Leu Tyr Ala Leu Met Ser Arg Cys
       770                 775                 780
   Trp Glu Leu Asn Pro Gln Asp Arg Pro Ser Phe Thr Glu Leu Arg Glu
   785                 790                 795                 800
   Asp Leu Glu Asn Thr Leu Lys Ala Leu Pro Pro Ala Gln Glu Pro Asp
                   805                 810                 815
   Glu Ile Leu Tyr Val Asn Met Asp Glu Gly Gly Gly Tyr Pro Glu Pro
               820                 825                 830
   Pro Gly Ala Ala Gly Gly Ala Asp Pro Pro Thr Gln Leu Asp Pro Lys
           835                 840                 845
   Asp Ser Cys Ser Cys Leu Thr Ser Ala Glu Val His Pro Ala Gly Arg
       850                 855                 860
   Tyr Val Leu Cys Pro Ser Thr Ala Pro Ser Pro Ala Gln Pro Ala Asp
   865                 870                 875                 880
   Arg Gly Ser Pro Ala Ala Pro Gly Gln Glu Asp Gly Ala
                   885                 890
<210> 148
   <211> 903
   <212> PRT
   <213> Homo sapiens
<400> 148
   Met Ala Trp Arg Cys Pro Arg Met Gly Arg Val Pro Leu Ala Trp Cys
   1               5                   10                  15
   Leu Ala Leu Cys Gly Trp Ala Cys Met Tyr Pro Tyr Asp Val Pro Asp
               20                  25                  30
   Tyr Ala Ala Pro Arg Gly Thr Gln Ala Glu Glu Ser Pro Phe Val Gly
           35                  40                  45
   Asn Pro Gly Asn Ile Thr Gly Ala Arg Gly Leu Thr Gly Thr Leu Arg
       50                  55                  60
   Cys Gln Leu Gln Val Gln Gly Glu Pro Pro Glu Val His Trp Leu Arg
   65                  70                  75                  80
   Asp Gly Gln Ile Leu Glu Leu Ala Asp Ser Thr Gln Thr Gln Val Pro
                   85                  90                  95
   Leu Gly Glu Asp Glu Gln Asp Asp Trp Ile Val Val Ser Gln Leu Arg
               100                 105                 110
   Ile Thr Ser Leu Gln Leu Ser Asp Thr Gly Gln Tyr Gln Cys Leu Val
           115                 120                 125
   Phe Leu Gly His Gln Thr Phe Val Ser Gln Pro Gly Tyr Val Gly Leu
       130                 135                 140
   Glu Gly Leu Pro Tyr Phe Leu Glu Glu Pro Glu Asp Arg Thr Val Ala
   145                 150                 155                 160
   Ala Asn Thr Pro Phe Asn Leu Ser Cys Gln Ala Gln Gly Pro Pro Glu
                   165                 170                 175
   Pro Val Asp Leu Leu Trp Leu Gln Asp Ala Val Pro Leu Ala Thr Ala
               180                 185                 190
   Pro Gly His Gly Pro Gln Arg Ser Leu His Val Pro Gly Leu Asn Lys
           195                 200                 205
   Thr Ser Ser Phe Ser Cys Glu Ala His Asn Ala Lys Gly Val Thr Thr
       210                 215                 220
   Ser Arg Thr Ala Thr Ile Thr Val Leu Pro Gln Gln Pro Arg Asn Leu
   225                 230                 235                 240
   His Leu Val Ser Arg Gln Pro Thr Glu Leu Glu Val Ala Trp Thr Pro
                   245                 250                 255
   Gly Leu Ser Gly Ile Tyr Pro Leu Thr His Cys Thr Leu Gln Ala Val
               260                 265                 270
   Leu Ser Asn Asp Gly Met Gly Ile Gln Ala Gly Glu Pro Asp Pro Pro
           275                 280                 285
   Glu Glu Pro Leu Thr Ser Gln Ala Ser Val Pro Pro His Gln Leu Arg
       290                 295                 300
   Leu Gly Ser Leu His Pro His Thr Pro Tyr His Ile Arg Val Ala Cys
   305                 310                 315                 320
   Thr Ser Ser Gln Gly Pro Ser Ser Trp Thr His Trp Leu Pro Val Glu
                   325                 330                 335
   Thr Pro Glu Gly Val Pro Leu Gly Pro Pro Glu Asn Ile Ser Ala Thr
               340                 345                 350
   Arg Asn Gly Ser Gln Ala Phe Val His Trp Gln Glu Pro Arg Ala Pro
           355                 360                 365
   Leu Gln Gly Thr Leu Leu Gly Tyr Arg Leu Ala Tyr Gln Gly Gln Asp
       370                 375                 380
   Thr Pro Glu Val Leu Met Asp Ile Gly Leu Arg Gln Glu Val Thr Leu
   385                 390                 395                 400
   Glu Leu Gln Gly Asp Gly Ser Val Ser Asn Leu Thr Val Cys Val Ala
                   405                 410                 415
   Ala Tyr Thr Ala Ala Gly Asp Gly Pro Trp Ser Leu Pro Val Pro Leu
               420                 425                 430
   Glu Ala Trp Arg Pro Gly Gln Ala Gln Pro Val His Gln Leu Val Lys
           435                 440                 445
   Glu Pro Ser Thr Pro Ala Phe Ser Trp Pro Trp Trp Tyr Val Leu Leu
       450                 455                 460
   Gly Ala Val Val Ala Ala Ala Cys Val Leu Ile Leu Ala Leu Phe Leu
   465                 470                 475                 480
   Val His Arg Arg Lys Lys Glu Thr Arg Tyr Gly Glu Val Phe Glu Pro
                   485                 490                 495
   Thr Val Glu Arg Gly Glu Leu Val Val Arg Tyr Arg Val Arg Lys Ser
               500                 505                 510
   Tyr Ser Arg Arg Thr Thr Glu Ala Thr Leu Asn Ser Leu Gly Ile Ser
           515                 520                 525
   Glu Glu Leu Lys Glu Lys Leu Arg Asp Val Met Val Asp Arg His Lys
       530                 535                 540
   Val Ala Leu Gly Lys Thr Leu Gly Glu Gly Glu Phe Gly Ala Val Met
   545 550 555 560
   Glu Gly Gln Leu Asn Gln Asp Asp Ser Ile Leu Lys Val Ala Val Lys
   565 570 575
   Thr Met Lys Ile Ala Ile Cys Thr Arg Ser Glu Leu Glu Asp Phe Leu
   580 585 590
   Ser Glu Ala Val Cys Met Lys Glu Phe Asp His Pro Asn Val Met Arg
   595 600 605
   Leu Ile Gly Val Cys Phe Gln Gly Ser Glu Arg Glu Ser Phe Pro Ala
   610 615 620
   Pro Val Val Ile Leu Pro Phe Met Lys His Gly Asp Leu His Ser Phe
   625 630 635 640
   Leu Leu Tyr Ser Arg Leu Gly Asp Gln Pro Val Tyr Leu Pro Thr Gln
   645 650 655
   Met Leu Val Lys Phe Met Ala Asp Ile Ala Ser Gly Met Glu Tyr Leu
   660 665 670
   Ser Thr Lys Arg Phe Ile His Arg Asp Leu Ala Ala Arg Asn Cys Met
   675 680 685
   Leu Asn Glu Asn Met Ser Val Cys Val Ala Asp Phe Gly Leu Ser Lys
   690 695 700
   Lys Ile Tyr Asn Gly Asp Tyr Tyr Arg Gln Gly Arg Ile Ala Lys Met
   705 710 715 720
   Pro Val Lys Trp Ile Ala Ile Glu Ser Leu Ala Asp Arg Val Tyr Thr
   725 730 735
   Ser Lys Ser Asp Val Trp Ser Phe Gly Val Thr Met Trp Glu Ile Ala
   740 745 750
   Thr Arg Gly Gln Thr Pro Tyr Pro Gly Val Glu Asn Ser Glu Ile Tyr
   755 760 765
   Asp Tyr Leu Arg Gln Gly Asn Arg Leu Lys Gln Pro Ala Asp Cys Leu
   770 775 780
   Asp Gly Leu Tyr Ala Leu Met Ser Arg Cys Trp Glu Leu Asn Pro Gln
   785 790 795 800
   Asp Arg Pro Ser Phe Thr Glu Leu Arg Glu Asp Leu Glu Asn Thr Leu
   805 810 815
   Lys Ala Leu Pro Pro Ala Gln Glu Pro Asp Glu Ile Leu Tyr Val Asn
   820 825 830
   Met Asp Glu Gly Gly Gly Tyr Pro Glu Pro Pro Gly Ala Ala Gly Gly
   835 840 845
   Ala Asp Pro Pro Thr Gln Pro Asp Pro Lys Asp Ser Cys Ser Cys Leu
   850 855 860
   Thr Ala Ala Glu Val His Pro Ala Gly Arg Tyr Val Leu Cys Pro Ser
   865 870 875 880
   Thr Thr Pro Ser Pro Ala Gln Pro Ala Asp Arg Gly Ser Pro Ala Ala
   885 890 895
   Pro Gly Gln Glu Asp Gly Ala
   900
<210> 149
   <211> 904
   <212> PRT
   <213> Mus musculus
<400> 149
   Met Ala Trp Arg Cys Pro Arg Met Gly Arg Val Pro Leu Ala Trp Cys
   1 5 10 15
   Leu Ala Leu Cys Gly Trp Ala Cys Met Tyr Pro Tyr Asp Val Pro Asp
   20 25 30
   Tyr Ala Ala His Lys Asp Thr Gln Thr Glu Ala Gly Ser Pro Phe Val
   35 40 45
   Gly Asn Pro Gly Asn Ile Thr Gly Ala Arg Gly Leu Thr Gly Thr Leu
   50 55 60
   Arg Cys Glu Leu Gln Val Gln Gly Glu Pro Pro Glu Val Val Trp Leu
   65 70 75 80
   Arg Asp Gly Gln Ile Leu Glu Leu Ala Asp Asn Thr Gln Thr Gln Val
   85 90 95
   Pro Leu Gly Glu Asp Trp Gln Asp Glu Trp Lys Val Val Ser Gln Leu
   100 105 110
   Arg Ile Ser Ala Leu Gln Leu Ser Asp Ala Gly Glu Tyr Gln Cys Met
           115                 120                 125
   Val His Leu Glu Gly Arg Thr Phe Val Ser Gln Pro Gly Phe Val Gly
       130                 135                 140
   Leu Glu Gly Leu Pro Tyr Phe Leu Glu Glu Pro Glu Asp Lys Ala Val
   145                 150                 155                 160
   Pro Ala Asn Thr Pro Phe Asn Leu Ser Cys Gln Ala Gln Gly Pro Pro
                   165                 170                 175
   Glu Pro Val Thr Leu Leu Trp Leu Gln Asp Ala Val Pro Leu Ala Pro
               180                 185                 190
   Val Thr Gly His Ser Ser Gln His Ser Leu Gln Thr Pro Gly Leu Asn
           195                 200                 205
   Lys Thr Ser Ser Phe Ser Cys Glu Ala His Asn Ala Lys Gly Val Thr
       210                 215                 220
   Thr Ser Arg Thr Ala Thr Ile Thr Val Leu Pro Gln Arg Pro His His
   225                 230                 235                 240
   Leu His Val Val Ser Arg Gln Pro Thr Glu Leu Glu Val Ala Trp Thr
                   245                 250                 255
   Pro Gly Leu Ser Gly Ile Tyr Pro Leu Thr His Cys Asn Leu Gln Ala
               260                 265                 270
   Val Leu Ser Asp Asp Gly Val Gly Ile Trp Leu Gly Lys Ser Asp Pro
           275                 280                 285
   Pro Glu Asp Pro Leu Thr Leu Gln Val Ser Val Pro Pro His Gln Leu
       290                 295                 300
   Arg Leu Glu Lys Leu Leu Pro His Thr Pro Tyr His Ile Arg Ile Ser
   305                 310                 315                 320
   Cys Ser Ser Ser Gln Gly Pro Ser Pro Trp Thr His Trp Leu Pro Val
                   325                 330                 335
   Glu Thr Thr Glu Gly Val Pro Leu Gly Pro Pro Glu Asn Val Ser Ala
               340                 345                 350
   Met Arg Asn Gly Ser Gln Val Leu Val Arg Trp Gln Glu Pro Arg Val
   355 360 365
   Pro Leu Gln Gly Thr Leu Leu Gly Tyr Arg Leu Ala Tyr Arg Gly Gln
   370 375 380
   Asp Thr Pro Glu Val Leu Met Asp Ile Gly Leu Thr Arg Glu Val Thr
   385 390 395 400
   Leu Glu Leu Arg Gly Asp Arg Pro Val Ala Asn Leu Thr Val Ser Val
   405 410 415
   Thr Ala Tyr Thr Ser Ala Gly Asp Gly Pro Trp Ser Leu Pro Val Pro
   420 425 430
   Leu Glu Pro Trp Arg Pro Gly Gln Gly Gln Pro Leu His His Leu Val
   435 440 445
   Ser Glu Pro Pro Pro Arg Ala Phe Ser Trp Pro Trp Trp Tyr Val Leu
   450 455 460
   Leu Gly Ala Val Val Ala Ala Ala Cys Val Leu Ile Leu Ala Leu Phe
   465 470 475 480
   Leu Val His Arg Arg Lys Lys Glu Thr Arg Tyr Gly Glu Val Phe Glu
   485 490 495
   Pro Thr Val Glu Arg Gly Glu Leu Val Val Arg Tyr Arg Val Arg Lys
   500 505 510
   Ser Tyr Ser Arg Arg Thr Thr Glu Ala Thr Leu Asn Ser Leu Gly Ile
   515 520 525
   Ser Glu Glu Leu Lys Glu Lys Leu Arg Asp Val Met Val Asp Arg His
   530 535 540
   Lys Val Ala Leu Gly Lys Thr Leu Gly Glu Gly Glu Phe Gly Ala Val
   545 550 555 560
   Met Glu Gly Gln Leu Asn Gln Asp Asp Ser Ile Leu Lys Val Ala Val
   565 570 575
   Lys Thr Met Lys Ile Ala Ile Cys Thr Arg Ser Glu Leu Glu Asp Phe
   580 585 590
   Leu Ser Glu Ala Val Cys Met Lys Glu Phe Asp His Pro Asn Val Met
   595 600 605
   Arg Leu Ile Gly Val Cys Phe Gln Gly Ser Glu Arg Glu Ser Phe Pro
       610                 615                 620
   Ala Pro Val Val Ile Leu Pro Phe Met Lys His Gly Asp Leu His Ser
   625                 630                 635                 640
   Phe Leu Leu Tyr Ser Arg Leu Gly Asp Gln Pro Val Tyr Leu Pro Thr
                   645                 650                 655
   Gln Met Leu Val Lys Phe Met Ala Asp Ile Ala Ser Gly Met Glu Tyr
               660                 665                 670
   Leu Ser Thr Lys Arg Phe Ile His Arg Asp Leu Ala Ala Arg Asn Cys
           675                 680                 685
   Met Leu Asn Glu Asn Met Ser Val Cys Val Ala Asp Phe Gly Leu Ser
       690                 695                 700
   Lys Lys Ile Tyr Asn Gly Asp Tyr Tyr Arg Gln Gly Arg Ile Ala Lys
   705                 710                 715                 720
   Met Pro Val Lys Trp Ile Ala Ile Glu Ser Leu Ala Asp Arg Val Tyr
                   725                 730                 735
   Thr Ser Lys Ser Asp Val Trp Ser Phe Gly Val Thr Met Trp Glu Ile
               740                 745                 750
   Ala Thr Arg Gly Gln Thr Pro Tyr Pro Gly Val Glu Asn Ser Glu Ile
           755                 760                 765
   Tyr Asp Tyr Leu Arg Gln Gly Asn Arg Leu Lys Gln Pro Ala Asp Cys
       770                 775                 780
   Leu Asp Gly Leu Tyr Ala Leu Met Ser Arg Cys Trp Glu Leu Asn Pro
   785                 790                 795                 800
   Gln Asp Arg Pro Ser Phe Thr Glu Leu Arg Glu Asp Leu Glu Asn Thr
                   805                 810                 815
   Leu Lys Ala Leu Pro Pro Ala Gln Glu Pro Asp Glu Ile Leu Tyr Val
               820                 825                 830
   Asn Met Asp Glu Gly Gly Gly Tyr Pro Glu Pro Pro Gly Ala Ala Gly
           835                 840                 845
   Gly Ala Asp Pro Pro Thr Gln Pro Asp Pro Lys Asp Ser Cys Ser Cys
       850                 855                 860
   Leu Thr Ala Ala Glu Val His Pro Ala Gly Arg Tyr Val Leu Cys Pro
   865 870 875 880
   Ser Thr Thr Pro Ser Pro Ala Gln Pro Ala Asp Arg Gly Ser Pro Ala
   885 890 895
   Ala Pro Gly Gln Glu Asp Gly Ala
   900
<210> 150
   <211> 888
   <212> PRT
   <213> Mus musculus
<400> 150
   Met Gly Arg Val Pro Leu Ala Trp Trp Leu Ala Leu Cys Cys Trp Gly
   1 5 10 15
   Cys Ala Ala His Lys Asp Thr Gln Thr Glu Ala Gly Ser Pro Phe Val
   20 25 30
   Gly Asn Pro Gly Asn Ile Thr Gly Ala Arg Gly Leu Thr Gly Thr Leu
   35 40 45
   Arg Cys Glu Leu Gln Val Gln Gly Glu Pro Pro Glu Val Val Trp Leu
   50 55 60
   Arg Asp Gly Gln Ile Leu Glu Leu Ala Asp Asn Thr Gln Thr Gln Val
   65 70 75 80
   Pro Leu Gly Glu Asp Trp Gln Asp Glu Trp Lys Val Val Ser Gln Leu
   85 90 95
   Arg Ile Ser Ala Leu Gln Leu Ser Asp Ala Gly Glu Tyr Gln Cys Met
   100 105 110
   Val His Leu Glu Gly Arg Thr Phe Val Ser Gln Pro Gly Phe Val Gly
   115 120 125
   Leu Glu Gly Leu Pro Tyr Phe Leu Glu Glu Pro Glu Asp Arg Thr Val
   130 135 140
   Ala Ala Asn Thr Pro Phe Asn Leu Ser Cys Gln Ala Gln Gly Pro Pro
   145 150 155 160
   Glu Pro Val Asp Leu Leu Trp Leu Gln Asp Ala Val Pro Leu Ala Thr
   165 170 175
   Ala Pro Gly His Gly Pro Gln Arg Ser Leu His Val Pro Gly Leu Asn
               180                 185                 190
   Lys Thr Ser Ser Phe Ser Cys Glu Ala His Asn Ala Lys Gly Val Thr
           195                 200                 205
   Thr Ser Arg Thr Ala Thr Ile Thr Val Leu Pro Gln Gln Pro Arg Asn
       210                 215                 220
   Leu His Leu Val Ser Arg Gln Pro Thr Glu Leu Glu Val Ala Trp Thr
   225                 230                 235                 240
   Pro Gly Leu Ser Gly Ile Tyr Pro Leu Thr His Cys Thr Leu Gln Ala
                   245                 250                 255
   Val Leu Ser Asp Asp Gly Met Gly Ile Gln Ala Gly Glu Pro Asp Pro
               260                 265                 270
   Pro Glu Glu Pro Leu Thr Ser Gln Ala Ser Val Pro Pro His Gln Leu
           275                 280                 285
   Arg Leu Gly Ser Leu His Pro His Thr Pro Tyr His Ile Arg Val Ala
       290                 295                 300
   Cys Thr Ser Ser Gln Gly Pro Ser Ser Trp Thr His Trp Leu Pro Val
   305                 310                 315                 320
   Glu Thr Pro Glu Gly Val Pro Leu Gly Pro Pro Glu Asn Ile Ser Ala
                   325                 330                 335
   Thr Arg Asn Gly Ser Gln Ala Phe Val His Trp Gln Glu Pro Arg Ala
               340                 345                 350
   Pro Leu Gln Gly Thr Leu Leu Gly Tyr Arg Leu Ala Tyr Gln Gly Gln
           355                 360                 365
   Asp Thr Pro Glu Val Leu Met Asp Ile Gly Leu Arg Gln Glu Val Thr
       370                 375                 380
   Leu Glu Leu Gln Gly Asp Gly Ser Val Ser Asn Leu Thr Val Cys Val
   385                 390                 395                 400
   Ala Ala Tyr Thr Ala Ala Gly Asp Gly Pro Trp Ser Leu Pro Val Pro
                   405                 410                 415
   Leu Glu Ala Trp Arg Pro Gly Gln Ala Gln Pro Val His Gln Leu Val
               420                 425                 430
   Lys Glu Pro Ser Thr Pro Ala Phe Ser Trp Pro Trp Trp Tyr Val Leu
   435 440 445
   Leu Gly Ala Val Val Ala Ala Ala Cys Val Leu Ile Leu Ala Leu Phe
   450 455 460
   Leu Val His Arg Arg Lys Lys Glu Thr Arg Tyr Gly Glu Val Phe Glu
   465 470 475 480
   Pro Thr Val Glu Arg Gly Glu Leu Val Val Arg Tyr Arg Val Arg Lys
   485 490 495
   Ser Tyr Ser Arg Arg Thr Thr Glu Ala Thr Leu Asn Ser Leu Gly Ile
   500 505 510
   Ser Glu Glu Leu Lys Glu Lys Leu Arg Asp Val Met Val Asp Arg His
   515 520 525
   Lys Val Ala Leu Gly Lys Thr Leu Gly Glu Gly Glu Phe Gly Ala Val
   530 535 540
   Met Glu Gly Gln Leu Asn Gln Asp Asp Ser Ile Leu Lys Val Ala Val
   545 550 555 560
   Lys Thr Met Lys Ile Ala Ile Cys Thr Arg Ser Glu Leu Glu Asp Phe
   565 570 575
   Leu Ser Glu Ala Val Cys Met Lys Glu Phe Asp His Pro Asn Val Met
   580 585 590
   Arg Leu Ile Gly Val Cys Phe Gln Gly Ser Glu Arg Glu Ser Phe Pro
   595 600 605
   Ala Pro Val Val Ile Leu Pro Phe Met Lys His Gly Asp Leu His Ser
   610 615 620
   Phe Leu Leu Tyr Ser Arg Leu Gly Asp Gln Pro Val Tyr Leu Pro Thr
   625 630 635 640
   Gln Met Leu Val Lys Phe Met Ala Asp Ile Ala Ser Gly Met Glu Tyr
   645 650 655
   Leu Ser Thr Lys Arg Phe Ile His Arg Asp Leu Ala Ala Arg Asn Cys
   660 665 670
   Met Leu Asn Glu Asn Met Ser Val Cys Val Ala Asp Phe Gly Leu Ser
   675 680 685
   Lys Lys Ile Tyr Asn Gly Asp Tyr Tyr Arg Gln Gly Arg Ile Ala Lys
   690 695 700
   Met Pro Val Lys Trp Ile Ala Ile Glu Ser Leu Ala Asp Arg Val Tyr
   705 710 715 720
   Thr Ser Lys Ser Asp Val Trp Ser Phe Gly Val Thr Met Trp Glu Ile
   725 730 735
   Ala Thr Arg Gly Gln Thr Pro Tyr Pro Gly Val Glu Asn Ser Glu Ile
   740 745 750
   Tyr Asp Tyr Leu Arg Gln Gly Asn Arg Leu Lys Gln Pro Ala Asp Cys
   755 760 765
   Leu Asp Gly Leu Tyr Ala Leu Met Ser Arg Cys Trp Glu Leu Asn Pro
   770 775 780
   Gln Asp Arg Pro Ser Phe Thr Glu Leu Arg Glu Asp Leu Glu Asn Thr
   785 790 795 800
   Leu Lys Ala Leu Pro Pro Ala Gln Glu Pro Asp Glu Ile Leu Tyr Val
   805 810 815
   Asn Met Asp Glu Gly Gly Gly Tyr Pro Glu Pro Pro Gly Ala Ala Gly
   820 825 830
   Gly Ala Asp Pro Pro Thr Gln Pro Asp Pro Lys Asp Ser Cys Ser Cys
   835 840 845
   Leu Thr Ala Ala Glu Val His Pro Ala Gly Arg Tyr Val Leu Cys Pro
   850 855 860
   Ser Thr Thr Pro Ser Pro Ala Gln Pro Ala Asp Arg Gly Ser Pro Ala
   865 870 875 880
   Ala Pro Gly Gln Glu Asp Gly Ala
   885
<210> 151
   <211> 894
   <212> PRT
   <213> Artificial
<220>
   <223> Chimeric protein construct
<400> 151
   Met Ala Trp Arg Cys Pro Arg Met Gly Arg Val Pro Leu Ala Trp Cys
   1               5                   10                  15
   Leu Ala Leu Cys Gly Trp Ala Cys Met Ala Pro Arg Gly Thr Gln Ala
               20                  25                  30
   Glu Glu Ser Pro Phe Val Gly Asn Pro Gly Asn Ile Thr Gly Ala Arg
           35                  40                  45
   Gly Leu Thr Gly Thr Leu Arg Cys Gln Leu Gln Val Gln Gly Glu Pro
       50                  55                  60
   Pro Glu Val His Trp Leu Arg Asp Gly Gln Ile Leu Glu Leu Ala Asp
   65                  70                  75                  80
   Ser Thr Gln Thr Gln Val Pro Leu Gly Glu Asp Glu Gln Asp Asp Trp
                   85                  90                  95
   Ile Val Val Ser Gln Leu Arg Ile Thr Ser Leu Gln Leu Ser Asp Thr
               100                 105                 110
   Gly Gln Tyr Gln Cys Leu Val Phe Leu Gly His Gln Thr Phe Val Ser
           115                 120                 125
   Gln Pro Gly Tyr Val Gly Leu Glu Gly Leu Pro Tyr Phe Leu Glu Glu
       130                 135                 140
   Pro Glu Asp Lys Ala Val Pro Ala Asn Thr Pro Phe Asn Leu Ser Cys
   145                 150                 155                 160
   Gln Ala Gln Gly Pro Pro Glu Pro Val Thr Leu Leu Trp Leu Gln Asp
                   165                 170                 175
   Ala Val Pro Leu Ala Pro Val Thr Gly His Ser Ser Gln His Ser Leu
               180                 185                 190
   Gln Thr Pro Gly Leu Asn Lys Thr Ser Ser Phe Ser Cys Glu Ala His
           195                 200                 205
   Asn Ala Lys Gly Val Thr Thr Ser Arg Thr Ala Thr Ile Thr Val Leu
       210                 215                 220
   Pro Gln Gln Pro Arg Asn Leu His Leu Val Ser Arg Gln Pro Thr Glu
   225                 230                 235                 240
   Leu Glu Val Ala Trp Thr Pro Gly Leu Ser Gly Ile Tyr Pro Leu Thr
   245 250 255
   His Cys Thr Leu Gln Ala Val Leu Ser Asp Asp Gly Met Gly Ile Gln
   260 265 270
   Ala Gly Glu Pro Asp Pro Pro Glu Glu Pro Leu Thr Ser Gln Ala Ser
   275 280 285
   Val Pro Pro His Gln Leu Arg Leu Gly Ser Leu His Pro His Thr Pro
   290 295 300
   Tyr His Ile Arg Val Ala Cys Thr Ser Ser Gln Gly Pro Ser Ser Trp
   305 310 315 320
   Thr His Trp Leu Pro Val Glu Thr Pro Glu Gly Val Pro Leu Gly Pro
   325 330 335
   Pro Glu Asn Ile Ser Ala Thr Arg Asn Gly Ser Gln Ala Phe Val His
   340 345 350
   Trp Gln Glu Pro Arg Ala Pro Leu Gln Gly Thr Leu Leu Gly Tyr Arg
   355 360 365
   Leu Ala Tyr Gln Gly Gln Asp Thr Pro Glu Val Leu Met Asp Ile Gly
   370 375 380
   Leu Arg Gln Glu Val Thr Leu Glu Leu Gln Gly Asp Gly Ser Val Ser
   385 390 395 400
   Asn Leu Thr Val Cys Val Ala Ala Tyr Thr Ala Ala Gly Asp Gly Pro
   405 410 415
   Trp Ser Leu Pro Val Pro Leu Glu Ala Trp Arg Pro Gly Gln Ala Gln
   420 425 430
   Pro Val His Gln Leu Val Lys Glu Pro Ser Thr Pro Ala Phe Ser Trp
   435 440 445
   Pro Trp Trp Tyr Val Leu Leu Gly Ala Val Val Ala Ala Ala Cys Val
   450 455 460
   Leu Ile Leu Ala Leu Phe Leu Val His Arg Arg Lys Lys Glu Thr Arg
   465 470 475 480
   Tyr Gly Glu Val Phe Glu Pro Thr Val Glu Arg Gly Glu Leu Val Val
   485 490 495
   Arg Tyr Arg Val Arg Lys Ser Tyr Ser Arg Arg Thr Thr Glu Ala Thr
   500 505 510
   Leu Asn Ser Leu Gly Ile Ser Glu Glu Leu Lys Glu Lys Leu Arg Asp
   515 520 525
   Val Met Val Asp Arg His Lys Val Ala Leu Gly Lys Thr Leu Gly Glu
   530 535 540
   Gly Glu Phe Gly Ala Val Met Glu Gly Gln Leu Asn Gln Asp Asp Ser
   545 550 555 560
   Ile Leu Lys Val Ala Val Lys Thr Met Lys Ile Ala Ile Cys Thr Arg
   565 570 575
   Ser Glu Leu Glu Asp Phe Leu Ser Glu Ala Val Cys Met Lys Glu Phe
   580 585 590
   Asp His Pro Asn Val Met Arg Leu Ile Gly Val Cys Phe Gln Gly Ser
   595 600 605
   Glu Arg Glu Ser Phe Pro Ala Pro Val Val Ile Leu Pro Phe Met Lys
   610 615 620
   His Gly Asp Leu His Ser Phe Leu Leu Tyr Ser Arg Leu Gly Asp Gln
   625 630 635 640
   Pro Val Tyr Leu Pro Thr Gln Met Leu Val Lys Phe Met Ala Asp Ile
   645 650 655
   Ala Ser Gly Met Glu Tyr Leu Ser Thr Lys Arg Phe Ile His Arg Asp
   660 665 670
   Leu Ala Ala Arg Asn Cys Met Leu Asn Glu Asn Met Ser Val Cys Val
   675 680 685
   Ala Asp Phe Gly Leu Ser Lys Lys Ile Tyr Asn Gly Asp Tyr Tyr Arg
   690 695 700
   Gln Gly Arg Ile Ala Lys Met Pro Val Lys Trp Ile Ala Ile Glu Ser
   705 710 715 720
   Leu Ala Asp Arg Val Tyr Thr Ser Lys Ser Asp Val Trp Ser Phe Gly
   725 730 735
   Val Thr Met Trp Glu Ile Ala Thr Arg Gly Gln Thr Pro Tyr Pro Gly
   740 745 750
   Val Glu Asn Ser Glu Ile Tyr Asp Tyr Leu Arg Gln Gly Asn Arg Leu
   755 760 765
   Lys Gln Pro Ala Asp Cys Leu Asp Gly Leu Tyr Ala Leu Met Ser Arg
   770 775 780
   Cys Trp Glu Leu Asn Pro Gln Asp Arg Pro Ser Phe Thr Glu Leu Arg
   785 790 795 800
   Glu Asp Leu Glu Asn Thr Leu Lys Ala Leu Pro Pro Ala Gln Glu Pro
   805 810 815
   Asp Glu Ile Leu Tyr Val Asn Met Asp Glu Gly Gly Gly Tyr Pro Glu
   820 825 830
   Pro Pro Gly Ala Ala Gly Gly Ala Asp Pro Pro Thr Gln Pro Asp Pro
   835 840 845
   Lys Asp Ser Cys Ser Cys Leu Thr Ala Ala Glu Val His Pro Ala Gly
   850 855 860
   Arg Tyr Val Leu Cys Pro Ser Thr Thr Pro Ser Pro Ala Gln Pro Ala
   865 870 875 880
   Asp Arg Gly Ser Pro Ala Ala Pro Gly Gln Glu Asp Gly Ala
   885 890
<210> 152
   <211> 894
   <212> PRT
   <213> Artificial
<220>
   <223> Chimeric protein construct
<400> 152
   Met Ala Trp Arg Cys Pro Arg Met Gly Arg Val Pro Leu Ala Trp Cys
   1 5 10 15
   Leu Ala Leu Cys Gly Trp Ala Cys Met Ala Pro Arg Gly Thr Gln Ala
   20 25 30
   Glu Glu Ser Pro Phe Val Gly Asn Pro Gly Asn Ile Thr Gly Ala Arg
   35 40 45
   Gly Leu Thr Gly Thr Leu Arg Cys Gln Leu Gln Val Gln Gly Glu Pro
   50 55 60
   Pro Glu Val His Trp Leu Arg Asp Gly Gln Ile Leu Glu Leu Ala Asp
   65 70 75 80
   Ser Thr Gln Thr Gln Val Pro Leu Gly Glu Asp Glu Gln Asp Asp Trp
   85 90 95
   Ile Val Val Ser Gln Leu Arg Ile Thr Ser Leu Gln Leu Ser Asp Thr
   100 105 110
   Gly Gln Tyr Gln Cys Leu Val Phe Leu Gly His Gln Thr Phe Val Ser
   115 120 125
   Gln Pro Gly Tyr Val Gly Leu Glu Gly Leu Pro Tyr Phe Leu Glu Glu
   130 135 140
   Pro Glu Asp Arg Thr Val Ala Ala Asn Thr Pro Phe Asn Leu Ser Cys
   145 150 155 160
   Gln Ala Gln Gly Pro Pro Glu Pro Val Asp Leu Leu Trp Leu Gln Asp
   165 170 175
   Ala Val Pro Leu Ala Thr Ala Pro Gly His Gly Pro Gln Arg Ser Leu
   180 185 190
   His Val Pro Gly Leu Asn Lys Thr Ser Ser Phe Ser Cys Glu Ala His
   195 200 205
   Asn Ala Lys Gly Val Thr Thr Ser Arg Thr Ala Thr Ile Thr Val Leu
   210 215 220
   Pro Gln Arg Pro His His Leu His Val Val Ser Arg Gln Pro Thr Glu
   225 230 235 240
   Leu Glu Val Ala Trp Thr Pro Gly Leu Ser Gly Ile Tyr Pro Leu Thr
   245 250 255
   His Cys Asn Leu Gln Ala Val Leu Ser Asp Asp Gly Val Gly Ile Trp
   260 265 270
   Leu Gly Lys Ser Asp Pro Pro Glu Asp Pro Leu Thr Leu Gln Val Ser
   275 280 285
   Val Pro Pro His Gln Leu Arg Leu Glu Lys Leu Leu Pro His Thr Pro
   290 295 300
   Tyr His Ile Arg Ile Ser Cys Ser Ser Ser Gln Gly Pro Ser Pro Trp
   305 310 315 320
   Thr His Trp Leu Pro Val Glu Thr Thr Glu Gly Val Pro Leu Gly Pro
                   325                 330                 335
   Pro Glu Asn Ile Ser Ala Thr Arg Asn Gly Ser Gln Ala Phe Val His
               340                 345                 350
   Trp Gln Glu Pro Arg Ala Pro Leu Gln Gly Thr Leu Leu Gly Tyr Arg
           355                 360                 365
   Leu Ala Tyr Gln Gly Gln Asp Thr Pro Glu Val Leu Met Asp Ile Gly
       370                 375                 380
   Leu Arg Gln Glu Val Thr Leu Glu Leu Gln Gly Asp Gly Ser Val Ser
   385                 390                 395                 400
   Asn Leu Thr Val Cys Val Ala Ala Tyr Thr Ala Ala Gly Asp Gly Pro
                   405                 410                 415
   Trp Ser Leu Pro Val Pro Leu Glu Ala Trp Arg Pro Gly Gln Ala Gln
               420                 425                 430
   Pro Val His Gln Leu Val Lys Glu Pro Ser Thr Pro Ala Phe Ser Trp
           435                 440                 445
   Pro Trp Trp Tyr Val Leu Leu Gly Ala Val Val Ala Ala Ala Cys Val
       450                 455                 460
   Leu Ile Leu Ala Leu Phe Leu Val His Arg Arg Lys Lys Glu Thr Arg
   465                 470                 475                 480
   Tyr Gly Glu Val Phe Glu Pro Thr Val Glu Arg Gly Glu Leu Val Val
                   485                 490                 495
   Arg Tyr Arg Val Arg Lys Ser Tyr Ser Arg Arg Thr Thr Glu Ala Thr
               500                 505                 510
   Leu Asn Ser Leu Gly Ile Ser Glu Glu Leu Lys Glu Lys Leu Arg Asp
           515                 520                 525
   Val Met Val Asp Arg His Lys Val Ala Leu Gly Lys Thr Leu Gly Glu
       530                 535                 540
   Gly Glu Phe Gly Ala Val Met Glu Gly Gln Leu Asn Gln Asp Asp Ser
   545                 550                 555                 560
   Ile Leu Lys Val Ala Val Lys Thr Met Lys Ile Ala Ile Cys Thr Arg
                   565                 570                 575
   Ser Glu Leu Glu Asp Phe Leu Ser Glu Ala Val Cys Met Lys Glu Phe
               580                 585                 590
   Asp His Pro Asn Val Met Arg Leu Ile Gly Val Cys Phe Gln Gly Ser
           595                 600                 605
   Glu Arg Glu Ser Phe Pro Ala Pro Val Val Ile Leu Pro Phe Met Lys
       610                 615                 620
   His Gly Asp Leu His Ser Phe Leu Leu Tyr Ser Arg Leu Gly Asp Gln
   625                 630                 635                 640
   Pro Val Tyr Leu Pro Thr Gln Met Leu Val Lys Phe Met Ala Asp Ile
                   645                 650                 655
   Ala Ser Gly Met Glu Tyr Leu Ser Thr Lys Arg Phe Ile His Arg Asp
               660                 665                 670
   Leu Ala Ala Arg Asn Cys Met Leu Asn Glu Asn Met Ser Val Cys Val
           675                 680                 685
   Ala Asp Phe Gly Leu Ser Lys Lys Ile Tyr Asn Gly Asp Tyr Tyr Arg
       690                 695                 700
   Gln Gly Arg Ile Ala Lys Met Pro Val Lys Trp Ile Ala Ile Glu Ser
   705                 710                 715                 720
   Leu Ala Asp Arg Val Tyr Thr Ser Lys Ser Asp Val Trp Ser Phe Gly
                   725                 730                 735
   Val Thr Met Trp Glu Ile Ala Thr Arg Gly Gln Thr Pro Tyr Pro Gly
               740                 745                 750
   Val Glu Asn Ser Glu Ile Tyr Asp Tyr Leu Arg Gln Gly Asn Arg Leu
           755                 760                 765
   Lys Gln Pro Ala Asp Cys Leu Asp Gly Leu Tyr Ala Leu Met Ser Arg
       770                 775                 780
   Cys Trp Glu Leu Asn Pro Gln Asp Arg Pro Ser Phe Thr Glu Leu Arg
   785                 790                 795                 800
   Glu Asp Leu Glu Asn Thr Leu Lys Ala Leu Pro Pro Ala Gln Glu Pro
                   805                 810                 815
   Asp Glu Ile Leu Tyr Val Asn Met Asp Glu Gly Gly Gly Tyr Pro Glu
   820 825 830
   Pro Pro Gly Ala Ala Gly Gly Ala Asp Pro Pro Thr Gln Pro Asp Pro
   835 840 845
   Lys Asp Ser Cys Ser Cys Leu Thr Ala Ala Glu Val His Pro Ala Gly
   850 855 860
   Arg Tyr Val Leu Cys Pro Ser Thr Thr Pro Ser Pro Ala Gln Pro Ala
   865 870 875 880
   Asp Arg Gly Ser Pro Ala Ala Pro Gly Gln Glu Asp Gly Ala
   885 890
<210> 153
   <211> 894
   <212> PRT
   <213> Artificial
<220>
   <223> Chimeric protein construct
<400> 153
   Met Ala Trp Arg Cys Pro Arg Met Gly Arg Val Pro Leu Ala Trp Cys
   1               5                   10                  15
   Leu Ala Leu Cys Gly Trp Ala Cys Met Ala Pro Arg Gly Thr Gln Ala
               20                  25                  30
   Glu Glu Ser Pro Phe Val Gly Asn Pro Gly Asn Ile Thr Gly Ala Arg
           35                  40                  45
   Gly Leu Thr Gly Thr Leu Arg Cys Gln Leu Gln Val Gln Gly Glu Pro
       50                  55                  60
   Pro Glu Val His Trp Leu Arg Asp Gly Gln Ile Leu Glu Leu Ala Asp
   65                  70                  75                  80
   Ser Thr Gln Thr Gln Val Pro Leu Gly Glu Asp Glu Gln Asp Asp Trp
                   85                  90                  95
   Ile Val Val Ser Gln Leu Arg Ile Thr Ser Leu Gln Leu Ser Asp Thr
               100                 105                 110
   Gly Gln Tyr Gln Cys Leu Val Phe Leu Gly His Gln Thr Phe Val Ser
           115                 120                 125
   Gln Pro Gly Tyr Val Gly Leu Glu Gly Leu Pro Tyr Phe Leu Glu Glu
       130                 135                 140
   Pro Glu Asp Arg Thr Val Ala Ala Asn Thr Pro Phe Asn Leu Ser Cys
   145                 150                 155                 160
   Gln Ala Gln Gly Pro Pro Glu Pro Val Asp Leu Leu Trp Leu Gln Asp
                   165                 170                 175
   Ala Val Pro Leu Ala Thr Ala Pro Gly His Gly Pro Gln Arg Ser Leu
               180                 185                 190
   His Val Pro Gly Leu Asn Lys Thr Ser Ser Phe Ser Cys Glu Ala His
           195                 200                 205
   Asn Ala Lys Gly Val Thr Thr Ser Arg Thr Ala Thr Ile Thr Val Leu
       210                 215                 220
   Pro Gln Gln Pro Arg Asn Leu His Leu Val Ser Arg Gln Pro Thr Glu
   225                 230                 235                 240
   Leu Glu Val Ala Trp Thr Pro Gly Leu Ser Gly Ile Tyr Pro Leu Thr
                   245                 250                 255
   His Cys Thr Leu Gln Ala Val Leu Ser Asp Asp Gly Met Gly Ile Gln
               260                 265                 270
   Ala Gly Glu Pro Asp Pro Pro Glu Glu Pro Leu Thr Ser Gln Ala Ser
           275                 280                 285
   Val Pro Pro His Gln Leu Arg Leu Gly Ser Leu His Pro His Thr Pro
       290                 295                 300
   Tyr His Ile Arg Val Ala Cys Thr Ser Ser Gln Gly Pro Ser Ser Trp
   305                 310                 315                 320
   Thr His Trp Leu Pro Val Glu Thr Pro Glu Gly Val Pro Leu Gly Pro
                   325                 330                 335
   Pro Glu Asn Val Ser Ala Met Arg Asn Gly Ser Gln Val Leu Val Arg
               340                 345                 350
   Trp Gln Glu Pro Arg Val Pro Leu Gln Gly Thr Leu Leu Gly Tyr Arg
           355                 360                 365
   Leu Ala Tyr Arg Gly Gln Asp Thr Pro Glu Val Leu Met Asp Ile Gly
       370                 375                 380
   Leu Thr Arg Glu Val Thr Leu Glu Leu Arg Gly Asp Arg Pro Val Ala
   385 390 395 400
   Asn Leu Thr Val Ser Val Thr Ala Tyr Thr Ser Ala Gly Asp Gly Pro
   405 410 415
   Trp Ser Leu Pro Val Pro Leu Glu Pro Trp Arg Pro Gly Gln Gly Gln
   420 425 430
   Pro Leu His His Leu Val Ser Glu Pro Pro Pro Arg Ala Phe Ser Trp
   435 440 445
   Pro Trp Trp Tyr Val Leu Leu Gly Ala Val Val Ala Ala Ala Cys Val
   450 455 460
   Leu Ile Leu Ala Leu Phe Leu Val His Arg Arg Lys Lys Glu Thr Arg
   465 470 475 480
   Tyr Gly Glu Val Phe Glu Pro Thr Val Glu Arg Gly Glu Leu Val Val
   485 490 495
   Arg Tyr Arg Val Arg Lys Ser Tyr Ser Arg Arg Thr Thr Glu Ala Thr
   500 505 510
   Leu Asn Ser Leu Gly Ile Ser Glu Glu Leu Lys Glu Lys Leu Arg Asp
   515 520 525
   Val Met Val Asp Arg His Lys Val Ala Leu Gly Lys Thr Leu Gly Glu
   530 535 540
   Gly Glu Phe Gly Ala Val Met Glu Gly Gln Leu Asn Gln Asp Asp Ser
   545 550 555 560
   Ile Leu Lys Val Ala Val Lys Thr Met Lys Ile Ala Ile Cys Thr Arg
   565 570 575
   Ser Glu Leu Glu Asp Phe Leu Ser Glu Ala Val Cys Met Lys Glu Phe
   580 585 590
   Asp His Pro Asn Val Met Arg Leu Ile Gly Val Cys Phe Gln Gly Ser
   595 600 605
   Glu Arg Glu Ser Phe Pro Ala Pro Val Val Ile Leu Pro Phe Met Lys
   610 615 620
   His Gly Asp Leu His Ser Phe Leu Leu Tyr Ser Arg Leu Gly Asp Gln
   625 630 635 640
   Pro Val Tyr Leu Pro Thr Gln Met Leu Val Lys Phe Met Ala Asp Ile
   645 650 655
   Ala Ser Gly Met Glu Tyr Leu Ser Thr Lys Arg Phe Ile His Arg Asp
   660 665 670
   Leu Ala Ala Arg Asn Cys Met Leu Asn Glu Asn Met Ser Val Cys Val
   675 680 685
   Ala Asp Phe Gly Leu Ser Lys Lys Ile Tyr Asn Gly Asp Tyr Tyr Arg
   690 695 700
   Gln Gly Arg Ile Ala Lys Met Pro Val Lys Trp Ile Ala Ile Glu Ser
   705 710 715 720
   Leu Ala Asp Arg Val Tyr Thr Ser Lys Ser Asp Val Trp Ser Phe Gly
   725 730 735
   Val Thr Met Trp Glu Ile Ala Thr Arg Gly Gln Thr Pro Tyr Pro Gly
   740 745 750
   Val Glu Asn Ser Glu Ile Tyr Asp Tyr Leu Arg Gln Gly Asn Arg Leu
   755 760 765
   Lys Gln Pro Ala Asp Cys Leu Asp Gly Leu Tyr Ala Leu Met Ser Arg
   770 775 780
   Cys Trp Glu Leu Asn Pro Gln Asp Arg Pro Ser Phe Thr Glu Leu Arg
   785 790 795 800
   Glu Asp Leu Glu Asn Thr Leu Lys Ala Leu Pro Pro Ala Gln Glu Pro
   805 810 815
   Asp Glu Ile Leu Tyr Val Asn Met Asp Glu Gly Gly Gly Tyr Pro Glu
   820 825 830
   Pro Pro Gly Ala Ala Gly Gly Ala Asp Pro Pro Thr Gln Pro Asp Pro
   835 840 845
   Lys Asp Ser Cys Ser Cys Leu Thr Ala Ala Glu Val His Pro Ala Gly
   850 855 860
   Arg Tyr Val Leu Cys Pro Ser Thr Thr Pro Ser Pro Ala Gln Pro Ala
   865 870 875 880
   Asp Arg Gly Ser Pro Ala Ala Pro Gly Gln Glu Asp Gly Ala
   885 890
<210> 154
   <211> 30
   <212> DNA
   <213> Artificial
<220>
   <223> Primer
<400> 154
   aagcagtggt atcaacgcag agtacgcggg 30
<210> 155
   <211> 18
   <212> DNA
   <213> Artificial
<220>
   <223> Primer
<400> 155
   acggacggca ggaccact 18
<210> 156
   <211> 39
   <212> DNA
   <213> Artificial
<220>
   <223> Primer
<400> 156
   acggacggca ggaccactaa gcagtggtat caacgcaga 39
<210> 157
   <211> 34
   <212> DNA
   <213> Artificial
<220>
   <223> Primer
<400> 157
   cagcaggcac accactgagg cagttccaga tttc 34
<210> 158
   <211> 18
   <212> DNA
   <213> Artificial
<220>
   <223> Primer
<400> 158
   acggacggca ggaccagt 18
<210> 159
   <211> 41
   <212> DNA
   <213> Artificial
<220>
   <223> Primer
<400> 159
   acggacggca ggaccagtaa gcagtggtat caacgcagag t 41
<210> 160
   <211> 27
   <212> DNA
   <213> Artificial
<220>
   <223> Primer
<400> 160
   ggaggagggc gccagtggga agaccga 27
<210> 161
   <211> 20
   <212> DNA
   <213> Artificial
<220>
   <223> Primer
<400> 161
   gccagatata cgcgttgaca 20
<210> 162
   <211> 20
   <212> DNA
   <213> Artificial
<220>
   <223> Primer
<400> 162
   gatctgctat ggcagggcct 20